# EUROPEAN PATENT APPLICATION

(11) **EP 4 310 084 A1**
(43) Date of publication of application: **24.01.2024**
(21) Application number: 22766278.0
(22) Date of filing: 07.03.2022
(51) Int. Cl.: C07D 471/04, A61K 31/519, A61P 35/00

(54) **PYRIDOPYRIMIDINE-BASED COMPOUND AND APPLICATION THEREOF**

(30) Priority: 08.03.2021 CN 202110251924
(71) Applicant: Jinan University, Tianhe District Guangzhou Guangdong 510632 (CN); Shanghai Institute of Organic Chemistry, Chinese Academy of Sciences, Shanghai 200032 (CN)
(72) Inventor: DING, Ke, Guangzhou, Guangdong 510632 (CN); ZHANG, Xin, Guangzhou, Guangdong 510632 (CN); XU, Fang, Guangzhou, Guangdong 510632 (CN); REN, Xiaomei, Guangzhou, Guangdong 510632 (CN); LU, Xiaoyun, Guangzhou, Guangdong 510632 (CN); MA, Dawei, Guangzhou, Guangdong 510632 (CN); HUANG, Weixue, Guangzhou, Guangdong 510632 (CN)
(74) Representative: Fuchs Patentanwälte Partnerschaft mbB
(86) International application number: PCT/CN2022/079597
(87) International publication number: WO 2022/188755

(57) **Abstract**

The present disclosure provides a class of Pyridopyrimidine compounds having a structure shown in Formula (I) or their pharmaceutically acceptable salts, or stereoisomers or prodrug molecules and applications thereof. The compounds in the present disclosure can efficiently and selectively degrade AKT3 protein in cells without affecting AKT1/2, thereby significantly inhibiting tumor cell proliferation mediated by high expression of AKT3 protein. It can be used to prepare therapeutic drugs for cancer and other diseases related to abnormal expression of AKT3 protein.

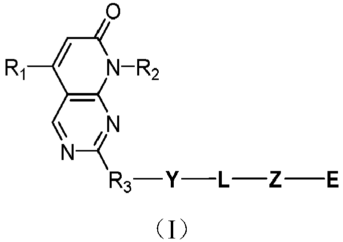

## Description

### Technical Field

The present disclosure relates to pharmaceutical chemistry technology, in particular to a class of Pyridopyrimidine compounds and the applications thereof.

### Background Art

AKT (AK mouse plus Transforming or Thymoma), also known as Protein kinase B (PKB), is a serine/threonine protein kinase with a molecular weight of approximately 57 kD. This family includes three subtypes: AKT1, AKT2, and AKT3. There are many similarities and differences in the functional and histological distribution of AKT subtypes, and their abnormal expression is closely related to the occurrence and development of various diseases. AKT1 widely exists in various tissues, including heart, liver, muscles, etc; AKT2 is mainly distributed in insulin sensitive tissues, such as skeletal muscle and adipose tissue; AKT3 is mainly distributed in tissues such as brain, heart, and kidneys. AKT1's expression increased in about 40% in breast cancer and ovarian cancer and more than 50% in prostate cancer; AKT2 is overexpressed in 40% of liver cancer and 57% of colorectal cancer; AKT2 deficiency will lead to hyperglycemia, Type 2 diabetes and glucose intake disorders; the overexpression of AKT1 and AKT2 is also associated with paclitaxel resistance in ovarian cancer; the overexpression of AKT3 exists in breast cancer, prostate cancer and some Osimertinib resistant non-small cell lung cancer. Therefore, selective regulation of AKT subtype proteins may play a positive role in the treatment of related diseases.

AKT is an important target for tumor treatment. At present, multiple kinase inhibitors of AKT have entered clinical trials and have shown good anti-tumor effects. However, these inhibitors lack selectivity towards various subtypes of AKT proteins, and undifferentiated inhibition may have certain clinical toxic side effects. In addition, similar to traditional targeted drugs, prolonged drug treatment may lead to patient resistance to AKT inhibitors, thereby weakening the efficacy. Moreover, AKT protein has both kinase and non-kinase functions, and simply inhibiting kinase function may be difficult to fully exert anti-tumor effects. Targeted degradation of the intact AKT3 protein can not only inhibit its kinase function, but also regulate its non-kinase function, thereby exerting a stronger anti-tumor effect.

Therefore, the development and synthesis of small molecule degrading agents which can selectively degrade AKT3 protein is of great significance for the development of therapeutic drugs for AKT3-mediated related diseases.

### Summary of the Disclosure

In response to the above-mentioned issues, the present disclosure provides a new class of Pyridopyrimidine compounds, which can selectively degrade AKT3 protein with high activity, inhibit the proliferation of various tumor cells, and can be used to treat diseases and tumors related to AKT3 protein.

The detailed technical solution is as follows:

Pyridopyrimidine compounds having a structure shown in Formula (I) or their pharmaceutically acceptable salts, or stereoisomers or prodrug molecules, 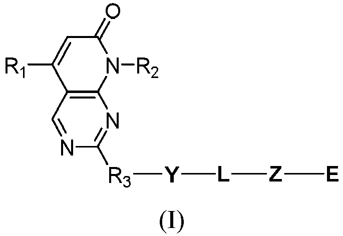 wherein,
E is selected from: H, C₁-C₁₅ alkyl, substituted C₁-C₁₅ alkyl, C₃-C₁₅ cycloalkyl, substituted C₃-C₁₅ cycloalkyl, 3-15 membered heterocycloalkyl, substituted 3-15 membered heterocycloalkyl;
L is absent or is a connecting unit consisting of one or more of the following groups: alkylene, ether, thioether, ester, amine, amide, heteroaryl, cycloalkyl, heterocycloalkyl, -N=N-;
Y is absent or is selected from -O-, -NH-, -NHCO-, -CH₂-, -S-, -CO-;
Z is absent or is selected from: -O-, -NH-, -N(C₁-C₆ alkyl)-, -NHCO-, -CH₂-, -S-, -CO-, -SO-;
R₁ is selected from: H, C₁-C₆ alkyl, halogen or halogen substituted C₁-C₆ alkyl;
R₂ is selected from: C₃-C₁₅ cycloalkyl, substituted C₃-C₁₅ cycloalkyl, 3-15 membered heterocycloalkyl, substituted 3-15 membered heterocycloalkyl, C₆-C₁₀ aryl, substituted C₆-C₁₀ aryl, 5-10 membered heteroaryl, substituted 5-10 membered heteroaryl;
R₃ is 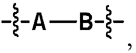 wherein B is connected to Y through a covalent bond.
A is selected from: -NH-, -NHR-; R is selected from: C₆-C₁₀ aryl, substituted C₆-C₁₀ aryl, 5-10 membered heteroaryl, substituted 5-10 membered heteroaryl;
B is absent or is selected from: C₃-C₁₅ cycloalkyl, substituted C₃-C₁₅ cycloalkyl, 3-15 membered heterocycloalkyl, substituted 3-15 membered heterocycloalkyl, 3-15 membered heterocycloalkyl ketone group, R₈ substituted 3-15 membered heterocycloalkyl ketone group, C₃-C₁₂ cycloalkyl substituted amine group, 3-12 membered heterocycloalkyl substituted amine group,

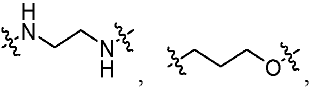

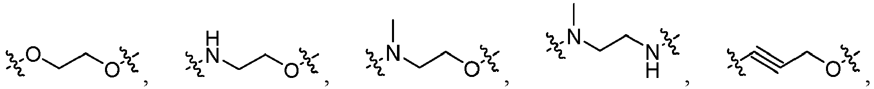

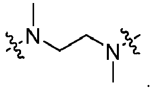

In some of embodiments, E is selected from: H, C₁-C₈ alkyl, R₅ substituted C₁-C₈ alkyl, C₃-C₁₂ cycloalkyl, R₆ substituted C₃-C₁₂ cycloalkyl, 3-12 membered heterocycloalkyl, R₆ substituted 3-12 membered heterocycloalkyl;
R₅ is selected from: halogen, C₃-C₁₂ cycloalkyl, C₁-C₃ alkyl substituted C₃-C₁₂ cycloalkyl, halogen substituted C₃-C₁₂ cycloalkyl;
R₆ is selected from: halogen, C₁-C₆ alkyl, halogen substituted C₁-C₆ alkyl.

In some embodiments, E is selected from: H, C₁-C₆ alkyl, R₅ substituted C₁-C₆ alkyl, C₃-C₁₀ cycloalkyl, R₆ substituted C₃-C₁₀ cycloalkyl;
R₅ is selected from: halogen, C₆-C₁₀ cycloalkyl, methyl substituted C₆-C₁₀ cycloalkyl, halogen substituted C₆-C₁₀ cycloalkyl;
R₆ is selected from: halogen, C₁-C₃ alkyl.

In some embodiments, E is selected from: H, cyclopropyl, 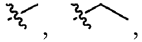 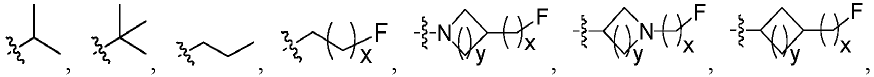 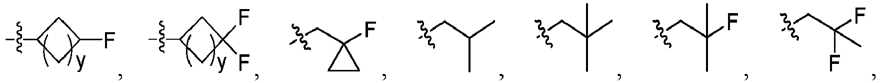 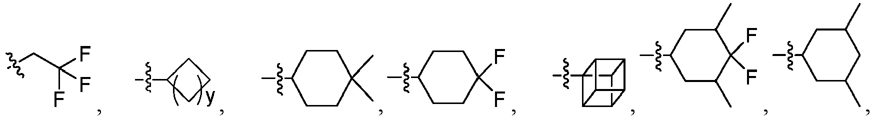 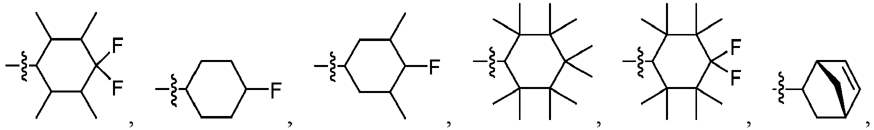 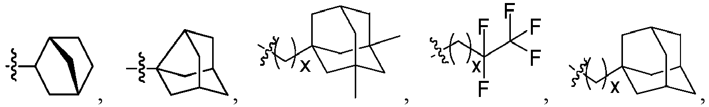 herein x is an integer from 0 to 3, and y is an integer from 0 to 3.

In some embodiments, L is selected from: 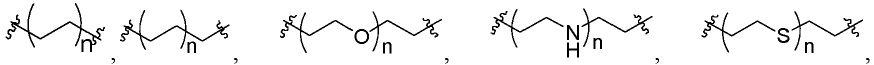 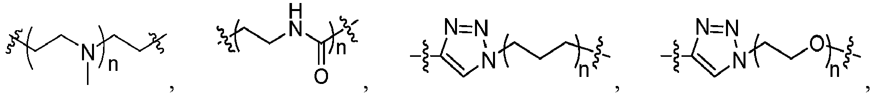 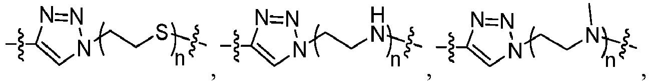 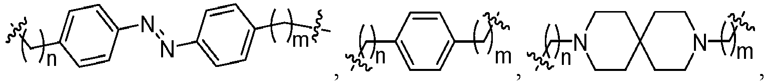 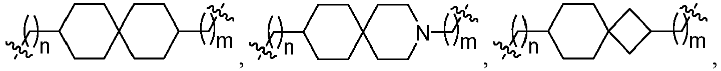 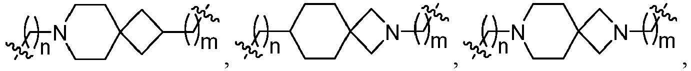 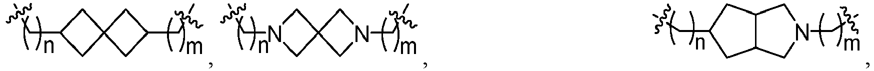 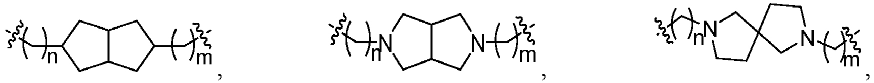 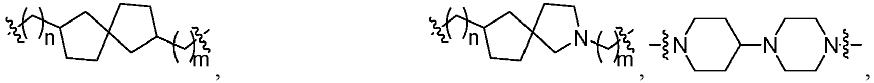 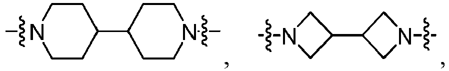 wherein n and m are independently integers from 0 to 14.

In some embodiments, L is selected from: 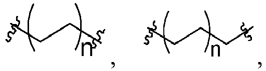 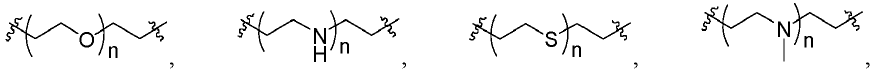 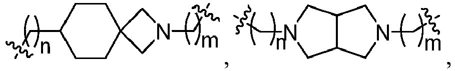 wherein n is an integer from 0 to 7, and m is an integer from 0 to 3.

In some embodiments, L is selected from: 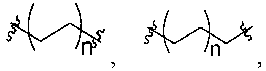 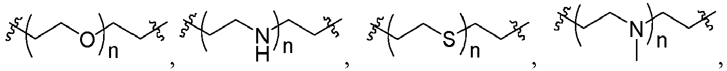 or L is absent, wherein n is an integer from 2 to 7.

In some embodiments, Y is selected from: -CH₂-, -CO-, -O-, or Y is absent; Z is selected from: -NHCO-, -NH-, or Z is absent.

In some embodiments, R₁ is selected from: H, halogen, C₁-C₃ alkyl.

In some embodiments, R₂ is selected from: C₅-C₁₀ cycloalkyl, R₉ substituted C₅-C₁₀ cycloalkyl, 5-10 membered heterocycloalkyl, R₉ substituted 5-10 membered heterocycloalkyl, C₆-C₁₀ aryl, R₉ substituted C₆-C₁₀ aryl, 5-10 membered heteroaryl, R₉ substituted 5-10 membered heteroaryl, 5-10 membered heteroaryl ketone, and R₉ substituted 5-10 membered heteroaryl ketone;
R₉ is selected from: amino, -N(C₁-C₆ alkyl)₂, halogen, C₁-C₆ alkyl, C₁-C₆ alkoxy, halogen substituted C₁-C₆ alkyl, halogen substituted C₁-C₆ alkoxy, -NH(R₄), -N(R₄)₂, - O(R4), -C=O-NH(R₄), -C=O-NH(C₃-C₆ cycloalkyl), R₁₀ substituted C₁-C₆ alkyl, C₃-C₁₀ cycloalkyl, 3-10 membered heterocycloalkyl, R₁₀ substituted C₃-C₁₀ cycloalkyl, R₁₀ substituted 3-10 membered heterocycloalkyl, -NH(R4) substituted 3-10 membered heterocycloalkyls, 5-10 membered heteroaryl groups, -COR₁₁;
R₄ is absent or is selected from: H, amino, ester, carboxyl, hydroxyl, thiol, sulfone, sulfoxide, C₁-C₁₅ alkyl, R₁₀ substituted C₁-C₁₅ alkyl, C₃-C₁₅ cycloalkyl, 3-15 membered heterocycloalkyl, R₁₀ substituted C₃-C₁₅ cycloalkyl, R₁₀ substituted 3-15 membered heterocycloalkyl, - COR₁₁;
R₁₀ is selected from: C₁-C₆ alkyl, amino substituted C₁-C₆ alkyl, C₃-C₆ cycloalkyl, dimethylamino substituted C₁-C₆ alkyl, 3-6 membered heterocycloalkyl, dimethylamino, C₁-C₃ alkoxy substituted C₁-C₆ alkyl, hydroxyl substituted C₁-C₆ alkyl, C₁-C₆ alkyl substituted 3-6 membered heterocycloalkyl, C₁-C₆ alkyl acyl, hydroxyl, C1-C6 alkyl substituted C₃-C₆ cycloalkyl, dimethylaminoethyl substituted 5-6 membered heterocycloalkyl, C₁-C₆ alkoxy;
R₁₁ is selected from: vinyl, C₁-C₆ alkyl, amino substituted C₁-C₆ alkyl, C₃-C₆ cycloalkyl, amino substituted C₃-C₆ cycloalkyl, halogen substituted C₃-C₆ cycloalkyl, 3-6 membered heterocycloalkyl, C₁-C₆ alkyl substituted 3-6 membered heterocycloalkyl, dimethylamine substituted C₁-C₆ alkyl, and dimethylamine ethyl substituted 5-6 membered heterocycloalkyl.

In some embodiments, R₂ is selected from: phenyl, 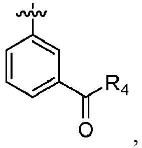 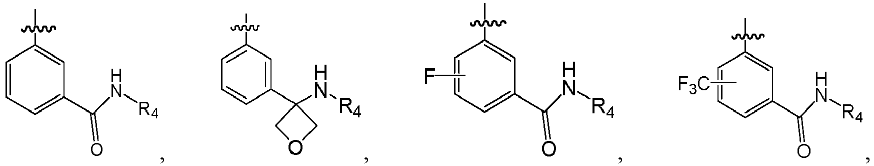 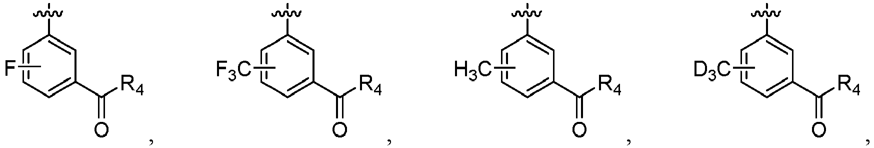 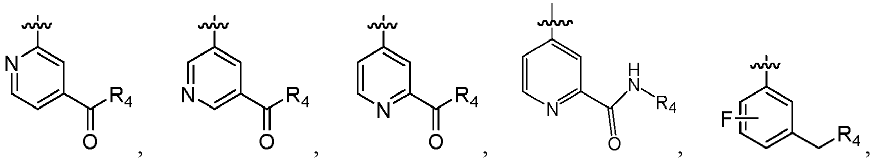 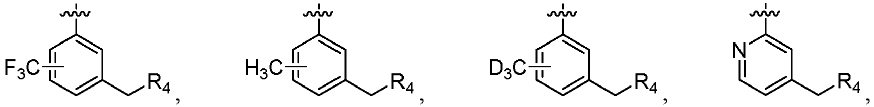 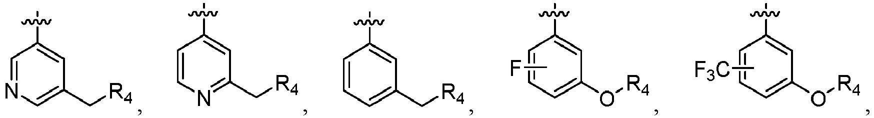 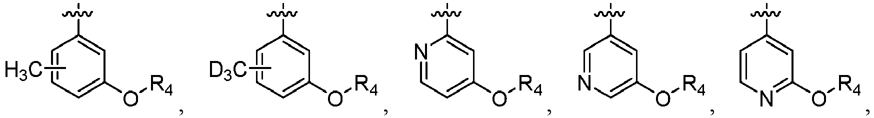 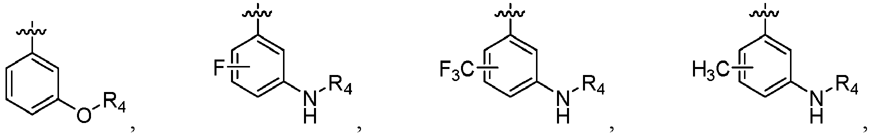 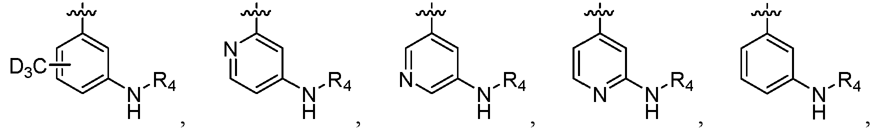 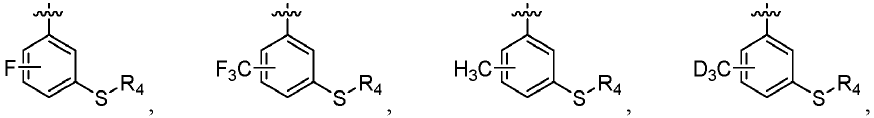 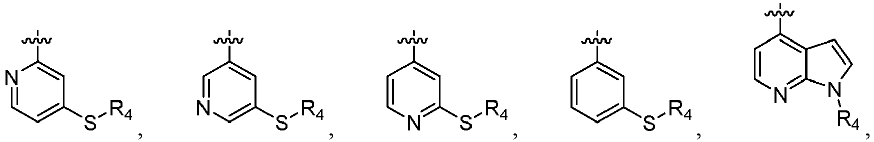 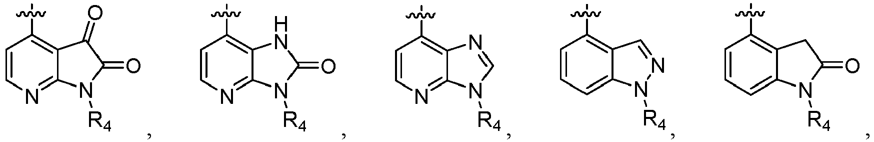 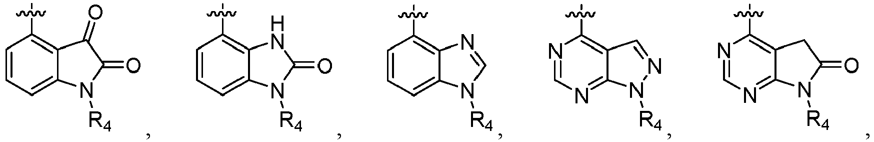 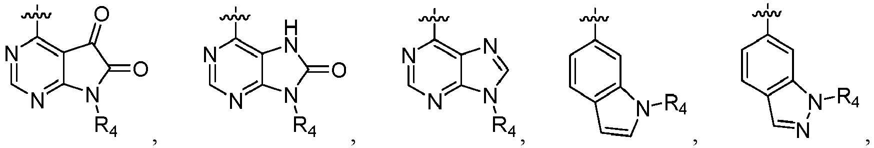 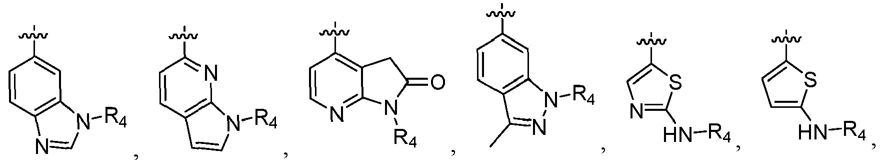 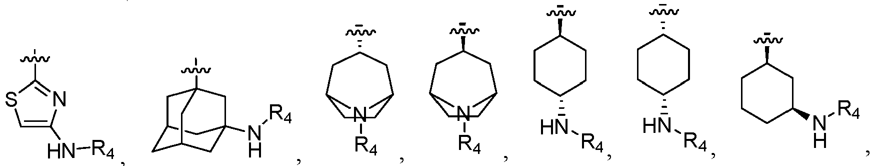 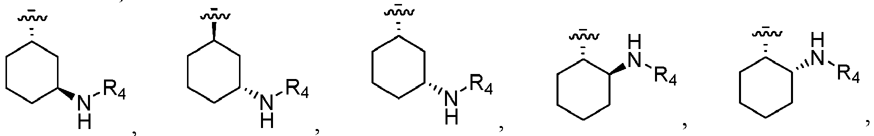 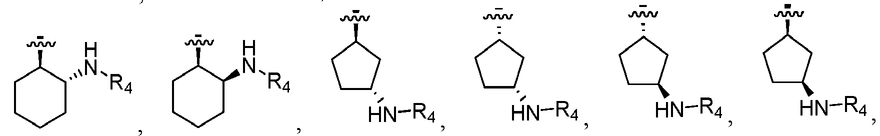 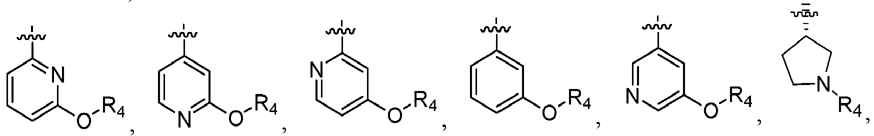 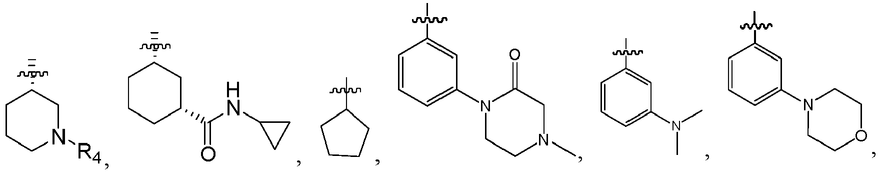 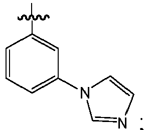 wherein R₄ is selected from: H,

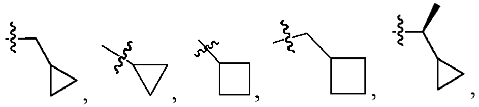

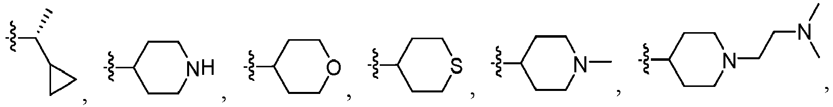

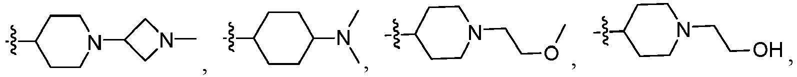

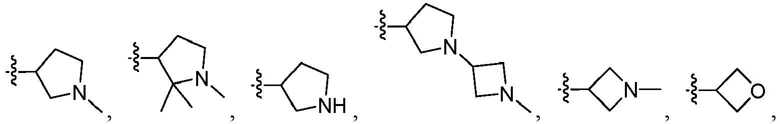

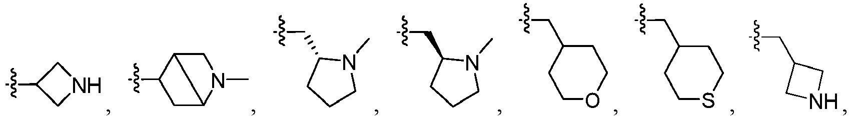

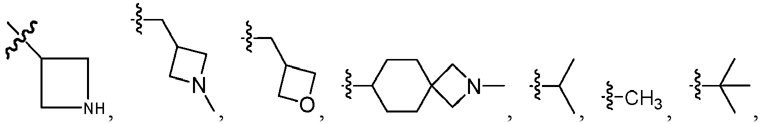

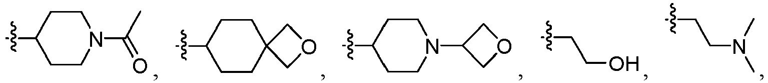

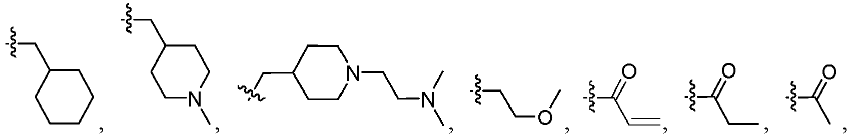

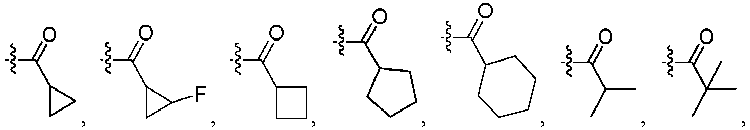

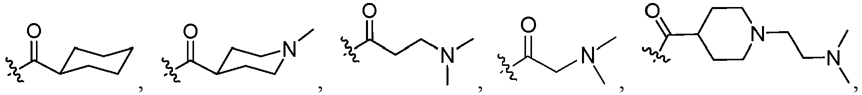

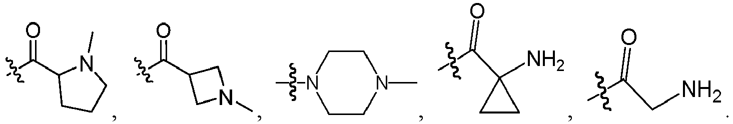

In some embodiments, R₂ is selected from: C₅-C₈ cycloalkyl, R₉ substituted C₅-C₈ cycloalkyl, 5-8-membered heterocycloalkyl, R₉ substituted 5-8-membered heterocycloalkyl, phenyl, R₉ substituted phenyl, 5-6-membered heteroaryl group, R₉ substituted 5-6-membered heteroaryl;
R₉ is selected from: H, dimethylamino, amino, halogen, C₁-C₃ alkyl, C₁-C₃ alkoxy, halogen substituted C₁-C₃ alkyl, halogen substituted C₁-C₃ alkoxy, -NH(R₄), -N(R₄)₂, - OR₄, -C=O-NH(cyclopropyl), R₁₀ substituted C₁-C₃ alkyl, C₃-C₆ cycloalkyl, 3-6 membered heterocycloalkyl, R₁₀ substituted C₃-C₆ cycloalkyl, R₁₀ substituted 3-6 membered heterocycloalkyl, -NH(R₄) substituted 3-6 membered heterocycloalkyl, - COR₁₁;
R₄ is selected from: H, C₁-C₆ alkyl, R₁₀ substituted C₁-C₆ alkyl, C₃-C₆ cycloalkyl, 3-6 membered heterocycloalkyl, R₁₀ substituted C₃-C₆ cycloalkyl, R₁₀ substituted 3-6 membered heterocycloalkyl, - CH₂R₁₁, - COR₁₁;
R₁₀ is selected from: C₁-C₃ alkyl, dimethylamino, C₃-C₆ cycloalkyl, 3-6 membered heterocycloalkyl, C₁-C₃ alkyl substituted 3-6 membered heterocycloalkyl, C₁-C₃ alkyl substituted C₃-C₆ cycloalkyl;
R₁₁ is selected from: vinyl, C₁-C₄ alkyl, C₃-C₆ cycloalkyl, and halogen substituted C₃-C₆ cycloalkyl.

In some embodiments, R₂ is selected from: C₅-C₆ cycloalkyl, R₉ substituted C₅-C₆ cycloalkyl, 5-6-membered heterocycloalkyl, R₉ substituted 5-6-membered heterocycloalkyl, phenyl, R₉ substituted phenyl;
R₉ is selected from: H, dimethylamino, amino, C₁-C₃ alkyl, -NH(R₄), -OR₄, - C=O-NH (cyclopropyl), R₁₀ substituted C₁-C₃ alkyl, C₃-C₆ cycloalkyl, 5-6 membered heterocycloalkyl, R₁₀ substituted C₃-C₆ cycloalkyl, R₁₀ substituted 5-6 membered heterocycloalkyl, - COR₁₁,
R₄ is selected from: H, C₁-C₃ alkyl, R₁₀ substituted C₁-C₃ alkyl, C₃-C₆ cycloalkyl, 5-6 membered heterocycloalkyl, R₁₀ substituted C₃-C₆ cycloalkyl, R₁₀ substituted 5-6 membered heterocycloalkyl, -CH₂R₁₁, - COR₁₁;
R₁₀ is selected from: C₁-C₃ alkyl, C₃-C₆ cycloalkyl;
R₁₁ is selected from: vinyl, C₁-C₄ alkyl, C₃-C₆ cycloalkyl.

In some embodiments, R₂ is selected from: 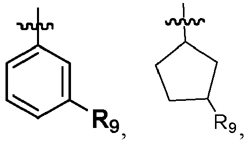 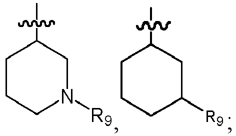
wherein, R₉ is selected from: H, dimethylamino, C₁-C₃ alkyl, -NH(R₄), -OR₄, -COR₁₁;
R₄ is selected from: H, methylpiperidyl, - CH₂R₁₁, - COR₁₁; R₁₁ is selected from: vinyl, C₁-C₄ alkyl, cyclopropyl, cyclobutyl, and cyclopentyl.

In some embodiments, A is selected from: -NH-, -NHR-; R is selected from: phenyl, R₇ substituted phenyl, 6-membered heteroaryl, R₇ substituted 6-membered heteroaryl;
R₇ is selected from: C₁-C₆ alkyl, C₃-C₆ cycloalkyl, halogen, C₁-C₆ alkoxy, halogen substituted C₁-C₆ alkoxy, deuterated C₁-C₆ alkoxy, halogen substituted C₁-C₆ alkyl, cyano substituted C₁-C₆ alkyl, deuterated C₁-C₆ alkyl, trifluoromethyl substituted C₃-C₆ cycloalkyl, C₁-C₆ cycloalkyl substituted by C₁-C₆ alkyl, hydroxyl, amino, -SO (C₁-C₆ alkyl), -S(O)₂ (C₁-C₆ alkyl), C₁-C₆ alkylthio;
B is absent or is selected from: C₃-C₁₂ cycloalkyl, R₈ substituted C₃-C₁₂ cycloalkyl, 3-12 membered heterocycloalkyl, R₈ substituted 3-12 membered heterocycloalkyl, 3-12 membered heterocycloalkyl ketone group, R₈ substituted 3-12 membered heterocycloalkyl ketone group, C₃-C₁₂ cycloalkyl substituted amine group, 3-12 membered heterocycloalkyl substituted amine group 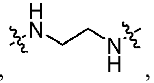 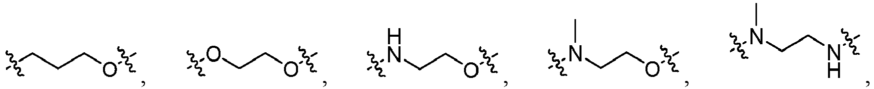 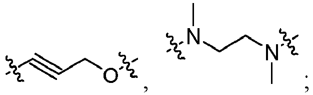
R₈ is selected from: C₁-C₆ alkyl, C₃-C₁₂ cycloalkyl, 3-12 membered heterocycloalkyl, amino, cyano substituted C₁-C₆ alkyl, halogen, C₁-C₆ alkoxy, halogen substituted C₁-C₆ alkyl, hydroxyl, amino.

In some embodiments, A is selected from: -NH-, 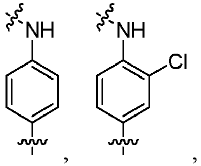 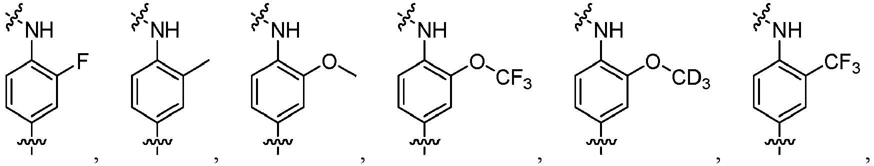 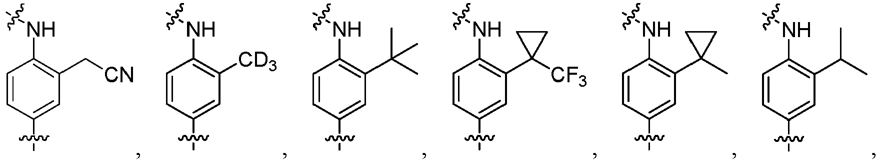 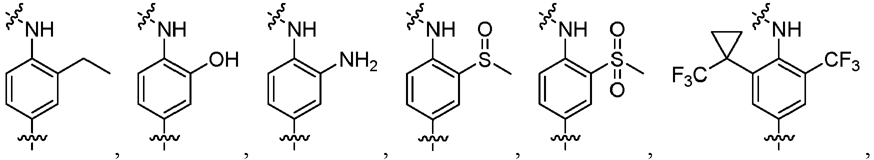 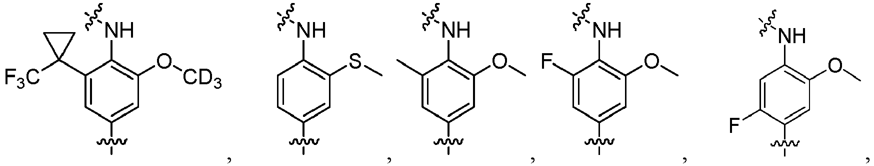 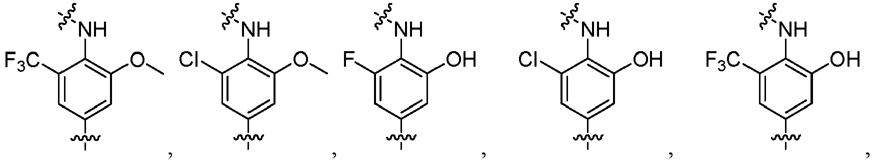 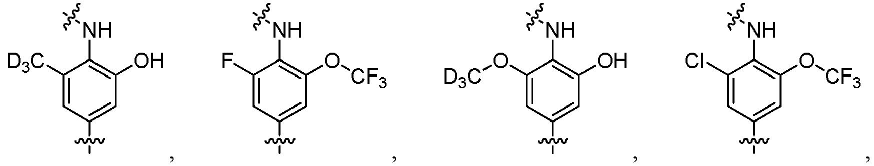 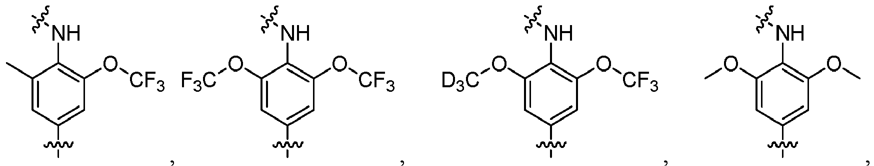 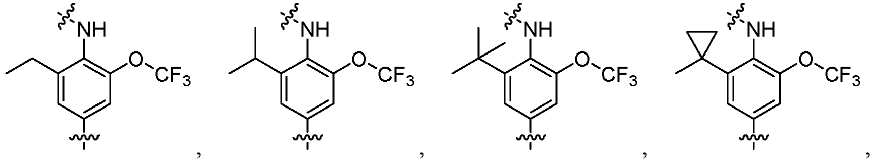 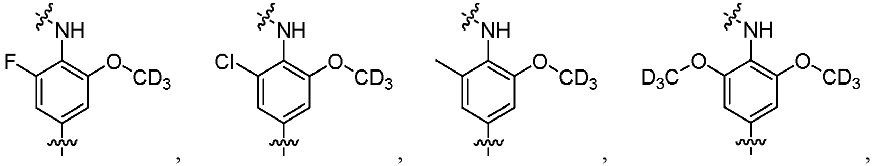 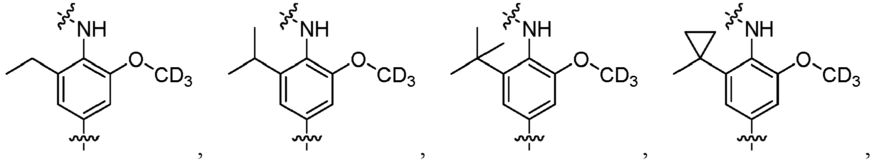 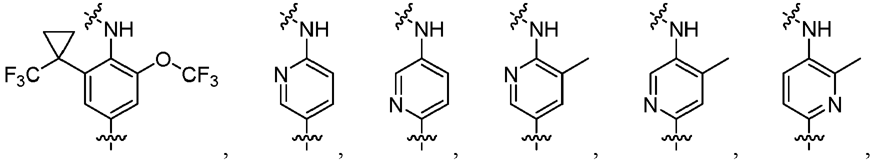 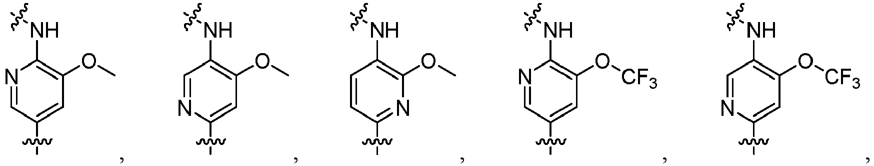 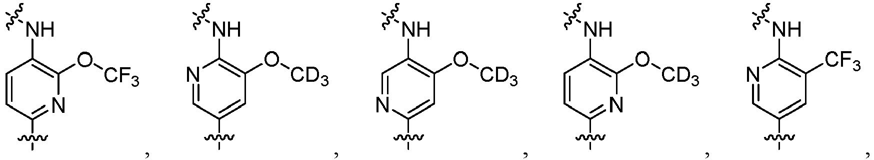 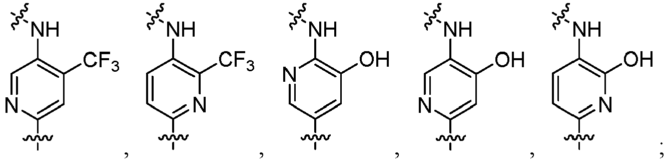 B is absent or is selected from:

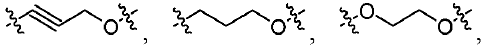

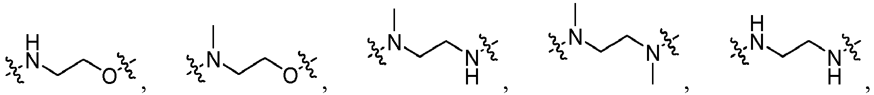

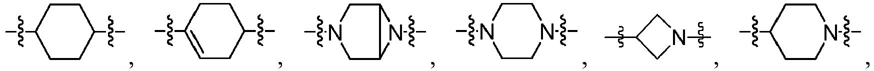

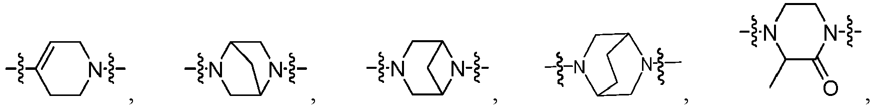

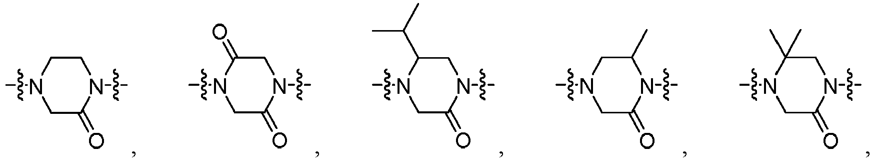

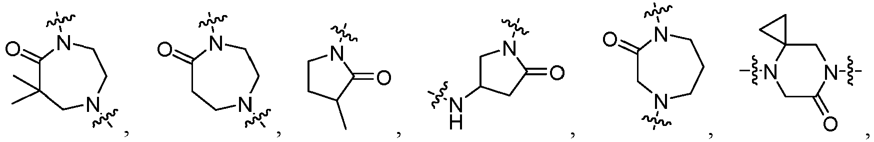

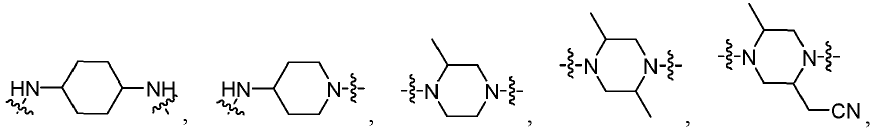

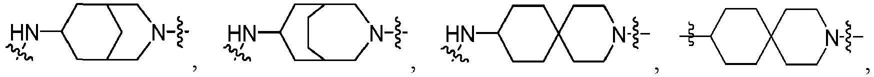

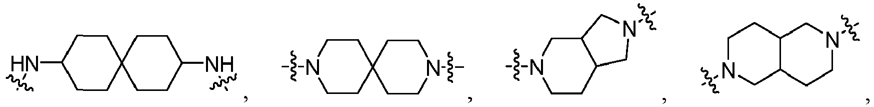

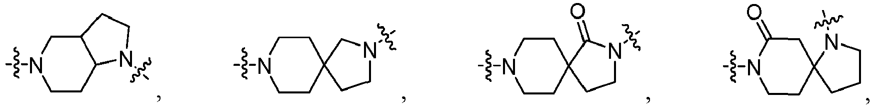

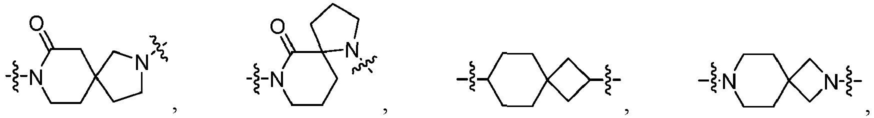

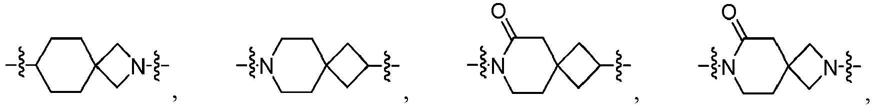

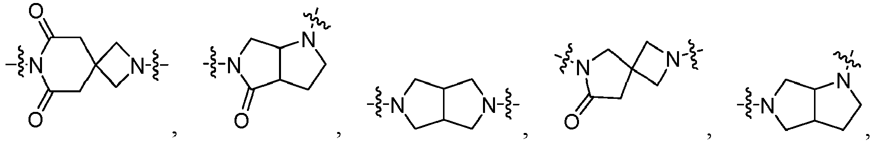

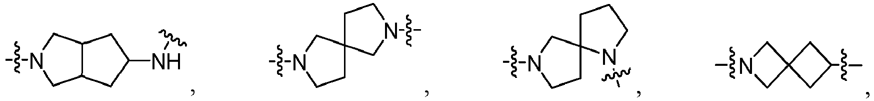

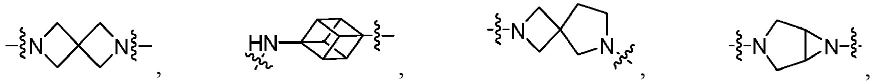

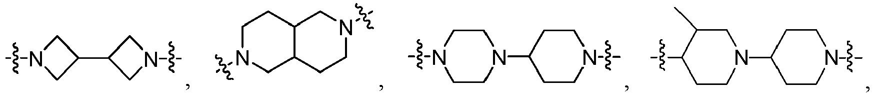

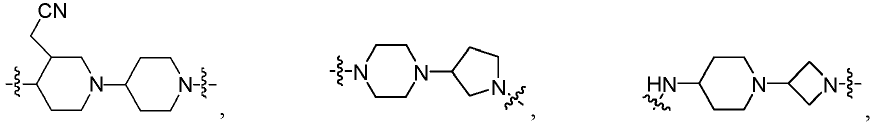

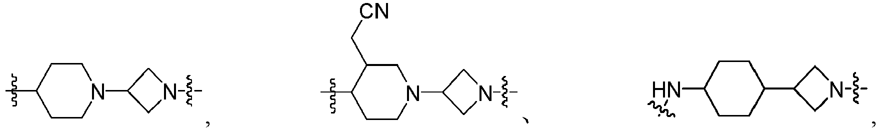

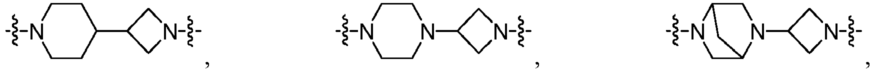

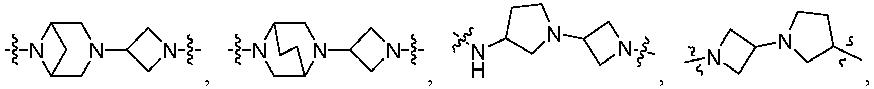

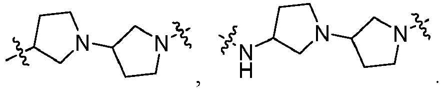

In some embodiments, A is selected from: -NH-, -NHR-; wherein R is selected from: phenyl, 6-membered heteroaryl, R₇ substituted phenyl, R₇ substituted 6-membered heteroaryl;
R₇ is selected from: C₁-C₃ alkyl, halogen, C₁-C₃ alkoxy, halogen substituted C₁-C₃ alkoxy, halogen substituted C₁-C₃ alkyl, cyano substituted C₁-C₃ alkyl;
B is absent or is selected from: C₄-C₁₂ cycloalkyl, R₈ substituted C₄-C₁₂ cycloalkyl, 4-12 membered heterocycloalkyl, R₈ substituted 4-12 membered heterocycloalkyl, 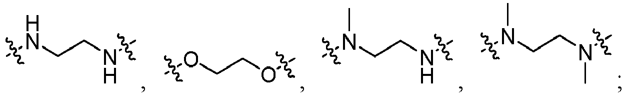
R₈ is selected from: C₁-C₃ alkyl, C₄-C₆ cycloalkyl, 4-6 membered heterocycloalkyl, cyano substituted C₁-C₃ alkyl, halogen, C₁-C₃ alkoxy, halogen substituted C₁-C₃ alkyl.
In some embodiments, A is selected from: -NH-, -NHR-; R is selected from: phenyl, 6-membered heteroaryl, R₇ substituted phenyl, R₇ substituted 6-membered heteroaryl;
R₇ is selected from: C₁-C₃ Alkoxy;
B is absent or is selected from: C₄-C₈ cycloalkyl, R₈ substituted C₄-C₁₀ cycloalkyl, 4-10 membered heterocyclic alkyl, R₈ substituted 4-10 membered heterocyclic alkyl, 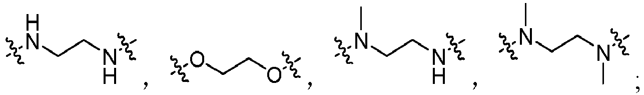
R₈ is selected from: C₁-C₃ alkyl, C₁-C₃ alkoxy.

In some embodiments, R₃ is selected from:

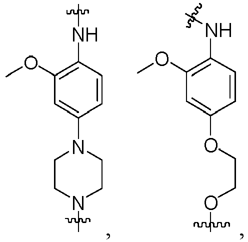

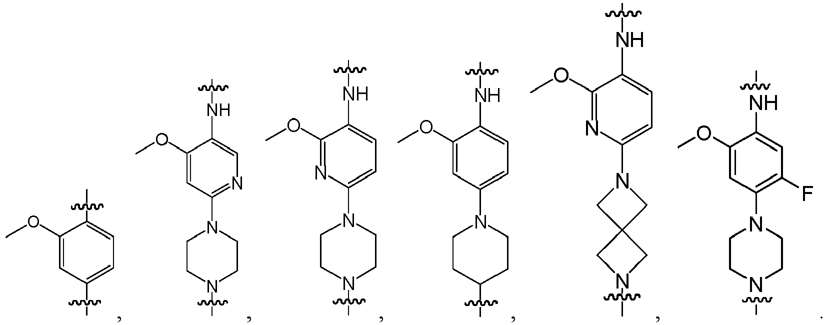

The present disclosure also provides applications of the above-mentioned Pyridopyrimidine compounds or their pharmaceutically acceptable salts or stereoisomers or prodrug molecules, including the following technical solution:

An application of the above-mentioned Pyridopyrimidine compounds or their pharmaceutically acceptable salts or stereoisomers or prodrug molecules in the preparation of AKT3 protein degradation agent.

An application of the above-mentioned Pyridopyrimidine compounds or their pharmaceutically acceptable salts or stereoisomers or prodrug molecules in the preparation of drugs for preventing and/or treating diseases related to abnormal expression of AKT3 protein.

In some embodiments, the diseases related to abnormal expression of AKT3 protein include: tumor, cardiovascular disease, diabetes, hypertension, muscular dystrophy, Parkinson's disease and Alzheimer's disease.

An application of the above-mentioned Pyridopyrimidine compounds or their pharmaceutically acceptable salts or stereoisomers or prodrug molecules in the preparation of drugs for preventing and/or treating tumors or preventing postoperative recurrence of tumors.

In some embodiments, the tumors are: non-small cell lung cancer, malignant melanoma, prostate cancer, kidney cancer, liver cancer, bladder cancer, ovarian cancer, colon cancer, rectal cancer, breast cancer, cervical cancer, lung cancer, laryngeal cancer, nasopharyngeal cancer, pancreatic cancer, multiple myeloma, B lymphoma, leukemia, skin squamous cell carcinoma.

The present disclosure also provides an AKT3 protein degrading agent, including the following technical solution:

An AKT3 protein degrading agent, wherein its active ingredient comprises the above-mentioned Pyridopyrimidine compounds or their pharmaceutically acceptable salts or stereoisomers or prodrug molecules.

The present disclosure also provides a pharmaceutical composition for treating and/or preventing tumors or preventing postoperative recurrence of tumors, including the following technical solution:

A pharmaceutical composition for treating and/or preventing tumors or preventing postoperative recurrence of tumors, is prepared from active ingredients and pharmaceutically acceptable carriers or excipients, wherein the active ingredients include the Pyridopyrimidine compounds or their pharmaceutically acceptable salts or stereoisomers or prodrug molecules.

The present disclosure provides a class of Pyridopyrimidine compounds or their pharmaceutically acceptable salts, or stereoisomers or prodrug molecules, which can efficiently and selectively degrade AKT3 protein in cells without affecting AKT1/2, thereby being able to significantly inhibit tumor cells proliferation mediated by high expression of AKT3, and can be used to prepare therapeutic drugs for diseases related to abnormal expression of AKT3 protein and various tumors.

### BRIEF DESCRIPTION OF THE DRAWINGS

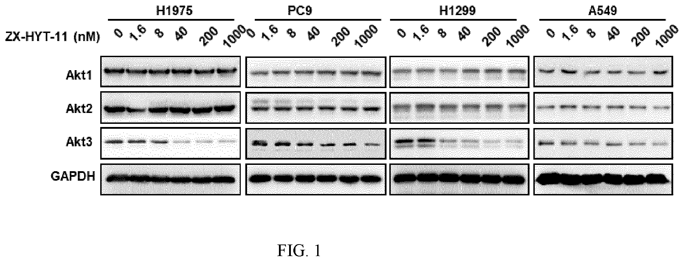

Fig.1 shows the degradation activity of compound **ZX-HYT-11** on AKT1/2/3 in H1975, PC-9, H1299, and A549 cells.

### DETAILED DESCRIPTION OF THE DISCLOSURE

In the compounds of the present disclosure, when any variable (eg. R⁴, R⁵, etc.) occurs more than once in any component, its definition at each occurrence is independent from the definition at each other occurrences. Similarly, combinations of substituents and variables are permissible as long as the combination stabilizes the compound. A line of self substituent to a ring system indicates that the indicated bond may be attached to any substitutable ring atom. If the ring system is polycyclic, it means that such bonds are only attached to any suitable carbon atoms adjacent to the ring. It is understood that an ordinary skilled in the art can select substituents and substitution patterns of the compounds of the present disclosure to provide compounds that are chemically stable and can be readily synthesized from the available starting materials by techniques in the art and by the methods described below. If a substituent itself is substituted by more than one group, it should be understood that these groups may be on the same carbon atom or on different carbon atoms, so long as the structure is stabel.

The term "alkyl" in the present disclosure means saturated aliphatic hydrocarbon groups including branched and straight chain having the specified number of carbon atoms. For example, the definition of "C1-C6" in "C1-C6 alkyl" includes groups having 1, 2, 3, 4, 5 or 6 carbon atoms arranged in a straight or branched chain. For example, "C1-C6 alkyl" specifically includes methyl, ethyl, n-propyl, isopropyl, n-butyl, tert-butyl, isobutyl, pentyl, hexyl.

The term "cycloalkyl" used in this article refers to a saturated or partially unsaturated monocyclic, bicyclic or polycyclic hydrocarbon group composed of carbon atoms. Double ring or multi ring includes spiral ring, fused ring, and bridge ring. For example, "cycloalkyl" includes but is not limited to the following groups: cyclopropyl, cyclobutyl, cyclopentyl, cyclohexyl, 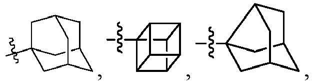 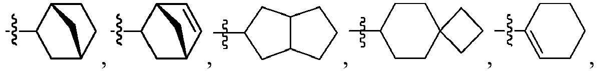 etc.

The term "alkoxy" refers to a group with an -O-alkyl structure, such as -OCH₃, -OCH₂CH₃, -OCH₂CH₂CH₃, -O-CH₂CH(CH₃)₂, -OCH₂CH₂CH₂CH₃, -O-CH(CH₃)₂, etc.

The term "heterocycloalkyl" is a saturated or partially unsaturated monocyclic, bicyclic or polycyclic substituent wherein one or more ring atoms are heteroatoms selected from N, O or S(O)ₘ (wherein m is an integer from 0 to 2 ), and the remaining ring atoms are carbon, bicyclic or polycyclic including spiral cyclic, thick cyclic, and bridge cyclic, such as: morpholinyl, piperidinyl, tetrahydropyrrolyl, pyrrolidinyl, dihydroimidazolyl, dihydroisoxazolyl, dihydroisothiazolyl, dihydro oxadiazolyl, dihydrooxazolyl, dihydropyrazinyl, dihydropyrazolyl, dihydropyridyl, dihydropyrimidinyl, dihydropyrrolyl, dihydrotetrazolyl, dihydrothiadiazolyl , dihydrothiazolyl, dihydrothienyl, dihydrotriazolyl, dihydroazetidine, tetrahydrofuranyl, tetrahydrothienyl, 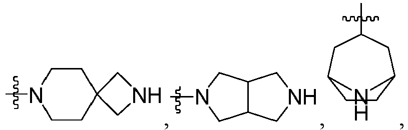 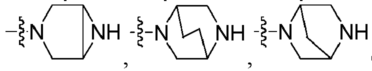 etc., and their N-oxides. The connection of heterocyclic substituents can be achieved through carbon atoms or through heteroatoms.

The term "heteroaryl" as used herein refers to an aromatic ring containing one or more heteroatoms selected from O, N or S. The aromatic ring can be monocyclic, bicyclic or polycyclic, include but not limited to: quinolinyl, pyrazolyl, pyrrolyl, thienyl, furanyl, pyridyl, pyrimidinyl, pyrazinyl, triazolyl, imidazolyl, oxazolyl, isoxazolyl, pyridazinyl, benzoyl Furanyl, benzothienyl, benzoxazole, indolyl, etc.; "heteroaryl" can also be understood to include the N-oxide derivatives of any nitrogen-containing heteroaryl groups. The attachment of heteroaryl substituents can be through carbon atoms or through heteroatoms.

The term "heteroaromatic ketone substituents" as used herein refers to an aromatic ring containing one or more cyclic carbonyl groups and one or more heteroatoms selected from O, N, or S, which can be monocyclic, bicyclic or polycyclic, such as but not limited to: 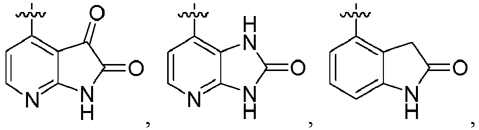 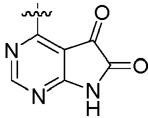 etc. The attachment of heteroaromatic ketone substituents can be achieved by carbon atoms or heteroatoms.

The term "heterocyclic alkane ketone substituents" used in this article refers to a saturated or partially unsaturated single ring, double ring, or multi ring substituent group containing one or more cyclic carbonyl groups, wherein one or more ring atoms are heteroatoms selected from N, O, or S (O) m (where m is an integer of 0-2) heteroatoms, and the remaining ring atoms are carbon. Double rings or multi rings include spiral rings, fused rings, and bridge rings, such as but not limited to: 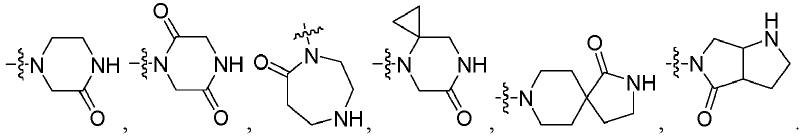 The attachment of heterocyclic alkane ketone substituents can be achieved by carbon atoms or heteroatoms.

As understood by the skilled in the art, "halogen" or "halo" as used herein means chlorine, fluorine, bromine and iodine.

The present disclosure provides a class of Pyridopyrimidine compounds having a structure shown in Formula (I), 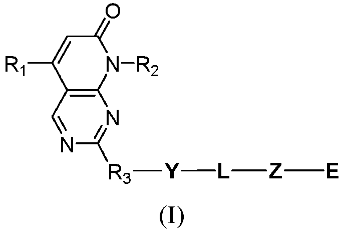 wherein,
E is selected from: H, C₁-C₁₅ alkyl, substituted C₁-C₁₅ alkyl, C₃-C₁₅ cycloalkyl, substituted C₃-C₁₅ cycloalkyl, 3-15 membered heterocycloalkyl, substituted 3-15 membered heterocycloalkyl;
L is absent or is a connecting unit consistingof one or more of the following groups: alkylene, ether, thioether, ester, amine, amide, heteroaryl, cycloalkyl, heterocycloalkyl, -N=N-;
Y is absent or is selected from -O-, -NH-, -NHCO-, -CH₂-, -S-, -CO-;
Z is absent or is selected from: -O-, -NH-, -N(C₁-C₆ alkyl)-, -NHCO-, -CH₂-, -S-, -CO-, -SO-;
R₁ is selected from: H, C₁-C₆ alkyl, halogen or halogen substituted C₁-C₆ alkyl;
R₂ is selected from: C₃-C₁₅ cycloalkyl, substituted C₃-C₁₅ cycloalkyl, 3-15 membered heterocycloalkyl, substituted 3-15 membered heterocycloalkyl, C₆-C₁₀ aryl, substituted C₆-C₁₀ aryl, 5-10 membered heteroaryl, substituted 5-10 membered heteroaryl;
R₃ is 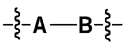 , wherein B is connected to Y through a covalent bond.
A is selected from: -NH-, -NHR-; R is selected from: C₆-C₁₀ aryl, substituted C₆-C₁₀ aryl, 5-10 membered heteroaryl, substituted 5-10 membered heteroaryl;
B is absent or is selected from: C₃-C₁₅ cycloalkyl, substituted C₃-C₁₅ cycloalkyl, 3-15 membered heterocycloalkyl, substituted 3-15 membered heterocycloalkyl, 3-15 membered heterocycloalkyl ketone group, R₈ substituted 3-15 membered heterocycloalkyl ketone group, C₃-C₁₂ cycloalkyl substituted amine group, 3-12 membered heterocycloalkyl substituted amine group,

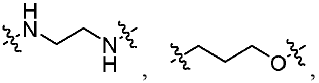

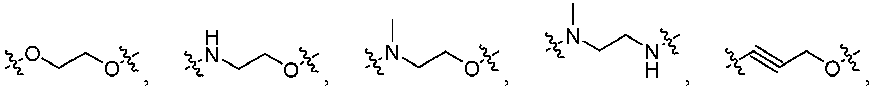

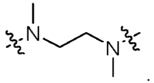

The present disclosure includes free forms of compounds of Formula I, as well as pharmaceutically acceptable salts and stereoisomers thereof. Some specific exemplary compounds in the present disclosure are protonation salts of amine compounds. The term "free form" refers to the amine compound in non-salt form. The pharmaceutically acceptable salts include not only exemplary salts of the particular compounds described herein, but also typical pharmaceutically acceptable salts of free form of all compounds of Formula I. The free forms of specific salts of the compounds can be isolated using techniques known in the art. For example, the free form can be regenerated by treating the salt with appropriate dilute aqueous base, such as dilute aqueous NaOH, dilute aqueous potassium carbonate, dilute aqueous ammonia, and dilute aqueous sodium bicarbonate. The free forms differ somewhat from their respective salt forms in certain physical properties such as solubility in polar solvents, but for the purposes of the disclosure, such salts of acid or base are otherwise pharmaceutically equivalent to their respective free forms.

The pharmaceutically acceptable salts of the present disclosure can be synthesized from the compounds containing a basic or acidic moiety in the present disclosure by conventional chemical methods. Generally, salts of basic compounds can be prepared by ion exchanged chromatography or by reacting the free base with a stoichiometric or excess amount of inorganic or organic acid in the desired salt form in a suitable solvent or combination of solvents. Similarly, salts of acidic compounds can be formed by reaction with a suitable inorganic or organic base.

Accordingly, the pharmaceutically acceptable salts of the compounds in the present disclosure include conventional non-toxic salts of the compounds in the present disclosure formed by reacting a basic compound of the present disclosure with an inorganic or organic acid. For example, conventional non-toxic salts include those derived from inorganic acids such as hydrochloric acid, hydrobromic acid, sulfuric acid, sulfamic acid, phosphoric acid, nitric acid, etc. They also include those derived from organic acids such as acetic acid, propionic acid, succinic acid, glycolic acid, hard Fatty acid, lactic acid, malic acid, tartaric acid, citric acid, ascorbic acid, pamoic acid, maleic acid, hydroxymaleic acid, phenylacetic acid, glutamic acid, benzoic acid, salicylic acid, p-aminobenzenesulfonic acid, 2 - acetoxy - benzoic acid, fumaric acid, toluenesulfonic acid, methanesulfonic acid, ethanedisulfonic acid, oxalic acid, isethionic acid, and trifluoroacetic acid, etc.

If the compounds of the present disclosure are acidic, appropriate "pharmaceutically acceptable salts" refer to salts prepared from pharmaceutically acceptable non-toxic bases including inorganic bases and organic bases. The salts derived from inorganic bases include aluminum salts, ammonium salts, calcium salts, copper salts, iron salts, ferrous salts, lithium salts, magnesium salts, manganese salts, manganous salts, potassium salts, sodium salts, zinc salts, etc. Particularly preferably, ammonium salts, calcium salts, magnesium salts, potassium salts, and sodium salts. The salts derived from pharmaceutically acceptable organic non-toxic bases include salts of primary, secondary and tertiary amines. Substituted amines include naturally occurring substituted amines, cyclic amines and basic ion exchange resins such as Amino acid, betaine, caffeine, choline, N, N'-dibenzylethylenediamine, diethylamine, 2-diethylaminoethanol, 2-dimethylaminoethanol, aminoethanol, ethanolamine, ethyl Diamine, N-ethylmorpholine, N-ethylpiperidine, Glucosamine, Glucosamine, Histidine, Hydroxocobalamin, Isopropylamine, Lysine, Methylglucamine, Morpholine, Piperazine, Piperidine, quack, polyamine resin, procaine, purine, theobromine, triethylamine, trimethylamine, tripropylamine, tromethamine, etc.

The preparation of the pharmaceutically acceptable salts described above and other typical pharmaceutically acceptable salts was described in more detail by Berg et al., in "Pharmaceutical Salts," J. Pharm. Sci. 1977:66: 1-19.

In one embodiment, the present disclosure provides a method of treating tumors, cardiovascular diseases, diabetes, hypertension, muscular dystrophy, Parkinson's disease, Alzheimer's disease and other diseases related to abnormal expression of AKT3 protein in humans or other mammals by using compounds of Formula I, as well as pharmaceutically acceptable salts and stereoisomers thereof.

In one embodiment, the compounds , as well as pharmaceutically acceptable salts and stereoisomers thereof in the present disclosure can be used for treating and/or preventing tumors, such as non-small cell lung cancer, malignant melanoma, prostate cancer, kidney cancer, liver cancer, bladder cancer, ovarian cancer, colon cancer, rectal cancer, breast cancer, cervical cancer, lung cancer, laryngeal cancer, nasopharyngeal cancer, pancreatic cancer, multiple myeloma, B lymphoma, leukemia, or preventing postoperative recurrence of tumors.

### Drug metabolites and prodrugs:

The metabolites of the compounds of the present disclosure and their pharmaceutically acceptable salts, and prodrugs that can be converted into the structures of the compounds of the present disclosure or their pharmaceutically acceptable salts in vivo, also fall within the scope of protection defined by the claims of this application.

### Pharmaceutical composition

The present disclosure also provides a pharmaceutical composition, comprising active ingredients within a safe and effective dosage range, as well as pharmaceutically acceptable carriers or excipients.

The "active ingredient" mentioned in the present disclosure refers to the compounds of Formula I according to the present disclosure or their pharmaceutically acceptable salts, stereoisomers, or prodrug.

The "active ingredient" and pharmaceutical composition of the present disclosure can be used as AKT3 protein degradation agent, and can be used to prepare drugs to prevent and/or treat tumors, cardiovascular diseases, diabetes, hypertension, muscular dystrophy, Parkinson's disease, Alzheimer's disease, etc.

"Safe and effective dosage" refers to the amount of the active ingredients that are sufficient to significantly improve the condition without causing serious side effects. Typically, the pharmaceutical compositions contain 1-2000 mg of active ingredients/formulation, and more preferably, 10-200 mg of active ingredients/formulation. Preferably, the 'one dose' is one tablet.

"Pharmaceutically acceptable carrier or excipient" refers to one or more compatible solid or liquid fillers or gel substances, which are suitable for human use, and must have sufficient purity and sufficiently low toxicity.

"Compatibility" here refers to that each component in the composition can be mixed with the active ingredients of the present disclosure and intermingled between each other without significantly reducing the efficacy of the active ingredients.

Embodiments of pharmaceutically acceptable carriers or excipients include cellulose and its derivatives (such as sodium carboxymethyl cellulose, sodium ethylcellulose, cellulose acetate, etc.), gelatin, talc, solid lubricants (such as stearic acid, magnesium stearate), calcium sulfate, vegetable oil (such as soybean oil, sesame oil, peanut oil, olive oil, etc.), polyols (such as propylene glycol, glycerin, mannitol, sorbitol, etc.), emulsifier (such as Tween ^{®}), wetting agent (such as sodium dodecyl sulfate), colorant, flavoring agent, stabilizer, antioxidant, preservative, pyrogen-free water, etc.

In another preferred embodiment, the compounds of Formula I of the present disclosure can form complexes with macromolecular compounds or macromolecule through nonbonding cooperation. In another preferred embodiment, the compound of Formula I of the present disclosure, as a small molecule, can also be connected with a macromolecular compound or a polymer through a chemical bond. The macromolecular compounds can be biological macromolecules such as polysaccharides, proteins, nucleic acids, peptides, etc.

There is no special restriction on application methods of the active ingredients or drug composition of the present disclosure, and typical administration methods include (but not limited to) oral, intratumoral, rectal, parenteral (intravenous, intramuscular or subcutaneous), etc.

The solid dosage forms used for oral administration include capsules, tablets, pills, powders and granules.

In these solid dosage forms, the active ingredient is mixed with at least one conventional inert excipient (or carrier), such as sodium citrate or dicalcium phosphate, or with the following ingredients:
(a) fillers or compatibilizers, such as starch, lactose, sucrose, glucose, mannitol, and silicic acid;
(b) adhesives, such as hydroxymethyl cellulose, alginate, gelatin, polyvinylpyrrolidone, sucrose, and Arabic gum;
(c) humectants, such as glycerin;
(d) disintegrating agents, such as agar, calcium carbonate, potato starch or cassava starch, algic acid, some composite silicates, and sodium carbonate;
(e) slow solvent, such as paraffin;
(f) absorption accelerators, such as quaternary amine compounds;
(g) wetting agents, such as cetyl alcohol and glyceryl monostearate;
(h) adsorbents, such as kaolin; and
(i) lubricants, such as talc, calcium stearate, magnesium stearate, solid polyethylene glycol, sodium dodecyl sulfate, or their mixtures. In the case of capsules, tablets and pills, the dosage form may also include buffers.

The solid dosage form can also be prepared with coating and shell materials, such as casings and other materials known in the art. They may comprise an opaque agent. Furthermore, the active ingredients from such compositions may be released in certain part of the digestive tract in a delayed manner. Embodiments of embedding components that can be used are polymers and waxes.

Liquid dosage forms for oral administration include pharmaceutically acceptable lotion, solutions, suspensions, syrups or tinctures. In addition to the active ingredients, the liquid dosage form may include inert diluents commonly used in the art, such as water or other solvents, solubilizers, emulsifiers (such as ethanol, isopropanol, ethyl carbonate, ethyl acetate, propylene glycol, 1,3-butanediol, dimethylformamide), and oil (especially cottonseed oil, peanut oil, corn germ oil, olive oil, castor oil and sesame oil or mixtures of these substances). In addition to these inert diluents, the composition may also include auxiliary agents, such as wetting agents, emulsifiers and suspending agents, sweeteners, flavoring agents and spices.

In addition to the active ingredients, the suspension may contain suspension agents, such as ethoxylated isooctadecanol, polyoxyethylene sorbitol and dehydrated sorbitol ester, microcrystalline cellulose, aluminum methoxide and agar, or mixtures of these substances.

Compositions for parenteral injection may include physiologically acceptable sterile aqueous or anhydrous solutions, dispersions, suspensions or emulsions, and sterile powders for re-dissolution into sterile injectable solutions or dispersions. Suitable aqueous and non-aqueous carriers, diluents, solvents or excipients include water, ethanol, polyols, and their suitable mixtures.

The compound of the present disclosure can be administered separately or in combination with other therapeutic drugs.

When using a pharmaceutical composition, a safe and effective amount of the compound of the present disclosure is applied to mammals (such as humans) in need of treatment, wherein the dosage at the time of application is a pharmaceutically effective dosage. For an individual weighing 60 kg, the daily dosage is usually 1-2000 mg, preferably 20-500 mg. Of course, the specific dosage should also consider factors such as the route of administration and the patient's health status, etc., which are within the skill range of a skilled physician.

### Drug Combinations

The compounds of Formula I may be used in combination with other drugs known to treat or improve similar conditions. In the case of combined administration, the administration mode and dosage of the original drug remain unchanged, while the compounds of Formula I are administered simultaneously or subsequently. When the compounds of Formula I are administered concomitantly with one or more other drugs, it is preferred to use a pharmaceutical composition containing one or more known drugs and the compounds of Formula I. Drug combination also includes administration of the compounds of Formula I with one or more other known drugs in overlapping time periods. When the compounds of Formula I are used in combination with one or more other drugs, the compounds of Formula I or known drugs may be administered at lower doses than that when they are administered alone.

Drugs or active ingredients that can be used in combination with the compounds of Formula I include, but not limited to: estrogen receptor modulators, androgen receptor modulators, retinal-like receptor modulators, cytotoxic/cytostatics, antiproliferative agents, protein transferase inhibitors, HMG-CoA reductase inhibitors, HIV protein kinase inhibitors agents, reverse transcriptase inhibitors, angiogenesis inhibitors, cell proliferation and survival signaling inhibitors, drugs that interfere with cell cycle checkpoints and apoptosis inducers, cytotoxic drugs, tyrosine protein inhibitors, EGFR inhibitors, VEGFR inhibitors, serine/threonine protein inhibitors, Bcr-Abl inhibitors, c-Kit inhibitors, Met inhibitors, Raf inhibitors, MEK inhibitors, MMP inhibitors, topoisomerase inhibitors, histidine Acid deacetylase inhibitors, proteasome inhibitors, CDK inhibitors, Bcl-2 family protein inhibitors, MDM2 family protein inhibitors, IAP family protein inhibitors, STAT family protein inhibitors, PI3K inhibitors, AKT inhibitors , integrin blocker, interferon-α, interleukin-12, COX-2 inhibitor, p53, p53 activator, VEGF antibody, EGF antibody, JAK inhibitors, etc.

In one embodiment, the drugs or active ingredients that can be used in combination with the compounds of Formula (I) include, but are not limited to: Aldesleukin, Alendronic Acid, Interferon, Altranoin, Allopurinol, Sodium Allopurinol, Palonosetron Hydrochloride, Hexamelamine, Aminoglumitide, Amifostine, Ammonium rubicin, ampicillin, anastrozole, dolasetron, aranesp, arglabin, arsenic trioxide, anoxin, 5-azacytidine, azathioprine, bacille Calmette-Guerin or tic BCG, betadine, beta-acetate Metasone, betamethasone sodium phosphate preparation, bexarotene, bleomycin sulfate, bromouridine, bortezomib, busulfan, calcitonin, alezolizumab injection, capecitabine, carboplatin, kangshi cefesone, cymoleukin, daunorubicin, chlorambucil, cisplatin, cladribine, cladribine, clodronate, cyclophosphamide, cytarabine, dacarbazine, actinobacteria D, Daunorubicin Liposome, Dexamethasone, Dexamethasone Phosphate, Estradiol Valerate, Denisole 2, Dipomet, Delorelin, Delazoxan, Diethylstilbestrol, Dafucon, Docetaxel, Deoxyfluridine, Doxorubicin, Dronabinol, Chin-166-chitosan complex, eligard, rasburicase, epirubicin hydrochloride, aprepitant, epirubicin, epoetin alfa, erythropoietin, eplatin, Levamisole tablets, estradiol preparations, 17-beta-estradiol, estramustine sodium phosphate, ethinyl estradiol, amifostine, hydroxyphosphate, fenbifu, etoposide, fadrozole, tamoxib fenestrate, filgrastim, finasteride, filgrastim, floxuridine, fluconazole, fludarabine, 5-fluorodeoxyuridine monophosphate, 5-fluorouracil, fluoxymesterone, Flutamide, Formestan, 1-β-D-D-arabinofuranocytothidine-5'-stearoyl phosphate, Formustine, Fulvestrant, Gammaglobulin, Gemcitabine, Gemtox Monoclonal antibody, imatinib mesylate, carbazide, wax paper capsules, goserelin, granisilone hydrochloride, histrelin, and methoxine, hydrocortisone, erythro-hydroxynonyl adrenal gland Purine, hydroxyurea, titan isibemumab, idarubicin, ifosfamide, interferon alpha, interferon-alpha2, interferon alpha-2A, interferon alpha-2B, interferon alpha-nl, Interferon alpha-n3, interferon beta, interferon gamma-la, interleukin-2, intron A, Iressa, irinotecan, keteri, lentinan sulfate, letrozole, tetrahydroform Folic acid, leuprolide, leuprolide acetate, levothyroxine, levothyroxine calcium salt, levothyroxine sodium, levothyroxine sodium preparations, lomustine, lonidamine, dronabinol, Nitrogen mustard, mecobalamin, medroxyprogesterone acetate, megestrol acetate, melphalan, esterified estrogen, 6-mercaptopurine, mesna, methotrexate, methyl aminolevulinate , mitifoxine, minocycline, mitomycin C, mitotane, mitosodium quinone, trolosteine, doxorubicin citrate liposome, nedaplatin, pegylated filgras kiosk, opryleukin, neupogen, nilutamide, tamoxifen, NSC-631570, Recombinant Human Interleukin 1-β, Octreotide, Ondansetron Hydrochloride, Dehydrocortisone Oral Solution, Oxaliplatin, Paclitaxel, Prednisone Sodium Phosphate, Pegaspargase, Pegasin, Pentostatin, Streptolyticum, Pilocarpine Hydrochloride, Pirarubicin, Pukamycin, Porfimer Sodium, Prednimustine, Steprednisolone, Prednisone, Premax Li, Procarb, Recombinant Human Erythropoietin, Raltitrexed, Ribi, Etidronate, Rhenium-186, Rituxan, Strength-A, Romotide, Pilocarpine Hydrochloride Tablets, Octreotide, Samostim, Semustine, Sizoran, Sobuzoxan, Methylprednisolone, Paphos Acid, Stem Cell Therapy, Streptozocin, Strontium Chloride-89, Levothyroxine Sodium, Tamoxifen, Tansu Loxin, Tasonamin, Tastolactone, Taxotere, Tecethiazine, Temozolomide, Teniposide, Testosterone Propionate, Methyltestosterone, Thioguanine, Thiatepa, Thyroid Stimulating Hormone, Tiludronic Acid, Topotecan, Toremifene, Tosilimumab, Trastuzumab, Triosulfan, Tretinoin, Methotrexate Tablets, Trimethylmelamine, Trimethrexate, Tripro acetate Relin, triptorelin pamoate, eufradine, uridine, valrubicin, veslinone, vinblastine, vincristine, vincristine, vinorelbine, velulizine, dextran Propionimine, net statin, zofenin, paclitaxel protein stabilizer, acolbifene, interferon r-lb, affinitak, aminopterin, azoxifene, asoprisnil, atamestane, atrasentan, BAY 43-9006, Avastin, CCI-779, CDC-501, Celebrex, Cetuximab, Crinator, Cyproterone Acetate, Decitabine, DN-101, Doxorubicin- MTC, dSLIM, dutasteride, edotecarin, eflunomine, ixitecan, fenretinide, histamine dihydrochloride, histidine hydrogel implant, holmium-166 DOTMP, ibandronic acid, interferon gamma, intron-PEG, ixabepilone, keyhole limpet hemocyanin, L-651582, lanreotide, Lasoxifene, libra, lonafamib, imiprexifene, minoxifene, MS-209, liposomal MTP-PE, MX-6, nafarelin, nemorubicin, novarestat , nolatrexide, olimerson, onco-TCS, osidem, paclitaxel polyglutamate, sodium paclitaxel, PN-401, QS-21, quasiyang, R-1549, raloxifene, leopard Ranazyme, 13-cis-retinoic acid, satraplatin, siocalcidol, T-138067, tarceva, docosahexaenoate paclitaxel, thymosin alphal, gazofurin, tipifarnib, tirapazamine, TLK-286, toremifene, trans MID-lo7R, valspoda, vapretide, vatalanib, verteporfin, vinflunine, Z-100 and zolelinic acid or their combination.

The benefits of the present disclosure are:
(1) A novel structure of Pyridopyrimidine compounds is provided.
(2) This type of compounds can efficiently and highly selectively degrade AKT3 protein in cells without affecting AKT1/2. They can effectively inhibit the growth of various tumor cells and can be used to prepare anti-tumor drugs.

A further description about the present disclosure is given below in conjunction with specific embodiments. It should be understood that these embodiments are only used to illustrate the present disclosure, but not to limit its scope. The following embodiments, which have not specified specific conditions, are usually performed in accordance with conventional conditions, such as those described in Sambrook, et al., "Molecular Cloning: Laboratory Manual" (New York: Cold Spring Harbor Laboratory Press, 1989) , or as recommended by the manufacturers. Unless otherwise specified, percentages and portions are calculated by weight.

Unless otherwise defined, all professional and scientific terms used herein have the same meanings as those are familiar to the skilled in the art. In addition, any methods and materials similar or equivalent to those described herein can be applied to the methods of the present disclosure. The preferred implementation methods and materials described herein are for demonstration purposes only.

The raw materials in the following embodiments can be obtained commercially, or prepared by methods known in the art, or according to the methods described herein.

The structure of the compound is determined by nuclear magnetic resonance (¹H-NMR) and/or mass spectrometry (MS). NMR determination is carried out using the Bruker AV-400 nuclear magnetic resonance instrument, with deuterated chloroform (CDCl3) or deuterated dimethyl sulfoxide (DMSO-D6) as the solvent and TMS as the internal standard. The LCQAD-40000 mass spectrometer is used for the determination of MS. 200-300 mesh silica gel (produced by Qingdao Ocean Chemical Factory) is used for Column chromatography.

### Embodiment 1: N-(3-(2-((4-(4-(2-(2-(2-(2-(2-((((3R, 5R, 7R) - adamantan-1-yl) acetamido) ethoxy) ethoxy) ethoxy) ethyl) piperazin-1-yl) -2-methoxyphenyl) amino) -5-methyl-7-oxypyridino [2,3-d] pyrimidin-8 (7H) - yl) phenyl) acrylamide (ZX-HYT-01)

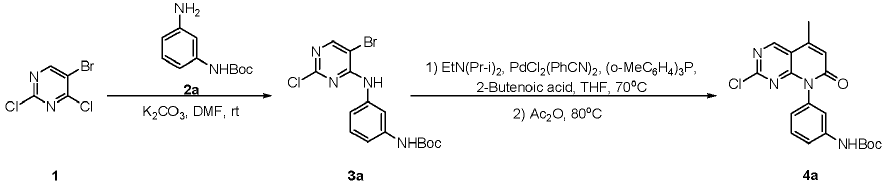

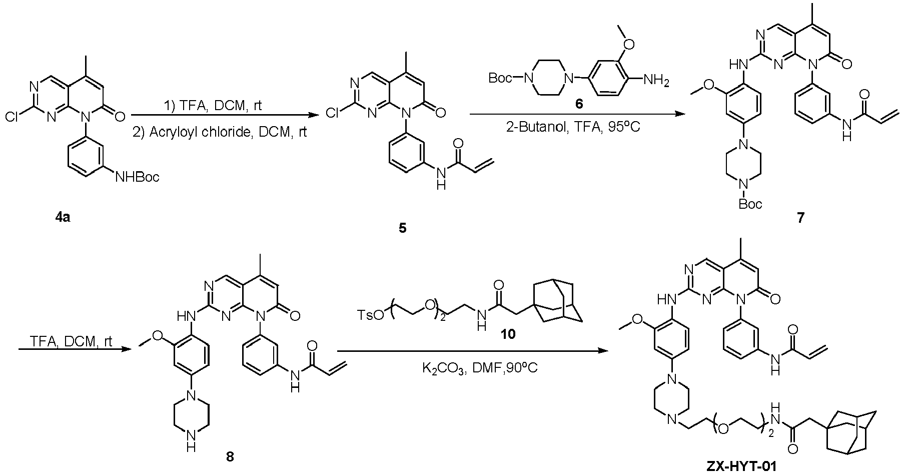

### Step 1: Synthesis of (3- ((5-bromo-2-chloropyrimidin-4-yl) amino) phenyl) tert butyl carbamate (3a)

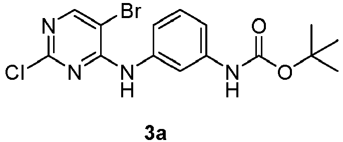

5-bromo-2,4-dichloropyrimidine **(1)** (0.45 g, 2.0 mmol) and (3-aminophenyl) tert butyl carbamate **(2a)** (0.42 g, 2.0 mmol) were sequentially dissolved in N, N-dimethylformamide (DMF) (10 mL) solution, and then potassium carbonate (0.55 g, 4.0 mmol) was added. The suspension was stirred overnight at room temperature. After the completion of the reaction monitored by TLC , 50 mL of ice water was added to the reaction solution, and a large amount of white precipitate was generated. Filtered under reduced pressure, and the filter cake was washed with ice water and anhydrous ether separately (3 × 20 mL). After the filter cake was drained, it was added to 10 mL of acetone for pulping, and then filtered under reduced pressure again. The resulting filter cake was dried to obtain a relatively pure white solid compound **3a** (0.67 g, yield 84.2%). MS (ESI), *m*/*z:* 399.1 [M+H]⁺ . ¹H NMR (400 MHz, DMSO-*d*₆) δ 8.29 (s, 1 H), 7.78 (s, 1 H), 7.45 (d, 1 H, *J* = 7.2 Hz), 7.32-7.28 (m, 2 H), 7.03 (dd, 1 H, *J* = 1.2, 8.0 Hz), 6.56 (s, 1 H), 1.53 (s, 9 H).

### Step 2: Synthesis of (3- (2-chloro-5-methyl-7-oxypyridino [2,3-d] pyrimidin-8 (7H) - yl) phenyl) tert butyl carbamate (4a)

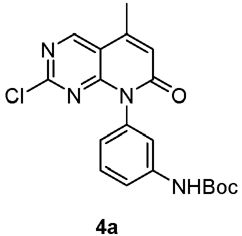

Compound **3a** (2.57 g, 6.43 mmol) and crotonic acid (5.54 g, 64.3 mmol) were placed in a 250 mL two-necked round-bottom flask, and anhydrous tetrahydrofuran (40 mL) and N, N- diisopropylethylamine (11.2 mL) were added under argon protection. After stirring the mixture evenly, replaced with argon three times. Added Bis (cyanobenzene) palladium dichloride (II) (0.12 g, 5%) and tri (o-methylphenyl) phosphorus (96 mg, 5%) again, and replaced with argon three times. Then the temperature of the mixture was slowly raised to 70° C. After the complete reaction of the raw material **3a** was detected by TLC, 1.5 mL of acetic anhydride was added. The reaction solution was heated to 80 °C and continued stirring for 8 hours. After the reaction was completed monitored by TLC, most of the organic solvent was evaporated under reduced pressure, and the residue was diluted with 100 mL of ethyl acetate. The organic layer was washed with 1N HCl (3 × 100 mL) and saturated sodium chloride solution (2 × 100 mL), and the separated organic phase was dried with anhydrous sodium sulfate, and then concentrated and purified through column chromatography (petroleum ether/ethyl acetate=2:1 elution) to obtain a white solid compound **4a** (0.71 g, yield 30%). MS (ESI), *m*/*z:* 387.3 [M+H]⁺. ¹H NMR (400 MHz, DMSO-*d*₆) δ 9.61 (s, 1H), 9.10 (s, 1H), 7.51 - 7.38 (m, 3H), 6.89 (dt, *J* = 7.4, 1.7 Hz, 1H), 6.73 (d, *J* = 1.4 Hz, 1H), 2.53 (d, *J* = 1.3 Hz, 3H), 1.47 (s, 9H).

### Step 3: Synthesis of N - (3- (2-chloro-5-methyl-7-oxypyridino [2,3-d] pyrimidin-8 (7H) - yl) phenyl) acrylamide (5)

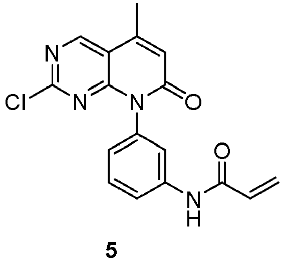

TFA (2 mL) was added into a DCM (10 mL) solution of compound **4a** (0.50 g, 1.29 mmol) , stirred at room temperature for 0.5 hours. After the reaction was complete, the organic solvent was removed by evaporating under reduced pressure. The obtained residue was dissolved in 10 mL of acetonitrile and the solution was sequentially added anhydrous potassium carbonate (0.36 g, 2.58 mmol) and acryloyl chloride (0.17 g, 1.94 mmol), continued stirring for 0.5 hours at room temperature. After the reaction was completed monitored by TLC, the organic solvent was evaporated and 50 mL of ice water was added. Continued stirring at room temperature for 1 hour and filtered under reduced pressure. The filter cake was washed with anhydrous acetone (2 × 25 mL). A white solid intermediate 5 (0.41 g, yield 93%) was obtained after the filter cake was dried. The crude intermediate could be used in the next reaction without purification. MS (ESI), *m*/*z:* 341.0 [M+H]⁺ . ¹H NMR (400 MHz, DMSO-*d*₆) δ ppm 10.69 (br, 1 H), 9.11 (s, 1 H), 7.78 (d, J = 8.22 Hz, 1 H), 7.71 (br, 1 H), 7.49 (t, J = 7.92 Hz, 1 H), 7.00 (d, J = 7.63 Hz, 1 H), 6.74 (s, 1 H), 6.56 (dd, J = 10.27, 16.92 Hz, 1 H), 6.26 (d, J = 16.82 Hz, 1 H), 5.76 (d, J = 10.17 Hz, 1 H), 2.55 (s, 3 H).

### Step 4: Synthesis of tert butyl 4- (4- (8- (3-acryloylphenyl) -5-methyl-7-oxy-7,8-dihydropyridino [2,3-d] pyrimidin-2-yl) amino) -3-ethoxyphenyl) piperazin-1-carboxylate (7)

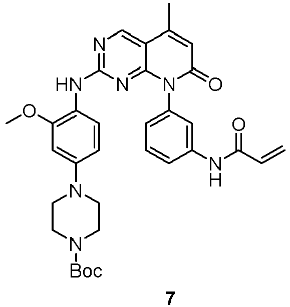

25 mL of sec-butanol was added to compound **5** (0.68 g, 2 mmol) and compound **6** (0.67 g, 2.2 mmol), and the mixture was added dropwise a catalytic amount of trifluoroacetic acid. Under argon protection, the reaction solution was heated to 95° C and stirred overnight. After the reaction was completed monitored by TLC, the mixture was cooled to room temperature and the organic solvent was evaporated under reduced pressure. The residue was separated and purified by column chromatography (chloroform/methanol=25:1 elution) to obtain a yellow solid compound 7 (1.11 g, yield 92%). MS (ESI), *m*/*z:* 612.1 [M+H]⁺ . ¹H NMR (400 MHz, DMSO-*d*₆) δ 10.35 (s, 1H), 8.81 (s, 1H), 8.14 (s, 1H), 7.90 (d, *J* = 8.2 Hz, 1H), 7.56 (t, *J* = 2.0 Hz, 1H), 7.51 (t, *J* = 8.1 Hz, 1H), 7.28 (d, *J* = 8.9 Hz, 1H), 6.98 (ddd, *J* = 7.9, 2.0, 1.0 Hz, 1H), 6.56 (d, *J* = 2.5 Hz, 1H), 6.50 - 6.38 (m, 1H), 6.33 (d, *J* = 1.3 Hz, 1H), 6.30 - 6.21 (m, 1H), 6.03 (s, 1H), 5.77 (dd, *J* = 10.1, 2.1 Hz, 1H), 3.78 (s, 3H), 3.51 - 3.40 (m, 4H), 3.05 - 2.89 (m, 4H), 2.46 (s, 3H), 1.44 (s, 9H).

### Step 5: Synthesis of N - (3- (2- ((2-methoxy-4- (piperazin-1-yl) phenyl) amino) -5-methyl-7-oxypyridino [2,3-d] pyrimidin-8 (7H) - yl) phenyl) acrylamide (8)

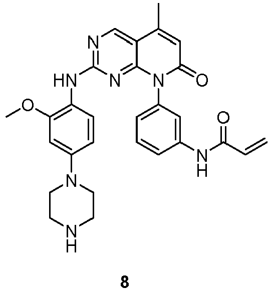

TFA (2 mL) was added into a DCM (20 mL) solution of compound 7 (1.22 g, 2 mmol) and the resulting solution was stirred at room temperature for 0.5 hours. After the reaction was completed monitored by TLC, the organic solvent was evaporated and the crude product obtained was purified by column chromatography (chloroform/methanol/ammonia=25:1:0.1 elution) to obtain a yellow solid product of **8** (0.91 g, yield 90%). MS (ESI), *m*/*z:* 510.3 [M-H]⁻. ¹H NMR (400 MHz, DMSO-*d*₆) δ 10.41 (s, 1H), 8.90 (s, 1H), 8.82 (s, 1H), 8.18 (s, 1H), 7.90 (d, *J* = 8.3 Hz, 1H), 7.56 - 7.45 (m, 2H), 7.31 (d, *J* = 8.8 Hz, 1H), 7.00 (dd, *J* = 7.9, 2.0 Hz, 1H), 6.60 (d, *J* = 2.6 Hz, 1H), 6.45 (dd, *J* = 16.9, 10.1 Hz, 1H), 6.35 - 6.23 (m, 2H), 6.06 (s, 1H), 5.77 (dd, *J* = 10.1, 2.1 Hz, 1H), 3.79 (s, 3H), 3.24 (s, 8H), 2.48 - 2.44 (m, 3H)

### Step 6: Synthesis of N-(3-(2-((4-(4-(2-(2-(2-(2-(2-(((3R, 5R, 7R) - adamantan-1-yl) acetylamino) ethoxy) ethoxy) ethyl) piperazin-1-yl) -2-methoxyphenyl) amino) -5-methyl-7-oxypyridino [2,3-d] pyrimidin-8 (7H) - yl) phenyl) acrylamide (ZX-HYT-01)

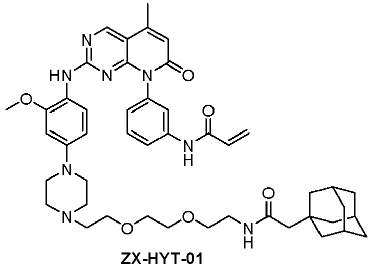

Compound **8** (0.20 g, 0.39 mmol), 2-(2-(2-(2-(2-(((3R, 5R, 7R) - adamantan-1-yl) acetamido) ethoxy) ethoxy) ethyl 4-methylbenzenesulfonate **10a** (0.23 g, 0.47 mmol), and anhydrous potassium carbonate (0.11 g, 0.8 mmol) were sequentially added to DMF (10 mL), and the reaction system was heated to 90 ° C and stirred overnight. After the reaction was completed monitored by TLC, the system was cooled to room temperature and added a saturated sodium chloride solution (50 mL), and extracted with ethyl acetate (40 mL). The organic phase was dried and concentrated with anhydrous sodium sulfate, and the resulting crude product was purified through column chromatography (chloroform/methanol=30/1 elution) to obtain a yellow solid target compound **ZX-HYT-01** (0.21 g, yield 66%). HRMS (ESI) for C₄₆H₅₈N₈O₆Na [M+Na]⁺: calcd, 841.4377; found, 841.4372. HPLC analysis: MeOH-H₂O (97:3), RT = 4.747 min, 98.07% purity. ¹H NMR (400 MHz, DMSO-*d*₆) δ 10.36 (s, 1H), 8.81 (s, 1H), 8.12 (s, 1H), 7.89 (d, *J* = 8.1 Hz, 1H), 7.72 (t, *J* = 5.7 Hz, 1H), 7.57 (t, *J* = 2.1 Hz, 1H), 7.51 (t, *J* = 8.1 Hz, 1H), 7.27 (d, *J* = 8.9 Hz, 1H), 6.98 (dd, *J* = 7.8, 2.0 Hz, 1H), 6.56 - 6.49 (m, 1H), 6.49 - 6.39 (m, 1H), 6.37 - 6.21 (m, 2H), 6.00 (s, 1H), 5.77 (dd, *J* = 10.0, 2.1 Hz, 1H), 3.78 (s, 3H), 3.60 - 3.49 (m, 6H), 3.42 (t, *J =* 5.9 Hz, 2H), 3.19 (q, *J* = 5.9 Hz, 2H), 3.02 (s, 4H), 2.63 - 2.52 (m, 6H), 2.46 (s, 3H), 1.91 (s, 3H), 1.83 (s, 2H), 1.70 - 1.61 (m, 3H), 1.60 - 1.50 (m, 9H). ¹³C NMR (101 MHz, DMSO) δ 170.52, 163.73, 162.63, 157.05, 156.73, 147.43, 140.39, 137.52, 132.15, 129.97, 127.66, 124.52, 120.13, 119.20, 116.98, 106.60, 100.06, 70.15, 69.99, 69.65, 56.15, 53.52, 50.47, 49.12, 42.54, 38.82, 36.93, 32.61, 28.52, 17.48.

### Embodiment 2: N-(3-(2-((4-(4-(6-(2-(((3R, 5R, 7R) - adamantan-1-yl) acetamido) hexyl) piperazin-1-yl) -2-methoxyphenyl) amino) -5-methyl-7-oxypyridino [2,3-d] pyrimidin-8 (7H) - yl) phenyl) acrylamide (ZX-HYT-02)

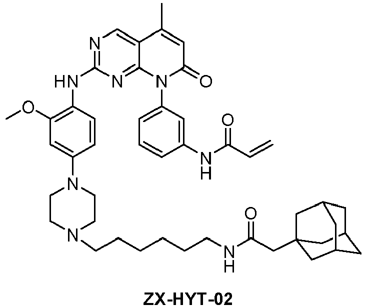

The synthesis method of compound **ZX-HYT-02** was similar to that of **ZX-HYT-01.** Compounds **8** (0.20 g, 0.39 mmol) and 6- (2- ((((3R, 5R, 7R) - adamantan-1-yl) acetamido) -4-methylbenzenesulfonic acid hexyl ester (0.21 g, 0.47 mmol) was used as raw materials, a yellow solid compound **ZX-HYT-02** (0.18 g, yield 59%) was obtained through nucleophilic substitution reaction. HRMS (ESI) for C₄₆H₅₈N₈O₄Na [M+Na]⁺: calcd, 809.4479; found, 809.4473. HPLC analysis: MeOH-H₂O (97:3), RT = 5.260 min, 98.05% purity. ¹H NMR (400 MHz, DMSO-*d*₆) δ 10.35 (s, 1H), 8.81 (s, 1H), 8.11 (s, 1H), 7.90 (d, *J* = 8.2 Hz, 1H), 7.65 (t, *J* = 5.6 Hz, 1H), 7.59 - 7.55 (m, 1H), 7.51 (t, *J* = 8.0 Hz, 1H), 7.27 (d, *J* = 8.9 Hz, 1H), 6.98 (ddd, *J* = 7.9, 2.1, 1.0 Hz, 1H), 6.52 (d, *J* = 2.5 Hz, 1H), 6.49 - 6.39 (m, 1H), 6.37 - 6.21 (m, 2H), 6.00 (s, 1H), 5.77 (dd, *J* = 10.1, 2.1 Hz, 1H), 3.78 (s, 3H), 3.09 - 2.95 (m, 6H), 2.48 - 2.43 (m, 6H), 2.30 (t, *J* = 7.3 Hz, 2H), 1.91 (d, *J* = 5.0 Hz, 3H), 1.81 (s, 2H), 1.69 - 1.63 (m, 3H), 1.61 - 1.54 (m, 9H), 1.49 - 1.43 (m, 2H), 1.42 - 1.36 (m, 2H), 1.33 - 1.27 (m, 4H). ¹³C NMR (101 MHz, DMSO) δ 170.22, 163.72, 162.64, 157.06, 156.73, 147.44, 140.39, 137.52, 132.16, 129.98, 127.63, 124.52, 120.12, 119.20, 116.97, 106.60, 100.06, 58.24, 56.14, 53.17, 50.60, 49.14, 42.62, 38.67, 36.94, 32.62, 29.65, 28.52, 27.11, 26.84, 26.70, 17.48.

### Embodiment 3: N-(3-(2-((4-(4-(8-(2-(((3R, 5R, 7R) - adamantan-1-yl) acetamido) octyl) piperazin-1-yl) -2-methoxyphenyl) amino) -5-methyl-7-oxypyridino [2,3-d] pyrimidin-8 (7H) - yl) phenyl) acrylamide (ZX-HYT-03)

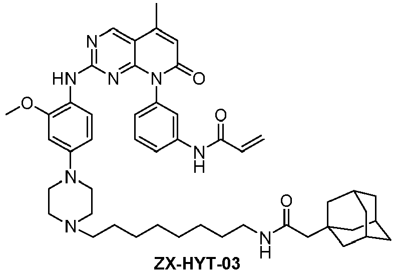

The synthesis method of compound **ZX-HYT-03** was similar to that of **ZX-HYT-01.** Compounds **8** (0.20 g, 0.39 mmol) and 8- (2- (((3R, 5R, 7R) - adamantan-1-yl) acetylamino) octyl-4-methylbenzenesulfonate (0.20 g, 0.47 mmol) were used as raw materials, a yellow solid compound **ZX-HYT-03** (0.17 g, yield 55%) was obtained through nucleophilic substitution. HRMS (ESI) for C₄₈H₆₂N₈O₄Na [M+Na]⁺: calcd, 837.4792; found, 837.4786. HPLC analysis: MeOH-H₂O (97:3), RT = 5.925 min, 98.11% purity. ¹H NMR (400 MHz, DMSO-*d*₆) δ 10.35 (s, 1H), 8.81 (s, 1H), 8.11 (s, 1H), 7.90 (d, *J* = 8.0 Hz, 1H), 7.63 (t, *J* = 5.6 Hz, 1H), 7.56 (t, *J* = 2.0 Hz, 1H), 7.51 (t, *J* = 8.0 Hz, 1H), 7.27 (d, *J=* 8.9 Hz, 1H), 6.98 (dd, *J* = 8.0, 2.0 Hz, 1H), 6.52 (d, *J* = 2.5 Hz, 1H), 6.49 - 6.39 (m, 1H), 6.36 - 6.21 (m, 2H), 6.00 (s, 1H), 5.79 - 5.74 (m, 1H), 3.78 (s, 3H), 3.12 - 2.94 (m, 6H), 2.49 - 2.43 (m, 6H), 2.31 (q, *J* = 8.5, 7.4 Hz, 2H), 1.91 (s, 3H), 1.81 (s, 2H), 1.71 - 1.62 (m, 3H), 1.61 - 1.52 (m, 9H), 1.48 - 1.42 (m, 2H), 1.41 - 1.35 (m, 2H), 1.33 - 1.21 (m, 11H). ¹³C NMR (101 MHz, DMSO) δ 170.15, 163.71, 162.57, 156.97, 156.77, 147.32, 140.41, 137.53, 132.22, 130.11, 129.89, 127.45, 124.48, 120.19, 119.20, 116.99, 106.75, 106.61, 100.08, 58.32, 56.17, 53.20, 50.61, 49.22, 42.64, 42.59, 38.69, 36.98, 36.93, 32.62, 29.64, 29.43, 29.15, 28.57, 28.52, 27.36, 26.87, 26.76, 17.44.

### Embodiment 4: N-(3-(2-((4-(4-(10-(2-(((3R, 5R, 7R) - adamantan-1-yl) acetylamino) decyl) piperazin-1-yl) -2-methoxyphenyl) amino) -5-methyl-7-oxypyridino [2,3-d] pyrimidin-8 (7H) - yl) phenyl) acrylamide (ZX-HYT-04)

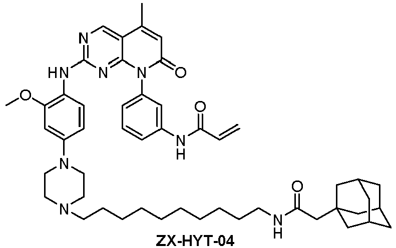

The synthesis method of compound **ZX-HYT-04** was similar to that of **ZX-HYT-01.** Compounds **8** (0.20 g, 0.39 mmol) and 10- (2- (((3R, 5R, 7R) - adamantan-1-yl) acetylamino) octyl-4-methylbenzenesulfonate (0.24 g, 0.47 mmol) were used as raw materials, a yellow solid compound **ZX-HYT-04** (0.19 g, yield 59%) was obtained by nucleophilic substitution reaction. HRMS (ESI) for C₅₀H₆₆N₈O₄Na [M+Na]⁺: calcd, 865.5105; found, 865.5099. HPLC analysis: MeOH-H₂O (97:3), RT = 6.906 min, 97.22% purity. ¹H NMR (400 MHz, DMSO-*d*₆) δ 10.35 (s, 1H), 8.81 (s, 1H), 8.11 (s, 1H), 7.96 - 7.82 (m, 1H), 7.63 (t, *J* = 5.6 Hz, 1H), 7.58 - 7.54 (m, 1H), 7.51 (t, *J* = 8.0 Hz, 1H), 7.27 (d, *J* = 8.9 Hz, 1H), 6.98 (dd, *J* = 7.7, 1.9 Hz, 1H), 6.52 (d, *J* = 2.5 Hz, 1H), 6.44 (dd, *J* = 16.9, 10.1 Hz, 1H), 6.35 - 6.21 (m, 2H), 6.00 (s, 1H), 5.79 - 5.74 (m, 1H), 3.78 (s, 3H), 3.01 (q, *J* = 6.3 Hz, 6H), 2.46 (s, 6H), 2.35 - 2.23 (m, 2H), 1.93 - 1.87 (m, 3H), 1.80 (s, 2H), 1.66 (d, *J* = 12.2 Hz, 3H), 1.61 - 1.52 (m, 9H), 1.45 (d, *J* = 6.6 Hz, 2H), 1.41 - 1.35 (m, 2H), 1.32 - 1.23 (m, 13H). ¹³C NMR (101 MHz, DMSO) δ 170.18, 163.71, 162.63, 157.04, 156.73, 147.42, 140.39, 137.52, 132.17, 129.97, 127.61, 124.51, 120.13, 119.20, 116.97, 106.60, 100.06, 58.30, 56.14, 55.37, 53.15, 50.59, 49.13, 42.62, 38.66, 36.94, 32.61, 29.64, 29.48, 29.46, 29.43, 29.17, 28.53, 27.42, 26.87, 26.67, 17.48.

### Embodiment 5: N-(3-(2-((4-(4-(12-(2-(((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) acrylamide (ZX-HYT-05)

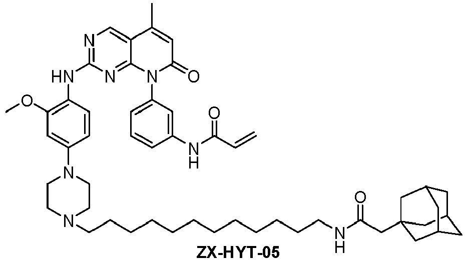

The synthesis method of compound **ZX-HYT-05** was similar to that of **ZX-HYT-01.** Compounds 8 (0.20 g, 0.39 mmol) and 12- (2- (((3R, 5R, 7R) - adamantan-1-yl) acetylamino) dodecyl 4-methylbenzenesulfonate (0.25 g, 0.47 mmol) were used as raw materials, a yellow solid compound **ZX-HYT-05** (0.18 g, yield 54%) was obtained through nucleophilic substitution reaction. HRMS (ESI) for C₅₂H₇0N₈O₄Na [M+Na]⁺: calcd, 893.5418; found, 893.5412. HPLC analysis: MeOH-H₂O (95:5), RT = 9.127 min, 97.17% purity. ¹H NMR (400 MHz, DMSO-*d*₆) δ 10.35 (s, 1H), 8.81 (s, 1H), 8.11 (s, 1H), 7.90 (d, *J* = 8.2 Hz, 1H), 7.62 (t, *J* = 5.7 Hz, 1H), 7.56 (t, *J* = 2.0 Hz, 1H), 7.51 (t, *J* = 8.0 Hz, 1H), 7.27 (d, *J* = 8.9 Hz, 1H), 6.98 (ddd, *J* = 7.9, 2.0, 1.0 Hz, 1H), 6.52 (d, *J* = 2.5 Hz, 1H), 6.44 (dd, *J* = 16.9, 10.1 Hz, 1H), 6.33 (d, *J* = 1.3 Hz, 1H), 6.26 (dd, *J* = 17.0, 2.1 Hz, 1H), 6.00 (s, 1H), 5.76 (dd, *J* = 10.1, 2.1 Hz, 1H), 3.78 (s, 3H), 3.09 - 2.94 (m, 6H), 2.49 - 2.41 (m, 7H), 2.30 (t, *J =* 7.4 Hz, 2H), 1.94 - 1.87 (m, 3H), 1.80 (s, 2H), 1.66 (d, *J* = 12.1 Hz, 3H), 1.58 (s, 2H), 1.56 - 1.52 (m, 7H), 1.50 - 1.42 (m, 2H), 1.40 - 1.33 (m, 2H), 1.31 - 1.21 (m, 16H). ¹³C NMR (101 MHz, DMSO) δ 170.14, 163.69, 162.61, 157.01, 156.73, 147.37, 140.41, 137.52, 132.19, 129.95, 127.55, 124.50, 120.24, 120.14, 119.19, 116.98, 106.65, 106.58, 100.02, 58.36, 56.13, 53.22, 50.59, 49.22, 42.62, 38.66, 36.95, 32.61, 29.64, 29.54, 29.52, 29.48, 29.43, 29.19, 28.54, 27.47, 26.88, 26.77, 17.48.

### Embodiment 6: N-(3-(2-((4-(4-(14-(2-((3R, 5R, 7R) - adamantan-1-yl) acetylamino) tetradecyl) piperazin-1-yl) -2-methoxyphenyl) amino) -5-methyl-7-oxypyridino [2,3-d] pyrimidin-8 (7H) - yl) phenyl) acrylamide (ZX-HYT-06)

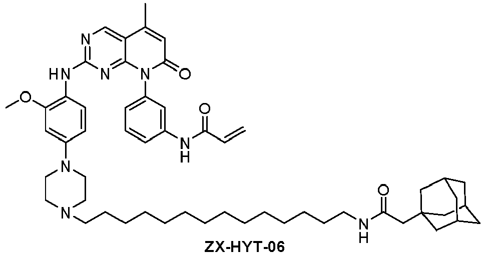

The synthesis method of compound **ZX-HYT-06** is similar to that of **ZX-HYT-01.** Compounds **8** (0.20 g, 0.39 mmol) and 14- (2- (((3R, 5R, 7R) - adamantan-1-yl) acetylamino) dodecyl 4-methylbenzenesulfonate (0.26 g, 0.47 mmol) were used as raw materials, a yellow solid compound **ZX-HYT-06** (0.15 g, yield 43%) was obtained by nucleophilic substitution reaction. HRMS (ESI) for C₅₄H₇₄N₈O₄Na [M+Na]⁺: calcd, 921.5731; found, 921.5725. HPLC analysis: MeOH-H₂O (95:5), RT = 14.347 min, 98.68% purity. ¹H NMR (400 MHz, DMSO-*d*₆) δ 10.35 (s, 1H), 8.80 (s, 1H), 8.11 (s, 1H), 7.90 (d, *J* = 8.1 Hz, 1H), 7.62 (t, *J* = 5.6 Hz, 1H), 7.56 (t, *J* = 2.0 Hz, 1H), 7.51 (t, *J* = 8.1 Hz, 1H), 7.27 (d, *J* = 8.9 Hz, 1H), 6.98 (ddd, *J* = 7.8, 2.0, 1.0 Hz, 1H), 6.51 (d, *J* = 2.5 Hz, 1H), 6.48 - 6.39 (m, 1H), 6.36 - 6.21 (m, 2H), 6.00 (s, 1H), 5.76 (dd, *J* = 10.1, 2.1 Hz, 1H), 3.78 (s, 3H), 3.06 - 2.93 (m, 6H), 2.49 - 2.40 (m, 7H), 2.30 (t, *J* = 7.4 Hz, 2H), 1.90 (s, 3H), 1.80 (s, 2H), 1.69 - 1.61 (m, 3H), 1.60 - 1.53 (m, 9H), 1.50 - 1.41 (m, 2H), 1.40 - 1.33 (m, 2H), 1.32 - 1.20 (m, 20H). ¹³C NMR (101 MHz, DMSO) δ 170.22, 163.72, 162.64, 157.04, 156.72, 147.44, 140.39, 137.51, 132.15, 129.97, 127.62, 124.51, 120.12, 119.20, 116.96, 106.60, 100.04, 58.29, 56.51, 56.14, 53.15, 50.59, 49.12, 42.60, 38.65, 36.93, 32.61, 29.60, 29.51, 29.48, 29.43, 29.40, 29.15, 28.53, 27.41, 26.84, 26.65, 18.99, 17.47.

### Embodiment 7: N-(3-(2-((4-(4-(12-(2-((3R, 5R, 7R) - adamantan-1-yl) acetylamino) dodecanoyl) piperazin-1-yl) -2-methoxyphenyl) amino) -5-methyl-7-oxypyridino [2,3-d] pyrimidin-8 (7H) - yl) phenyl) acrylamide (ZX-HYT-07)

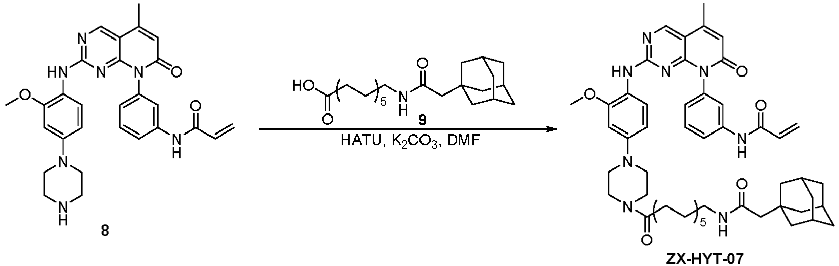

A DMF (10 mL) solution of compound **8** (0.15 g, 0.29 mmol) was added 12-(2- (((3R, 5R, 7R) - adamantan-1-yl) acetamido) dodecanoic acid **9** (0.12 g, 0.29 mmol), HATU (0.17 g, 0.43 mmol), and potassium carbonate (82 mg, 0.58 mmol), and the reaction system was stirred at room temperature for 0.5 hours. After the reaction was completed monitored by TLC, the reaction system was added ethyl acetate (20 mL) for dilution, and then saturated sodium chloride solution (1 × 30 mL) and water (3 × 30 mL) were used to wash the organic layer. The residue obtained after drying and concentration over anhydrous sodium sulfate was purified by column chromatography (chloroform/methanol=30/1 elution) to obtain a yellow solid compound **ZX-HYT-07** (0.13 g, yield 51%). HRMS (ESI) for C₅₂H₆₈N₈O₅Na [M+Na]⁺: calcd, 907.5210; found, 907.5205. HPLC analysis: MeOH-H₂O (95:5), RT = 6.582 min, 100.00% purity . ¹H NMR (400 MHz, DMSO-*d*₆) δ 10.35 (s, 1H), 8.81 (s, 1H), 8.11 (s, 1H), 7.90 (d, *J* = 8.2 Hz, 1H), 7.62 (t, *J* = 5.7 Hz, 1H), 7.56 (t, *J* = 2.0 Hz, 1H), 7.51 (t, *J* = 8.0 Hz, 1H), 7.27 (d, *J* = 8.9 Hz, 1H), 6.98 (ddd, *J* = 7.9, 2.0, 1.0 Hz, 1H), 6.52 (d, *J* = 2.5 Hz, 1H), 6.44 (dd, *J* = 16.9, 10.1 Hz, 1H), 6.33 (d, *J* = 1.3 Hz, 1H), 6.26 (dd, *J* = 17.0, 2.1 Hz, 1H), 6.00 (s, 1H), 5.76 (dd, *J* = 10.1, 2.1 Hz, 1H), 3.78 (s, 3H), 3.09 - 2.94 (m, 6H), 2.49 - 2.41 (m, 7H), 2.30 (t, *J* = 7.4 Hz, 2H), 1.94 - 1.87 (m, 3H), 1.80 (s, 2H), 1.66 (d, *J* = 12.1 Hz, 3H), 1.58 (s, 2H), 1.56 - 1.52 (m, 7H), 1.50 - 1.42 (m, 2H), 1.40 - 1.33 (m, 2H), 1.31 - 1.21 (m, 16H). ¹³C NMR (101 MHz, DMSO) δ 174.77, 171.05, 170.10, 163.66, 162.59, 157.04, 156.70, 147.38, 140.39, 137.52, 132.20, 129.97, 127.61, 124.51, 120.88, 120.15, 119.21, 117.05, 107.22, 106.64, 100.82, 56.15, 50.57, 50.02, 49.59, 45.24, 42.61, 41.25, 38.65, 36.93, 35.58, 32.71, 32.61, 29.65, 29.54, 29.51, 29.45, 29.42, 29.32, 29.21, 28.52, 26.90, 25.60, 25.34, 17.50.

### Embodiment 8: N-(3-(2-((4-(4-(12-(2-((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) propionamide (ZX-HYT-08)

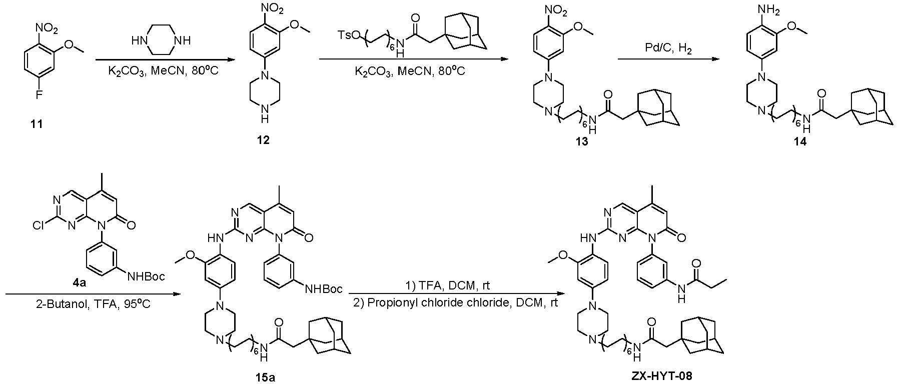

### Step 1: 1- (3-methoxy-4-nitrophenyl) piperazine (12)

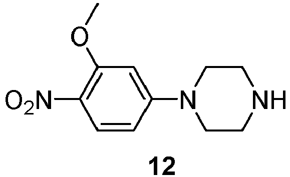

5-Fluoro-2-nitroanisole **11** (3.00 g, 17.5 mmol), piperazine (7.53 g, 87.5 mmol), and anhydrous potassium carbonate (3.63 g, 26.3 mmol) were sequentially added to acetonitrile (50 mL), and the mixture was stirred at 80 ° C for 6 hours. After the reaction was completed monitored by TLC, the organic solvent was evaporated under reduced pressure. The obtained residue was added to 20 mL of distilled water and stirred for 1 hour, then filtered under reduced pressure. The obtained filter cake was added to 40 mL of anhydrous ether, continued stirring for 1 hour, and filtered under reduced pressure. The filter cake was washed with iced anhydrous ether (3 × 20 mL) . The dried filter cake was the relatively pure compound **12** (3.95 g, yield 95%). MS (ESI), *m*/*z:* 238.1 [M+H]⁺. ¹H NMR (400 MHz, DMSO-*d*₆) δ 9.21 (s, 2H), 7.97 - 7.87 (m, 1H), 6.69 - 6.58 (m, 2H), 3.93 (s, 3H), 3.70 - 3.63 (m, 4H), 3.28 - 3.19 (m, 4H).

### Step 2: 2- ((3R, 5R, 7R) - adamantan-1-yl) - N - (12- (4-(3-methoxy-4-nitrophenyl) piperazin-1-yl) dodecyl) acetamide (13)

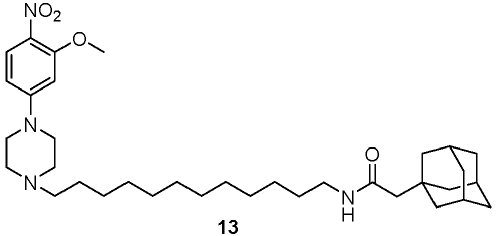

Compound **12** (0.95 g, 4 mmol), potassium carbonate (0.83 g, 6 mmol), and 12- (2- ((((3R, 5R, 7R) - adamantan-1-yl) acetamido) dodecyl 4-methylbenzenesulfonate (2.34 g, 4.4 mmol) were sequentially added to DMF (30 mL), and the mixture was heated and stirred overnight at 80 ° C. After the reaction was complete, the reaction solution was cooled to room temperature, then added 50 mL of saturated sodium chloride aqueous solution, and extracted with ethyl acetate. The organic phase was separated, and concentrated after dried with anhydrous sodium sulfate. The remaining residue was purified by column chromatography (chloroform/methanol=100/1 elution) to obtain a yellow oily target compound **13** (2.05 g, yield 86%). MS (ESI), *m*/*z:* 619.3 [M+Na]⁺. ¹H NMR (400 MHz, DMSO-*d*₆) δ 7.88 (d, *J* = 9.4 Hz, 1H), 7.62 (t, *J* = 5.6 Hz, 1H), 6.58 (dd, *J* = 9.5, 2.5 Hz, 1H), 6.52 (d, *J* = 2.6 Hz, 1H), 3.91 (s, 3H), 3.42 (t, *J* = 5.1 Hz, 4H), 3.00 (q, *J* = 6.5 Hz, 2H), 2.45 (t, *J* = 5.1 Hz, 4H), 2.33 - 2.25 (m, 2H), 1.94 - 1.86 (m, 3H), 1.80 (s, 2H), 1.65 (dt, *J* = 12.2, 2.9 Hz, 3H), 1.56 (dd, *J* = 12.7, 2.3 Hz, 9H), 1.44 (t, *J* = 7.2 Hz, 2H), 1.36 (t, *J* = 6.5 Hz, 2H), 1.26 - 1.20 (m, 16H).

### Step 3: 2- ((3R, 5R, 7R) - adamantan-1-yl) - N - (12- (4-(4-amino-3-methoxyphenyl) piperazin-1-yl) dodecyl) acetamide (14)

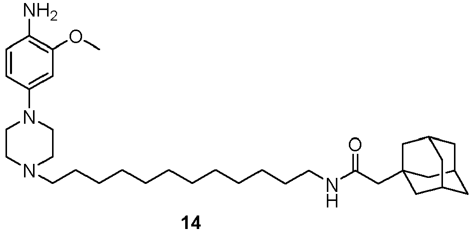

The methanol solution of compound **13** (2.98 g, 5 mmol) was added palladium carbon (0.55 g) and replaced with hydrogen gas three times. The reaction solution was stirred overnight at room temperature. After the reaction was complete, filtered under reduced pressure, the filtrate was evaporated to dryness. The obtained colorless oily liquid was compound **14** (2.8 g, yield 99%). Due to its high susceptibility to oxidation in the air, this compound did not require purification and could be directly used in the next step. MS (ESI), m/z: 567.4 [M+H]⁺

### Step 4: tert butyl(3-(2-((4-(4-(12-(2-(((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) carbamate (15a)

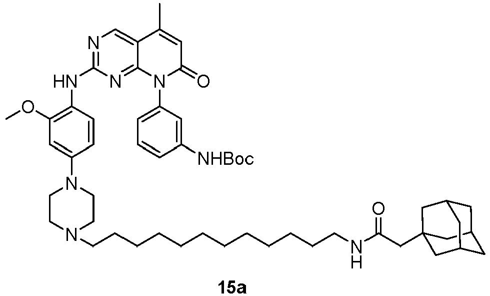

Intermediate **14** (1.13 g, 2 mmol) and compound **4a** (0.72 g, 2 mmol) were quickly added to 30 mL of sec-butanol, and added one drop of TFA. The reaction solution was replaced with argon three times and stirred overnight at 95 °C. After the reaction was completed monitored by TLC, the reaction solution was cooled to room temperature and evaporated under reduced pressure to remove organic solvent. The obtained residue was purified by column chromatography (chloroform/methanol=50/1 elution) to obtain a yellow solid compound **15a** (1.11 g, yield 61%). MS (ESI), *m*/*z:* 917.5 [M+H]⁺. ¹H NMR (400 MHz, Chloroform-*d*) δ 8.64 (s, 1H), 7.85 (s, 1H), 7.63 (s, 1H), 7.37 (t, *J* = 7.9 Hz, 1H), 6.85 (ddd, *J* = 8.1, 2.4, 0.9 Hz, 1H), 6.69 (ddd, *J* = 7.8, 2.0, 0.9 Hz, 1H), 6.61 (t, *J* = 2.1 Hz, 1H), 6.48 (d, *J* = 2.5 Hz, 1H), 6.39 (d, *J* = 1.3 Hz, 1H), 6.21 (s, 1H), 5.36 (s, 1H), 3.85 (s, 3H), 3.81 (s, 1H), 3.25 (td, *J* = 7.2, 5.8 Hz, 2H), 3.14 (t, *J* = 5.0 Hz, 4H), 2.64 (t, *J* = 5.0 Hz, 4H), 2.47 (d, *J* = 1.2 Hz, 3H), 2.45 - 2.39 (m, 2H), 1.99 (p, *J* = 3.1 Hz, 3H), 1.92 (s, 2H), 1.72 (dt, *J* = 12.2, 3.1 Hz, 3H), 1.65 (dd, *J* = 10.9, 2.5 Hz, 9H), 1.52 (dq, *J* = 20.4, 6.8, 6.3 Hz, 4H), 1.36 - 1.26 (m, 16H).

### Step 5: N-(3-(2-((4-(4-(12-(2-((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) propionamide (ZX-HYT-08)

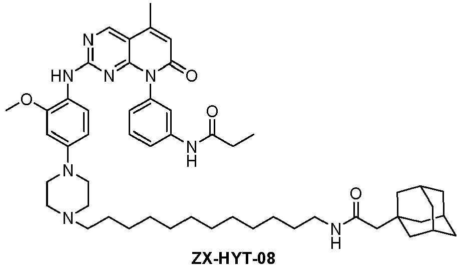

10 mL of DCM and 0.50 mL of TFA were added to a round bottomed flask containing compound **15a** (0.25 g, 0.27 mmol), and the reaction system was stirred at room temperature for 0.5 hours. After the reaction was complete, the organic solvent was removed by evaporation under reduced pressure. The obtained residue was dissolved directly in 5 mL of anhydrous acetonitrile, and the solution was sequentially added anhydrous potassium carbonate (0.14 g, 1.01 mmol) and propionyl chloride (0.04 g, 0.43 mmol). The reaction solution was stirred at room temperature for 1 hour, and monitor by TLC. After the reaction was complete, the organic solvent was evaporated to dryness. The obtained residue was directly mixed with silica gel and purified by column chromatography (chloroform/methanol=30/1 elution) to obtain a yellow powdered compound **ZX-HYT-08** (0.18 g, yield 78%). HRMS (ESI) for C₅₂H₇₂N₈O₄Na [M+Na]⁺: calcd, 895.5574; found, 895.5569. HPLC analysis: MeOH-H₂O (97:3), RT = 8.712 min, 95.41% purity. ¹H NMR (400 MHz, DMSO-*d*₆) δ 10.06 (s, 1H), 8.79 (s, 1H), 8.09 (s, 1H), 7.79 (d, *J* = 8.0 Hz, 1H), 7.62 (t, *J* = 5.8 Hz, 1H), 7.53 - 7.41 (m, 2H), 7.27 (d, *J* = 8.8 Hz, 1H), 6.92 (d, *J* = 7.8 Hz, 1H), 6.52 (s, 1H), 6.31 (s, 1H), 6.01 (s, 1H), 3.78 (s, 3H), 3.10 - 2.94 (m, 6H), 2.45 (m, 5H), 2.37 - 2.25 (m, 4H), 1.90 (s, 3H), 1.80 (s, 2H), 1.68 - 1.62 (m, 3H), 1.61 - 1.51 (m, 9H), 1.48 - 1.41 (m, 2H), 1.41 - 1.33 (m, 2H), 1.32 - 1.21 (m, 18H), 1.07 (t, *J* = 7.5 Hz, 3H). ¹³C NMR (101 MHz, DMSO) δ 172.54, 170.10, 162.60, 157.00, 156.75, 147.32, 140.79, 137.45, 130.11, 129.79, 123.85, 120.27, 119.70, 118.79, 116.98, 106.68, 106.57, 100.04, 58.35, 56.13, 53.25, 50.59, 49.26, 42.62, 38.65, 36.96, 32.61, 30.05, 29.65, 29.57, 29.54, 29.52, 29.48, 29.43, 29.19, 28.54, 27.46, 26.88, 26.77, 17.47, 10.08.

### Embodiment 9: N-(12-(4-(4-((8-(3-acetylaminophenyl) -5-methyl-7-oxy-7,8-dihydropyridino [2,3-d] pyrimidin-2-yl] amino) -3-methoxyphenyl) piperazin-1-yl) dodecyl) -2- ((3R, 5R, 7R) - adamantan-1-yl) acetamide (ZX-HYT-09)

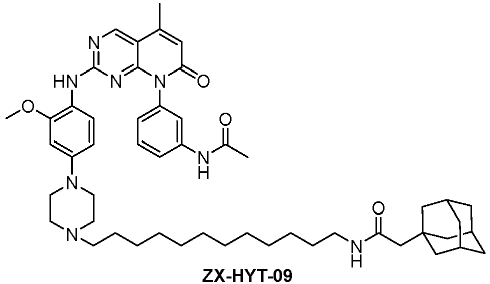

Similar to the synthesis method of **ZX-HYT-08,** a yellow powdery target compound **ZX-HYT-09** (0.11 g, yield 47%) could be obtained through a two-step reaction using compound **15a** (0.25 g, 0.27 mmol) and acetyl chloride (0.04 g, 0.51 mmol) as raw materials. HRMS (ESI) for C₅₁H₇₁N₈O₄ [M+H]⁺: calcd, 859.5598; found, 859.5593. HPLC analysis: MeOH-H₂O (97:3), RT = 9.171 min, 98.91% purity. ¹H NMR (400 MHz, DMSO-*d*₆) δ 10.17 (s, 1H), 8.80 (s, 1H), 8.13 (s, 1H), 7.76 (d, *J* = 8.0 Hz, 1H), 7.63 (t, *J* = 5.7 Hz, 1H), 7.55 - 7.42 (m, 2H), 7.27 (d, *J* = 8.8 Hz, 1H), 7.02 - 6.90 (m, 1H), 6.56 (s, 1H), 6.32 (s, 1H), 6.05 (s, 1H), 3.79 (s, 3H), 3.20 - 2.93 (m, 3H), 2.49 - 2.41 (m, 6H), 2.05 (s, 4H), 1.95 - 1.87 (m, 3H), 1.80 (s, 2H), 1.75 - 1.61 (m, 4H), 1.61 - 1.44 (m, 11H), 1.42 - 1.02 (m, 22H). ¹³C NMR (101 MHz, DMSO) δ 170.14, 168.91, 162.60, 157.00, 156.73, 147.33, 140.74, 137.44, 129.76, 123.96, 120.63, 119.69, 118.81, 117.02, 106.87, 106.62, 100.29, 57.54, 56.18, 52.51, 50.59, 48.30, 42.62, 38.66, 36.95, 32.61, 29.65, 29.53, 29.50, 29.47, 29.44, 29.33, 29.20, 28.69, 28.55, 27.17, 26.90, 25.66, 24.52, 17.47.

### Embodiment 10: N-(3-(2-((4-(4-(12-(2-(((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) isobutylamide (ZX-HYT-10)

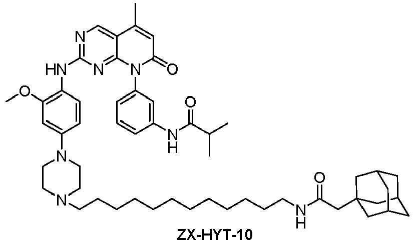

Similar to the synthesis method of **ZX-HYT-08,** a yellow powdery target compound **ZX-HYT-10** (0.16 g, yield 67%) could be obtained through a two-step reaction using compound **15a** (0.25 g, 0.27 mmol) and isobutyryl chloride (0.04 g, 0.37 mmol) as raw materials. HRMS (ESI) for C₅₃H₇₄N₈O4Na [M+Na]⁺: calcd, 909.5731; found, 909.5725. HPLC analysis: MeOH-H₂O (97:3), RT = 8.363 min, 98.82% purity. ¹H NMR (400 MHz, DMSO-*d*₆) δ 10.03 (s, 1H), 8.80 (s, 1H), 8.11 (s, 1H), 7.81 (d, *J* = 8.2 Hz, 1H), 7.62 (t, *J* = 5.6 Hz, 1H), 7.56 - 7.42 (m, 2H), 7.27 (d, *J* = 8.8 Hz, 1H), 6.93 (dd, *J* = 7.8, 1.9 Hz, 1H), 6.53 (d, *J* = 2.5 Hz, 1H), 6.32 (s, 1H), 6.02 (s, 1H), 3.78 (s, 3H), 3.14 - 2.91 (m, 6H), 2.59 (p, *J* = 6.9 Hz, 2H), 2.49 - 2.23 (m, 7H), 1.91 (s, 3H), 1.80 (s, 2H), 1.68 - 1.62 (m, 3H), 1.61 - 1.52 (m, 9H), 1.50 - 1.41 (m, 2H), 1.40 - 1.34 (m, 2H), 1.33 - 1.18 (m, 17H), 1.11 - 1.05 (m, 6H). ¹³C NMR (101 MHz, DMSO) δ 178.31, 175.78, 170.12, 162.60, 157.00, 156.75, 147.33, 140.86, 137.45, 129.79, 123.88, 120.31, 119.83, 118.94, 116.98, 106.68, 106.56, 100.04, 58.21, 56.13, 53.14, 50.59, 49.11, 42.62, 42.46, 38.65, 36.96, 35.43, 33.59, 32.61, 29.65, 29.52, 29.45, 29.43, 29.19, 28.54, 27.40, 26.88, 26.61, 19.93, 19.90, 19.38, 17.47.

### Embodiment 11: N-(3-(2-((4-(4-(12-(2-(((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) cyclopropane formamide (ZX-HYT-11)

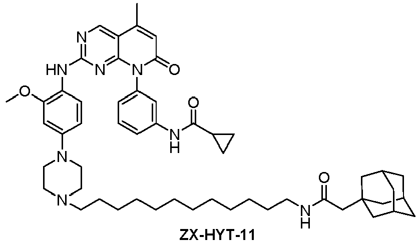

Similar to the synthesis method of **ZX-HYT-08,** a yellow powdery target compound **ZX-HYT-11** (0.15 g, yield 63%) could be obtained through a two-step reaction using compound **15a** (0.25 g, 0.27 mmol) and cyclopropyl chloride (0.04 g, 0.38 mmol) as raw materials. HRMS (ESI) for C₅₃H₇₂N₈O₄Na [M+Na]⁺: calcd, 907.5574; found, 907.5569. HPLC analysis: MeOH-H₂O (95:5), RT = 10.249 min, 100% purity. ¹H NMR (400 MHz, DMSO-*d*₆) δ 10.39 (s, 1H), 8.80 (s, 1H), 8.11 (s, 1H), 7.81 (d, *J* = 8.0 Hz, 1H), 7.62 (t, *J* = 5.7 Hz, 1H), 7.55 - 7.41 (m, 2H), 7.28 (d, *J* = 8.9 Hz, 1H), 6.99 - 6.88 (m, 1H), 6.53 (d, *J* = 2.5 Hz, 1H), 6.32 (d, *J* = 1.4 Hz, 1H), 6.02 (s, 1H), 3.79 (s, 3H), 3.12 - 2.94 (m, 6H), 2.49 - 2.41 (m, 4H), 2.32 (q, *J=* 13.4, 7.5 Hz, 2H), 1.94 - 1.87 (m, 3H), 1.80 (s, 2H), 1.69 - 1.62 (m, 3H), 1.61 - 1.52 (m, 9H), 1.52 - 1.44 (m, 2H), 1.42 - 1.33 (m, 2H), 1.32 - 1.15 (m, 18H), 0.88 - 0.75 (m, 6H). ¹³C NMR (101 MHz, DMSO) δ 176.01, 172.17, 170.08, 162.58, 157.02, 156.73, 147.32, 140.77, 137.47, 129.85, 123.83, 119.68, 118.75, 116.99, 106.72, 106.57, 100.12, 56.15, 53.19, 50.58, 49.20, 42.62, 38.64, 36.95, 32.61, 29.65, 29.51, 29.45, 29.42, 29.18, 28.53, 27.41, 26.87, 17.48, 15.04, 12.87, 8.16, 7.64.

### Embodiment 12: N-(3-(2-((4-(4-(12-(2-(((3R, 5R, 7R) - adamantan-1-yl) acetamido) dodecyl) piperazin-1-yl) -2-methoxyphenyl) amino) -5-methyl-7-oxypyridino [2,3-d] pyrimidin-8 (7H) - yl) phenyl) cyclopentamide (ZX-HYT-12)

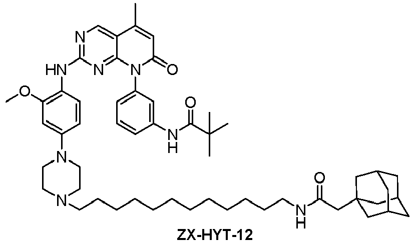

Similar to the synthesis method of **ZX-HYT-08,** a yellow powdery target compound **ZX-HYT-12** (0.18 g, yield 73%) could be obtained through a two-step reaction using compound **15a** (0.25 g, 0.27 mmol) and pivaloyl chloride (0.04 g, 0.33 mmol) as raw materials. HRMS (ESI) for C₅₄H₇₆N₈O₄Na [M+Na]⁺: calcd, 923.5887; found, 923.5882. HPLC analysis: MeOH-H₂O (97:3), RT = 10.083 min, 97.50% purity. ¹H NMR (400 MHz, DMSO-*d*₆) δ 9.37 (s, 1H), 8.81 (s, 1H), 8.10 (s, 1H), 7.91 (d, *J* = 7.8 Hz, 1H), 7.62 (t, *J* = 5.7 Hz, 1H), 7.56 (t, *J* = 2.0 Hz, 1H), 7.46 (t, *J* = 8.0 Hz, 1H), 7.28 (d, *J* = 8.9 Hz, 1H), 6.93 (dt, *J* = 7.9, 1.2 Hz, 1H), 6.52 (d, *J* = 2.5 Hz, 1H), 6.40 - 6.26 (m, 1H), 6.02 (s, 1H), 3.78 (s, 3H), 3.11 - 2.94 (m, 6H), 2.49 - 2.42 (m, 7H), 2.36 - 2.25 (m, 2H), 1.91 (t, *J* = 3.4 Hz, 3H), 1.80 (s, 2H), 1.70 - 1.62 (m, 3H), 1.61 - 1.52 (m, 9H), 1.49 - 1.42 (m, 2H), 1.40 - 1.33 (m, 2H), 1.33 - 1.23 (m, 18H), 1.23 - 1.18 (m, 9H). ¹³C NMR (101 MHz, DMSO) δ 179.87, 177.00, 170.12, 162.63, 157.01, 156.76, 147.34, 140.89, 137.28, 129.57, 123.96, 120.60, 120.24, 119.79, 116.95, 106.73, 106.54, 99.97, 58.31, 56.13, 53.23, 50.59, 49.25, 42.62, 38.65, 38.18, 36.95, 32.61, 29.64, 29.53, 29.51, 29.46, 29.42, 29.18, 28.54, 27.58, 27.48, 27.43, 26.87, 26.74, 17.48.

### Embodiment 13: N-(3-(2-((4-(4-(12-(2-(((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) cyclohexane formamide (ZX-HYT-13)

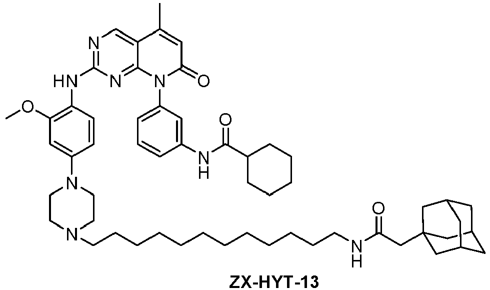

Similar to the synthesis method of **ZX-HYT-08,** a yellow powdery target compound **ZX-HYT-13** (0.13 g, yield 52%) could be obtained through a two-step reaction using compound **15a** (0.25 g, 0.27 mmol) and cyclohexyl chloride (0.05 g, 0.35 mmol) as raw materials. HRMS (ESI) for C₅₆H₇₈N₈O₄Na [M+Na]⁺: calcd, 949.6044; found, 949.6038. HPLC analysis: MeOH-H₂O (97:3), RT = 12.082 min, 96.21% purity. ¹H NMR (400 MHz, DMSO-*d*₆) δ 10.01 (s, 1H), 8.80 (s, 1H), 8.10 (s, 1H), 7.78 (d, *J* = 8.2 Hz, 1H), 7.62 (t, *J* = 5.7 Hz, 1H), 7.54 (t, *J* = 2.0 Hz, 1H), 7.46 (t, *J=* 8.1 Hz, 1H), 7.27 (d, *J=* 8.9 Hz, 1H), 6.91 (ddd, *J=* 7.8, 2.0, 1.0 Hz, 1H), 6.52 (d, *J* = 2.5 Hz, 1H), 6.32 (d, *J* = 1.3 Hz, 1H), 6.01 (s, 1H), 3.78 (s, 3H), 3.11 - 2.95 (m, 6H), 2.50 - 2.40 (m, 6H), 2.36 - 2.24 (m, 3H), 1.90 (s, 3H), 1.85 - 1.70 (m, 6H), 1.68 - 1.62 (m, 4H), 1.60 - 1.52 (m, 8H), 1.50 - 1.44 (m, 2H), 1.41 - 1.33 (m, 4H), 1.32 - 1.13 (m, 21H). ¹³C NMR (101 MHz, DMSO) δ 174.86, 170.11, 162.61, 157.02, 156.74, 147.34, 140.92, 137.43, 130.12, 129.78, 123.79, 120.27, 119.72, 118.83, 116.99, 106.68, 106.57, 100.04, 58.30, 56.14, 53.25, 50.59, 49.29, 45.34, 42.62, 38.64, 36.95, 32.61, 29.64, 29.60, 29.53, 29.50, 29.48, 29.42, 29.18, 28.54, 27.43, 26.87, 26.77, 25.86, 25.65, 17.47.

### Embodiment 14: N-(4-(2-((4-(4-(12-(2-(((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) acrylamide (ZX-HYT-14)

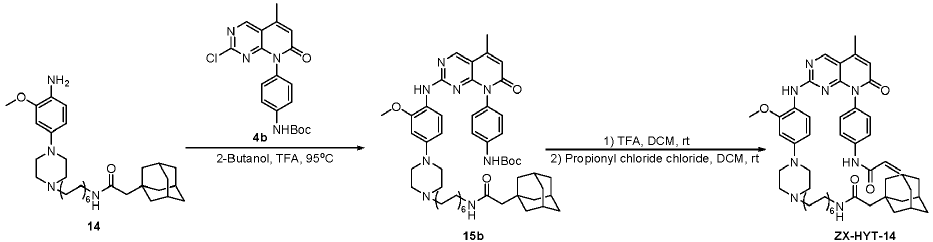

Similar to the synthesis method of **ZX-HYT-08,** compound **14** (1.13 g, 2 mmol), compound **4b** (0.72 g, 2 mmol), and acryloyl chloride (0.03 g, 0.38 mmol) were used as raw materials to obtain a yellow powdery target compound ZX-HYT-14 (0.21 g, yield 86%) through a three-step reaction. HRMS (ESI) for C₅₂H₇₀N₈O₄Na [M+Na]⁺: calcd, 893.5418; found, 893.5412. HPLC analysis: MeOH-H₂O (97:3), RT = 10.934 min, 96.59% purity. ¹H NMR (400 MHz, DMSO-*d*₆) δ 10.42 (s, 1H), 8.79 (s, 1H), 8.07 (s, 1H), 7.85 (m, 2H), 7.62 (t, *J* = 5.7 Hz, 1H), 7.26 - 7.17 (m, 3H), 6.58 - 6.46 (m, 2H), 6.38 - 6.28 (m, 2H), 5.96 (s, 1H), 5.81 (dd, *J* = 10.1, 2.1 Hz, 1H), 3.78 (s, 3H), 3.04 - 2.91 (m, 6H), 2.45 (s, 3H), 2.41 (s, 4H), 2.28 (t, *J* = 7.3 Hz, 2H), 1.90 (s, 3H), 1.80 (s, 2H), 1.69 - 1.62 (m, 3H), 1.59 - 1.52 (m, 9H), 1.47 - 1.41 (m, 2H), 1.40 - 1.33 (m, 2H), 1.32 - 1.22 (m, 16H). ¹³C NMR (101 MHz, DMSO) δ 170.21, 163.68, 162.78, 157.00, 156.90, 147.24, 139.19, 132.48, 132.22, 129.86, 127.38, 120.46, 120.19, 117.00, 106.61, 99.72, 58.30, 56.11, 53.23, 50.60, 49.03, 42.61, 38.67, 36.94, 32.61, 29.63, 29.54, 29.52, 29.49, 29.44, 29.19, 28.54, 27.44, 26.88, 26.77, 17.43.

### Embodiment 15: N-(4-(2-((4-(4-(12-(2-(((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) phenyl) cyclopropane formamide (ZX-HYT-15)

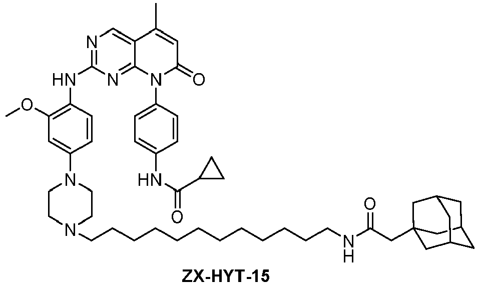

Similar to the synthesis method of **ZX-HYT-08,** compound **14** (1.13 g, 2 mmol), compound **4b** (0.72 g, 2 mmol), and cyclopropanyl chloride (0.04 g, 0.38 mmol) were used as raw materials to obtain a yellow powdery target compound **ZX-HYT-15** (0.21 g, yield 86%) through a three-step reaction. HRMS (ESI) for C₅₃H₇₂N₈O₄Na [M+Na]⁺: calcd, 907.5574; found, 907.5569. HPLC analysis: MeOH-H₂O (97:3), RT = 9.243 min, 96.18% purity. ¹H NMR (400 MHz, DMSO-*d*₆) δ 10.45 (s, 1H), 8.79 (s, 1H), 8.07 (s, 1H), 7.80 - 7.71 (m, 2H), 7.63 (t, *J* = 5.7 Hz, 1H), 7.27 - 7.14 (m, 3H), 6.53 (d, *J* = 2.6 Hz, 1H), 6.31 (d, *J* = 1.4 Hz, 1H), 5.99 (s, 1H), 3.79 (s, 3H), 3.16 - 2.92 (m, 6H), 2.49 - 2.23 (m, 6H), 1.93 - 1.83 (m, 4H), 1.80 (s, 2H), 1.69 - 1.61 (m, 3H), 1.60 - 1.52 (m, 9H), 1.48 (s, 2H), 1.41 - 1.19 (m, 20H), 0.92 - 0.79 (m, 4H). ¹³C NMR (101 MHz, DMSO) δ 172.24, 170.20, 162.81, 157.01, 156.93, 147.21, 139.50, 131.85, 129.74, 120.42, 120.04, 117.05, 106.65, 100.03, 58.03, 56.17, 53.03, 50.59, 48.83, 42.61, 38.65, 36.94, 32.61, 29.62, 29.49, 29.41, 29.17, 28.53, 27.32, 26.86, 26.42, 17.44, 14.96, 7.68.

### Embodiment 16: 2- ((3R, 5R, 7R) - adamantan-1-yl) - N - (12- (4- (4- (8-(3-ethylphenyl) -5-methyl-7-oxy-7,8-dihydropyridino [2,3-d] pyrimidin-2-yl) amino) -3-methoxyphenyl) piperazin-1-yl) dodecyl) acetamide (ZX-HYT-16)

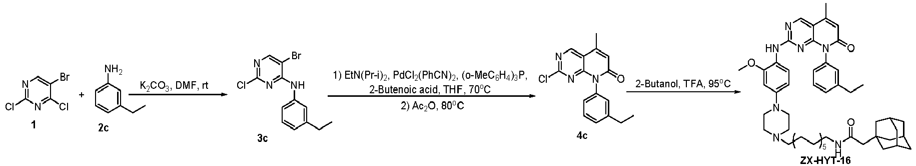

### Step 1: 5-Bromo-2-chloro-N - (3-ethylphenyl) pyrimidin-4-amine (3c)

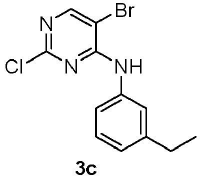

Compound 5-bromo-2,4-dichloropyrimidine (4.9 g, 21.5 mmol), 3-ethylaniline (2.6 g, 21.5 mmol), and potassium carbonate (5.9 g, 43 mmol) were sequentially added to 60 mL of DMF and reacted at room temperature for 6 hours. TLC monitoring, after the reaction was complete, the reaction system was added to 200 mL of ice water, and a large amount of white solids were generated. Filtered under reduced pressure, and the filter cake was washed with ice water. Then the dried filter cake was placed in 50 mL of acetone and stirred at room temperature for 2 hours for beating, then filtered under reduced pressure to obtain a filter cake, a white intermediate **3c** (6.5 g, yield 92%). MS (ESI), *m*/*z:* 312.1 [M+H]⁺ . ¹H NMR (400 MHz, Chloroform-*d*) δ 8.30 (s, 1H), 7.53 - 7.48 (m, 1H), 7.40 (t, *J* = 2.5 Hz, 1H), 7.33 (td, *J* = 7.8, 2.9 Hz, 1H), 7.28 (s, 1H), 7.06 (dt, *J* = 8.2, 1.9 Hz, 1H), 2.70 (qd, *J* = 7.7, 2.8 Hz, 2H), 1.29 (t, *J* = 7.7, 3.0 Hz, 3H).

### Step 2: 2-chloro-8- (3-ethylphenyl) -5-methylpyridino [2,3-d] pyrimidin-7 (8H) - one (3-4c)

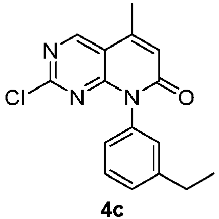

Compound 3c (2.19 g, 7.01 mmol), crotonic acid (6.04 g, 70.1 mmol), bis (cyanobenzene) palladium dichloride (II) (0.13 g, 5%), and tri (o-methylphenyl) phosphorus (104 mg, 5%) were placed in a 250 mL two-necked round-bottom flask, and replaced with argon 3 to 5 times. Anhydrous tetrahydrofuran (45 mL) and N, N-diisopropylethylamine (12 mL) were added with a syringe, and replaced with argon three times again. Then the reaction system was stirred at 70 ° C for 6 hours. Detected by TLC, after the reaction of raw material 3c was complete, 5 mL of acetic anhydride was added, and the reaction solution was heated to 80 °C and continued stirring for 8 hours. After the reaction was complete monitored by TLC, most of the organic solvent was evaporated under reduced pressure, and the residue was diluted with 100 mL of ethyl acetate. The organic layer was washed with 1N HCl (3 × 100 mL) and saturated sodium chloride solution (2 ×100 mL). The separated organic phase was dried with anhydrous sodium sulfate, concentrated and purified through column chromatography (petroleum ether/ethyl acetate=3:1 elution) to obtain a white solid compound **4c** (1.66 g, yield 80%). MS (ESI), *m*/*z:* 300.2 [M+H]⁺ . ¹H NMR (400 MHz, Chloroform-*d*) δ 8.83 (s, 1H), 7.49 (dd, *J* = 9.0, 7.3 Hz, 1H), 7.38 - 7.32 (m, 1H), 7.08 - 7.01 (m, 2H), 6.73 - 6.67 (m, 1H), 2.76 (q, *J* = 7.6 Hz, 2H), 2.56 (s, 3H), 1.30 (t, *J* = 7.6 Hz, 3H).

### Step 3: 2- ((3R, 5R, 7R) - adamantan-1-yl) - N - (12- (4- (4- (8-(3-ethylphenyl) -5-methyl-7-oxy-7,8-dihydropyridino [2,3-d] pyrimidin-2-yl) amino) -3-methoxyphenyl) piperazin-1-yl) dodecyl) acetamide (ZX-HYT-16)

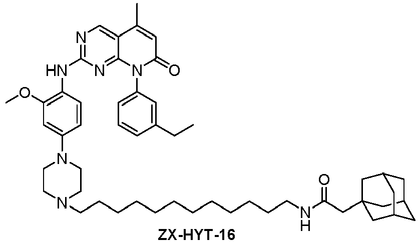

Compound **4c** (0.31 g, 1 mmol), 2- ((3R, 5R, 7R) - adamantan-1-yl) - N - (12-(4- (4-amino-3-methoxyphenyl) piperazin-1-yl) dodecyl) acetamide (0.57 g, 1 mmol), and a catalytic amount of trifluoroacetic acid were sequentially added to 20 mL of sec-butanol, and stirred the reaction solution at 90 ° C for 10 hours. After the reaction was complete monitored by TLC, the organic solvent was evaporated under reduced pressure, and the residue was separated by column chromatography (chloroform/methanol=30:1) to obtain a target compound **ZX-HYT-16** (0.24 g, yield 29%). HRMS (ESI) for C₅₁H₇₂N₇O₃ [M+H]⁺: calcd, 830.5618; found, 830.5622. HPLC analysis: MeOH-H₂O (97:3), RT = 13.173 min, 100% purity. ¹H NMR (400 MHz, DMSO-*d*₆) δ 8.78 (s, 1H), 8.05 (s, 1H), 7.61 (t, *J* = 5.7 Hz, 1H), 7.46 (t, *J* = 7.7 Hz, 1H), 7.37 (d, *J* = 7.8 Hz, 1H), 7.16 (d, *J* = 8.7 Hz, 1H), 7.10 (s, 1H), 7.06 (d, *J* = 7.8 Hz, 1H), 6.52 (s, 1H), 6.30 (s, 1H), 5.93 (s, 1H), 3.77 (s, 3H), 3.08 - 2.95 (m, 6H), 2.67 (q, *J* = 7.6 Hz, 2H), 2.49 - 2.38 (m, 7H), 2.29 (t, *J* = 7.4 Hz, 2H), 1.90 (s, 3H), 1.80 (s, 2H), 1.68 - 1.62 (m, 3H), 1.60 - 1.51 (m, 9H), 1.48 - 1.41 (m, 2H), 1.39 - 1.34 (m, 2H), 1.30 - 1.16 (m, 19H). ¹³C NMR (101 MHz, DMSO) δ 170.07, 162.72, 156.88, 147.13, 145.34, 137.26, 129.51, 128.68, 127.72, 126.70, 120.39, 117.07, 106.60, 100.19, 58.38, 56.14, 53.27, 50.59, 49.34, 42.63, 38.64, 36.96, 32.61, 29.65, 29.51, 29.47, 29.42, 29.18, 28.55, 28.42, 27.46, 26.88, 26.79, 17.44, 15.88.

### Embodiment 17: 2- ((3R, 5R, 7R) - adamantan-1-yl) - N - (12- (4- (4- (8-(3-aminophenyl) -5-methyl-7-oxy-7,8-dihydropyridino [2,3-d] pyrimidin-2-yl) amino) -3-methoxyphenyl) piperazin-1-yl) dodecyl) acetamide (ZX-HYT-17)

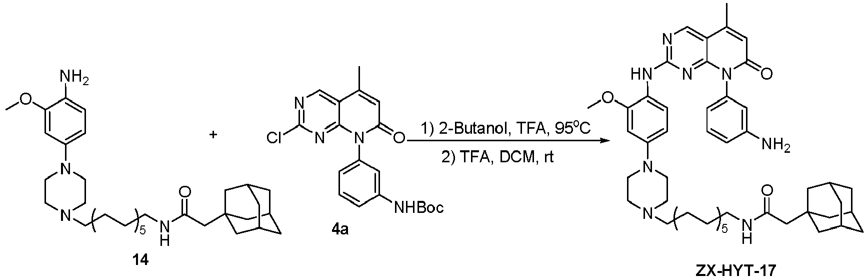

Intermediate **14** (1.13 g, 2 mmol), compound **4a** (0.72 g, 2 mmol), and a catalytic amount of trifluoroacetic acid were sequentially added to 30 mL of sec-butanol, and stirred the reaction solution at 95 ° C for 10 hours. After the reaction was complete monitored by TLC, the organic solvent was evaporated under reduced pressure. The residue was purified using column chromatography (chloroform/methanol=50/1 elution). The purified intermediate was redissolved in DCM (8 mL) and TFA (0.50 mL), stirred at room temperature for 0.5 hours, and evaporated the organic solvent to dryness. Then the residue was separated by column chromatography to obtain a yellow powdered compound **ZX-HYT-17** (0.13 g, yield 54%). HRMS (ESI) for C₄₉H₆₈N₈O₃Na [M+Na]⁺: calcd, 839.5312; found, 839.5307. HPLC analysis: MeOH-H₂O (97:3), 14.595 min, 100% purity. ¹H NMR (400 MHz, DMSO-*d*₆) δ 8.77 (s, 1H), 8.04 (s, 1H), 7.63 (t, *J* = 5.6 Hz, 1H), 7.45 (d, *J* = 8.9 Hz, 1H), 7.17 (t, *J* = 7.9 Hz, 1H), 6.70 (dd, *J* = 8.1, 2.3 Hz, 1H), 6.55 (d, *J* = 2.5 Hz, 1H), 6.40 (t, *J* = 2.1 Hz, 1H), 6.38 - 6.33 (m, 1H), 6.28 (d, *J* = 1.4 Hz, 1H), 6.14 (s, 1H), 5.26 (s, 2H), 3.80 (s, 3H), 3.06 (t, *J* = 5.0 Hz, 4H), 3.00 (q, *J* = 6.5 Hz, 2H), 2.51 - 2.48 (m, 4H), 2.43 (s, 3H), 2.38 - 2.25 (m, 2H), 1.94 - 1.87 (m, 3H), 1.80 (s, 2H), 1.69 - 1.62 (m, 3H), 1.60 - 1.53 (m, 9H), 1.45 (q, *J* = 6.9, 6.2 Hz, 2H), 1.36 (q, *J* = 6.5, 6.0 Hz, 2H), 1.30 - 1.23 (m, 16H). ¹³C NMR (101 MHz, DMSO) δ 170.11, 162.63, 156.84, 156.81, 150.26, 146.96, 137.84, 130.12, 129.81, 120.54, 117.12, 116.29, 114.57, 113.78, 107.13, 106.53, 100.12, 58.32, 56.16, 53.24, 50.59, 49.30, 46.16, 42.62, 38.65, 36.96, 32.61, 29.65, 29.52, 29.47, 29.43, 29.18, 28.54, 27.45, 26.88, 26.71, 17.42.

### Embodiment 18: 2- ((3R, 5R, 7R) - adamantan-1-yl) - N - (12- (4-(3-methoxy-4- (5-methyl-8- (3- (1-methylpipe-4-yl) amino) phenyl) -7-oxo-7,8-dihydropyridino [2,3-d] pyrimidin-2-yl) amino) phenyl) piperazin-1-yl) dodecyl) acetamide (ZX-HYT-18)

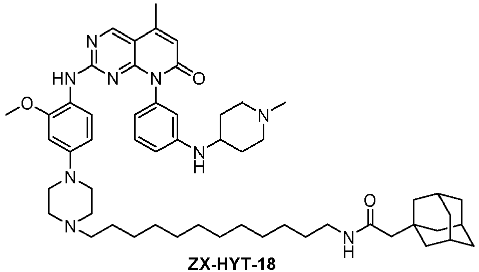

Compound **ZX-HYT-17** (200 mg, 0.24 mmol), 1-methylpiperidin-4-one (27 mg, 0.24 mmol), sodium triacetoxyborohydride (0.25 g, 1.2 mmol), and a catalytic amount of glacial acetic acid were placed in 20 mL of ultra dry toluene, and under argon protection, the reaction system was placed at 40 ° C and stirred for 2 hours. After the reaction was complete monitored by TLC, the organic solvent was evaporated to dryness, and the residue was directly mixed with silica gel for column chromatography (chloroform/methanol=25:1 elution). After purification, a yellow target compound **ZX-HYT-18** (0.16 mg, yield 73%) was obtained. HRMS (ESI) for C₅₅H₈₀N₉O₃ [M+H]⁺: calcd, 914.6384; found, 914.6379. HPLC analysis: MeOH-H₂O (97:3), RT = 13.264 min, 99.75% purity. ¹H NMR (400 MHz, DMSO-*d*₆) δ 8.77 (s, 1H), 8.03 (s, 1H), 7.63 (t, *J* = 5.7 Hz, 1H), 7.42 (d, *J* = 8.9 Hz, 1H), 7.22 (t, *J* = 7.9 Hz, 1H), 6.72 (d, *J* = 7.7 Hz, 1H), 6.54 (d, *J* = 2.5 Hz, 1H), 6.43 (t, *J* = 2.2 Hz, 1H), 6.34 (dd, *J* = 7.4, 1.9 Hz, 1H), 6.28 (s, 1H), 6.05 (s, 1H), 5.70 (d, *J* = 7.7 Hz, 1H), 3.79 (s, 3H), 3.05 - 2.97 (m, 6H), 2.79 (s, 3H), 2.49 - 2.46 (m, 4H), 2.44 (s, 3H), 2.31 (t, *J* = 7.4 Hz, 3H), 2.23 (s, 3H), 2.16 (s, 2H), 1.96 - 1.85 (m, 6H), 1.80 (s, 2H), 1.68 - 1.62 (m, 3H), 1.58 - 1.51 (m, 9H), 1.46 - 1.42 (m, 2H), 1.39 - 1.34 (m, 2H), 1.30 - 1.21 (m, 16H). ¹³C NMR (101 MHz, DMSO) δ 170.19, 162.67, 156.83, 149.34, 147.02, 138.08, 130.03, 117.14, 115.99, 113.36, 111.76, 106.92, 106.55, 100.20, 58.30, 56.18, 55.35, 54.28, 53.24, 50.59, 49.39, 45.85, 42.61, 38.65, 36.94, 32.61, 31.73, 29.61, 29.48, 29.39, 29.15, 28.53, 27.42, 26.84, 26.73, 17.40.

### Embodiment 19: 2- ((3R, 5R, 7R) - adamantan-1-yl) - N - (12- (4- (4- (8- (3-((cyclopropylmethyl) amino) phenyl) -5-methyl-7-oxy-7,8-dihydropyridino [2,3-d] pyrimidin-2-yl) amino) -3-methoxyphenyl) piperazin-1-yl) dodecyl) acetamide (ZX-HYT-19)

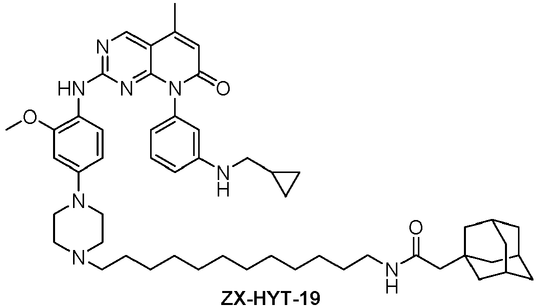

Similar to the synthesis method of compound **ZX-HYT-18,** compound **ZX-HYT-17** (0.20 g, 0.24 mmol) and cyclopropane formaldehyde (17 mg, 0.24 mmol) were used as raw materials to obtain a yellow solid compound **ZX-HYT-19** ( 0.18 g, yield 86%).. HRMS (ESI) for C₅₃H₇₅N₈O₃ [M+H]⁺: calcd, 871.5962; found, 871.5957. HPLC analysis: MeOH-H₂O (97:3), RT = 11.992 min, 97.65% purity. ¹H NMR (400 MHz, DMSO-*d*₆) δ 8.77 (s, 1H), 8.02 (s, 1H), 7.61 (t, *J* = 5.7 Hz, 1H), 7.43 (d, *J* = 8.8 Hz, 1H), 7.23 (t, *J* = 7.9 Hz, 1H), 6.72 (d, *J* = 8.2 Hz, 1H), 6.54 (d, *J* = 2.5 Hz, 1H), 6.44 (d, *J* = 2.2 Hz, 1H), 6.37 (dd, *J* = 7.4, 1.9 Hz, 1H), 6.29 (s, 1H), 6.07 (s, 1H), 5.86 (t, *J* = 5.5 Hz, 1H), 3.80 (s, 3H), 3.08 - 2.97 (m, 5H), 2.89 (d, *J* = 6.3 Hz, 2H), 2.49 - 2.40 (m, 7H), 2.35 - 2.26 (m, 2H), 1.94 - 1.86 (m, 3H), 1.80 (s, 2H), 1.70 - 1.62 (m, 3H), 1.60 - 1.57 (m, 2H), 1.56 - 1.52 (m, 7H), 1.49 - 1.43 (m, 2H), 1.39 - 1.33 (m, 2H), 1.30 - 1.21 (m, 16H), 1.04 (hept, *J* = 5.8 Hz, 2H), 0.43 (dt, *J* = 8.5, 3.0 Hz, 2H), 0.17 (t, *J* = 4.8 Hz, 2H). ¹³C NMR (101 MHz, DMSO) δ 170.09, 162.63, 156.84, 150.61, 146.94, 137.97, 129.89, 117.17, 116.08, 113.05, 111.55, 106.92, 106.56, 100.17, 56.18, 53.23, 50.59, 49.35, 48.11, 42.63, 38.64, 36.96, 32.61, 29.64, 29.50, 29.45, 29.41, 29.17, 28.54, 27.41, 26.87, 17.41, 11.03, 4.02, 3.93.

### Embodiment 20: N-((1R,3S)-3-(2-((4-(4-(12-(2-((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) cyclopentyl) cyclopropane formamide (ZX-HYT-20)

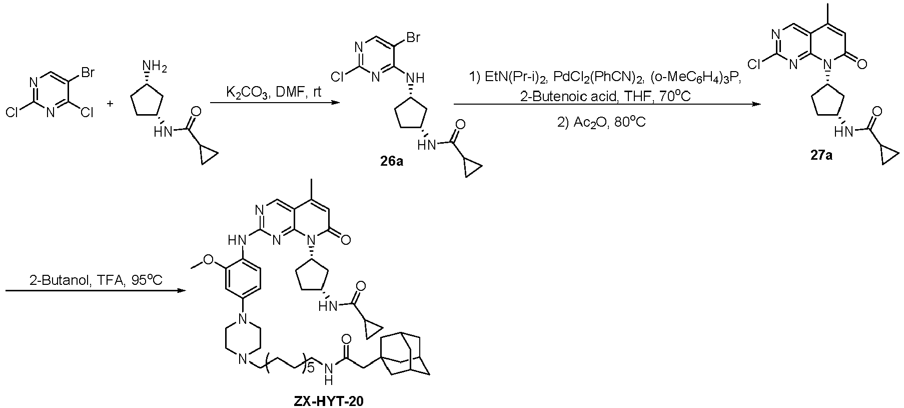

### Step 1: N - ((1R, 3S) -3- ((5-bromo-2-chloropyrimidin-4-yl) amino) cyclopentyl) cyclopropane formamide (26a)

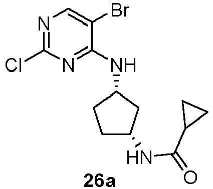

Compound 5-bromo-2,4-dichloropyrimidine (0.91 g, 4 mmol), N - ((1R, 3S) -3- ((5-bromo-2-chloropyrimidin-4-yl) amino) cyclopentanyl) cyclopropaneformamide (0.67 g, 4 mmol), and potassium carbonate (0.57 g, 8 mmol) were sequentially added to 25 mL of DMF and reacted at room temperature for 6 hours. TLC monitoring, after the reaction was complete, the reaction system was added to 200 mL of ice water, and a large amount of white solids were generated. Filtered under reduced pressure, and the filter cake was washed with ice water. Then the dried filter cake was placed in 15 mL of acetone and stirred at room temperature for 2 hours for beating, then filtered under reduced pressure to obtain a filter cake, a white intermediate **26a** (0.96 g, yield 67%). MS (ESI), *m*/*z:* 359.6 [M+H]⁺ . ¹H NMR (400 MHz, DMSO-*d*₆) δ 9.15 (s, 1H), 8.34 (d, *J* = 7.3 Hz, 1H), 7.23 (s, 1H), 5.67 (p, *J* = 9.4, 8.9 Hz, 1H), 4.17 - 4.04 (m, 1H), 2.31 - 2.21 (m, 2H), 2.02 - 1.92 (m, 2H), 1.89 - 1.79 (m, 2H), 1.63 - 1.54 (m, 1H), 0.81 - 0.62 (m, 4H).

### Step 2: N - ((1R, 3S) -3- (2-chloro-5-methyl-7-oxypyridino [2,3-d] pyrimidin-8 (7H) - yl) cyclopentyl) cyclopropaneformamide (27a)

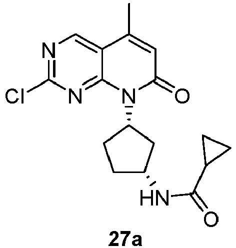

Compound **26a** (1.92 g, 5.35 mmol), crotonic acid (4.61 g, 70.1 mmol), bis (cyanobenzene) palladium dichloride (II) (0.14 g, 5%), and tri (o-methylphenyl) phosphorus (114 mg, 5%) were placed in a 250 mL two-necked round-bottom flask, and replaced with argon 3-5 times. Anhydrous tetrahydrofuran (30 mL) and N, N-diisopropylethylamine (5 mL) were added with a syringe, and replaced with argon three times again. Then the reaction system was stirred at 70 ° C for 6 hours. Detected by TLC, after the reaction of raw material 26a was complete, 5 mL of acetic anhydride was added and the reaction solution was heated to 80 °C and continued stirring for 8 hours. After the reaction was complete monitored by TLC, most of the organic solvent was evaporated under reduced pressure, and the residue was diluted with 100 mL of ethyl acetate. The organic layer was washed with 1N HCl (3 × 100 mL) and saturated sodium chloride solution (2 × 100 mL). The separated organic phase was dried with anhydrous sodium sulfate, concentrated and purified through column chromatography (petroleum ether/ethyl acetate=2:1 elution) to obtain a white solid compound **27a** (0.87 g, yield 47%). MS (ESI), *m*/*z:* 347.1 [M+H]⁺ . ¹H NMR (400 MHz, DMSO-*d*₆) δ 9.05 (s, 1H), 8.24 (d, *J* = 7.4 Hz, 1H), 6.63 (d, *J* = 1.5 Hz, 1H), 5.77 (p, *J* = 9.4, 8.9 Hz, 1H), 4.18 - 4.05 (m, 1H), 2.46 (s, 3H), 2.25 - 2.15 (m, 2H), 2.07 - 1.97 (m, 2H), 1.95 - 1.85 (m, 2H), 1.61 - 1.52 (m, 1H), 0.69 - 0.59 (m, 4H).

### Step 3: N-((1R,3S)-3-(2-((4-(4-(12-(2-((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) cyclopentyl) cyclopropane formamide (ZX-HYT-20)

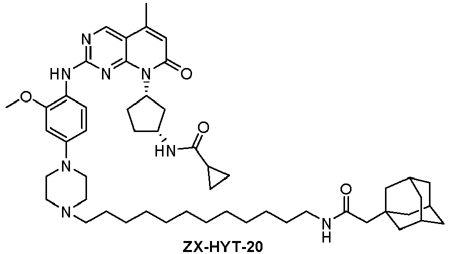

Compound **27a** (0.17 g, 0.5 mmol), compound **14** (0.28 g, 0.5 mmol), and a catalytic amount of trifluoroacetic acid were sequentially added to 10 mL of sec-butanol, and stirred the reaction solution at 90 °C for 10 hours. After the reaction was complete monitored by TLC, the organic solvent was evaporated under reduced pressure, and the residue was separated by column chromatography (chloroform/methanol=35: 1) to obtain a target compound **ZX-HYT-20** (0.13 g, yield 30%). HRMS (ESI) for C₅₂H₇₇N₈O₄ [M+H]⁺: calcd, 877.6068; found, 877.6062. HPLC analysis: MeOH-H₂O (95:5), 13.792 min, 100% purity. ¹H NMR (400 MHz, DMSO-*d*₆) δ 8.72 (s, 1H), 8.62 (s, 1H), 8.16 (s, 1H), 7.62 (t, *J* = 5.6 Hz, 1H), 7.51 (d, *J* = 8.6 Hz, 1H), 6.63 (d, *J* = 2.5 Hz, 1H), 6.51 (dd, *J* = 8.8, 2.5 Hz, 1H), 6.16 (d, *J* = 1.3 Hz, 1H), 5.84 (s, 1H), 4.08 (q, *J* = 7.8 Hz, 1H), 3.79 (s, 3H), 3.14 (t, *J* = 4.8 Hz, 4H), 3.00 (q, *J* = 6.5 Hz, 2H), 2.51 - 2.45 (m, 6H), 2.35 (s, 3H), 2.33 - 2.19 (m, 4H), 2.04 - 1.87 (m, 4H), 1.86 - 1.72 (m, 4H), 1.69 - 1.61 (m, 3H), 1.60 - 1.52 (m, 9H), 1.50 - 1.41 (m, 2H), 1.40 - 1.32 (m, 2H), 1.32 - 1.17 (m, 16H), 0.75 - 0.53 (m, 4H). ¹³C NMR (101 MHz, DMSO) δ 172.23, 170.08, 163.06, 160.29, 157.37, 155.88, 153.16, 149.92, 146.19, 119.63, 117.24, 107.10, 106.87, 100.36, 58.38, 55.93, 53.27, 50.57, 50.14, 49.89, 49.18, 42.60, 38.62, 36.94, 34.09, 32.60, 31.65, 29.65, 29.53, 29.49, 29.43, 29.19, 28.51, 27.46, 26.88, 26.78, 25.90, 17.19, 14.15, 12.89, 8.19, 6.67, 6.62.

### Embodiment 21: (1R, 3S) -3- (2- (4- (4- (12- (2- (((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) - N-cyclopropylcyclohexane-1-carboxylamide (ZX-HYT-21)

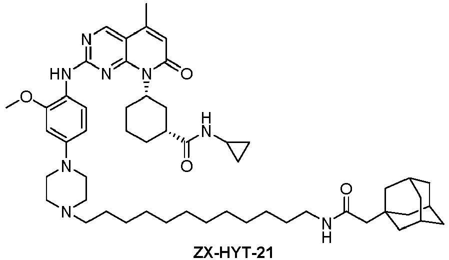

Similar to the synthesis method of compound **ZX-HYT-20,** compound **14** (0.28 g, 0.5 mmol) and N - (((1R, 3S) -3-aminocyclohexyl) cyclopropane formamide (0.18 g, 0.5 mmol) were used as raw materials to obtain a yellow solid compound **ZX-HYT-21** (0.13 g, yield 29%). HRMS (ESI) for C₅₃H₇₉N₈O₄ [M+H]⁺: calcd, 891.6224; found, 891.6219. HPLC analysis: MeOH-H₂O (95:5), RT = 11.542 min, 100% purity. ¹H NMR (400 MHz, DMSO-*d*₆) δ 8.82 - 8.44 (m, 2H), 7.82 - 7.08 (m, 3H), 6.65 (s, 1H), 6.52 (d, *J* = 8.7 Hz, 1H), 6.12 (s, 1H), 5.41 - 4.88 (m, 1H), 3.78 (s, 3H), 3.16 (s, 4H), 3.00 (q, *J* = 6.4 Hz, 2H), 2.71 - 2.53 (m, 4H), 2.44 - 2.21 (m, 7H), 2.17 - 1.94 (m, 2H), 1.90 (s, 3H), 1.80 (s, 2H), 1.71 - 1.61 (m, 4H), 1.59 - 1.51 (m, 10H), 1.50 - 1.42 (m, 3H), 1.39 - 1.33 (m, 2H), 1.30 - 1.18 (m, 19H), 0.60 - 0.51 (m, 2H), 0.38 - 0.28 (m, 2H). ¹³C NMR (101 MHz, DMSO) δ 175.49, 170.09, 157.15, 128.32, 128.23, 115.89, 107.36, 106.56, 100.65, 100.56, 100.49, 58.25, 55.50, 53.17, 52.03, 50.58, 48.92, 44.47, 42.62, 38.64, 36.95, 32.61, 29.64, 29.49, 29.42, 29.17, 28.54, 27.75, 27.38, 26.87, 25.49, 22.67, 17.13, 6.16, 6.05.

### Embodiment 22: 2- ((3R, 5R, 7R) - adamantan-1-yl) - N - (12- (4- (4- (8-((S) -1- (cyclopropanecarbonyl) piperidin-3-yl) -5-methyl-7-oxy-7,8-dihydropyridino [2,3-d] pyrimidin-2-yl) amino) -3-methoxyphenyl) piperazin-1-yl) dodecyl) acetamide (ZX-HYT-22)

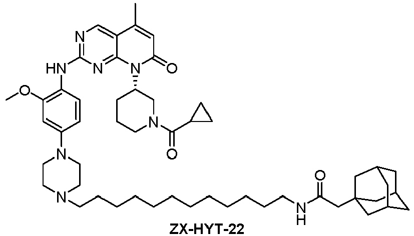

Similar to the synthesis method of compound **ZX-HYT-20,** compound **14** (0.28 g, 0.5 mmol) and (S) - (3-aminopiperidin-1-yl) (cyclopropyl) ketone (0.15 g, 0.5 mmol) were used as raw materials to obtain a yellow solid compound **ZX-HYT-22** (0.13 g, yield 29%). HRMS (ESI) for C₅₂H₇₇N₈O₄ [M+H]⁺: calcd, 877.6068; found, 877.6067. HPLC analysis: MeOH-H₂O (97:3), 11.956 min, 100% purity. ¹H NMR (400 MHz, DMSO-*d*₆) δ 8.96 (s, 1H), 8.70 (s, 1H), 7.61 (t, *J* = 5.6 Hz, 1H), 7.09 (s, 1H), 6.61 (s, 1H), 6.48 (s, 1H), 6.14 (s, 1H), 5.35 - 4.74 (m, 1H), 4.50 - 3.76 (m, 4H), 3.73 (s, 3H), 3.14 (s, 4H), 3.00 (q, *J* = 6.4 Hz, 2H), 2.50 - 2.39 (m, 4H), 2.38 - 2.29 (m, 5H), 2.00 - 1.86 (m, 4H), 1.80 (s, 3H), 1.69 - 1.61 (m, 4H), 1.59 - 1.52 (m, 10H), 1.48 - 1.42 (m, 3H), 1.39 - 1.34 (m, 2H), 1.29 - 1.21 (m, 16H), 0.77 - 0.55 (m, 4H). ¹³C NMR (101 MHz, DMSO) δ 171.40, 171.11, 170.08, 157.36, 155.90, 146.49, 119.19, 115.82, 107.01, 106.57, 100.16, 58.34, 55.75, 53.27, 50.58, 48.98, 47.11, 43.60, 42.62, 38.65, 36.95, 32.60, 29.65, 29.51, 29.47, 29.42, 29.18, 28.55, 27.45, 26.88, 26.73, 25.48, 17.16, 10.89, 7.28, 7.15.

### Embodiment 23: (1R,3S)-3-(2-((4-(4-(12-(2-((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) cyclohexyl) cyclopropane formamide (ZX-HYT-23)

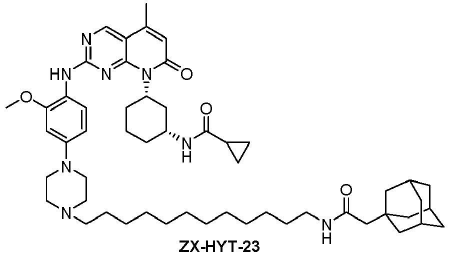

Similar to the synthesis method of compound **ZX-HYT-20,** compound **14** (0.28 g, 0.5 mmol) and (1R, 3S) -3-amino-N-cyclopropylcyclohexane-1-carboxamide (0.18 g, 0.5 mmol) were used as raw materials to obtain a yellow solid compound **ZX-HYT-23** (0.26 g, yield 58%). HRMS (ESI) for C₅₃H₇₉N₈O₄ [M+H]⁺: calcd, 891.6224; found, 891.6219. HPLC analysis: MeOH-H₂O (95:5), RT = 11.070 min, 100% purity. ¹H NMR (400 MHz, DMSO-*d*₆) δ 8.69 (s, 1H), 8.63 (s, 1H), 8.09 (s, 1H), 7.62 (t, *J* = 5.6 Hz, 1H), 6.66 (d, *J* = 2.5 Hz, 1H), 6.55 (d, *J* = 8.8 Hz, 1H), 6.10 (s, 1H), 5.33 (s, 1H), 3.79 (s, 3H), 3.67 (s, 1H), 3.17 (s, 4H), 3.00 (q, *J* = 6.5 Hz, 2H), 2.68 - 2.51 (m, 4H), 2.43 - 2.24 (m, 6H), 1.90 (s, 3H), 1.83 - 1.71 (m, 4H), 1.69 - 1.61 (m, 4H), 1.61 - 1.52 (m, 10H), 1.51 - 1.41 (m, 4H), 1.40 - 1.32 (m, 3H), 1.31 - 1.17 (m, 18H), 0.68 - 0.54 (m, 4H). ¹³C NMR (101 MHz, DMSO) δ 171.87, 170.09, 157.18, 145.86, 120.00, 117.81, 116.08, 107.22, 106.72, 100.51, 58.20, 55.90, 52.99, 50.58, 48.86, 48.13, 42.62, 38.64, 36.95, 34.95, 32.60, 32.41, 29.64, 29.49, 29.41, 29.17, 28.54, 27.38, 27.31, 26.87, 24.13, 17.12, 14.03, 6.57.

### Embodiment 24: 2- (((3R, 5R, 7R) - adamantan-1-yl) - N - (12- (4- (4-(8-cyclopentyl-5-methyl-7-oxy-7,8-dihydropyridinyl [2,3-d] pyrimidin-2-yl) amino) -3-methoxyphenyl) piperazin-1-yl) dodecyl) acetamide (ZX-HYT-24)

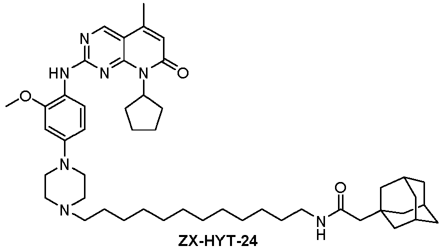

Similar to the synthesis method of compound **ZX-HYT-20,** compound **14** (0.28 g, 0.5 mmol) and cyclopentamine (0.13 g, 0.5 mmol) were used as raw materials to obtain a yellow solid compound **ZX-HYT-24** (0.21 g, yield 53%). HRMS (ESI) for C₄₈H₇₂N₇O₃ [M+H]⁺: calcd, 794.5697; found, 794.5699. HPLC analysis: MeOH-H₂O (97:3), 14.410 min, 100% purity. ¹H NMR (400 MHz, DMSO-*d*₆) δ 8.73 - 8.63 (m, 2H), 7.61 (t, *J* = 5.6 Hz, 1H), 7.31 (d, *J* = 8.7 Hz, 1H), 6.62 (d, *J* = 2.5 Hz, 1H), 6.49 (dd, *J* = 8.7, 2.5 Hz, 1H), 6.12 (s, 1H), 5.60 (s, 1H), 3.74 (s, 3H), 3.18 - 3.09 (m, 4H), 3.00 (q, *J* = 6.4 Hz, 2H), 2.51 - 2.42 (m, 4H), 2.36 - 2.26 (m, 5H), 2.19 - 2.08 (m, 2H), 1.90 (s, 3H), 1.80 (s, 2H), 1.69 - 1.61 (m, 4H), 1.60 - 1.52 (m, 12H), 1.48 - 1.42 (m, 3H), 1.40 - 1.32 (m, 3H), 1.29 - 1.22 (m, 16H). ¹³C NMR (101 MHz, DMSO) δ 170.08, 163.14, 160.65, 157.19, 155.91, 154.16, 150.52, 145.91, 126.66, 119.56, 116.67, 107.18, 106.80, 100.38, 58.38, 55.82, 53.24, 52.51, 50.58, 49.27, 42.62, 38.64, 36.96, 32.61, 29.64, 29.49, 29.45, 29.40, 29.17, 28.54, 27.66, 27.45, 26.87, 26.75, 25.07, 17.14.

### Embodiment 25:N-(3-(2-((4-(4-(12-(2-((3R, 5R, 7R) - adamantan-1-yl) acetylamino) dodecyl) piperazin-1-yl) phenyl) amino) -5-methyl-7-oxapyridino [2,3-d] pyrimidin-8 (7H) - yl) phenyl) cyclopropane formamide (ZX-HYT-25)

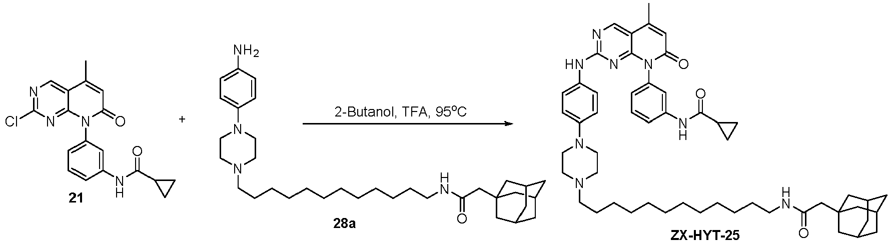

Compound **28a** (0.27 g, 0.5 mmol), compound **21** (0.18 g, 0.5 mmol), and a catalytic amount of trifluoroacetic acid were sequentially added to 10 mL of sec-butanol, and stirred at 95 ° C for 10 hours. After the reaction was complete monitored by TLC, the organic solvent was evaporated under reduced pressure, and the residue was separated by column chromatography (chloroform/methanol=45:1) to obtain a target compound **ZX-HYT-25** (0.25 g, yield 52%). HRMS (ESI) for C₅₂H₇₁N₈O₃ [M+H]⁺: calcd, 855.5649; found, 855.5644. ¹H NMR (400 MHz, DMSO-*d*₆) δ 10.40 (s, 1H), 9.83 (s, 1H), 8.82 (s, 1H), 7.86 (d, *J* = 8.3 Hz, 1H), 7.61 (t, *J* = 5.7 Hz, 1H), 7.52 - 7.43 (m, 2H), 7.19 (d, *J* = 8.4 Hz, 2H), 6.93 (d, *J* = 7.8 Hz, 1H), 6.55 (d, *J* = 8.3 Hz, 2H), 6.30 (s, 1H), 3.05 - 2.93 (m, 6H), 2.49 - 2.39 (m, 7H), 2.30 (t, *J* = 7.3 Hz, 2H), 1.90 (s, 3H), 1.82 - 1.74 (m, 3H), 1.68 - 1.63 (m, 3H), 1.59 - 1.52 (m, 9H), 1.47 - 1.41 (m, 2H), 1.39 - 1.34 (m, 2H), 1.30 - 1.22 (m, 16H), 0.83 - 0.74 (m, 4H). ¹³C NMR (101 MHz, DMSO) δ 172.18, 170.09, 162.61, 158.77, 156.91, 156.73, 147.36, 146.73, 140.86, 137.68, 132.24, 129.93, 123.84, 119.72, 118.75, 116.73, 115.74, 106.23, 58.35, 53.27, 50.59, 49.31, 42.63, 38.65, 36.96, 32.61, 29.65, 29.52, 29.48, 29.43, 29.19, 28.54, 27.45, 26.88, 26.78, 17.46, 15.03, 7.72, 7.60.

### Embodiment 26: N-(3-(2-((4-(4-(12-(2-((3R, 5R, 7R) - adamantan-1-yl) acetylamino) dodecyl) piperazin-1-yl) -2-methylphenyl) amino) -5-methyl-7-oxypyridino [2,3-d] pyrimidin-8 (7H) - yl) phenyl) cyclopropane formamide (ZX-HYT-26)

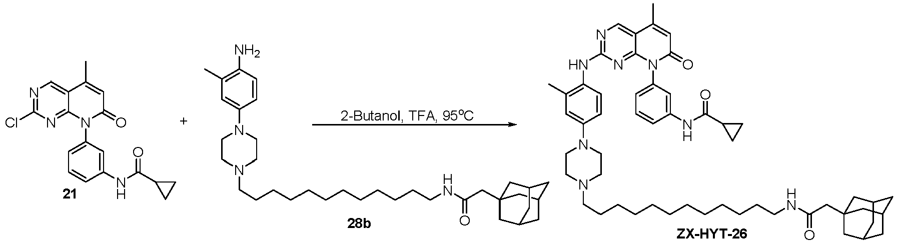

Similar to the synthesis method of compound **ZX-HYT-25,** compound **21** (0.18 g, 0.5 mmol) and compound **28b** (0.28 g, 0.5 mmol) were used as raw materials to obtain a yellow solid compound **ZX-HYT-26** (0.14 g, yield 33%). HRMS (ESI) for C₅₃H₇₃N₈O₃ [M+H]⁺: calcd, 869.5806; found, 869.5800. HPLC analysis: MeOH-H₂O (95:5), RT = 6.397 min, 96.91% purity. ¹H NMR (400 MHz, DMSO-*d*₆) δ 10.35 (s, 1H), 8.84 (s, 1H), 8.76 (s, 1H), 7.65 (dd, *J* = 11.6, 7.0 Hz, 2H), 7.49 (s, 1H), 7.38 (t, *J* = 8.1 Hz, 1H), 7.15 - 7.03 (m, 1H), 6.86 (d, *J* = 7.8 Hz, 1H), 6.64 (s, 1H), 6.42 (s, 1H), 6.26 (s, 1H), 3.00 (q, *J* = 6.3 Hz, 6H), 2.51 - 2.39 (m, 7H), 2.30 (t, *J* = 7.3 Hz, 2H), 2.10 (s, 3H), 1.91 (s, 3H), 1.85 - 1.75 (m, 3H), 1.71 - 1.62 (m, 3H), 1.61 - 1.51 (m, 9H), 1.50 - 1.42 (m, 2H), 1.40 - 1.34 (m, 2H), 1.32 - 1.21 (m, 16H), 0.89 - 0.69 (m, 4H). ¹³C NMR (101 MHz, DMSO) δ 172.15, 170.08, 162.70, 157.06, 156.72, 147.27, 140.56, 137.23, 129.56, 129.20, 123.92, 119.68, 118.52, 117.42, 116.50, 112.99, 106.37, 58.40, 53.27, 50.59, 49.14, 42.62, 38.64, 36.96, 32.61, 29.65, 29.52, 29.48, 29.43, 29.18, 28.54, 27.46, 26.88, 26.79, 18.84, 17.48, 15.04, 7.67.

### Embodiment 27: N - (3- (2- (4- (2- ((12- (2- (((3R, 5R, 7R) - adamantan-1-yl) acetylamino) dodecyl) oxy) ethoxy) -2-methoxyphenyl) amino) -5-methyl-7-oxypyridino [2,3-d] pyrimidin-8 (7H) - yl) phenyl) cyclopropaneformamide (ZX-HYT-27)

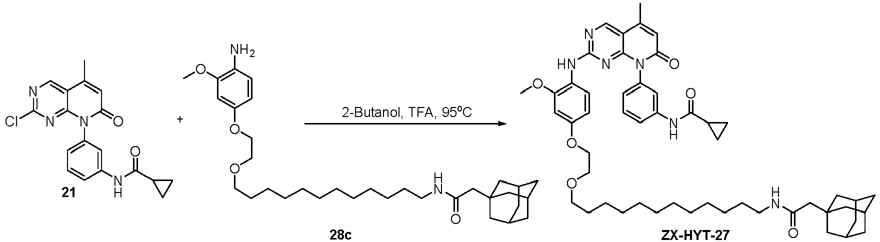

Similar to the synthesis method of compound **ZX-HYT-25,** compound **21** (0.18 g, 0.5 mmol) and compound **28c** (0.27 g, 0.5 mmol) were used as raw materials to obtain a yellow solid compound **ZX-HYT-27** (0.22 g, yield 51%). HRMS (ESI) for C₅₁H₆₉N₆O₆ [M+H]⁺: calcd, 861.5279; found, 861.5273. HPLC analysis: MeOH-H₂O (90:10), RT = 7.673 min, 100% purity. ¹H NMR (400 MHz, DMSO-*d*₆) δ 10.42 (s, 1H), 8.82 (s, 1H), 8.19 (s, 1H), 7.74 (d, *J* = 8.2 Hz, 1H), 7.64 (t, *J* = 5.7 Hz, 1H), 7.53 (t, *J* = 2.0 Hz, 1H), 7.47 (t, *J* = 8.0 Hz, 1H), 7.31 (d, *J* = 8.9 Hz, 1H), 6.93 (dd, *J* = 7.8, 2.0 Hz, 1H), 6.55 (d, *J* = 2.6 Hz, 1H), 6.33 (s, 1H), 6.03 (s, 1H), 4.01 (t, *J* = 4.6 Hz, 2H), 3.78 (s, 3H), 3.70 - 3.63 (m, 2H), 3.44 (t, *J* = 6.6 Hz, 2H), 2.99 (q, *J* = 6.4 Hz, 2H), 2.46 (s, 3H), 1.94 - 1.86 (m, 3H), 1.83 - 1.74 (m, 3H), 1.68 - 1.61 (m, 3H), 1.58 - 1.47 (m, 11H), 1.38 - 1.19 (m, 18H), 0.85 - 0.71 (m, 4H). ¹³C NMR (101 MHz, DMSO) δ 172.20, 170.09, 162.56, 157.06, 156.72, 147.31, 140.75, 137.47, 129.80, 123.87, 121.49, 119.76, 118.81, 117.13, 106.70, 104.65, 99.36, 70.87, 69.09, 67.72, 56.29, 50.58, 42.62, 38.65, 36.94, 32.60, 29.68, 29.64, 29.53, 29.51, 29.44, 29.38, 29.18, 28.53, 26.88, 26.14, 17.47, 15.03, 7.69, 7.63.

### Embodiment 28: N-(3-(2-((4-((12-(2-((3R, 5R, 7R) - adamantan-1-yl) acetylamino) dodecyl) oxy) -2-methoxyphenyl) amino) -5-methyl-7-oxypyrimidino [2,3-d] pyrimidin-8 (7H) - yl) phenyl) cyclopropane formamide (ZX-HYT-28)

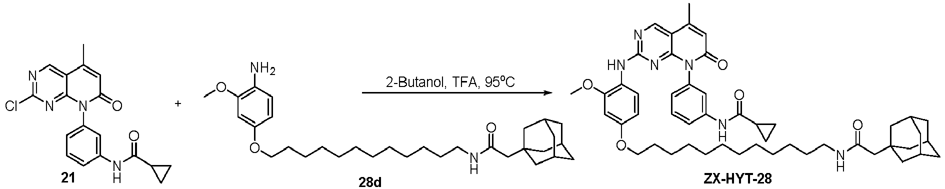

Similar to the synthesis method of compound **ZX-HYT-25,** compound **21** (0.18 g, 0.5 mmol) and compound **28d** (0.25 g, 0.5 mmol) were used as raw materials to obtain a yellow solid compound **ZX-HYT-28** (0.24 g, yield 59%). HRMS (ESI) for C₄₉H₆₅N₆O₅ [M+H]⁺: calcd, 817.5016; found, 817.5011. HPLC analysis: MeOH-H₂O (95:5), RT = 9.932 min, 95.18% purity. ¹H NMR (400 MHz, DMSO-*d*₆) δ 10.43 - 10.34 (m, 1H), 8.86 - 8.75 (m, 1H), 8.14 (s, 1H), 7.76 (d, *J* = 8.2 Hz, 1H), 7.60 (d, *J* = 6.2 Hz, 1H), 7.51 (d, *J* = 2.6 Hz, 1H), 7.46 (td, *J* = 8.1, 2.5 Hz, 1H), 7.31 (dd, *J* = 8.8, 2.5 Hz, 1H), 6.93 (d, *J* = 7.8 Hz, 1H), 6.51 (s, 1H), 6.32 (s, 1H), 6.04 (s, 1H), 3.87 (q, *J* = 5.7 Hz, 2H), 3.78 (s, 3H), 2.99 (td, *J* = 7.1, 3.3 Hz, 2H), 2.45 (s, 3H), 1.90 (s, 3H), 1.83 - 1.75 (m, 3H), 1.71 - 1.61 (m, 5H), 1.60 - 1.51 (m, 9H), 1.43 - 1.32 (m, 5H), 1.31 - 1.21 (m, 13H), 0.84 - 0.72 (m, 4H). ¹³C NMR (101 MHz, DMSO) δ 172.18, 170.10, 162.57, 157.06, 156.72, 147.31, 140.75, 137.47, 129.80, 123.87, 121.31, 119.74, 118.82, 117.11, 106.68, 104.55, 99.29, 67.98, 56.27, 50.59, 42.62, 38.65, 36.94, 32.61, 29.65, 29.51, 29.45, 29.29, 29.23, 29.19, 28.54, 26.89, 26.05, 17.46, 15.01, 7.64.

### Embodiment 29: N-(3-(2-((4-(4-(12-((2-((3R, 5R, 7R) - adamantan-1-yl) ethyl) amino) dodecyl) piperazin-1-yl) -2-methoxyphenyl) amino) -5-methyl-7-oxypyrano [2,3-d] pyrimidin-8 (7H) - yl) phenyl) cyclopropane formamide (ZX-HYT-29)

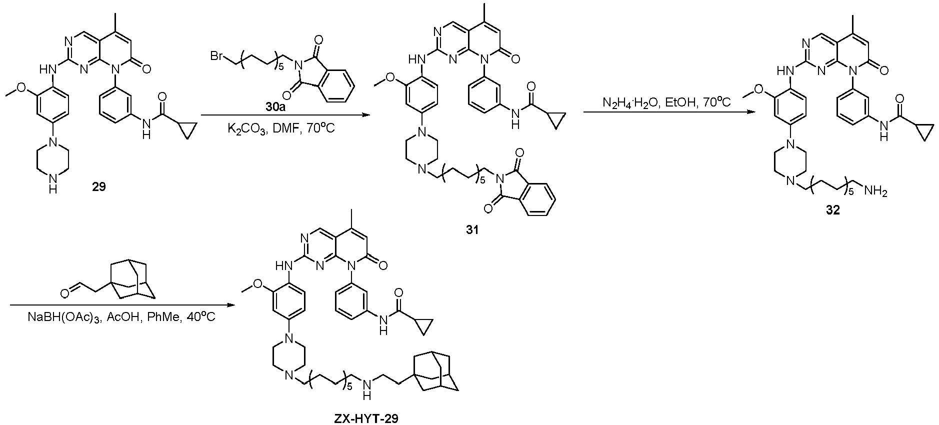

### Step 1: N-(3-(2-((4-(4-(12-(1,3-dioxoisoindole-2-yl) dodecyl) piperazin-1-yl) -2-methoxyphenyl) amino) -5-methyl-7-oxypyrano [2,3-d] pyrimidin-8 (7H) - yl) phenyl) cyclopropaneformamide (31)

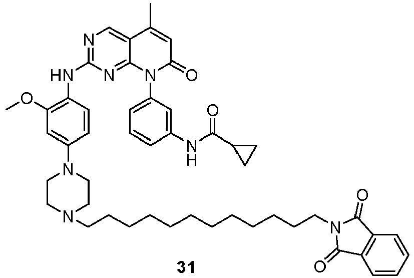

Compound **29** (0.26 mg, 0.5 mmol), compound **30a** (0.20 g, 0.5 mmol), and anhydrous potassium carbonate (0.14 g, 1 mmol) were placed in DMF (8 mL) and stirred at 70 ° C for 7 hours. Detected by TLC, after the reaction was complete, 100 mL of ethyl acetate was added. The organic layer was successively washed with saturated salt water (2 × 40 mL) and distilled water (3 ×40 mL), separated, dried, and evaporated to dryness. The residue obtained was purified by column chromatography (chloroform/methanol=45:1 elution) to obtain a yellow powdered compound **31** (0.31 g, yield 76%). HRMS (ESI) for C₄₉H₅₉N₈O₅ [M+H]⁺: calcd, 839.4608; found, 839.4602. HPLC analysis: MeOH-H₂O (95:5), RT = 9.870 min, 100% purity. ¹H NMR (400 MHz, DMSO-*d*₆) δ 10.37 (s, 1H), 8.78 (s, 1H), 8.07 (s, 1H), 7.83 (s, 5H), 7.56 - 7.40 (m, 2H), 7.26 (d, *J* = 8.9 Hz, 1H), 6.91 (s, 1H), 6.51 (s, 1H), 6.30 (s, 1H), 6.01 (s, 1H), 3.77 (s, 3H), 3.58 - 3.51 (m, 2H), 3.02 (s, 4H), 2.48 - 2.36 (m, 7H), 2.28 (s, 2H), 1.81 - 1.69 (m, 2H), 1.60 - 1.53 (m, 2H), 1.46 - 1.40 (m, 2H), 1.32 - 1.17 (m, 15H), 0.83 - 0.71 (m, 4H).

### Step 2: N-(3-(2-((4-(4-(12-aminododecyl) piperazin-1-yl) -2-methoxyphenyl) amino) -5-methyl-7-oxypyridino [2,3-d] pyrimidin-8 (7H) - yl) phenyl) cyclopropaneformamide (32)

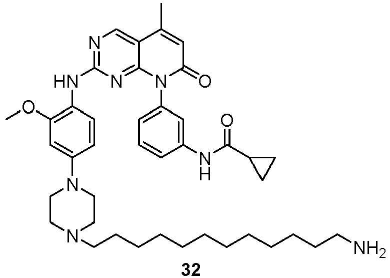

Compound **31** (0.84 mg, 1 mmol) and 80% hydrazine hydrate (2 mL) were placed in 40 mL of anhydrous ethanol and stirred at 70 ° C for 3 hours. Detected by TLC , and after the reaction was complete, the organic solvent was evaporated to dryness under reduced pressure. The residue was placed in 50 mL of DCM and thoroughly stirred before suction filtration. The filtrate was evaporated to dryness and purified by column chromatography (chloroform/methanol=10:1 elution) to obtain a yellow solid compound **32** (0.67 g, yield 94%). HRMS (ESI) for C₄₁H₅₇N₈O₃ [M+H]⁺: calcd, 709.4554; found, 709.4548. HPLC analysis: MeOH-H₂O (95:5), RT = 7.589 min, 97.81% purity. ¹H NMR (400 MHz, DMSO-*d*₆) δ 10.40 (s, 1H), 8.78 (s, 1H), 8.08 (s, 1H), 7.81 (s, 1H), 7.56 - 7.38 (m, 2H), 7.27 (d, *J* = 8.9 Hz, 1H), 6.91 (d, *J* = 7.8 Hz, 1H), 6.52 (s, 1H), 6.31 (s, 1H), 6.01 (s, 1H), 3.78 (s, 3H), 3.03 (s, 6H), 2.49 - 2.38 (m, 7H), 2.29 (t, *J* = 7.4 Hz, 2H), 1.78 (d, *J* = 9.6 Hz, 1H), 1.45 (s, 3H), 1.31 - 1.20 (m, 17H), 0.85 - 0.70 (m, 4H).

### Step 3: N-(3-(2-((4-(4-(12-((2- (((3R, 5R, 7R) - adamantan-1-yl) ethyl) amino) dodecyl) piperazin-1-yl) -2-methoxyphenyl) amino) -5-methyl-7-oxypyrano [2,3-d] pyrimidin-8 (7H) - yl) phenyl) cyclopropane formamide (ZX-HYT-29)

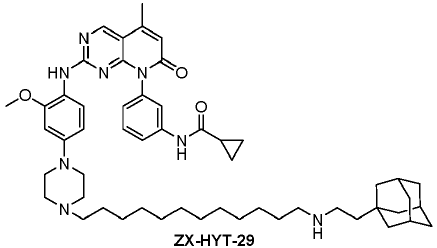

Compound **32** (177 mg, 0.25 mmol), adamantane acetaldehyde (45 mg, 0.25 mmol), sodium triacetoxyborohydride (0.27 g, 1.25 mmol), and a catalytic amount of glacial acetic acid were placed in 20 mL of ultra dry toluene; under argon protection, the reaction system was placed at 40 ° C and stirred for 2 hours. After the reaction was complete monitored by TLC, the organic solvent was evaporated to dryness, and the residue was directly mixed with silica gel for column chromatography (chloroform/methanol=15:1 elution) to obtain a yellow solid target compound **ZX-HYT-29** (0.12 mg, yield 55%). HRMS (ESI) for C₅₂H₇₃N₈O₃ [M+H]⁺: calcd, 857.5806; found, 857.5801. HPLC analysis: MeOH-H₂O (97:3), RT = 6.096 min, 99.17% purity. ¹H NMR (400 MHz, DMSO-*d*₆) δ 10.38 (s, 1H), 8.80 (s, 1H), 8.09 (s, 1H), 7.81 (d, *J* = 8.2 Hz, 1H), 7.51 - 7.42 (m, 2H), 7.27 (d, *J* = 8.9 Hz, 1H), 6.92 (dd, *J* = 7.7, 2.0 Hz, 1H), 6.53 (d, *J* = 2.5 Hz, 1H), 6.32 (s, 1H), 6.02 (s, 1H), 3.78 (s, 3H), 3.03 (t, *J* = 4.8 Hz, 4H), 2.50 - 2.42 (m, 11H), 2.30 (t, *J* = 7.4 Hz, 2H), 1.93 - 1.86 (m, 3H), 1.81 - 1.73 (m, 1H), 1.70 - 1.62 (m, 3H), 1.62 - 1.55 (m, 3H), 1.45 (s, 8H), 1.38 (t, *J* = 6.9 Hz, 2H), 1.33 - 1.22 (m, 17H), 1.21 - 1.16 (m, 2H), 0.82 - 0.74 (m, 4H). ¹³C NMR (101 MHz, DMSO) δ 172.17, 162.58, 157.00, 156.75, 147.31, 140.79, 137.47, 129.84, 123.82, 119.69, 118.74, 116.99, 106.70, 106.56, 100.07, 58.34, 56.15, 53.28, 50.01, 49.29, 44.49, 44.15, 42.62, 37.12, 31.86, 29.87, 29.80, 29.47, 28.52, 27.43, 27.32, 26.79, 17.47, 15.03, 7.70, 7.62.

### Embodiment 30: N-(3-(2-((2-methoxy-4- (4-octylpiperazin-1-yl) phenyl) amino) -5-methyl-7-oxypyridino [2,3-d] pyrimidin-8 (7H) - yl) phenyl) cyclopropane formamide (ZX-HYT-30)

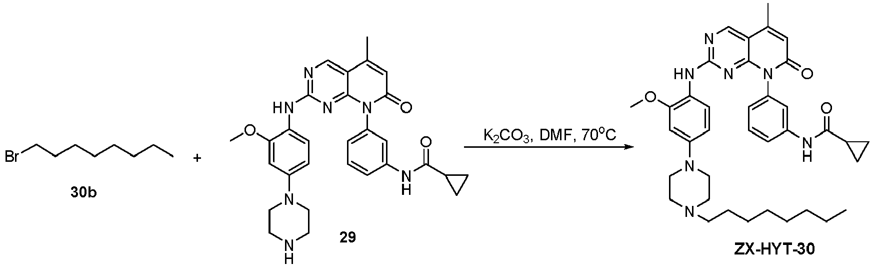

Compound **29** (0.13 mg, 0.25 mmol), compound **30b** (48 mg, 0.25 mmol), and anhydrous potassium carbonate (69 mg, 0.5 mmol) were placed in DMF (5 mL) and stirred the reaction solution at 70 ° C for 7 hours. Detected by TLC, and after the reaction was complete, 30 mL of ethyl acetate was added. The organic layer was sequentially washed with saturated salt water (2 × 20 mL) and distilled water (3 × 20 mL), separated, dried, and evaporated to dryness. The residue obtained was purified by column chromatography (chloroform/methanol=50:1 elution) to obtain a yellow powdered target compound **ZX-HYT-30** (0.11 g, yield 69%). HRMS (ESI) for C₃₇H₄₈N₇O₃ [M+H]⁺: calcd, 638.3819; found, 638.3813. HPLC analysis: MeOH-H₂O (95:5), RT = 6.803 min, 98.91% purity. ¹H NMR (400 MHz, DMSO-*d*₆) δ 10.38 (s, 1H), 8.80 (s, 1H), 8.10 (s, 1H), 7.80 (d, *J* = 8.0 Hz, 1H), 7.51 - 7.42 (m, 2H), 7.27 (d, *J* = 8.9 Hz, 1H), 6.92 (d, *J* = 8.2 Hz, 1H), 6.53 (d, *J* = 2.5 Hz, 1H), 6.31 (s, 1H), 6.02 (s, 1H), 3.78 (s, 3H), 3.03 (t, *J* = 4.8 Hz, 4H), 2.50 - 2.43 (m, 7H), 2.31 (t, *J* = 7.4 Hz, 2H), 1.82 - 1.73 (m, 1H), 1.46 (s, 2H), 1.30 - 1.23 (m, 10H), 0.90 - 0.85 (m, 3H), 0.82 - 0.74 (m, 4H). ¹³C NMR (101 MHz, DMSO) δ 172.19, 162.60, 157.02, 156.75, 147.33, 140.79, 137.48, 129.86, 123.83, 120.30, 119.68, 118.73, 116.99, 106.70, 106.57, 100.08, 58.35, 56.15, 53.27, 49.28, 31.76, 29.44, 29.20, 27.46, 26.79, 22.57, 17.46, 15.03, 14.44, 7.69, 7.62.

### Embodiment 31: N-(3-(2-((2-methoxy-4- (4-octylpiperazin-1-yl) phenyl) amino) -5-methyl-7-oxypyridino [2,3-d] pyrimidin-8 (7H) - yl) phenyl) cyclopropane formamide (ZX-HYT-31)

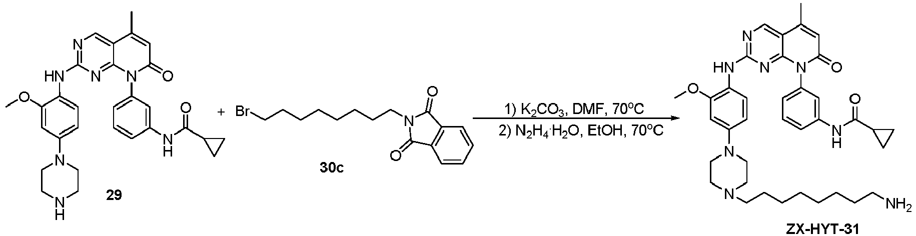

Compound **29** (0.53 mg, 1 mmol), compound **30c** (0.34 g, 1 mmol), and anhydrous potassium carbonate (0.28 g, 2 mmol) were placed in DMF (10 mL) and stirred at 70 ° C for 7 hours. Detected by TLC, and after the reaction was complete, 50 mL of ethyl acetate was added. The organic layer was successively washed with saturated salt water (2 × 50 mL), distilled water (3 ×50 mL), separated, dried, and evaporated to dryness. After purification by column chromatography (chloroform/methanol=50:1 elution), the residue was directly dissolved in 30 mL of anhydrous ethanol and added 1 mL of 80% hydrazine hydrate dropwise. The reaction system was stirred at 40 ° C for 2 hours. After the reaction was complete, the organic solvent was evaporated to dryness. After thoroughly stirring the residue with 50 mL of DCM, filtered under reduced pressure, and the filtrate was evaporated to dryness. After purification by column chromatography (elution with chloroform/methanol=10:1), a yellow solid compound **ZX-HYT-31** (0.57 g, yield 88%) was obtained. HRMS (ESI) for C₃₇H₄₉N₈O₃ [M+H]⁺: calcd, 653.3928; found, 653.3934. HPLC analysis: MeOH-H₂O (95:5), RT = 7.852 min, 97.44% purity. ¹H NMR (400 MHz, DMSO-*d*₆) δ 10.41 (s, 1H), 8.79 (s, 1H), 8.10 (s, 1H), 7.80 (d, *J* = 8.1 Hz, 1H), 7.53 - 7.41 (m, 2H), 7.27 (d, *J* = 8.9 Hz, 1H), 6.95 - 6.87 (m, 1H), 6.52 (d, *J* = 2.5 Hz, 1H), 6.31 (s, 1H), 6.02 (s, 1H), 3.78 (s, 3H), 3.03 (d, *J* = 5.4 Hz, 4H), 2.56 - 2.52 (m, 1H), 2.50 - 2.46 (m, 5H), 2.45 (s, 3H), 2.30 (t, *J* = 7.4 Hz, 2H), 1.78 (qd, *J* = 7.0, 5.2 Hz, 1H), 1.45 (t, *J* = 7.3 Hz, 2H), 1.36 (t, *J* = 6.8 Hz, 2H), 1.32 - 1.24 (m, 10H), 0.83 - 0.74 (m, 4H). ¹³C NMR (101 MHz, DMSO) δ 172.24, 162.63, 157.02, 156.73, 147.39, 140.77, 137.46, 129.88, 123.83, 120.25, 119.66, 118.75, 116.97, 106.69, 106.57, 100.06, 58.36, 56.15, 53.26, 49.25, 41.77, 33.00, 29.48, 29.44, 27.42, 26.83, 26.77, 17.46, 15.03, 7.72, 7.62.

### Embodiment 32: (1R,3R,SS,7R)-N-(8-(4-(4-((8-(3- (cyclopropaneylamino) phenyl) phenyl) -5-methyl-7-oxy-7,8-dihydropyridinyl [2,3-d] pyrimidin-2-yl) amino) -3-methoxyphenyl) piperazin-1-yl) octyl) -3,5-dimethyladamantan-1-carboxamide (ZX-HYT-32)

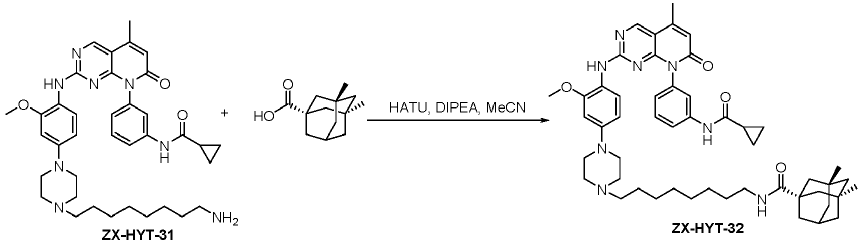

Compound **ZX-HYT-31** (0.13 mg, 0.2 mmol), (1R, 3R, 5S, 7R) -3,5-dimethyladamantan-1-carboxylic acid (42 mg, 0.2 mmol), HATU (91 mg, 0.24 mmol), and DIPEA (52 mg, 0.4 mmol) were placed in acetonitrile (6 mL) and stirred the reaction solution at room temperature for 1 hour, under TLC detection, after the reaction was complete, the organic solvent was evaporated to dryness. The residue was evaporated with silica gel and separated directly by column chromatography (chloroform/ methanol=50:1 elution) to obtain a yellow powdered target compound **ZX-HYT-32** (93 mg, yield 55%). HRMS (ESI) for C₅₀H₆₇N₈O₄ [M+H]⁺: calcd, 843.5285; found, 843.5280. HPLC analysis: MeOH-H₂O (95:5), 6.693 min, 99.44% purity. ¹H NMR (400 MHz, DMSO-*d*₆) δ 10.39 (s, 1H), 8.79 (s, 1H), 8.09 (s, 1H), 7.80 (d, *J* = 8.2 Hz, 1H), 7.50 - 7.42 (m, 2H), 7.31 (t, *J* = 5.6 Hz, 1H), 7.27 (d, *J* = 8.9 Hz, 1H), 6.91 (d, *J* = 7.9 Hz, 1H), 6.52 (d, *J* = 2.6 Hz, 1H), 6.31 (s, 1H), 6.01 (s, 1H), 3.78 (s, 3H), 3.09 - 2.97 (m, 6H), 2.50 - 2.46 (m, 4H), 2.45 (s, 3H), 2.30 (t, *J* = 7.5 Hz, 2H), 2.03 (p, *J* = 3.2 Hz, 1H), 1.77 (td, *J* = 7.2, 3.7 Hz, 1H), 1.60 - 1.53 (m, 2H), 1.51 - 1.41 (m, 3H), 1.40 - 1.34 (m, 5H), 1.33 (s, 1H), 1.30 - 1.22 (m, 11H), 1.14 - 1.05 (m, 2H), 0.82 - 0.75 (m, 10H). ¹³C NMR (101 MHz, DMSO) δ 176.90, 172.21, 162.61, 157.00, 156.73, 147.36, 140.77, 137.46, 129.86, 123.83, 120.29, 119.67, 118.75, 116.97, 106.72, 106.57, 100.07, 58.35, 56.15, 53.23, 50.80, 49.21, 45.51, 42.89, 42.17, 38.93, 37.91, 31.17, 30.92, 29.53, 29.37, 29.34, 29.18, 27.35, 26.70, 17.46, 15.03, 7.72, 7.63.

### Embodiment 33: N-(3-(2-((4-(4-(8-(3,3-dimethylsuccinamide) octyl) piperazin-1-yl) -2-methoxyphenyl) amino) -5-methyl-7-oxypyrano [2,3-d] pyrimidin-8 (7H) - yl) phenyl) cyclopropaneformamide (ZX-HYT-33)

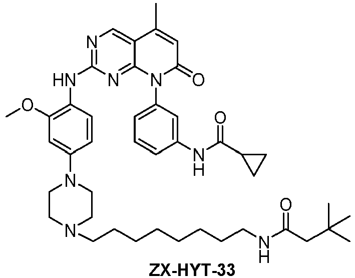

Similar to the synthesis method of compound **ZX-HYT-32,** compound **ZX-HYT-31** (0.13 mg, 0.2 mmol) and 3,3-dimethylbutyric acid (23 mg, 0.2 mmol) were used as raw materials to obtain compound **ZX-HYT-33** (60 mg, yield 40%) through amide condensation. HRMS (ESI) for C₄₃H₅₉N₈O₄ [M+H]⁺: calcd, 751.4659; found, 751.4654. HPLC analysis: MeOH-H₂O (90:10), 6.793 min, 98.37% purity. ¹H NMR (400 MHz, DMSO-*d*₆) δ 10.38 (s, 1H), 8.79 (s, 1H), 8.09 (s, 1H), 7.80 (d, *J* = 7.6 Hz, 1H), 7.67 (t, *J* = 5.5 Hz, 1H), 7.52 - 7.42 (m, 2H), 7.27 (d, *J* = 8.8 Hz, 1H), 6.92 (d, *J* = 7.9 Hz, 1H), 6.52 (d, *J* = 2.5 Hz, 1H), 6.36 - 6.28 (m, 1H), 6.02 (s, 1H), 3.78 (s, 3H), 3.08 - 2.98 (m, 6H), 2.50 - 2.46 (m, 4H), 2.45 (s, 3H), 2.30 (t, *J* = 7.4 Hz, 2H), 1.93 (s, 2H), 1.82 - 1.73 (m, 1H), 1.45 (t, *J* = 7.2 Hz, 2H), 1.38 (q, *J* = 6.6 Hz, 2H), 1.32 - 1.21 (m, 8H), 0.95 (s, 9H), 0.83 - 0.73 (m, 4H). ¹³C NMR (101 MHz, DMSO) δ 172.18, 170.98, 162.58, 157.00, 156.74, 147.31, 140.78, 137.48, 129.85, 123.83, 120.27, 119.68, 118.74, 116.99, 106.71, 106.56, 100.07, 58.36, 56.15, 53.28, 49.37, 49.28, 38.70, 30.82, 30.16, 29.67, 29.44, 29.21, 27.39, 26.89, 26.78, 17.46, 15.04, 7.71, 7.62.

### Embodiment 34: N-(8-(4-(4-((8-(3- (cyclopropaneformamido) phenyl) phenyl) -5-methyl-7-oxy-7,8-dihydropyridino [2,3-d] pyrimidin-2-yl) amino) -3-methoxyphenyl) piperazin-1-yl) octyl) -4,4-difluorocyclohexane-1-carboxylamide (ZX-HYT-34)

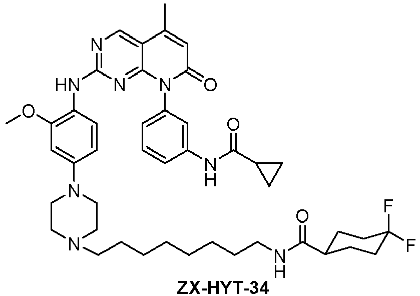

Similar to the synthesis method of compound **ZX-HYT-32,** compound **ZX-HYT-31** (0.13 mg, 0.2 mmol) and 4,4-difluorocyclohexan-1-carboxylic acid (33 mg, 0.2 mmol) were used as raw materials to obtain compound **ZX-HYT-34** (73 mg, yield 46%) through amide condensation. HRMS (ESI) for C₄₄H₅₇F₂N₈O₄ [M+H]⁺: calcd, 799.4471; found, 799.4465. HPLC analysis: MeOH-H₂O (97:3), RT = 4.194 min, 99.49% purity. ¹H NMR (400 MHz, DMSO-*d*₆) δ 10.38 (s, 1H), 8.80 (s, 1H), 8.10 (s, 1H), 7.86 - 7.74 (m, 2H), 7.50 - 7.43 (m, 2H), 7.27 (d, *J* = 8.9 Hz, 1H), 6.92 (d, *J* = 7.8 Hz, 1H), 6.53 (d, *J* = 2.5 Hz, 1H), 6.31 (s, 1H), 6.02 (s, 1H), 3.78 (s, 3H), 3.08 - 2.98 (m, 6H), 2.49 - 2.46 (m, 4H), 2.45 (s, 3H), 2.30 (t, *J* = 7.4 Hz, 2H), 2.21 (t, *J* = 11.8 Hz, 1H), 2.08 - 1.98 (m, 2H), 1.86 - 1.71 (m, 5H), 1.61 (t, *J* = 11.9 Hz, 2H), 1.49 - 1.42 (m, 2H), 1.41 - 1.35 (m, 2H), 1.31 - 1.23 (m, 8H), 0.82 - 0.75 (m, 4H). ¹³C NMR (101 MHz, DMSO) δ 173.95, 172.18, 162.58, 156.99, 156.74, 147.32, 140.77, 137.47, 129.84, 124.20, 123.82, 120.26, 119.67, 118.73, 116.98, 106.70, 106.56, 100.07, 58.36, 56.14, 53.27, 49.27, 41.49, 38.79, 32.97, 32.73, 32.50, 29.53, 29.42, 29.19, 27.39, 26.78, 26.17, 26.08, 17.46, 15.04, 7.69, 7.63.

### Embodiment 35: (3R,5R,7R)-N-(8-(4-(4-((8-(3-(cyclopropaneformamide) phenyl) -5-methyl-7-oxy-7,8-dihydropyridino [2,3-d-dipyrimidin-2-yl) amino) -3-methoxyphenyl) piperazin-1-yl) octyl) adamantan-1-carboxylamide (ZX-HYT-35)

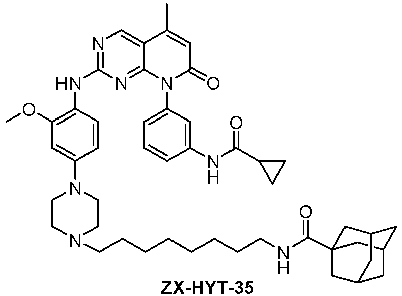

Similar to the synthesis method of compound **ZX-HYT-32,** compound **ZX-HYT-31** (0.13 mg, 0.2 mmol) and (3R, 5R, 7R) - adamantane-1-carboxylic acid (36 mg, 0.2 mmol) were used as raw materials to obtain compound **ZX-HYT-35** (74 mg, yield 45%) through amide condensation. HRMS (ESI) for C₄₈H₆₃N₈O₄ [M+H]⁺: calcd, 815.4972; found, 815.4967. HPLC analysis: MeOH-H₂O (95:5), 5.824 min, 100% purity. ¹H NMR (400 MHz, DMSO-*d*₆) δ 10.38 (s, 1H), 8.79 (s, 1H), 8.09 (s, 1H), 7.80 (s, 1H), 7.53 - 7.41 (m, 2H), 7.28 (dd, *J* = 12.6, 7.2 Hz, 2H), 6.91 (d, *J* = 7.9 Hz, 1H), 6.58 - 6.48 (m, 1H), 6.31 (s, 1H), 6.02 (s, 1H), 3.78 (s, 3H), 3.09 - 2.97 (m, 6H), 2.47 (d, *J* = 19.2 Hz, 7H), 2.34 - 2.26 (m, 2H), 1.95 (s, 3H), 1.75 (t, *J* = 6.2 Hz, 7H), 1.70 - 1.58 (m, 6H), 1.45 (s, 2H), 1.38 (t, *J* = 6.6 Hz, 2H), 1.33 - 1.20 (m, 8H), 0.89 - 0.70 (m, 4H). ¹³C NMR (101 MHz, DMSO) δ 177.08, 172.16, 162.57, 157.00, 156.73, 147.31, 140.76, 137.46, 129.83, 129.76, 123.81, 119.66, 118.72, 116.97, 116.84, 106.69, 106.55, 100.13, 100.06, 58.38, 56.14, 53.28, 49.28, 38.90, 36.65, 29.59, 29.41, 29.21, 28.15, 27.39, 26.78, 26.72, 17.46, 15.07, 15.03, 7.71, 7.70.

### Embodiment 36: 2- ((3R, 5R, 7R) - adamantan-1-yl) - N - (12- (4-(3-methoxy-4- ((5-methyl-7-oxy-8-phenyl-7,8)) - dihydropyridino [2,3-d] pyrimidin-2-yl) amino) phenyl) piperazin-1-yl) dodecyl) acetamide (ZX-HYT-36)

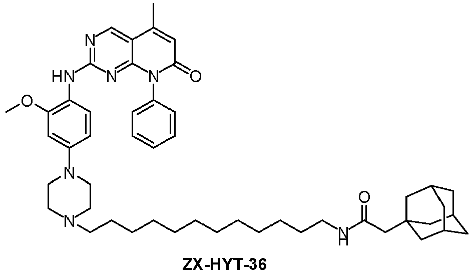

The synthesis method of compound **ZX-HYT-36** referred to Embodiment 16. HRMS (ESI) for C₄₉H₆₈N₇O₃ [M+H]⁺: calcd, 803.5384; found, 803.5379. HPLC analysis: MeOH-H₂O (95:5), RT= 11.983 min, 99.29% purity. ¹H NMR (400 MHz, DMSO-*d*₆) δ 8.79 (s, 1H), 8.07 (s, 1H), 7.61 (t, *J* = 5.6 Hz, 1H), 7.53 (dq, *J* = 14.0, 7.3 Hz, 3H), 7.27 (d, *J* = 7.3 Hz, 2H), 7.17 (d, *J* = 8.8 Hz, 1H), 6.52 (s, 1H), 6.31 (s, 1H), 5.97 (s, 1H), 3.77 (s, 3H), 3.08 - 2.94 (m, 6H), 2.50 - 2.37 (m, 7H), 2.31 (t, *J* = 7.8 Hz, 2H), 1.90 (s, 3H), 1.80 (s, 2H), 1.69 - 1.62 (m, 3H), 1.60 - 1.52 (m, 9H), 1.45 (t, *J* = 7.2 Hz, 2H), 1.37 (t, *J* = 6.7 Hz, 2H), 1.29 - 1.22 (m, 16H). ¹³C NMR (101 MHz, DMSO) δ 170.08, 162.70, 156.92, 156.85, 147.20, 137.25, 129.55, 129.52, 128.33, 120.30, 117.02, 106.75, 106.60, 100.14, 79.66, 58.33, 56.12, 53.24, 50.59, 49.28, 42.63, 38.65, 36.96, 32.61, 29.65, 29.51, 29.48, 29.43, 29.18, 28.55, 27.45, 26.88, 26.74, 17.46.

### Embodiment 37: 2- ((3R, 5R, 7R) - adamantan-1-yl) - N - (12- (4- (4- (8-((S) -1- (cyclopropanecarbonyl) pyrrolidin-3-yl)) -5-methyl-7-oxy-7,8-dihydropyridino [2,3-d] pyrimidin-2-yl) amino) -3-methoxyphenyl) piperazin-1-yl) dodecyl) acetamide (ZX-HYT-37)

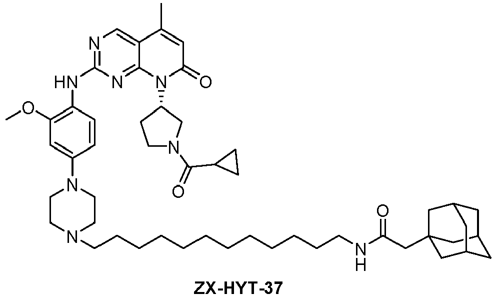

The synthesis method of compound **ZX-HYT-37** referred to Embodiment 20. RMS (ESI) for C₅₁H₇₅N₈O₄ [M+H]⁺: calcd, 863.5911; found, 863.5906. HPLC analysis: MeOH-H₂O (97:3), 9.734 min, 100% purity. ¹H NMR (400 MHz, Methanol-*d*₄) δ 8.70 (s, 1H), 7.24 (s, 1H), 6.67 (s, 1H), 6.58 (d, *J* = 6.0 Hz, 1H), 6.19 (s, 1H), 5.75 (s, 1H), 5.39 (s, 1H), 3.80 (s, 3H), 3.27 - 3.20 (m, 4H), 3.14 (t, *J* = 6.9 Hz, 2H), 2.81 (d, *J* = 30.8 Hz, 1H), 2.66 (t, *J* = 5.0 Hz, 4H), 2.52 (s, 1H), 2.46 - 2.35 (m, 5H), 2.23 - 2.11 (m, 1H), 1.95 (s, 3H), 1.91 (s, 2H), 1.74 (d, *J* = 12.3 Hz, 3H), 1.67 (s, 2H), 1.63 (d, *J* = 2.9 Hz, 7H), 1.59 - 1.54 (m, 2H), 1.51 - 1.45 (m, 2H), 1.38 - 1.25 (m, 16H), 0.90 - 0.78 (m, 1H), 0.55 - 0.44 (m, 2H), 0.18 - 0.03 (m, 2H). ¹³C NMR (101 MHz, DMSO) δ 171.17, 170.09, 163.11, 157.46, 155.88, 150.21, 150.05, 146.67, 131.96, 125.70, 125.40, 119.48, 116.53, 107.01, 100.18, 58.39, 55.92, 53.31, 50.58, 50.31, 49.18, 48.95, 42.61, 38.65, 36.95, 32.59, 29.63, 29.49, 29.17, 28.54, 27.45, 26.86, 17.19, 14.00, 12.41, 11.86, 7.64, 7.41.

### Embodiment 38: N-(3-(2-((4-(2-((12-(2-((3R, 5R, 7R) - adamantan-1-yl) ethoxy) dodecyl) oxy) ethoxy) -2-methoxyphenyl) amino) -5-methyl-7-oxypyridino [2,3-d] pyrimidin-8 (7H) - yl) phenyl) cyclopropane formamide (ZX-HYT-38)

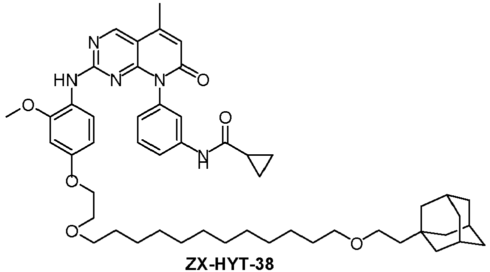

The synthesis method of compound **ZX-HYT-38** referred to Embodiment 27. HRMS (ESI) for C₅₁H₇₅N₈O₄ [M+H]⁺: calcd, 847.5248; found, 847.5351. HPLC analysis: MeOH-H₂O (97:3), 4.685 min, 100% purity. ¹H NMR (400 MHz, DMSO-*d*₆) δ 10.42 (s, 1H), 8.81 (s, 1H), 8.18 (s, 1H), 7.80 - 7.66 (m, 1H), 7.54 (t, *J* = 2.1 Hz, 1H), 7.47 (t, *J* = 8.0 Hz, 1H), 7.32 (d, *J* = 8.9 Hz, 1H), 6.93 (d, *J* = 7.8 Hz, 1H), 6.55 (d, *J* = 2.5 Hz, 1H), 6.33 (s, 1H), 6.03 (s, 1H), 4.07 - 3.95 (m, 2H), 3.78 (s, 3H), 3.71 - 3.63 (m, 2H), 3.45 - 3.42 (m, 2H), 3.36 - 3.32 (m, 2H), 3.27 (t, *J* = 6.4 Hz, 2H), 2.45 (s, 3H), 1.88 (s, 2H), 1.77 (p, *J* = 6.4 Hz, 1H), 1.61 (q, *J* = 12.0 Hz, 4H), 1.54 - 1.37 (m, 8H), 1.31 - 1.17 (m, 23H), 0.87 - 0.81 (m, 2H), 0.79 - 0.77 (m, 4H). ¹³C NMR (101 MHz, DMSO) δ 172.22, 162.59, 157.09, 156.69, 147.36, 140.74, 137.46, 130.12, 129.84, 123.86, 121.43, 119.71, 118.78, 117.10, 106.68, 104.53, 99.27, 70.83, 70.36, 69.07, 67.66, 66.26, 56.26, 43.73, 42.59, 37.04, 31.79, 31.74, 29.68, 29.56, 29.52, 29.48, 29.45, 29.37, 29.33, 29.26, 29.22, 28.48, 26.19, 26.13, 22.59, 17.49, 15.03, 14.45, 7.74, 7.66.

### Embodiment 39: 2- ((3R, 5R, 7R) - adamantan-1-yl) - N-(12-(4-(4-((8-(3-((cyclobutylmethyl) amino) phenyl) -5-methyl-7) - oxo-7,8-dihydropyridino [2,3-d] pyrimidin-2-yl) amino) -3-methoxyphenyl) piperazin-1-yl) dodecyl) acetamide (ZX-HYT-39)

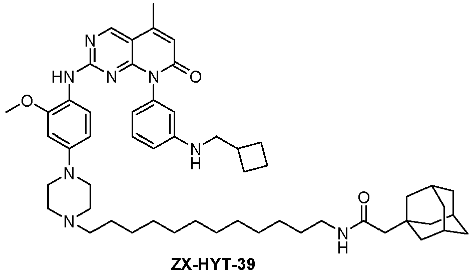

The synthesis method of compound **ZX-HYT-39** referred to Embodiment 19. HRMS (ESI) for C₅₄H₇₇N₈O₃ [M+H]⁺: calcd, 885.6119; found, 885.6114. HPLC analysis: MeOH-H₂O (97:3), 13.507 min, 100% purity. ¹H NMR (400 MHz, **DMSO-*d*₆)** δ 8.77 (s, 1H), 8.01 (s, 1H), 7.61 (t, *J* = 5.9 Hz, 1H), 7.43 (d, *J* = 8.8 Hz, 1H), 7.22 (t, *J* = 8.0 Hz, 1H), 6.69 (d, *J* = 8.2 Hz, 1H), 6.54 (s, 1H), 6.42 (s, 1H), 6.35 (d, *J* = 7.7 Hz, 1H), 6.28 (s, 1H), 6.05 (s, 1H), 5.72 (t, *J* = 5.7 Hz, 1H), 3.79 (s, 3H), 3.09 - 2.95 (m, 8H), 2.60 - 2.52 (m, 2H), 2.50 - 2.45 (m, 4H), 2.43 (s, 3H), 2.29 (t, *J* = 7.4 Hz, 2H), 2.00 (q, *J* = 8.0 Hz, 2H), 1.90 (s, 3H), 1.85 - 1.77 (m, 4H), 1.73 - 1.65 (m, 3H), 1.65 - 1.61 (m, 2H), 1.59 - 1.56 (m, 2H), 1.56 - 1.52 (m, 6H), 1.47 - 1.41 (m, 2H), 1.39 - 1.34 (m, 2H), 1.30 - 1.21 (m, 16H). ¹³C NMR (101 MHz, DMSO) δ 170.12, 162.63, 156.82, 150.68, 146.95, 137.95, 129.90, 120.56, 117.16, 115.92, 113.01, 111.30, 106.86, 106.55, 100.17, 58.32, 56.17, 53.27, 50.59, 49.42, 49.25, 42.62, 38.64, 36.95, 34.72, 32.61, 29.63, 29.49, 29.45, 29.40, 29.16, 28.54, 27.42, 26.86, 26.76, 26.17, 18.42, 17.41.

### Embodiment 40: 2- ((3R, 5R, 7R) - adamantan-1-yl) - N-(12-(4-(4-((8-(3-(cyclopropylmethoxy) phenyl) -5-methyl-7-oxo -) 7,8-dihydropyridino [2,3-d] pyrimidin-2-yl) amino) -3-methoxyphenyl) piperazin-1-yl) dodecyl) acetamide (ZX-HYT-40)

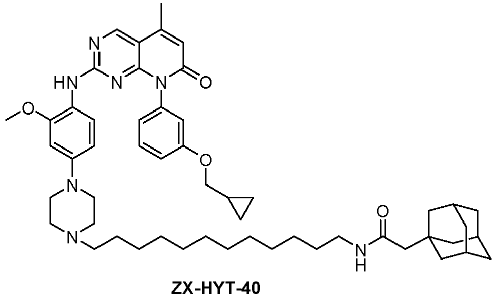

The synthesis method of compound **ZX-HYT-40** referred to Embodiment 16. HRMS (ESI) for C₅₃H₇₃N₇O₄ [M+H]⁺: calcd, 872.5797; found, 872.5789. ¹H NMR (400 MHz, DMSO-*d*₆) δ 8.78 (s, 1H), 8.08 (s, 1H), 7.61 (t, *J* = 5.7 Hz, 1H), 7.44 (t, *J* = 8.1 Hz, 1H), 7.28 (d, *J* = 8.8 Hz, 1H), 7.08 (dd, *J* = 8.4, 2.6 Hz, 1H), 6.86 (t, *J* = 2.2 Hz, 1H), 6.81 (dd, *J* = 7.6, 1.9 Hz, 1H), 6.54 (d, *J* = 2.5 Hz, 1H), 6.30 (d, *J* = 1.4 Hz, 1H), 6.00 (s, 1H), 3.86 - 3.73 (m, 5H), 3.10 - 2.94 (m, 6H), 2.50 - 2.40 (m, 7H), 2.30 (s, 2H), 1.95 - 1.85 (m, 3H), 1.80 (s, 2H), 1.69 - 1.62 (m, 3H), 1.60 - 1.52 (m, 9H), 1.49 - 1.42 (m, 2H), 1.37 (t, *J* = 6.7 Hz, 2H), 1.30 - 1.23 (m, 17H), 0.53 (dt, *J* = 10.2, 3.0 Hz, 2H), 0.28 (dt, *J* = 6.1, 3.0 Hz, 2H). ¹³C NMR (101 MHz, DMSO) δ 170.08, 162.59, 159.85, 156.88, 156.81, 147.13, 138.33, 130.22, 121.48, 117.06, 115.85, 114.49, 106.60, 100.18, 72.76, 58.34, 56.13, 53.25, 50.59, 49.32, 42.63, 38.64, 36.96, 32.61, 29.65, 29.51, 29.47, 29.42, 29.18, 28.54, 27.44, 26.87, 17.44, 10.57, 3.54.

### Embodiment 41: N-((1R,3S,5R,7S)-3-(2-((4-(4-(12-(2-((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) adamantan-1-yl) cyclopropane formamide (ZX-HYT-41)

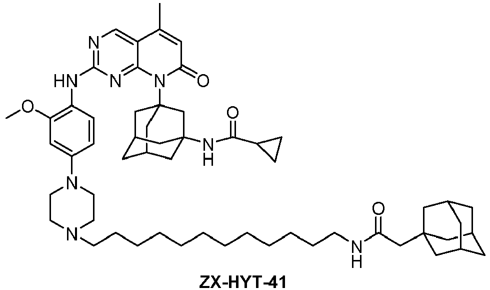

The synthesis method of compound **ZX-HYT-41** referred to Embodiment 16. HRMS (ESI) for C₅₇H₈₂N₈O₄ [M+H]⁺: calcd, 943.6532; found, 943.6541. ¹H NMR (400 MHz, DMSO-*d*₆) δ 10.55 (s, 1H), 9.15 (s, 1H), 8.79 (s, 1H), 8.08 (s, 1H), 7.64 (t, *J* = 5.7 Hz, 1H), 6.53 (d, *J* = 2.6 Hz, 1H), 6.31 (d, *J* = 1.4 Hz, 1H), 5.99 (s, 1H), 3.79 (s, 3H), 3.16 - 2.92 (m, 6H), 2.49 - 2.23 (m, 6H), 1.93 - 1.83 (m, 4H), 1.80 (s, 2H), 1.69 - 1.61 (m, 6H), 1.60 - 1.52 (m, 9H), 1.48 (s, 2H), 1.41 - 1.19 (m, 32H), 1.02 - 0.79 (m, 4H).

### Embodiment 42: 2- ((3R, 5R, 7R) - adamantan-1-yl) - N-(12-(4-(4-((8-(8-(cyclopropanecarbonyl) -8-azabicyclic [3.2.1] octane) -3-yl) -5-methyl-7-oxy-7,8-dihydropyridino [2,3-d] pyrimidin-2-yl) amino) -3-methoxyphenyl) piperazin-1-yl) dodecyl) acetamide (ZX-HYT-42)

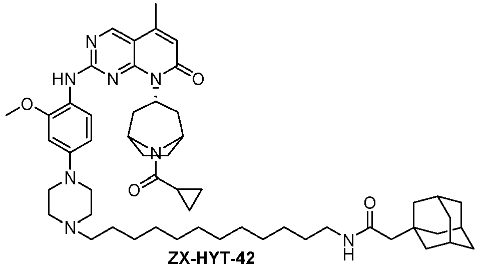

The synthesis method of compound **ZX-HYT-42** referred to Embodiment 16. HRMS (ESI) for C₅₄H₇₈N₈O₄ [M+H]⁺: calcd, 903.6224; found, 903.6231. HPLC analysis: MeOH-H₂O (97:3), 12.782 min, 100% purity. ¹H NMR (400 MHz, DMSO-*d*₆) δ 8.73 (s, 1H), 8.37 (s, 1H), 7.61 (t, *J* = 5.7 Hz, 2H), 6.65 (d, *J* = 2.5 Hz, 1H), 6.57 (d, *J* = 8.9 Hz, 1H), 6.15 (s, 1H), 5.31 (s, 1H), 4.65 - 4.47 (m, 2H), 3.81 (s, 3H), 3.23 - 3.07 (m, 4H), 3.00 (q, *J* = 6.5 Hz, 2H), 2.69 - 2.52 (m, 4H), 2.38 - 2.26 (m, 5H), 2.22 - 2.13 (m, 2H), 1.90 (s, 4H), 1.80 (s, 2H), 1.68 - 1.62 (m, 3H), 1.59 - 1.52 (m, 8H), 1.50 - 1.44 (m, 2H), 1.39 - 1.33 (m, 2H), 1.30 - 1.21 (m, 22H), 0.84 - 0.74 (m, 2H), 0.71 - 0.60 (m, 2H). ¹³C NMR (101 MHz, DMSO) δ 170.36, 170.09, 162.97, 157.33, 146.18, 107.72, 106.90, 100.54, 58.19, 56.09, 53.19, 51.44, 50.58, 48.96, 42.62, 38.64, 36.95, 33.32, 32.60, 32.35, 31.76, 31.13, 30.93, 29.64, 29.48, 29.41, 29.17, 28.54, 27.37, 26.87, 23.32, 22.57, 18.30, 17.19, 14.42, 11.72, 7.21, 7.15, 6.53.

### Embodiment 43: 2- ((3R, 5R, 7R) - adamantan-1-yl) - N-(12-(4-(4 - ((8-(3-(dimethylamino) phenyl) -5-methyl-7-oxy-7,8-dihydropyridino [2,3-d] pyrimidin-2-yl) amino) -3-methoxyphenyl) piperazin-1-yl) dodecyl) acetamide (ZX-HYT-43)

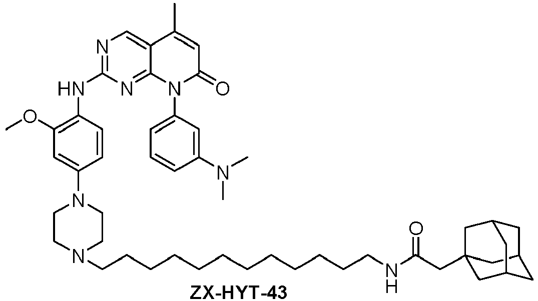

The synthesis method of compound **ZX-HYT-43** referred to Embodiment 16. HRMS (ESI) for C₅₁H₇₂N₈O₃ [M+H]⁺: calcd, 845.5806; found, 845.5797. HPLC analysis: MeOH-H₂O (95:5), RT = 12.901 min, 99.52 % purity. ¹H NMR (400 MHz, DMSO-*d*₆) δ 8.78 (s, 1H), 8.03 (s, 1H), 7.61 (t, *J* = 5.7 Hz, 1H), 7.40 - 7.30 (m, 2H), 6.85 (dd, *J* = 8.4, 2.5 Hz, 1H), 6.62 (t, *J* = 2.2 Hz, 1H), 6.54 (d, *J* = 2.5 Hz, 1H), 6.50 (dd, *J* = 7.5, 1.8 Hz, 1H), 6.29 (s, 1H), 5.98 (s, 1H), 3.79 (s, 3H), 3.09 - 2.97 (m, 6H), 2.90 (s, 6H), 2.50 - 2.46 (m, 4H), 2.44 (s, 3H), 2.32 (d, *J* = 8.7 Hz, 2H), 1.94 - 1.87 (m, 3H), 1.80 (s, 2H), 1.65 (d, *J* = 12.2 Hz, 3H), 1.58 (s, 2H), 1.56 - 1.54 (m, 6H), 1.45 (t, *J* = 7.1 Hz, 2H), 1.36 (q, *J* = 6.5, 5.9 Hz, 2H), 1.32 - 1.23 (m, 17H). ¹³C NMR (101 MHz, DMSO) δ 170.07, 162.68, 156.91, 156.77, 151.82, 146.91, 138.12, 129.87, 120.57, 117.20, 116.85, 113.36, 112.11, 106.62, 100.22, 58.33, 56.16, 55.38, 53.24, 50.59, 49.32, 42.63, 38.65, 36.96, 32.61, 29.65, 29.51, 29.46, 29.43, 29.19, 28.55, 27.44, 26.88, 26.72, 17.41.

### Embodiment 44: 2- ((3R, 5R, 7R) - adamantan-1-yl) - N-(12-(4-(4-((8-(3-((azacyclobutane-3-ylmethyl) amino) phenyl) -5-methyl-7-oxy-7,8-dihydropyridino [2,3-d] pyrimidin-2-yl) amino) -3-methoxyphenyl) piperazin-1-yl) dodecanyl) acetamide (ZX-HYT-44)

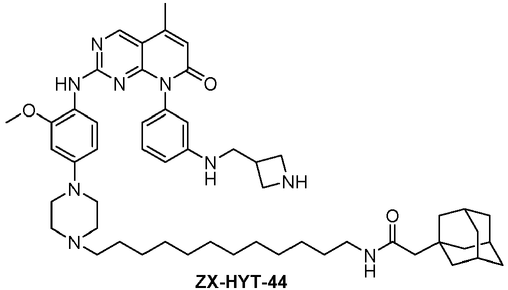

The synthesis method of compound **ZX-HYT-44** referred to Embodiment 17. HRMS (ESI) for C₅₃H₇₅N₉O₃ [M+H]⁺: calcd, 886.6071; found, 886.6066. HPLC analysis: MeOH-H₂O (95:5), RT = 8.652 min, 98.25% purity. ¹H NMR (400 MHz, DMSO-*d*₆) δ 9.95 (s, 1H), 8.79 (s, 1H), 8.69 (s, 1H), 8.13 (s, 1H), 7.64 (t, *J* = 5.7 Hz, 1H), 7.45 (d, *J* = 8.8 Hz, 1H), 7.25 (t, *J* = 8.0 Hz, 1H), 6.72 (d, *J* = 8.3 Hz, 1H), 6.64 (d, *J* = 2.5 Hz, 1H), 6.50 - 6.39 (m, 2H), 6.30 (s, 1H), 6.06 (s, 1H), 3.95 (s, 2H), 3.81 (s, 3H), 3.76 - 3.64 (m, 4H), 3.57 (s, 2H), 3.30 - 3.23 (m, 4H), 3.13 (s, 4H), 3.04 - 2.89 (m, 5H), 2.45 (s, 3H), 1.90 (s, 3H), 1.80 (s, 2H), 1.70 - 1.61 (m, 4H), 1.60 - 1.51 (m, 8H), 1.40 - 1.34 (m, 2H), 1.33 - 1.19 (m, 16H). ¹³C NMR (101 MHz, DMSO) δ 170.15, 162.64, 158.69, 158.40, 156.89, 156.81, 150.09, 147.02, 138.15, 129.96, 121.57, 119.19, 117.24, 116.80, 116.21, 112.57, 112.28, 107.27, 106.71, 100.91, 56.31, 55.97, 51.27, 50.58, 49.41, 46.70, 45.42, 42.61, 38.65, 36.94, 32.61, 31.46, 29.64, 29.49, 29.41, 29.27, 29.16, 28.97, 28.52, 26.88, 26.49, 23.66, 17.42.

### Embodiment 45: 2- ((3R, 5R, 7R) - adamantan-1-yl) -N-(12-(4-(4-((8-(3-(azacyclobutane-3-ylamino) phenyl) -5-methyl-7-oxy-7,8-dihydropyridino [2,3-d] pyrimidin-2-yl) amino) -3-methoxyphenyl) piperazin-1-yl) dodecyl) acetamide (ZX-HYT-45)

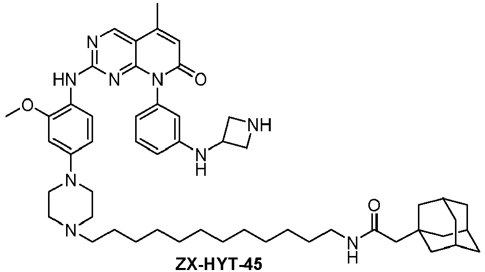

The synthesis method of compound **ZX-HYT-45** referred to Embodiment 17. HRMS (ESI) for C₅₂H₇₃N₉O₃ [M+H]⁺: calcd, 872.5915; found, 872.5926. HPLC analysis: MeOH-H₂O (95:5), RT = 4.763 min, 98.21% purity. ¹H NMR (400 MHz, DMSO-*d*₆) δ 8.76 (s, 1H), 8.01 (s, 1H), 7.61 (s, 1H), 7.43 - 7.32 (m, 1H), 7.23 (s, 1H), 6.67 - 6.57 (m, 1H), 6.53 (s, 1H), 6.46 - 6.22 (m, 4H), 6.03 (s, 1H), 4.18 (s, 1H), 3.94 - 3.64 (m, 7H), 3.06 - 2.96 (m, 6H), 2.48 - 2.35 (m, 7H), 2.29 (s, 2H), 1.89 (s, 3H), 1.79 (s, 2H), 1.67 - 1.61 (m, 3H), 1.59 - 1.50 (m, 9H), 1.47 - 1.41 (m, 2H), 1.38 - 1.34 (m, 2H), 1.29 - 1.17 (m, 16H). ¹³C NMR (101 MHz, DMSO) δ 170.11, 162.61, 156.82, 148.94, 147.00, 138.05, 130.10, 120.51, 117.14, 113.44, 111.57, 106.87, 106.55, 100.11, 58.40, 56.16, 54.58, 54.44, 53.29, 50.59, 49.35, 47.79, 42.62, 38.65, 36.95, 32.60, 29.64, 29.51, 29.42, 29.18, 28.54, 27.48, 26.88, 26.80, 17.41.

### Embodiment 46: N-((1S,4S)-4-(2-((4-(4-(12-(2-((3R, 5R, 7R) - adamantan-1-yl) acetylamino) dodecyl) piperazin-1-yl) -2-methoxyphenyl) amino) -5-methyl-7-oxypyridino [2,3-d] pyrimidin-8 (7H) - yl) cyclohexyl) cyclopropane formamide (ZX-HYT-46)

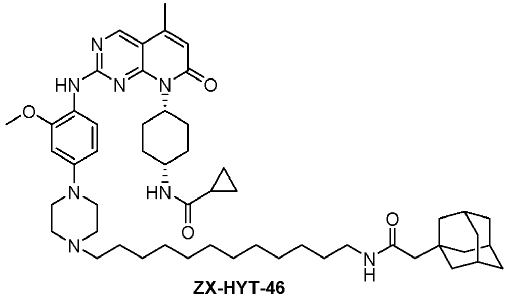

The synthesis method of compound **ZX-HYT-46** referred to Embodiment 20. HRMS (ESI) for C₅₃H₇₈N₈O₄ [M+H]⁺: calcd, 891.6219; found, 891.6223. ¹H NMR (500 MHz, Chloroform-*d*) δ 9.07 (s, 1H), 8.51 (s, 1H), 7.48 (t, *J* = 5.9 Hz, 1H), 7.07 (d, *J* = 7.5 Hz, 1H), 6.91 (d, *J* = 11.5 Hz, 1H), 6.76 (dd, *J* = 7.5, 1.5 Hz, 1H), 6.45 (q, *J* = 1.1 Hz, 1H), 6.31 (d, *J* = 1.6 Hz, 1H), 4.11 (p, *J* = 7.0 Hz, 1H), 3.86 (s, 3H), 3.59 (dp, *J* = 11.4, 7.0 Hz, 1H), 3.27 (t, *J* = 7.1 Hz, 4H), 3.07 (td, *J* = 7.1, 5.9 Hz, 2H), 2.63 (t, *J* = 7.1 Hz, 4H), 2.50 - 2.41 (m, 4H), 2.38 (t, *J* = 7.1 Hz, 2H), 2.28 (s, 2H), 2.10 (s, 3H), 1.78 - 1.70 (m, 2H), 1.70 - 1.54 (m, 15H), 1.54 - 1.48 (m, 5H), 1.41 - 1.38 (m, 4H), 1.34 - 1.18 (m, 14H), 1.00 - 0.86 (m, 4H).

### Embodiment 47: N-(3-(2-((4-(4-(8-(2-((3R, 5R, 7R) - adamantan-1-yl) acetylamino) octyl) piperazin-1-yl) -2-methoxyphenyl)) amino) -5-methyl-7-oxypyridino [2,3-d] pyrimidin-8 (7H) - yl) phenyl) cyclopropane formamide (ZX-HYT-47)

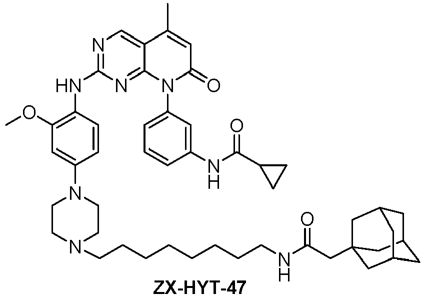

The synthesis method of compound **ZX-HYT-47** referred to Embodiment 8. HRMS (ESI) for C₄₉H₆₄N₈O₄ [M+H]⁺: calcd, 829.5123; found, 829.5135. ¹H NMR (400 MHz, DMSO-*d*₆) δ 10.39 (s, 1H), 8.80 (s, 1H), 8.11 (s, 1H), 7.81 (d, *J* = 8.0 Hz, 1H), 7.62 (t, *J* = 5.7 Hz, 1H), 7.55 - 7.41 (m, 2H), 7.28 (d, *J* = 8.9 Hz, 1H), 6.99 - 6.88 (m, 1H), 6.53 (d, *J* = 2.5 Hz, 1H), 6.32 (d, *J* = 1.4 Hz, 1H), 6.02 (s, 1H), 3.79 (s, 3H), 3.12 - 2.94 (m, 6H), 2.49 - 2.41 (m, 4H), 2.32 (q, *J* = 13.4, 7.5 Hz, 2H), 1.94 - 1.87 (m, 3H), 1.80 (s, 3H), 1.69 - 1.62 (m, 4H), 1.61 - 1.52 (m, 9H), 1.52 - 1.44 (m, 2H), 1.42 - 1.33 (m, 2H), 1.32 - 1.15 (m, 10H), 0.88 - 0.75 (m, 4H).

### Embodiment 48: N-(3-(2-((4-(4-(8-(2-((3R, 5R, 7R) - adamantan-1-yl) acetylamino) octyl) piperazin-1-yl) -2-methoxyphenyl)) amino) -5-methyl-7-oxypyridino [2,3-d] pyrimidin-8 (7H) - yl) phenyl) neopentanamide (ZX-HYT-48)

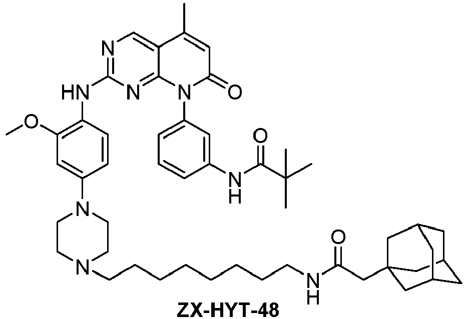

The synthesis method of compound **ZX-HYT-48** referred to Embodiment 8. HRMS (ESI) for C₅₀H₆₈N₈O₄ [M+H]⁺: calcd, 845.5436; found, 845.5441. ¹H NMR (400 MHz, DMSO-*d*₆) δ 9.38 (s, 1H), 8.80 (s, 1H), 8.11 (s, 1H), 7.91 (d, *J* = 8.2 Hz, 1H), 7.64 (t, *J* = 5.6 Hz, 1H), 7.55 (t, *J* = 2.1 Hz, 1H), 7.46 (t, *J* = 8.1 Hz, 1H), 7.28 (d, *J* = 8.9 Hz, 1H), 6.93 (dd, *J* = 7.7, 2.0 Hz, 1H), 6.52 (d, *J* = 2.5 Hz, 1H), 6.32 (s, 1H), 6.03 (s, 1H), 3.78 (s, 3H), 3.15 - 3.04 (m, 4H), 3.01 (q, *J* = 6.5 Hz, 2H), 2.71 - 2.52 (m, 4H), 2.48 - 2.38 (m, 5H), 1.90 (s, 3H), 1.80 (s, 2H), 1.68 - 1.62 (m, 3H), 1.58 - 1.53 (m, 9H), 1.51 - 1.43 (m, 2H), 1.41 - 1.35 (m, 2H), 1.30 - 1.25 (m, 8H), 1.21 (s, 9H).

### Embodiment 49: N-(3-(2-((4-(4-(8-(2-((3R, 5R, 7R) - adamantan-1-yl) acetylamino) octyl) piperazin-1-yl) -2-methoxyphenyl)) amino) -5-methyl-7-oxypyridino [2,3-d] pyrimidin-8 (7H) - yl) phenyl) propionamide (ZX-HYT-49)

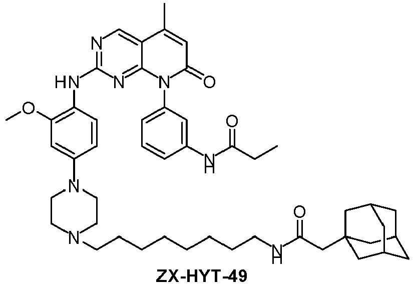

The synthesis method of compound **ZX-HYT-49** referred to Embodiment 8. HRMS (ESI) for C₄₈H₆₄N₈O₄ [M+H]⁺: calcd, 817.5123; found, 817.5134. ¹H NMR (400 MHz, DMSO-*d*₆) δ 10.05 (s, 1H), 8.80 (s, 1H), 8.10 (s, 1H), 7.82 - 7.75 (m, 1H), 7.63 (t, *J* = 5.7 Hz, 1H), 7.51 - 7.43 (m, 2H), 7.26 (d, *J* = 8.9 Hz, 1H), 6.96 - 6.89 (m, 1H), 6.53 (d, *J* = 2.6 Hz, 1H), 6.35 - 6.29 (m, 1H), 6.01 (s, 1H), 3.78 (s, 3H), 3.10 - 2.96 (m, 6H), 2.49 - 2.41 (m, 7H), 2.36 - 2.25 (m, 4H), 1.91 (s, 3H), 1.80 (s, 2H), 1.70 - 1.62 (m, 3H), 1.60 - 1.52 (m, 9H), 1.49 - 1.43 (m, 2H), 1.40 - 1.35 (m, 2H), 1.31 - 1.23 (m, 8H), 1.07 (t, *J* = 7.5 Hz, 3H).

### Embodiment 50: N-(3-(2-((4-(4-(2-((3R, 5R, 7R) - adamantan-1-yl) acetyl) piperazin-1-yl) -2-methoxyphenyl) amino) -5-methyl-7-oxypyridino [2,3-d] pyrimidin-8 (7H) - yl) phenyl) cyclopropane formamide (ZX-HYT-50)

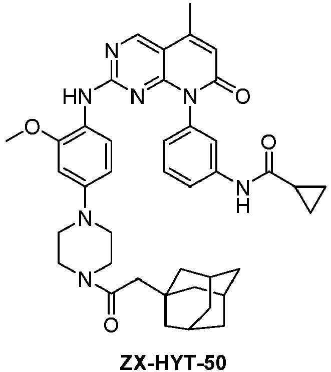

The synthesis method of compound **ZX-HYT-50** referred to Embodiment 8. HRMS (ESI) for C₄₁H₄₇N₇O₄ [M+H]⁺: calcd, 702.3702; found, 702.3713. ¹H NMR (600 MHz, DMSO-*d*₆) δ 10.39 (s, 1H), 8.81 (s, 1H), 8.14 (s, 1H), 7.80 (s, 1H), 7.54 - 7.41 (m, 2H), 7.29 (d, *J* = 8.8 Hz, 1H), 6.98 - 6.87 (m, 1H), 6.66 - 6.50 (m, 1H), 6.33 (s, 1H), 6.03 (s, 1H), 3.80 (s, 3H), 3.70 - 3.57 (m, 4H), 3.09 - 2.94 (m, 4H), 2.46 (s, 3H), 2.16 (s, 2H), 1.94 (s, 3H), 1.78 (p, *J* = 6.3 Hz, 1H), 1.69 - 1.60 (m, 12H), 0.82 - 0.73 (m, 4H).

### Embodiment 51: N-(3-(2-((4-(4-(2-(2-((3R, 5R, 7R) - adamantan-1-yl) acetylamino) ethyl) piperazin-1-yl) -2-methoxyphenyl)) amino) -5-methyl-7-oxypyridino [2,3-d] pyrimidin-8 (7H) - yl) phenyl) cyclopropane formamide (ZX-HYT-51)

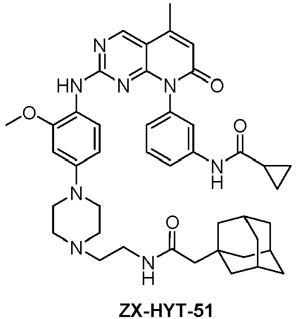

The synthesis method of compound **ZX-HYT-51** referred to Embodiment 8. HRMS (ESI) for C₄₃H₅₂N₈O₄ [M+H]⁺: calcd, 745.4184; found, 745.4191. ¹H NMR (600 MHz, DMSO-*d*₆) δ 10.40 (s, 1H), 8.80 (s, 1H), 8.11 (s, 1H), 7.79 (s, 1H), 7.61 (t, *J* = 5.7 Hz, 1H), 7.54 - 7.42 (m, 2H), 7.28 (d, *J* = 8.9 Hz, 1H), 6.93 (dt, *J* = 8.1, 1.6 Hz, 1H), 6.53 (d, *J* = 2.5 Hz, 1H), 6.32 (s, 1H), 6.00 (s, 1H), 3.79 (s, 3H), 3.20 (q, *J* = 6.4 Hz, 2H), 3.09 - 2.98 (m, 4H), 2.57 - 2.52 (m, 4H), 2.46 (s, 3H), 2.40 (t, *J* = 6.6 Hz, 2H), 1.91 (s, 3H), 1.86 - 1.82 (m, 2H), 1.78 (tt, *J* = 7.3, 3.7 Hz, 1H), 1.69 - 1.64 (m, 3H), 1.60 - 1.56 (m, 9H), 0.80 - 0.74 (m, 4H).

### Embodiment 52: N-(3-(2-((4-(4-(4-(2-((3R, 5R, 7R) - adamantan-1-yl) acetylamino) butyl) piperazin-1-yl) -2-methoxyphenyl)) amino) -5-methyl-7-oxypyridino [2,3-d] pyrimidin-8 (7H) - yl) phenyl) cyclopropane formamide (ZX-HYT-52)

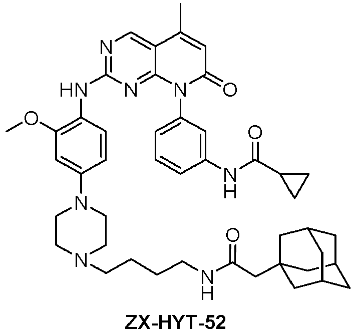

The synthesis method of compound **ZX-HYT-52** referred to Embodiment 8. HRMS (ESI) for C₄₅H₅₆N₈O₄ [M+H]⁺: calcd, 773.4497; found, 773.4485. ¹H NMR (600 MHz, DMSO-*d*₆) δ 10.39 (s, 1H), 8.81 (s, 1H), 8.14 (s, 1H), 7.81 (s, 1H), 7.71 (s, 1H), 7.50 - 7.43 (m, 2H), 7.29 (d, *J* = 8.8 Hz, 1H), 6.95 - 6.91 (m, 1H), 6.56 (s, 1H), 6.33 (s, 1H), 6.02 (s, 1H), 3.79 (s, 3H), 3.18 - 2.86 (m, 8H), 2.64 - 2.51 (m, 6H), 2.46 (s, 3H), 1.95 - 1.90 (m, 3H), 1.83 (s, 2H), 1.80 - 1.76 (m, 1H), 1.69 - 1.63 (m, 3H), 1.61 - 1.52 (m, 9H), 1.46 - 1.40 (m, 2H), 0.83 - 0.75 (m, 4H).

### Embodiment 53: N-(3-(2-((4-(4-(6-(2-((3R, 5R, 7R) - adamantan-1-yl) acetylamino) hexyl) piperazin-1-yl) -2-methoxyphenyl)) amino) -5-methyl-7-oxypyridino [2,3-d] pyrimidin-8 (7H) - yl) phenyl) cyclopropane formamide (ZX-HYT-53)

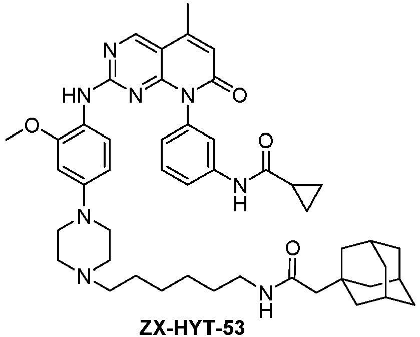

The synthesis method of compound **ZX-HYT-53** referred to Embodiment 8. HRMS (ESI) for C₄₇H₆₀N₈O₄ [M+H]⁺: calcd, 801.4810; found, 801.4823. ¹H NMR (600 MHz, DMSO-*d*₆) δ 10.40 (s, 1H), 8.81 (s, 1H), 8.17 (s, 1H), 7.91 (s, 1H), 7.76 (s, 1H), 7.50 - 7.45 (m, 2H), 7.19 (d, *J* = 8.8 Hz, 1H), 6.95 - 6.93 (m, 1H), 6.57 (s, 1H), 6.34 (s, 1H), 6.12 (s, 1H), 3.79 (s, 3H), 3.18 - 2.86 (m, 8H), 2.64 - 2.51 (m, 8H), 2.46 (s, 3H), 1.95 - 1.90 (m, 3H), 1.83 (s, 2H), 1.80 - 1.76 (m, 1H), 1.69 - 1.63 (m, 3H), 1.61 - 1.52 (m, 9H), 1.48 - 1.37 (m, 4H), 0.83 - 0.75 (m, 4H).

### Embodiment 54: N-(3-(2-((4-(4-(10-(2-((3R, 5R, 7R) - adamantan-1-yl) acetylamino) decyl) piperazin-1-yl) -2-methoxyphenyl)) amino) -5-methyl-7-oxypyridino [2,3-d] pyrimidin-8 (7H) - yl) phenyl) cyclopropane formamide (ZX-HYT-54)

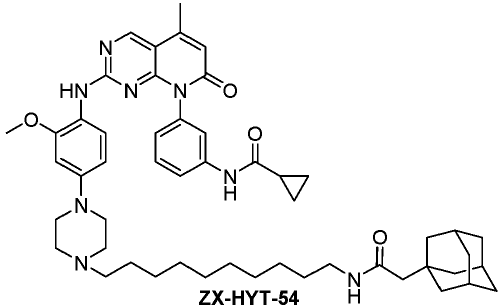

The synthesis method of compound **ZX-HYT-54** referred to Embodiment 8. HRMS (ESI) for C₅₁H₆₈N8O₄ [M+H]⁺: calcd, 857.5436; found, 857.5441. ¹H NMR (400 MHz, DMSO-*d*₆) δ 10.39 (s, 1H), 8.80 (s, 1H), 8.11 (s, 1H), 7.81 (d, *J* = 8.0 Hz, 1H), 7.62 (t, *J* = 5.7 Hz, 1H), 7.57 - 7.41 (m, 2H), 7.28 (d, *J* = 8.9 Hz, 1H), 6.99 - 6.88 (m, 1H), 6.53 (d, *J* = 2.5 Hz, 1H), 6.32 (d, *J* = 1.4 Hz, 1H), 6.02 (s, 1H), 3.79 (s, 3H), 3.12 - 2.94 (m, 6H), 2.49 - 2.41 (m, 4H), 2.32 (q, *J* = 13.4, 7.5 Hz, 2H), 1.94 - 1.87 (m, 3H), 1.80 (s, 2H), 1.69 - 1.62 (m, 3H), 1.61 - 1.52 (m, 9H), 1.52 - 1.44 (m, 2H), 1.42 - 1.33 (m, 2H), 1.31 - 1.11 (m, 14H), 0.88 - 0.75 (m, 6H).

### Embodiment 55: N-(3-(2-((4-(4-(14-(2-((3R, 5R, 7R) - adamantan-1-yl) acetylamino) tetradecyl) piperazin-1-yl) -2-methoxyphenyl)) amino) -5-methyl-7-oxypyridino [2,3-d] pyrimidin-8 (7H) - yl) phenyl) cyclopropane formamide (ZX-HYT-55)

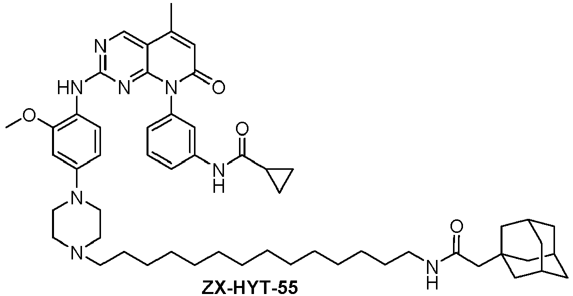

The synthesis method of compound **ZX-HYT-55** referred to Embodiment 8. HRMS (ESI) for C₅₅H₇₆N₈O₄ [M+H]⁺: calcd, 913.6062; found, 913.6062. ¹H NMR (400 MHz, DMSO-*d*₆) δ 10.39 (s, 1H), 8.80 (s, 1H), 8.11 (s, 1H), 7.81 (d, *J* = 8.0 Hz, 1H), 7.62 (t, *J* = 5.7 Hz, 1H), 7.55 - 7.41 (m, 2H), 7.28 (d, *J* = 8.9 Hz, 1H), 6.99 - 6.88 (m, 1H), 6.53 (d, *J* = 2.5 Hz, 1H), 6.32 (d, *J* = 1.4 Hz, 1H), 6.02 (s, 1H), 3.79 (s, 3H), 3.12 - 2.94 (m, 6H), 2.49 - 2.41 (m, 4H), 2.32 (q, *J* = 13.4, 7.5 Hz, 2H), 1.94 - 1.87 (m, 3H), 1.80 (s, 2H), 1.69 - 1.62 (m, 3H), 1.61 - 1.52 (m, 9H), 1.52 - 1.44 (m, 2H), 1.42 - 1.33 (m, 2H), 1.32 - 1.12 (m, 22H), 0.88 - 0.75 (m, 6H).

### Embodiment 56: N-(3-(2-((4-(4-(2-((3R, 5R, 7R) - adamantan-1-yl) acetyl) piperazin-1-yl) -2-methoxyphenyl) amino) -5-methyl-7-oxypyridino [2,3-d] pyrimidin-8 (7H) - yl) phenyl) acrylamide (ZX-HYT-56)

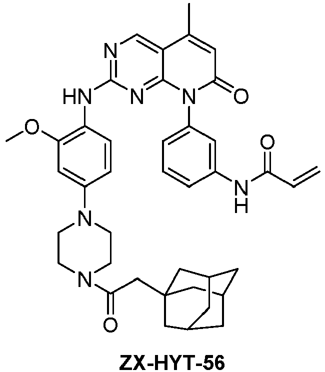

The synthesis method of compound **ZX-HYT-56** referred to Embodiment 8. HRMS (ESI) for C₄₀H₄₅N₇O₄ [M+H]⁺: calcd, 688.3606; found, 688.3673.. ¹H NMR (400 MHz, DMSO-*d*₆) δ 10.35 (s, 1H), 8.80 (s, 1H), 8.11 (s, 1H), 7.90 (d, *J* = 8.1 Hz, 1H), 7.62 (t, *J* = 5.6 Hz, 1H), 7.56 (t, *J* = 2.0 Hz, 1H), 7.51 (t, *J* = 8.1 Hz, 1H), 7.27 (d, *J* = 8.9 Hz, 1H), 6.98 (ddd, *J* = 7.8, 2.0, 1.0 Hz, 1H), 6.51 (d, *J* = 2.5 Hz, 1H), 6.48 - 6.39 (m, 1H), 6.36 - 6.21 (m, 2H), 6.00 (s, 1H), 5.76 (dd, *J* = 10.1, 2.1 Hz, 1H), 3.78 (s, 3H), 3.06 - 2.93 (m, 4H), 2.49 - 2.40 (m, 4H), 2.41 (s, 3H), 2.30 - 2.12 (m, 2H), 1.79 - 1.61 (m, 3H), 1.42 - 1.20 (m, 12H).

### Embodiment 57: N-(3-(2-((4-(4-(2-(2-((3R, 5R, 7R) - adamantan-1-yl) acetylamino) ethyl) piperazin-1-yl) -2-methoxyphenyl)) amino) -5-methyl-7-oxypyridino [2,3-d] pyrimidin-8 (7H) - yl) phenyl) acrylamide (ZX-HYT-57)

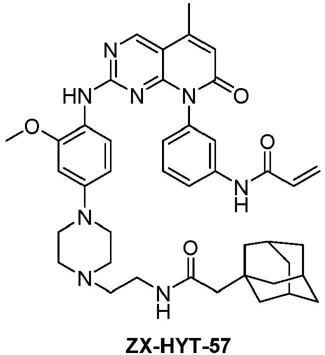

The synthesis method of compound **ZX-HYT-57** referred to Embodiment 8. HRMS (ESI) for C₄₂H₅₀N₈O₄ [M+H]⁺: calcd, 731.4028; found, 731.4031. ¹H NMR (400 MHz, DMSO-*d*₆) δ 10.35 (s, 1H), 8.80 (s, 1H), 8.11 (s, 1H), 7.90 (d, *J* = 8.1 Hz, 1H), 7.62 (t, *J* = 5.6 Hz, 1H), 7.56 (t, *J* = 2.0 Hz, 1H), 7.51 (t, *J* = 8.1 Hz, 1H), 7.27 (d, *J* = 8.9 Hz, 1H), 6.98 (ddd, *J* = 7.8, 2.0, 1.0 Hz, 1H), 6.51 (d, *J* = 2.5 Hz, 1H), 6.48 - 6.39 (m, 1H), 6.36 - 6.21 (m, 2H), 6.00 (s, 1H), 5.76 (dd, *J* = 10.1, 2.1 Hz, 1H), 3.78 (s, 3H), 3.06 - 2.93 (m, 4H), 2.49 - 2.40 (m, 4H), 2.41 (s, 3H), 2.30 - 2.12 (m, 4H), 1.69 - 1.61 (m, 3H), 1.60 - 1.53 (m, 4H), 1.32 - 1.20 (m, 10H).

### Embodiment 58: N-(3-(2-((4-(4-(4-(2-((3R, 5R, 7R) - adamantan-1-yl) acetylamino) butyl) piperazin-1-yl) -2-methoxyphenyl)) amino) -5-methyl-7-oxypyridino [2,3-d] pyrimidin-8 (7H) - yl) phenyl) acrylamide (ZX-HYT-58)

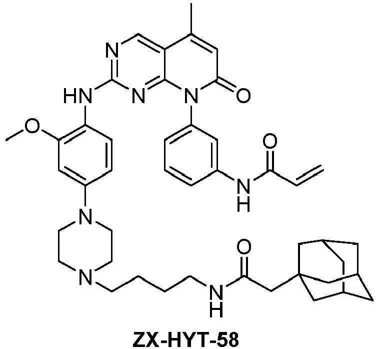

The synthesis method of compound **ZX-HYT-58** referred to Embodiment 8. HRMS (ESI) for C₄₄H₅₄N₈O₄ [M+H]⁺: calcd, 759.4341; found, 759.4356.
¹H NMR (400 MHz, DMSO-*d*₆) δ 10.35 (s, 1H), 8.80 (s, 1H), 8.11 (s, 1H), 7.90 (d, *J* = 8.1 Hz, 1H), 7.62 (t, *J* = 5.6 Hz, 1H), 7.56 (t, *J* = 2.0 Hz, 1H), 7.51 (t, *J* = 8.1 Hz, 1H), 7.27 (d, *J* = 8.9 Hz, 1H), 6.98 (ddd, *J* = 7.8, 2.0, 1.0 Hz, 1H), 6.51 (d, *J* = 2.5 Hz, 1H), 6.48 - 6.39 (m, 1H), 6.36 - 6.21 (m, 2H), 6.00 (s, 1H), 5.76 (dd, *J* = 10.1, 2.1 Hz, 1H), 3.78 (s, 3H), 3.06 - 2.93 (m, 6H), 2.49 - 2.40 (m, 7H), 2.30 (t, *J* = 7.4 Hz, 2H), 1.90 (s, 3H), 1.69 - 1.61 (m, 3H), 1.60 - 1.53 (m, 3H), 1.50 - 1.41 (m, 2H), 1.32 - 1.20 (m, 10H).

### Embodiment 59: N-(3-(2-((4-(4-(8-(2-((1S, 3R, 5S, 7R) -3,5-dimethyladamantan-1-yl) acetamido) octyl) piperazin-1-yl) -2-methoxyphenyl) amino) -5-methyl-7-oxypyridino [2,3-d] pyrimidin-8 (7H) - yl) phenyl) cyclopropanilamide (ZX-HYT-59)

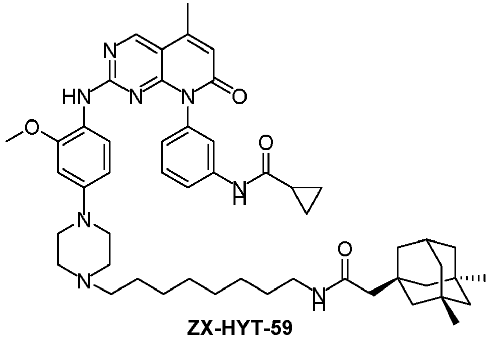

The synthesis method of compound **ZX-HYT-59** referred to Embodiment 32. HRMS (ESI) for C₅₁H₆₈N₈O₄ [M+H]⁺: calcd, 857.5442; found, 857.5436. HPLC analysis: MeOH-H₂O (85:15), RT = 13.264 min, 96.02% purity. ¹H NMR (400 MHz, DMSO-*d*₆) δ 10.41 (s, 1H), 8.79 (s, 1H), 8.14 (s, 1H), 7.81 (d, *J* = 7.9 Hz, 1H), 7.66 (t, *J* = 5.7 Hz, 1H), 7.50 - 7.43 (m, 2H), 7.29 (d, *J* = 8.8 Hz, 1H), 6.92 (dd, *J* = 7.9, 2.0 Hz, 1H), 6.55 (d, *J* = 2.5 Hz, 1H), 6.32 (s, 1H), 6.04 (s, 1H), 3.78 (s, 3H), 3.12 (q, *J* = 7.4 Hz, 4H), 3.01 (q, *J* = 6.4 Hz, 2H), 2.82 (s, 2H), 2.52 - 2.37 (m, 7H), 2.02 - 1.95 (m, 1H), 1.83 (s, 1H), 1.77 (p, *J* = 6.2 Hz, 1H), 1.54 (s, 1H), 1.41 - 1.34 (m, 3H), 1.28 - 1.26 (m, 7H), 1.25 - 1.23 (m, 8H), 1.20 - 1.16 (m, 3H), 1.13 - 1.07 (m, 2H), 1.03 - 0.98 (m, 1H), 0.86 - 0.62 (m, 10H). ¹³C NMR (101 MHz, DMSO) δ 172.23, 170.21, 162.60, 157.03, 156.74, 147.36, 140.72, 137.48, 129.84, 123.89, 119.74, 118.84, 117.04, 106.89, 106.63, 100.37, 56.22, 54.03, 52.51, 51.10, 49.88, 48.94, 43.18, 42.29, 41.21, 38.62, 34.20, 31.32, 31.01, 29.63, 29.58, 29.32, 29.13, 27.05, 26.87, 18.53, 17.46, 17.23, 15.05, 12.99, 7.73, 7.63.

### Embodiment 60: (1S, 2R, 4S) - N - (8- (4- (4- ((8- (3-(cyclopropaneylamino) phenyl) -5-methyl-7-oxy-7,8-dihydropyridinyl [2,3-d] pyrimidin-2-yl) amino) -3-methoxyphenyl) piperazin-1-yl) octyl) bicyclic [2.2.1] hept-5-en-2-carboxylamide (ZX-HYT-60)

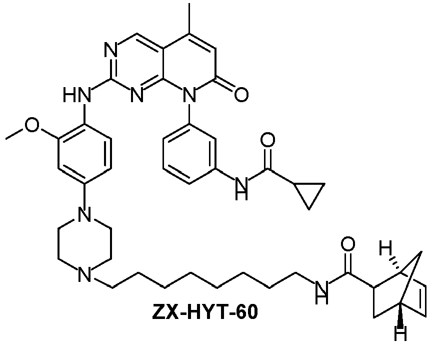

The synthesis method of compound **ZX-HYT-60** referred to Embodiment 32. HRMS (ESI) for C₄₅H₅₆N₈O₄ [M+H]⁺: calcd, 773.4503; found, 773.4497. HPLC analysis: MeOH-H₂O (97:3), RT = 4.703 min, 98.47% purity. ¹H NMR (400 MHz, DMSO-*d*₆) δ 10.43 (s, 1H), 9.87 (s, 1H), 9.27 (s, 1H), 8.81 (s, 1H), 8.17 (s, 1H), 7.78 (d, *J* = 8.0 Hz, 1H), 7.56 (t, *J* = 5.7 Hz, 1H), 7.48 - 7.44 (m, 1H), 7.32 (d, *J* = 8.8 Hz, 1H), 6.93 (d, *J* = 7.8 Hz, 1H), 6.61 (s, 1H), 6.33 (s, 1H), 6.10 (dd, *J* = 5.7, 3.0 Hz, 1H), 5.82 - 5.76 (m, 1H), 3.80 (s, 3H), 3.76 - 3.70 (m, 1H), 3.60 - 3.55 (m, 1H), 3.14 - 3.08 (m, 8H), 3.04 - 3.00 (m, 1H), 2.98 - 2.92 (m, 2H), 2.82 - 2.74 (m, 2H), 2.45 (s, 3H), 1.79 (t, *J* = 6.1 Hz, 1H), 1.73 - 1.67 (m, 2H), 1.38 - 1.35 (m, 1H), 1.32 - 1.29 (m, 3H), 1.27 - 1.24 (m, 3H), 1.20 - 1.16 (m, 7H), 0.83 - 0.74 (m, 4H). ¹³C NMR (101 MHz, DMSO) δ 173.07, 172.27, 162.57, 157.06, 156.73, 147.34, 140.71, 137.48, 137.28, 132.58, 129.81, 123.93, 119.82, 118.92, 117.12, 107.10, 106.70, 100.79, 56.30, 51.27, 49.85, 46.67, 46.15, 46.11, 43.77, 42.56, 38.91, 29.68, 28.93, 28.84, 26.73, 26.46, 17.47, 15.03, 9.04, 7.65.

### Embodiment 61: (1R,2R,4S)-N-(8- (4- (4- ((8- (3- (cyclopropaneformamide) phenyl) phenyl) -5-methyl-7-oxy-7,8-dihydropyridinyl [2,3-d] pyrimidin-2-yl) amino) -3-methoxyphenyl) piperazin-1-yl) octyl) bicyclic [2.2.1] heptane-2-carboxylamide (ZX-HYT-61)

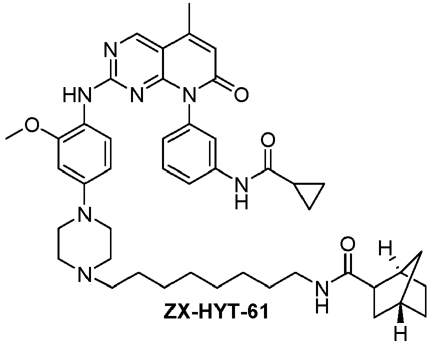

The synthesis method of compound **ZX-HYT-61** referred to Embodiment 32. HRMS (ESI) for C₄₅H₅₈N₈O₄ [M+H]⁺: calcd, 775.4659; found, 775.4654. HPLC analysis: MeOH-H₂O (95:5), 4.929 min, 96.20% purity. ¹H NMR (400 MHz, DMSO-*d*₆) δ 10.40 (s, 1H), 8.78 (s, 1H), 8.08 (s, 1H), 7.81 (d, *J* = 7.9 Hz, 1H), 7.70 - 7.58 (m, 1H), 7.53 - 7.42 (m, 2H), 7.27 (d, *J* = 8.9 Hz, 1H), 6.91 (d, *J* = 7.9 Hz, 1H), 6.52 (s, 1H), 6.30 (s, 1H), 6.02 (s, 1H), 3.78 (s, 3H), 3.11 - 2.93 (m, 7H), 2.59 - 2.53 (m, 1H), 2.49 - 2.45 (m, 4H), 2.44 - 2.40 (m, 4H), 2.29 (t, *J* = 7.4 Hz, 2H), 2.22 - 2.09 (m, 2H), 1.85 - 1.68 (m, 2H), 1.60 - 1.53 (m, 1H), 1.49 - 1.42 (m, 4H), 1.40 - 1.35 (m, 3H), 1.29 - 1.24 (m, 9H), 0.82 - 0.75 (m, 4H). ¹³C NMR (101 MHz, DMSO) δ 175.06, 173.17, 172.21, 162.60, 156.97, 156.73, 147.32, 140.79, 137.47, 130.11, 129.85, 123.82, 120.28, 119.68, 118.75, 116.98, 106.72, 106.56, 100.04, 58.37, 56.14, 53.27, 49.27, 46.87, 46.33, 41.94, 41.04, 38.98, 38.95, 37.06, 36.29, 35.86, 33.79, 31.13, 29.81, 29.73, 29.65, 29.44, 29.31, 29.21, 28.86, 27.39, 27.03, 26.82, 26.78, 24.32, 17.45, 15.04, 7.71, 7.63.

### Embodiment 62: N-(3-(2-((4-(4-(8-(cyclopropaneformamide) octyl) piperazin-1-yl) -2-methoxyphenyl) amino) -5-methyl-7-oxypyridino [2,3-d)] pyrimidin-8 (7H) - yl) phenyl) cyclopropaneformamide (ZX-HYT-62)

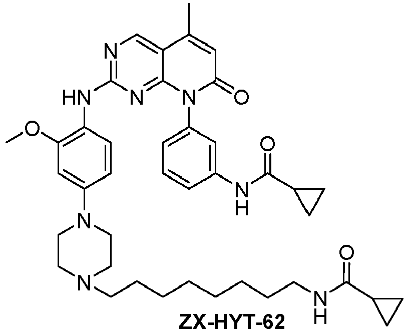

The synthesis method of compound **ZX-HYT-62** referred to Embodiment 32. HRMS (ESI) for C₄₁H₅₂N₈O₄ [M+H]⁺: calcd, 721.4184; found, 721.4192. ¹H NMR (400 MHz, DMSO-*d*₆) δ 10.39 (s, 1H), 8.80 (s, 1H), 8.11 (s, 1H), 7.81 (d, *J* = 8.0 Hz, 1H), 7.62 (t, *J* = 5.7 Hz, 1H), 7.55 - 7.41 (m, 2H), 7.28 (d, *J* = 8.9 Hz, 1H), 6.99 - 6.88 (m, 1H), 6.53 (d, *J* = 2.5 Hz, 1H), 6.32 (d, *J* = 1.4 Hz, 1H), 6.02 (s, 1H), 3.79 (s, 3H), 3.12 - 2.94 (m, 6H), 2.49 - 2.41 (m, 4H), 2.33 (s, 3H), 1.94 - 1.87 (m, 3H), 1.69 - 1.62 (m, 2H), 1.61 - 1.52 (m, 9H), 1.52 - 1.44 (m, 2H), 0.88 - 0.75 (m, 8H).

### Embodiment 63: N - (8- (4- (4- ((8- (3- (cyclopropaneformamido) phenyl) -5-methyl-7-oxy-7,8-dihydropyridino [2,3-d] pyrimidin-2-yl) amino) -3-methoxyphenyl) piperazin-1-yl) octyl) cyclohexane formamide (ZX-HYT-63)

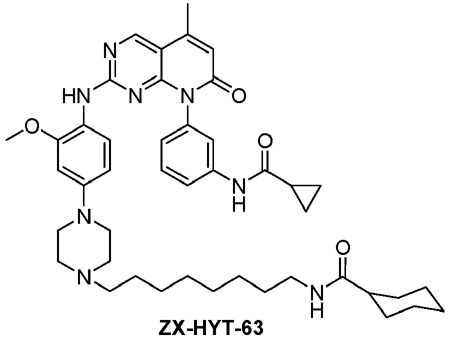

The synthesis method of compound **ZX-HYT-63** referred to Embodiment 32. HRMS (ESI) for C₄₄H₅₈N₈O₄ [M+H]⁺: calcd, 763.4654; found, 763.4661. ¹H NMR (400 MHz, DMSO-*d*₆) δ 10.39 (s, 1H), 8.80 (s, 1H), 8.11 (s, 1H), 7.81 (d, *J* = 8.0 Hz, 1H), 7.62 (t, *J* = 5.7 Hz, 1H), 7.55 - 7.41 (m, 2H), 7.28 (d, *J* = 8.9 Hz, 1H), 6.99 - 6.88 (m, 1H), 6.53 (d, *J* = 2.5 Hz, 1H), 6.32 (d, *J* = 1.4 Hz, 1H), 6.02 (s, 1H), 3.79 (s, 3H), 3.12 - 2.94 (m, 6H), 2.49 - 2.41 (m, 4H), 2.33 (s, 3H), 1.94 - 1.87 (m, 3H), 1.69 - 1.62 (m, 2H), 1.61 - 1.52 (m, 11H), 1.52 - 1.44 (m, 4H), 0.88 - 0.75 (m, 10H).

### Embodiment 64: N - ((3S, 5S, 7S) - adamantan-1-yl) -4- (4- ((8- (3-(cyclopropaneformamido) phenyl) -5-methyl-7-oxy-7,8-dihydropyridino [2,3-d] pyrimidin-2-yl) amino) -3-methoxyphenyl) piperazin-1-formamide (ZX-HYT-64)

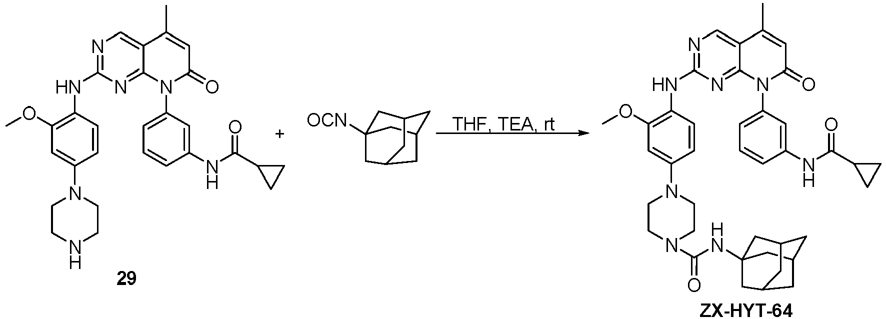

Compound **29** (0.13 mg, 0.25 mmol), 1-isocyandiamondane (44 mg, 0.25 mmol), and triethylamine (38 mg, 0.38 mmol) were placed in anhydrous ethanol (5 mL) and stirred the reaction solution at 25 ° C for 1 hour. Detected by TLC, and after the reaction was complete, the reaction solution was evaporated to dryness. The residue was purified by column chromatography (elution with chloroform/methanol=40:1) to obtain a yellow powdered target compound **ZX-HYT-64** (0.17 g, yield 97%). HRMS (ESI) for C₄₀H₄₆N₈O₄ [M+H]⁺: calcd, 703.3715; found, 703.3721. ¹H NMR (400 MHz, DMSO-*d*₆) δ 10.38 (s, 1H), 8.80 (s, 1H), 8.11 (s, 1H), 7.87 - 7.76 (m, 1H), 7.52 - 7.42 (m, 2H), 7.28 (d, *J* = 8.9 Hz, 1H), 6.98 - 6.85 (m, 1H), 6.61 - 6.52 (m, 1H), 6.32 (s, 1H), 6.04 (s, 1H), 5.77 (s, 1H), 3.80 (s, 3H), 3.45 - 3.36 (m, 4H), 3.02 - 2.93 (m, 4H), 2.46 (s, 3H), 2.05 - 1.98 (m, 3H), 1.98 - 1.91 (m, 6H), 1.82 - 1.74 (m, 1H), 1.67 - 1.57 (m, 6H), 0.82 - 0.75 (m, 4H).

### Embodiment 65: N-(3-(2-((4-(4-((3R, 5R, 7R) - adamantan-1-carbonyl) piperazin-1-yl) -2-methoxyphenyl) amino) -5-methyl-7-oxypyridino [2,3-d] pyrimidin-8 (7H) - yl) phenyl) cyclopropane formamide (ZX-HYT-65)

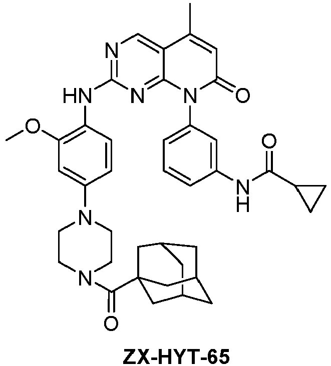

The synthesis method of compound **ZX-HYT-65** referred to Embodiment 32. HRMS (ESI) for C₄₀H₄₅N₇O₄ [M+H]⁺: calcd, 688.3606; found, 688.3611. HRMS (ESI) for C₄₀H₄₆N₈O₄ [M+H]⁺: calcd, 703.3715; found, 703.3723. ¹H NMR (600 MHz, DMSO-*d*₆) δ 10.39 (s, 1H), 8.81 (s, 1H), 8.13 (s, 1H), 7.81 (s, 1H), 7.53 - 7.43 (m, 2H), 7.29 (d, *J* = 8.8 Hz, 1H), 6.98 - 6.89 (m, 1H), 6.62 - 6.52 (m, 1H), 6.33 (s, 1H), 6.03 (s, 1H), 3.80 (s, 3H), 3.77 - 3.69 (m, 4H), 3.08 - 2.96 (m, 4H), 2.46 (s, 3H), 2.01 (s, 3H), 1.98 - 1.90 (m, 6H), 1.78 (p, *J* = 6.2, 5.4 Hz, 1H), 1.76 - 1.67 (m, 6H), 0.84 - 0.75 (m, 4H).

### Embodiment 66: 2-((4-(4-(2-((3R, 5R, 7R) - adamantan-1-yl) acetyl) piperazin-1-yl) -2-methoxyphenyl) amino) -5-methyl-8- (3-(4-methyl-2-oxopirazin-1-yl) phenyl) pyridino [2,3-d] pyrimidin-7 (8H) - one (ZX-HYT-66)

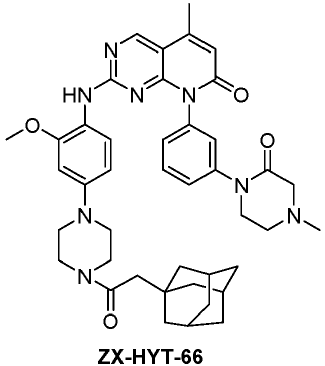

The synthesis method of compound **ZX-HYT-66** referred to Embodiment 18. MS (ESI), *m*/*z:* 731.6 [M+H]⁺. ¹H NMR (400 MHz, Chloroform-*d*) δ 9.07 (s, 1H), 8.48 (s, 1H), 7.87 (t, *J* = 1.5 Hz, 1H), 7.53 (d, *J* = 7.5 Hz, 1H), 7.26 (t, *J* = 7.5 Hz, 1H), 7.09 (dd, *J* = 24.7, 7.5 Hz, 2H), 6.74 (dd, *J* = 7.5, 1.5 Hz, 1H), 6.58 (q, *J* = 0.9 Hz, 1H), 6.30 (d, *J* = 1.5 Hz, 1H), 3.94 - 3.85 (m, 5H), 3.68 (t, *J* = 7.0 Hz, 4H), 3.51 (s, 2H), 3.32 (t, *J* = 7.1 Hz, 4H), 2.69 (t, *J* = 7.1 Hz, 2H), 2.47 (d, *J* = 1.1 Hz, 3H), 2.39 (s, 3H), 2.34 (s, 2H), 2.00 (p, *J* = 7.0 Hz, 3H), 1.69 - 1.58 (m, 12H).

### Embodiment 67: N - (3- (2- (6- (4- (2- ((3R, 5R, 7R) - adamantan-1-yl) acetyl) piperazin-1-yl) -2-methoxypyridin-3-yl)) amino) -5-methyl-7-oxypyridino [2,3-d] pyrimidin-8 (7H) - yl) phenyl) cyclopropane formamide (ZX-HYT-67)

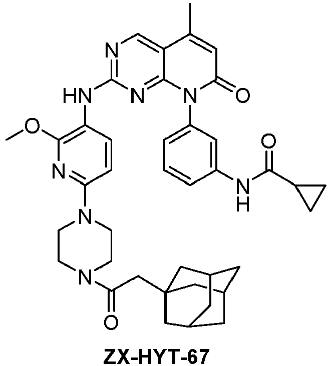

The synthesis method of compound **ZX-HYT-67** referred to Embodiment 32. HRMS (ESI) for C₄₀H₄₆N₈O₄ [M+H]⁺: calcd, 703.3715; found, 703.3723. ¹H NMR (400 MHz, DMSO-*d*₆) δ 10.35 (s, 1H), 8.79 (s, 1H), 8.34 (s, 1H), 7.77 - 7.66 (m, 1H), 7.50 - 7.40 (m, 3H), 6.90 (d, *J* = 7.8 Hz, 1H), 6.30 (s, 1H), 5.86 (s, 1H), 3.80 (s, 3H), 3.66 - 3.53 (m, 4H), 3.42 - 3.36 (m, 4H), 2.45 (s, 3H), 2.16 (s, 2H), 1.97 - 1.89 (m, 3H), 1.82 - 1.73 (m, 1H), 1.70 - 1.57 (m, 12H), 0.83 - 0.70 (m, 4H).

### Embodiment 68: N - ((3S, 5S, 7S) - adamantan-1-yl) -4- (5- ((8- (3-(cyclopropaneformamido) phenyl) -5-methyl-7-oxy-7,8-dihydropyridino [2,3-d] pyrimidin-2-yl) amino) -6-methoxypyridin-2-yl) piperazin-1-formamide (ZX-HYT-68)

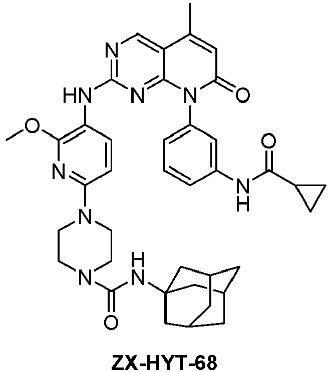

The synthesis method of compound **ZX-HYT-68** referred to Embodiment 65. HRMS (ESI) for C₃₉H₄₅N₉O₄ [M+H]⁺: calcd, 704.3667; found, 704.3653. ¹H NMR (400 MHz, DMSO-*d*₆) δ 10.36 (s, 1H), 8.79 (s, 1H), 8.32 (s, 1H), 7.78 - 7.70 (m, 1H), 7.50 - 7.40 (m, 3H), 6.90 (d, *J* = 7.8 Hz, 1H), 6.30 (s, 1H), 5.83 (s, 1H), 5.76 (s, 1H), 3.80 (s, 3H), 3.45 - 3.35 (m, 8H), 2.45 (s, 3H), 2.05 - 1.98 (m, 3H), 1.98 - 1.90 (m, 6H), 1.82 - 1.74 (m, 1H), 1.68 - 1.56 (m, 6H), 0.83 - 0.73 (m, 4H).

### Embodiment 69: N-(3-(2-((6-(4-((3R, 5R, 7R) - adamantan-1-carbonyl) piperazin-1-yl) -2-methoxypyridin-3-yl) amino) -5-methyl-7-oxypyridino [2,3-d] pyrimidin-8 (7H) - yl) phenyl) cyclopropane formamide (ZX-HYT-69)

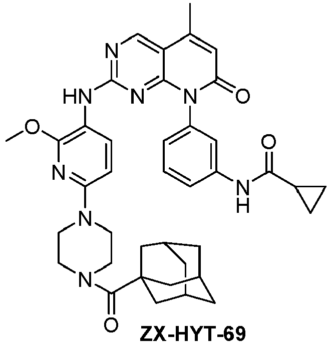

The synthesis method of compound **ZX-HYT-69** referred to Embodiment 32. HRMS (ESI) for C₃₉H₄₄N₈O₄ [M+H]⁺: calcd, 689.3558; found, 689.3562. ¹H NMR (400 MHz, DMSO-*d*₆) δ 10.36 (s, 1H), 8.79 (s, 1H), 8.33 (s, 1H), 7.81 - 7.66 (m, 1H), 7.50 - 7.40 (m, 3H), 6.91 (d, *J* = 7.8 Hz, 1H), 6.31 (s, 1H), 5.86 (s, 1H), 3.81 (s, 3H), 3.75 - 3.65 (m, 4H), 3.33 - 3.33 (m, 4H), 2.45 (s, 3H), 2.05 - 1.98 (m, 3H), 1.98 - 1.91 (m, 6H), 1.81 - 1.63 (m, 7H), 0.84 - 0.71 (m, 4H).

### Embodiment 70: N-(3-(2-((6-(4-(2-((3R, 5R, 7R) - adamantan-1-yl) acetyl) piperazin-1-yl) -4-methoxypyridin-3-yl)) amino) -5-methyl-7-oxypyridino [2,3-d] pyrimidin-8 (7H) - yl) phenyl) cyclopropane formamide (ZX-HYT-70)

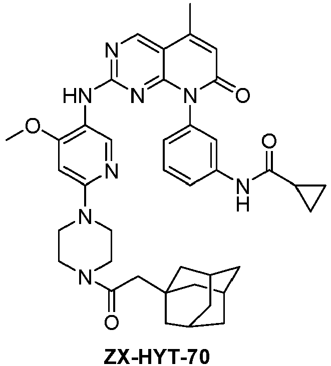

The synthesis method of compound **ZX-HYT-70** referred to Embodiment 32. HRMS (ESI) for C₄₀H₄₆N₈O₄ [M+H]⁺: calcd, 703.3715; found, 703.3723. ¹H NMR (400 MHz, DMSO-*d*₆) δ 10.29 (s, 1H), 8.72 (s, 1H), 8.52 (s, 1H), 7.83 (s, 1H), 7.66 - 7.56 (m, 1H), 7.46 (s, 1H), 7.36 (s, 1H), 6.91 - 6.74 (m, 1H), 6.40 - 6.20 (m, 2H), 3.73 (s, 3H), 3.64 - 3.55 (m, 4H), 3.51 - 3.39 (m, 4H), 2.42 (s, 3H), 2.21 - 2.12 (m, 2H), 1.93 (s, 3H), 1.81 - 1.73 (m, 1H), 1.72 - 1.54 (m, 12H), 0.83 - 0.72 (m, 4H).

### Embodiment 71: N - ((3S, 5S, 7S) - adamantan-1-yl) -4- (5-((8- (3-(cyclopropaneformamido) phenyl) -5-methyl-7-oxy-7,8-dihydropyridino [2,3-d] pyrimidin-2-yl) amino) -4-methoxypyridin-2-yl) piperazin-1-formamide (ZX-HYT-71)

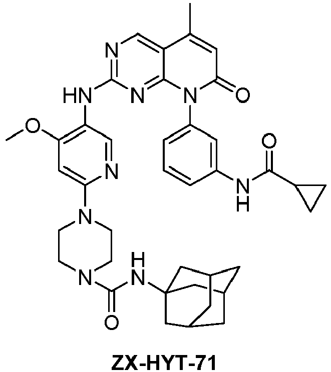

The synthesis method of compound **ZX-HYT-71** referred to Embodiment 65. HRMS (ESI) for C₃₉H₄₅N₉O₄ [M+H]⁺: calcd, 704.3667; found, 704.3653. ¹H NMR (400 MHz, DMSO-*d*₆) δ 10.30 (s, 1H), 8.72 (s, 1H), 8.50 (s, 1H), 7.82 (s, 1H), 7.63 - 7.55 (m, 1H), 7.47 (s, 1H), 7.37 (s, 1H), 6.85 (s, 1H), 6.30 (s, 1H), 6.27 (s, 1H), 5.74 (s, 1H), 3.73 (s, 3H), 3.45 - 3.35 (m, 8H), 2.42 (s, 3H), 2.06 - 1.99 (m, 3H), 1.99 - 1.93 (m, 6H), 1.82 - 1.74 (m, 1H), 1.65 - 1.58 (m, 6H), 0.84 - 0.74 (m, 4H).

### Embodiment 72: N-(3-(2-((6-(4-((3R, 5R, 7R) - adamantan-1-carbonyl) piperazin-1-yl) -4-methoxypyridin-3-yl) amino) -5-methyl-7-oxypyridino [2,3-d] pyrimidin-8 (7H) - yl) phenyl) cyclopropane formamide (ZX-HYT-72)

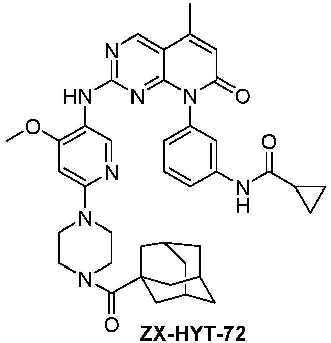

The synthesis method of compound **ZX-HYT-72** referred to Embodiment 32. HRMS (ESI) for C₃₉H₄₄N₈O₄ [M+H]⁺: calcd, 689.3558; found, 689.3562. ¹H NMR (400 MHz, DMSO-*d*₆) δ 10.29 (s, 1H), 8.72 (s, 1H), 8.52 (s, 1H), 7.84 (s, 1H), 7.66 - 7.54 (m, 1H), 7.46 (s, 1H), 7.37 (s, 1H), 6.84 (s, 1H), 6.34 - 6.24 (m, 2H), 3.78 - 3.65 (m, 7H), 3.49 - 3.39 (m, 4H), 2.42 (s, 3H), 2.05 - 1.88 (m, 9H), 1.80 - 1.63 (m, 7H), 0.82 - 0.73 (m, 4H).

### Embodiment 73: N-(3-(2-((4-((2-(2-((3R, 5R, 7R) - adamantan-1-yl) - N-methylacetamido) ethyl) (methyl) amino) -2-methoxyphenyl)) amino) -5-methyl-7-oxypyridino [2,3-d] pyrimidin-8 (7H) - yl) phenyl) cyclopropaneformamide (ZX-HYT-73)

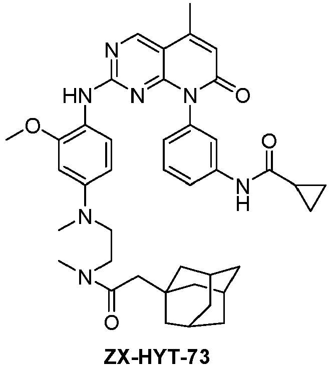

The synthesis method of compound **ZX-HYT-73** referred to Embodiment 32. HRMS (ESI) for C₄₁H₄₉N₇O₄ [M+H]⁺: calcd, 704.3919; found, 704.3923. ¹H NMR (400 MHz, DMSO-*d*₆) δ 10.35 (s, 1H), 8.79 (s, 1H), 8.34 (s, 1H), 7.77 - 7.66 (m, 1H), 7.50 - 7.40 (m, 3H), 6.90 (d, *J* = 7.8 Hz, 1H), 6.30 (s, 1H), 5.86 (s, 1H), 3.80 (s, 3H), 3.67 (s, 3H), 3.42 - 3.36 (m, 4H), 3.16 (s, 3H), 2.45 (s, 3H), 2.16 (s, 2H), 1.97 - 1.89 (m, 3H), 1.82 - 1.72 (m, 1H), 1.71 - 1.58 (m, 12H), 0.85 - 0.73 (m, 4H).

### Embodiment 74: 2-((4-(4-(2-((3R,5R,7R)- adamantan-1-yl) acetyl) piperazin-1-yl) -2-methoxyphenyl) amino) -5-methyl-8- (3- (4-methylpiperazin-1-yl) methyl) phenyl) pyridino [2,3-d] pyrimidin-7 (8H) - ketone (ZX-HYT-74)

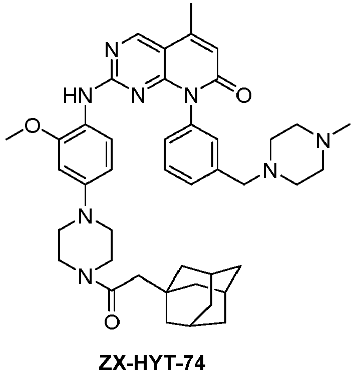

The synthesis method of compound **ZX-HYT-74** referred to Embodiment 18. MS (ESI), *m*/*z:* 730.9 [M+H]⁺. ¹H NMR (500 MHz, Chloroform-*d*) δ 9.07 (s, 1H), 8.49 (s, 1H), 7.81 (p, *J* = 1.3 Hz, 1H), 7.42 (dt, *J* = 7.5, 1.5 Hz, 1H), 7.34 (t, *J* = 7.5 Hz, 1H), 7.13 (dddd, *J* = 7.5, 2.6, 1.7, 1.1 Hz, 1H), 7.07 (d, *J* = 7.5 Hz, 1H), 6.74 (dd, *J* = 7.5, 1.5 Hz, 1H), 6.58 (q, *J* = 0.9 Hz, 1H), 6.30 (d, *J* = 1.5 Hz, 1H), 3.87 (s, 3H), 3.68 (t, *J* = 7.0 Hz, 4H), 3.54 (t, *J* = 1.0 Hz, 2H), 3.32 (t, *J* = 7.1 Hz, 4H), 3.02 (t, *J* = 7.1 Hz, 4H), 2.49 - 2.43 (m, 7H), 2.33 (d, *J* = 8.8 Hz, 5H), 2.00 (s, 3H), 1.67 - 1.57 (m, 12H).

### Embodiment 75: 2-((4-(4-(2-((3R,5R,7R)-adamantan-1-yl) acetyl) piperazin-1-yl) -2-methoxyphenyl) amino) -8- (4-fluoro-3- ((4-methylpiperazin-1-yl) methyl) phenyl) -5-methylpyridino [2,3-d] pyrimidin-7 (8H) - ketone (ZX-HYT-75)

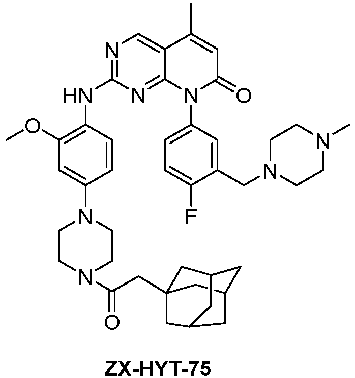

The synthesis method of compound **ZX-HYT-75** referred to Embodiment 18. MS (ESI), *m*/*z:* 748.9 [M+H]⁺. ¹H NMR (500 MHz, Chloroform-*d*) δ 9.07 (s, 1H), 8.78 (s, 1H), 7.85 (dq, *J* = 4.9, 1.1 Hz, 1H), 7.41(ddd, *J* = 7.5, 4.9, 1.5 Hz, 1H), 7.27 - 7.18 (m, 1H), 7.07 (d, *J* = 7.5 Hz, 1H), 6.74 (dd, *J* = 7.5, 1.5 Hz, 1H), 6.58 (q, *J* = 0.9 Hz, 1H), 6.30 (d, *J* = 1.5 Hz, 1H), 3.89 (s, 3H), 3.67 (t, *J* = 7.0 Hz, 4H), 3.61 (d, *J* = 1.1 Hz, 2H), 3.31 (t, *J* = 7.1 Hz, 4H), 2.59 (td, *J* = 6.9, 0.8 Hz, 4H), 2.48 - 2.42 (m, 7H), 2.33 (d, *J* = 9.0 Hz, 5H), 2.10 (s, 3H), 1.69 - 1.51 (m, 12H).

### Embodiment 76: 2-((4-(4-(2-((3R,5R,7R)-adamantan-1-yl) acetyl) piperazin-1-yl) -2-methoxyphenyl) amino) -5-methyl-8- (3- (4-methylpiperazin-1-yl) methyl) -4- (trifluoromethyl) phenyl) pyridino [2,3-d] pyrimidin-7 (8H) - ketone (ZX-HYT-76)

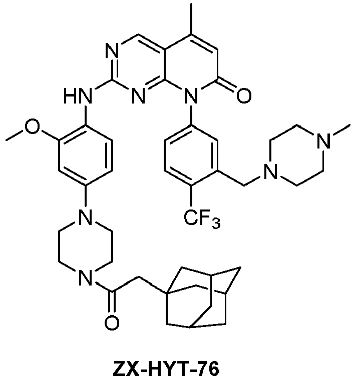

The synthesis method of compound **ZX-HYT-76** referred to Embodiment 18. MS (ESI), *m*/*z:* 798.9 [M+H]⁺. ¹H NMR (500 MHz, Chloroform-*d*) δ 9.07 (s, 1H), 8.49 (s, 1H), 7.60 - 7.54 (m, 2H), 7.42 (dd, *J* = 7.4, 1.5 Hz, 1H), 7.07 (d, *J* = 7.5 Hz, 1H), 6.74 (dd, *J* = 7.5, 1.5 Hz, 1H), 6.58 (q, *J* = 0.9 Hz, 1H), 6.30 (d, *J* = 1.5 Hz, 1H), 3.89 (s, 2H), 3.81 - 3.64 (m, 6H), 3.31 (t, *J* = 7.1 Hz, 4H), 2.67 (td, *J* = 7.0, 0.8 Hz, 4H), 2.49 - 2.42 (m, 7H), 2.34 (d, *J =* 10.2 Hz, 4H), 2.16 (s, 3H), 1.67 - 1.57 (m, 12H).

### Embodiment 77: 3-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) - N - (1-methylazacyclobutane-3-yl) benzamide (ZX-HYT-77)

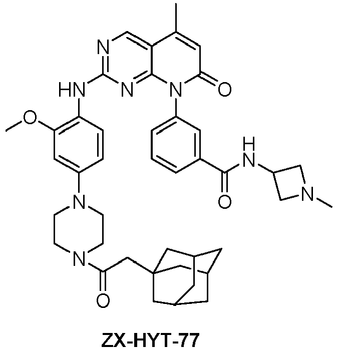

The synthesis method of compound **ZX-HYT-77** referred to Embodiment 18. MS (ESI), *m*/*z:* 730.9 [M+H]⁺. ¹H NMR (500 MHz, Chloroform-*d*) δ 9.09 (s, 1H), 8.68 (s, 1H), 8.29 (t, *J* = 1.5 Hz, 1H), 7.79 (d, *J* = 9.5 Hz, 1H), 7.71 (dt, *J* = 7.3, 1.5 Hz, 1H), 7.52 (dt, *J* = 7.5, 1.5 Hz, 1H), 7.41 (t, *J* = 7.5 Hz, 1H), 7.07 (d, *J* = 7.5 Hz, 1H), 6.74 (dd, *J* = 7.5, 1.5 Hz, 1H), 6.59 (q, *J* = 1.0 Hz, 1H), 6.30 (d, *J* = 1.5 Hz, 1H), 4.14 (dp, *J* = 9.5, 7.0 Hz, 1H), 3.87 (s, 3H), 3.68 (t, *J* = 7.0 Hz, 4H), 3.32 (t, *J* = 7.1 Hz, 4H), 3.16 (dd, *J* = 12.4, 7.0 Hz, 2H), 2.94 (dd, *J* = 12.4, 7.1 Hz, 2H), 2.47 (d, *J* = 1.1 Hz, 3H), 2.34 (s, 2H), 2.24 (s, 3H), 1.98 (s, 3H), 1.69 - 1.50 (m, 12H).

### Embodiment 78: 4-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) - N - (1-methylazacyclobutane-3-yl) pyridinamide (ZX-HYT-78)

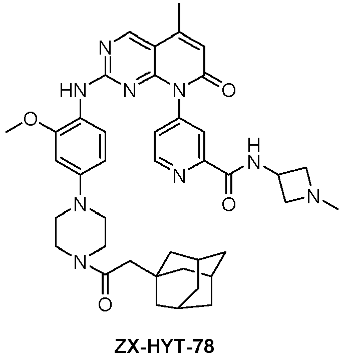

The synthesis method of compound **ZX-HYT-78** referred to Embodiment 18. MS (ESI), *m*/*z:* 732.3 [M+H]⁺. ¹H NMR (500 MHz, Chloroform-*d*) δ 9.07 (s, 1H), 8.75 (d, *J* = 1.5 Hz, 1H), 8.48 (s, 1H), 8.43 (d, *J* = 7.5 Hz, 1H), 8.33 (dd, *J* = 7.4, 1.5 Hz, 1H), 7.99 (d, *J* = 10.3 Hz, 1H), 7.07 (d, *J* = 7.5 Hz, 1H), 6.74 (dd, *J* = 7.5, 1.5 Hz, 1H), 6.58 (q, *J* = 1.0 Hz, 1H), 6.30 (d, *J* = 1.5 Hz, 1H), 4.14 (dp, *J* = 9.5, 7.0 Hz, 1H), 3.87 (s, 3H), 3.68 (t, *J* = 7.0 Hz, 4H), 3.32 (t, *J* = 7.1 Hz, 4H), 3.16 (dd, *J* = 12.4, 7.0 Hz, 2H), 2.94 (dd, *J* = 12.4, 7.1 Hz, 2H), 2.47 (d, *J* = 1.1 Hz, 3H), 2.34 (s, 2H), 2.24 (s, 3H), 1.98 (s, 3H), 1.69 - 1.50 (m, 12H).

### Embodiment 79: 5-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) -2-fluoro-N - (1-methylazacyclobutane-3-yl) benzamide (ZX-HYT-79)

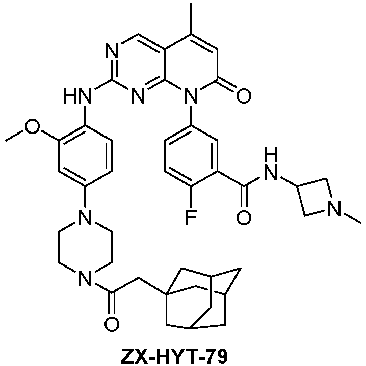

The synthesis method of compound **ZX-HYT-79** referred to Embodiment 18. MS (ESI), *m*/*z:* 749.3 [M+H]⁺. ¹H NMR (600 MHz, Chloroform-*d*) δ 8.65 (s, 1H), 8.01 (dd, *J* = 6.9, 2.7 Hz, 1H), 7.76 (s, 1H), 7.45 - 7.27 (m, 3H), 6.81 (dd, *J* = 12.2, 3.2 Hz, 1H), 6.44 (s, 1H), 6.37 (s, 1H), 6.05 (s, 1H), 3.84 (s, 3H), 3.82 - 3.78 (m, 4H), 3.72 - 3.67 (m, 4H), 3.51 - 3.16 (m, 4H), 2.93 (tq, *J* = 7.2, 3.6 Hz, 1H), 2.62 (s, 3H), 2.46 (s, 3H), 2.20 (s, 2H), 2.00 - 1.97 (m, 3H), 1.72 - 1.65 (m, 12H).

### Embodiment 80: 5-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) - N - (1-methylazacyclobutane-3-yl) -2- (trifluoromethyl) benzamide (ZX-HYT-80)

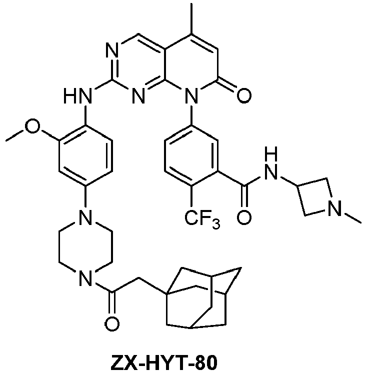

The synthesis method of compound **ZX-HYT-80** referred to Embodiment 18. MS (ESI), *m*/*z:* 799.3 [M+H]⁺. ¹H NMR (400 MHz, Chloroform-*d*) δ 9.07 (s, 1H), 8.48 (s, 1H), 8.02 (d, *J* = 1.5 Hz, 1H), 7.67 (d, *J* = 7.4 Hz, 1H), 7.56 (dd, *J* = 7.5, 1.6 Hz, 1H), 7.32 (d, *J* = 9.7 Hz, 1H), 7.07 (d, *J* = 7.5 Hz, 1H), 6.74 (dd, *J* = 7.5, 1.5 Hz, 1H), 6.57 (q, *J* = 0.9 Hz, 1H), 6.30 (d, *J* = 1.5 Hz, 1H), 4.31 - 4.14 (m, 1H), 3.86 (s, 2H), 3.68 (t, *J* = 7.0 Hz, 4H), 3.32 (t, *J* = 7.1 Hz, 4H), 3.17 (dd, *J =* 12.5, 7.0 Hz, 2H), 2.95 (dd, *J* = 12.4, 7.0 Hz, 2H), 2.47 (d, *J* = 1.1 Hz, 3H), 2.34 -2.25 (m, 4H), 2.12 (s, 3H), 1.7 - 1.57 (m, 12H).

### Embodiment 81: N-(3-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) phenyl) -1-aminocyclopropane-1-formamide (ZX-HYT-81)

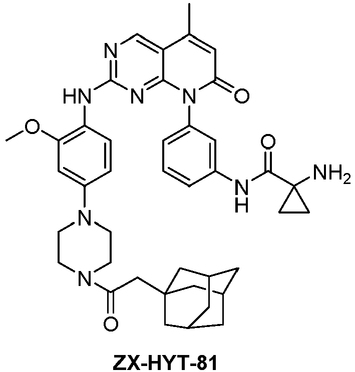

The synthesis method of compound **ZX-HYT-81** referred to Embodiment 18. MS (ESI), *m*/*z:* 717.3 [M+H]⁺. ¹H NMR (400 MHz, DMSO-*d*₆) δ 10.19 (s, 1H), 8.80 (s, 1H), 8.13 (s, 1H), 7.87 (s, 1H), 7.60 (s, 1H), 7.48 (t, *J* = 8.1 Hz, 1H), 7.28 (d, *J* = 8.8 Hz, 1H), 6.95 (d, *J* = 7.8 Hz, 1H), 6.57 (s, 1H), 6.32 (s, 1H), 6.06 (s, 1H), 3.79 (s, 3H), 3.70 - 3.55 (m, 4H), 3.11 - 2.92 (m, 4H), 2.46 (s, 3H), 2.16 (s, 2H), 1.93 (s, 3H), 1.74 - 1.50 (m, 12H), 1.21 - 1.09 (m, 2H), 0.93 - 0.80 (m, 2H).

### Embodiment 82: N-(3-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) phenyl) -2-aminoacetamide (ZX-HYT-82)

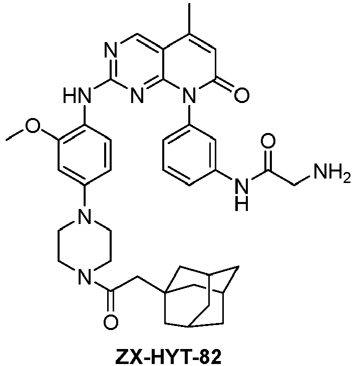

The synthesis method of compound **ZX-HYT-82** referred to Embodiment 18. MS (ESI), *m*/*z:* 691.3 [M+H]⁺. ¹H NMR (400 MHz, DMSO-*d*₆) δ 10.19 (s, 1H), 8.81 (s, 1H), 8.11 (s, 1H), 7.87 (s, 1H), 7.60 (s, 1H), 7.48 (t, *J* = 8.1 Hz, 1H), 7.28 (d, *J* = 8.8 Hz, 1H), 6.95 (d, *J* = 7.8 Hz, 1H), 6.57 (s, 1H), 6.32 (s, 1H), 6.06 (s, 1H), 4.12 (s, 2H), 3.79 (s, 3H), 3.70 - 3.55 (m, 4H), 3.11 - 2.92 (m, 4H), 2.46 (s, 3H), 2.16 (s, 2H), 1.93 (s, 3H), 1.74 - 1.50 (m, 12H).

### Embodiment 83: N-(3-(2-((4-(4-(((3R,5R,7R)- adamantan-1-yl) methyl) piperazin-1-yl) -2-methoxyphenyl) amino) -5-methyl) -7-oxopyridino [2,3-d] pyrimidin-8 (7H) - yl) phenyl) cyclopropane formamide (ZX-HYT-83)

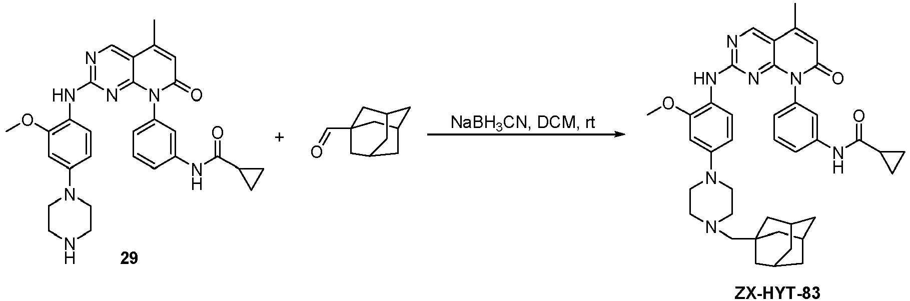

Compound **29** (0.13 g, 0.25 mmol), 1-adamantane formaldehyde (0.041 g, 0.025 mmol), and sodium cyanide borohydride (0.032 g, 0.5 mmol) were sequentially added to 10 mL of dichloromethane and stirred at room temperature for 2 hours. Detected by TLC, after the reaction was complete, the reaction solution was evaporated to drynes. The remaining solid residue was purified by column chromatography (chloroform/methanol=40:1 elution) to obtain a yellow powdered target compound **ZX-HYT-83** (0.11 g, yield 65%). HRMS (ESI) for C₄₀H₄₇N₇O₃ [M+H]⁺: calcd, 674.3813; found, 674.3824. ¹H NMR (400 MHz, DMSO-*d*₆) δ 10.49 (s, 1H), 8.82 (s, 1H), 8.24 (s, 1H), 7.81 (s, 1H), 7.64 - 7.43 (m, 2H), 7.29 (d, *J* = 8.8 Hz, 1H), 6.98 - 6.87 (m, 1H), 6.66 - 6.50 (m, 1H), 6.33 (s, 1H), 6.03 (s, 1H), 3.80 (s, 3H), 3.70 - 3.57 (m, 4H), 3.09 - 2.94 (m, 4H), 2.46 (s, 3H), 2.16 (s, 2H), 1.94 (s, 3H), 1.79 (p, *J* = 6.3 Hz, 1H), 1.72 - 1.61 (m, 12H), 0.87 - 0.71 (m, 4H).

### Embodiment 84: N-(3-(2-((4-(4-(2-((3R,5R,7R)- adamantan-1-yl) ethyl) piperazin-1-yl) -2-methoxyphenyl) amino) -5-methyl-7-oxypyridino [2,3-d] pyrimidin-8 (7H) - yl) phenyl) cyclopropane formamide (ZX-HYT-84)

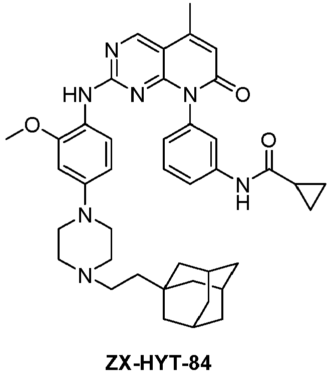

The synthesis method of compound **ZX-HYT-84** referred to Embodiment 83. HRMS (ESI) for C₄₁H₄₉N₇O₃ [M+H]⁺: calcd, 688.3970; found, 688.3981. ¹H NMR (400 MHz, DMSO-*d*₆) δ 10.49 (s, 1H), 8.82 (s, 1H), 8.24 (s, 1H), 7.81 (s, 1H), 7.64 - 7.43 (m, 2H), 7.29 (d, *J* = 8.8 Hz, 1H), 6.98 - 6.87 (m, 1H), 6.66 - 6.50 (m, 1H), 6.33 (s, 1H), 6.03 (s, 1H), 3.80 (s, 3H), 3.70 - 3.57 (m, 4H), 3.09 - 2.94 (m, 4H), 2.46 (s, 3H), 2.26 (s, 2H), 1.95 (s, 3H), 1.79 (p, *J* = 6.4 Hz, 1H), 1.72 - 1.63 (m, 14H), 0.88 - 0.70 (m, 4H).

### Embodiment 85: N-(3-(2-((4-(4-(2-((3R,5R,7R) - adamantan-1-yl) amino) -2-methoxyphenyl) amino) -5-methyl-7-oxypyridino [2,3-) d] pyrimidin-8 (7H) - yl) phenyl) cyclopropaneformamide (ZX-HYT-85)

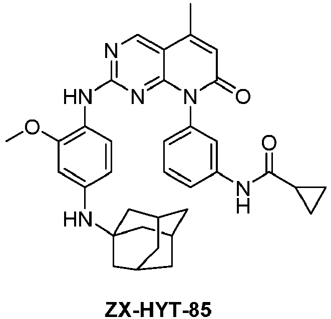

The synthesis method of compound **ZX-HYT-85** referred to Embodiment 25. HRMS (ESI) for C₃₅H₃₈N₆O₃ [M+H]⁺: calcd, 591.7355; found, 591.9362. ¹H NMR (400 MHz, DMSO-*d*₆) δ 10.49 (s, 1H), 8.82 (s, 1H), 8.24 (s, 1H), 7.81 (s, 1H), 7.64 - 7.43 (m, 2H), 7.29 (d, *J* = 8.8 Hz, 1H), 6.98 - 6.87 (m, 1H), 6.66 - 6.50 (m, 1H), 6.33 (s, 1H), 6.03 (s, 1H), 5.83 (s, 1H), 3.80 (s, 3H), 2.46 (s, 3H), 1.95 (s, 3H), 1.79 (p, *J* = 6.4 Hz, 1H), 1.74 - 1.63 (m, 12H), 0.88 - 0.71 (m, 4H).

### Embodiment 86: N-(3-(2-((4-((3R,5R,7R)- adamantan-1-yl) -2-methoxyphenyl) amino) -5-methyl-7-oxypyridino [2,3-d] pyrimidine) -8 (7H) - yl) phenyl) cyclopropaneformamide (ZX-HYT-86)

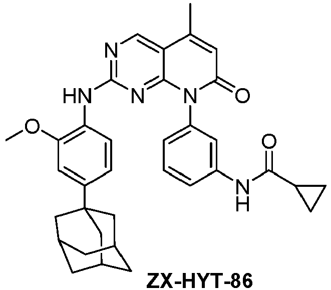

The synthesis method of compound **ZX-HYT-86** referred to Embodiment 25. HRMS (ESI) for C₃₅H₃₇N₅O₃ [M+H]⁺: calcd, 576.2969; found, 576.2971. ¹H NMR (400 MHz, DMSO-*d*₆) δ 10.49 (s, 1H), 8.82 (s, 1H), 8.24 (s, 1H), 7.81 (s, 1H), 7.64 - 7.43 (m, 2H), 7.29 (d, *J* = 8.8 Hz, 1H), 6.98 - 6.87 (m, 1H), 6.66 - 6.50 (m, 1H), 6.33 (s, 1H), 6.03 (s, 1H), 3.80 (s, 3H), 2.46 (s, 3H), 1.95 (s, 3H), 1.79 (p, *J* = 6.4 Hz, 1H), 1.74 - 1.63 (m, 12H), 0.88 - 0.71 (m, 4H).

### Embodiment 87: N - ((3S, 5S, 7S) - adamantan-1-yl) -1- (4-((8- (3-(cyclopropaneformamido) phenyl) -5-methyl-7-oxy-7,8-dihydropyridino [2,3-d] pyrimidin-2-yl) amino) -3-methoxyphenyl) azacyclobutane-3-formamide (ZX-HYT-87)

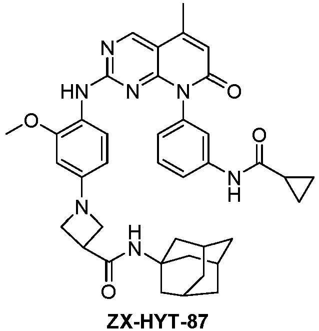

The synthesis method of compound **ZX-HYT-87** referred to Embodiment 25. HRMS (ESI) for C₃₉H₄₃N₇O₄ [M+H]⁺: calcd, 674.3449; found, 674.3452. ¹H NMR (400 MHz, DMSO-*d*₆) δ 10.37 (s, 1H), 8.77 (s, 1H), 8.02 (s, 1H), 7.79 - 7.65 (m, 1H), 7.53 - 7.47 (m, 1H), 7.44 (t, *J* = 8.0 Hz, 1H), 7.16 (d, *J* = 8.7 Hz, 1H), 6.93 (d, *J* = 7.8 Hz, 1H), 6.31 - 6.26 (m, 2H), 5.93 (s, 1H), 5.62 (s, 1H), 3.97 (s, 3H), 3.77 - 3.59 (m, 4H), 2.44 (s, 3H), 1.93 - 1.86 (m, 3H), 1.81 - 1.73 (m, 2H), 1.68 - 1.49 (m, 12H), 0.83 - 0.70 (m, 4H).

### Embodiment 88: N-(5-(2-((4-(4-(2-((3R,5R,7R) - adamantan-1-yl) acetyl) piperazin-1-yl) -2-methoxyphenyl) amino) -5-methyl-7-oxypyridino [2,3-d] pyrimidin-8 (7H) - yl) -2-fluorophenyl) -2-aminoacetamide (ZX-HYT-88)

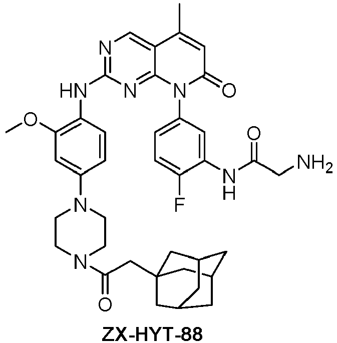

The synthesis method of compound **ZX-HYT-88** referred to Embodiment 18. MS (ESI), *m*/*z:* 709.8 [M+H]⁺. ¹H NMR (400 MHz, DMSO-*d*₆) δ 10.19 (s, 1H), 8.91 (s, 1H), 8.18 (s, 1H), 7.87 (s, 1H), 7.60 (s, 1H), 7.4 (d, *J* = 7.8 Hz, 1H), 6.98 (d, *J* = 7.8 Hz, 1H), 6.57 (s, 1H), 6.32 (s, 1H), 6.06 (s, 1H), 4.12 (s, 2H), 3.79 (s, 3H), 3.70 - 3.55 (m, 4H), 3.11 - 2.92 (m, 4H), 2.46 (s, 3H), 2.16 (s, 2H), 1.93 (s, 3H), 1.74 - 1.50 (m, 12H).

### Embodiment 89: N - (((3R, 5R, 7R) - adamantan-1-yl) methyl) -1- (4- ((8-(3- (cyclopropaneformamido) phenyl) -5-methyl-7-oxy-7,8-dihydropyridino [2,3-d] pyrimidin-2-yl) amino) -3-methoxyphenyl) piperidin-4-formamide (ZX-HYT-89)

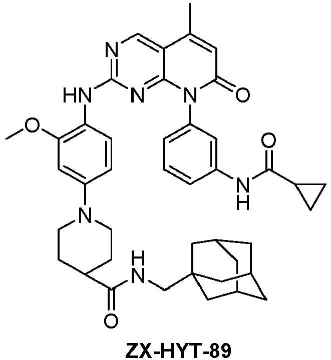

The synthesis method of compound **ZX-HYT-89** referred to Embodiment 25. HRMS (ESI) for C₄₂H₄₉N₇O₄ [M+H]⁺: calcd, 716.3919; found, 716.3921. ¹H NMR (400 MHz, DMSO-*d*₆) δ 10.39 (s, 1H), 8.80 (s, 1H), 8.10 (s, 1H), 7.84 - 7.75 (m, 1H), 7.70 - 7.63 (m, 1H), 7.51 - 7.42 (m, 2H), 7.26 (d, *J* = 8.8 Hz, 1H), 6.92 (d, *J* = 7.9 Hz, 1H), 6.53 (s, 1H), 6.31 (s, 1H), 6.03 (s, 1H), 3.78 (s, 3H), 3.67 - 3.56 (m, 2H), 2.81 - 2.75 (m, 2H), 2.64 - 2.54 (m, 2H), 2.45 (s, 3H), 2.37 - 2.28 (m, 1H), 1.97 - 1.87 (m, 3H), 1.82 - 1.53 (m, 11H), 1.49 - 1.37 (m, 6H), 0.83 - 0.71 (m, 4H).

### Embodiment 90: N-(3-(2-((4-(5-(2-((3R,5R,7R)- adamantan-1-yl) acetyl) -2,5-diazabicyclo) [2.2.2] octane-2-yl) -2-methoxyphenyl) amino) -5-methyl-7-oxypyridino [2,3-d] pyrimidin-8 (7H) - yl) phenyl) cyclopropane formamide (ZX-HYT-90)

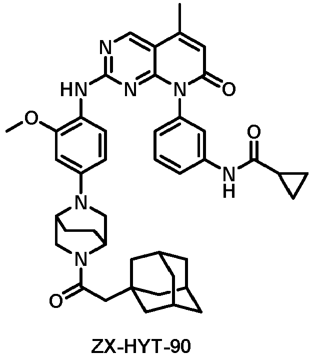

The synthesis method of compound **ZX-HYT-90** referred to Embodiment 25. HRMS (ESI) for C₄₃H₄₉N₇O₄ [M+H]⁺: calcd, 728.9175; found, 728.9182. ¹H NMR (400 MHz, DMSO-*d*₆) δ 10.36 (s, 1H), 8.76 (s, 1H), 8.02 (s, 1H), 7.79 - 7.65 (m, 1H), 7.53 - 7.47 (m, 1H), 7.44 (t, *J* = 8.0 Hz, 1H), 7.16 (d, *J* = 8.7 Hz, 1H), 6.93 (d, *J* = 7.8 Hz, 1H), 6.32 - 6.22 (m, 2H), 5.62 (s, 1H), 3.98 - 3.87 (m, 4H), 3.77 - 3.59 (m, 5H), 3.29 - 3.22 (m, 2H), 2.44 (s, 3H), 1.93 - 1.86 (m, 3H), 1.85 - 1.73 (m, 5H), 1.68 - 1.49 (m, 12H), 0.83 - 0.70 (m, 4H).

### Embodiment 91: N-(3-(2-((4-(6-(2-((3R,5R,7R) - adamantan-1-yl) acetyl) -2,6-diazospira [3.3] hept-2-yl)) -2-methoxyphenyl) amino) -5-methyl-7-oxypyridino [2,3-d] pyrimidin-8 (7H) - yl) phenyl) cyclopropane formamide (ZX-HYT-91)

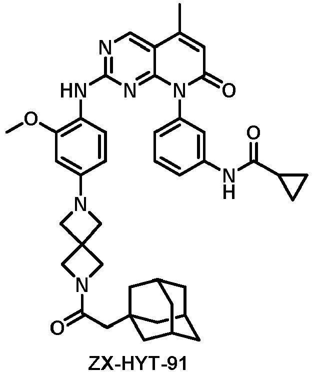

The synthesis method of compound **ZX-HYT-91** referred to Embodiment 25. HRMS (ESI) for C₄₂H₄₇N₇O₄ [M+H]⁺: calcd, 714.3762; found, 714.3774. ¹H NMR (400 MHz, DMSO-*d*₆) δ 10.36 (s, 1H), 8.76 (s, 1H), 8.02 (s, 1H), 7.79 - 7.65 (m, 1H), 7.53 - 7.47 (m, 1H), 7.44 (t, *J* = 8.0 Hz, 1H), 7.16 (d, *J* = 8.7 Hz, 1H), 6.93 (d, *J* = 7.8 Hz, 1H), 6.32 - 6.22 (m, 2H), 5.62 (s, 1H), 3.98 - 3.87 (m, 4H), 3.77 - 3.59 (m, 5H), 3.29 - 3.22 (m, 2H), 2.44 (s, 3H), 1.93 - 1.86 (m, 3H), 1.85 - 1.73 (m, 3H), 1.68 - 1.49 (m, 12H), 0.83 - 0.70 (m, 4H).

### Embodiment 92: N-(3-(2-((4-(2-(2-((3R,5R,7R)- adamantan-1-yl) acetyl) -2,7-) diazospira [3.5] non 7-yl) -2-methoxyphenyl) amino) -5-methyl-7-oxypyridino [2,3-d] pyrimidin-8 (7H) - yl) phenyl) cyclopropane formamide (ZX-HYT-92)

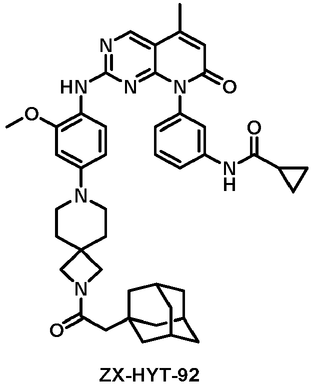

### Embodiment 92: N-(3-(2-((4-(2-(2-((3R,5R,7R)- adamantan-1-yl) acetyl) -2,7-) diazospira [3.5] non 7-yl) -2-methoxyphenyl) amino) -5-methyl-7-oxypyridino [2,3-d] pyrimidin-8 (7H) - yl) phenyl) cyclopropane formamide (ZX-HYT-92)

The synthesis method of compound **ZX-HYT-92** referred to Embodiment 25. HRMS (ESI) for C₄₄H₅₁N₇O₄ [M+H]⁺: calcd, 742.4075; found, 742.4082. ¹H NMR (400 MHz, DMSO-*d*₆) δ 10.36 (s, 1H), 8.76 (s, 1H), 8.02 (s, 1H), 7.79 - 7.65 (m, 1H), 7.53 - 7.47 (m, 1H), 7.44 (t, *J* = 8.0 Hz, 1H), 7.16 (d, *J* = 8.7 Hz, 1H), 6.93 (d, *J* = 7.8 Hz, 1H), 6.32 - 6.22 (m, 2H), 5.62 (s, 1H), 3.98 - 3.87 (m, 7H), 3.77 - 3.59 (m, 4H), 3.29 - 3.22 (m, 2H), 2.44 (s, 3H), 1.93 - 1.86 (m, 3H), 1.85 - 1.73 (m, 5H), 1.68 - 1.49 (m, 12H), 0.83 - 0.70 (m, 4H).

### Embodiment 93: N - (3- (2- ((4- (5- (2- ((3R, 5R, 7R) - adamantan-1-yl) acetyl) hexahydropyrrolo [3,4-c] pyrrole-2 (1H) - yl) -2-methoxyphenyl) amino) -5-methyl-7-oxypyridino [2,3-d] pyrimidin-8 (7H) - yl) phenyl) cyclopropane formamide (ZX-HYT-93)

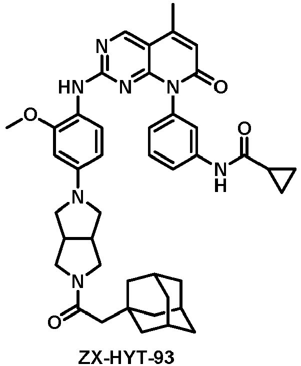

The synthesis method of compound **ZX-HYT-93** referred to Embodiment 25. HRMS (ESI) for C₄₃H₄₉N₇O₄ [M+H]⁺: calcd, 728.3919; found, 728.3919. ¹H NMR (400 MHz, DMSO-*d*₆) δ 10.37 (s, 1H), 8.81 (s, 1H), 8.14 (s, 1H), 7.80 (s, 1H), 7.54 - 7.41 (m, 2H), 7.29 (d, *J* = 8.8 Hz, 1H), 6.98 - 6.87 (m, 1H), 6.66 - 6.50 (m, 1H), 6.33 (s, 1H), 6.03 (s, 1H), 3.80 (s, 3H), 3.70 - 3.57 (m, 4H), 3.09 - 2.94 (m, 4H), 2.46 (s, 3H), 2.16 (s, 2H), 1.94 (s, 3H), 1.81-1.74 (m, 1H), 1.69 - 1.53 (m, 14H), 0.82 - 0.73 (m, 4H).

### Embodiment 94: N-(5-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) -2-fluorophenyl) cyclopropane formamide (ZX-HYT-94)

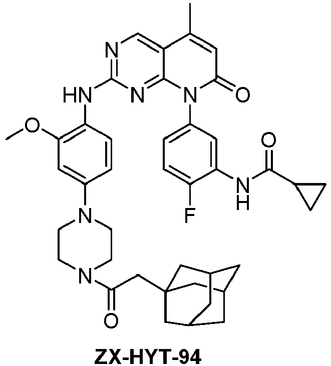

The synthesis method of compound **ZX-HYT-94** referred to Embodiment 20. HRMS (ESI) for C₄₁H₄₆FN₇O₄ [M+H]⁺: calcd, 720.3668; found, 720.3673. ¹H NMR (400 MHz, DMSO-*d*₆) δ 10.49 (s, 1H), 8.82 (s, 1H), 8.51 (s, 1H), 7.83 (s, 1H), 7.67 - 7.56 (m, 1H), 7.42 (s, 1H), 7.31 (s, 1H), 6.91 - 6.74 (m, 1H), 6.40 - 6.20 (m, 2H), 3.73 (s, 3H), 3.64 - 3.55 (m, 4H), 3.51 - 3.39 (m, 4H), 2.42 (s, 3H), 2.21 - 2.12 (m, 2H), 1.93 (s, 3H), 1.81 - 1.73 (m, 1H), 1.72 - 1.54 (m, 12H), 0.83 - 0.72 (m, 4H).

### Embodiment 95: N-(5-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) -2- (trifluoromethyl) phenyl) cyclopropane formamide (ZX-HYT-95)

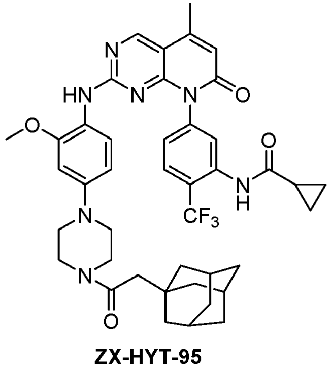

The synthesis method of compound **ZX-HYT-95** referred to Embodiment 20. HRMS (ESI) for C₄₂H₄₆F₃N₇O₄ [M+H]⁺: calcd, 770.3636; found, 770.3641. ¹H NMR (600 MHz, DMSO-*d*₆) δ 10.19 (s, 1H), 8.77 (s, 1H), 8.51 (s, 1H), 7.88 (s, 1H), 7.68 - 7.55 (m, 1H), 7.41 (s, 1H), 7.30 (s, 1H), 6.91 - 6.74 (m, 1H), 6.40 - 6.20 (m, 2H), 3.73 (s, 3H), 3.64 - 3.55 (m, 4H), 3.51 - 3.39 (m, 4H), 2.42 (s, 3H), 2.21 - 2.12 (m, 2H), 1.93 (s, 3H), 1.81 - 1.73 (m, 1H), 1.72 - 1.54 (m, 12H), 0.83 - 0.72 (m, 4H).

### Embodiment 96: N-(4-(2-((4-(4-(2-((3R,5R,7R) - adamantan-1-yl) acetyl) piperazin-1-yl) -2-methoxyphenyl) amino) -5-methyl-7-oxypyridino [2,3-d] pyrimidin-8 (7H) - yl) pyridin-2-yl) cyclopropane formamide (ZX-HYT-96)

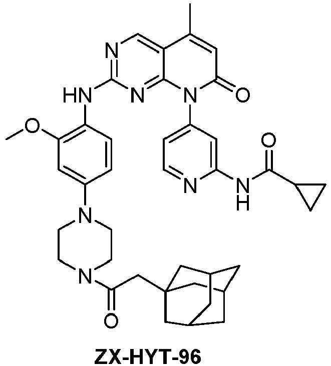

The synthesis method of compound **ZX-HYT-96** referred to Embodiment 20. HRMS (ESI) for C₄₀H₄₆N₈O₄ [M+H]⁺: calcd, 703.3715; found, 703.3723. ¹H NMR (400 MHz, DMSO-*d*₆) δ 10.19 (s, 1H), 8.77 (s, 1H), 8.51 (s, 1H), 7.88 (s, 1H), 7.68 - 7.59 (m, 1H), 7.31 (s, 1H), 7.21 (s, 1H), 6.91 - 6.74 (m, 1H), 6.40 - 6.20 (m, 2H), 3.73 (s, 3H), 3.64 - 3.55 (m, 4H), 3.51 - 3.39 (m, 4H), 2.42 (s, 3H), 2.21 - 2.12 (m, 2H), 1.93 (s, 3H), 1.81 - 1.73 (m, 1H), 1.72 - 1.54 (m, 12H), 0.83 - 0.72 (m, 4H).

### Embodiment 97: 2-((4-(4-(2-((3R,5R,7R)- adamantan-1-yl) acetyl) piperazin-1-yl) -2-methoxyphenyl) amino) -8- (3- ((cyclopropylmethyl) amino) phenyl) -5-methylpyridino [2,3-d] pyrimidin-7 (8H) - ketone (ZX-HYT-97)

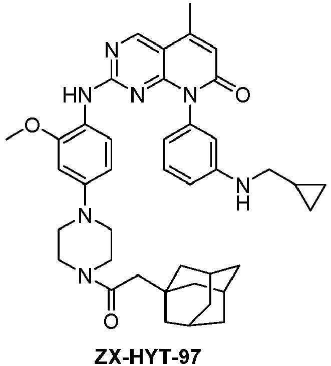

The synthesis method of compound **ZX-HYT-97** referred to Embodiment 18. HRMS (ESI) for C₄₁H₄₉N₇O₃ [M+H]⁺: calcd, 688.3970; found, 688.3965. ¹H NMR (400 MHz, DMSO-*d*₆) δ 10.39 (s, 1H), 8.81 (s, 1H), 8.14 (s, 1H), 7.80 (s, 1H), 7.54 - 7.41 (m, 2H), 7.29 (d, *J* = 8.8 Hz, 1H), 6.98 - 6.87 (m, 1H), 6.66 - 6.50 (m, 1H), 6.33 (s, 1H), 6.03 (s, 1H), 3.80 (s, 3H), 3.70 - 3.57 (m, 4H), 3.24 (dd, *J* = 7.0, 5.9 Hz, 2H), 3.09 - 2.94 (m, 4H), 2.46 (s, 3H), 2.16 (s, 2H), 1.94 (s, 3H), 1.71 - 1.60 (m, 1H), 1.69 - 1.60 (m, 12H), 0.82 - 0.73 (m, 4H).

### Embodiment 98: 2-((4-(4-(2-((3R,5R,7R)- adamantan-1-yl) acetyl) piperazin-1-yl) -2-methoxyphenyl) amino) -5-methyl-8- (3-morpholinophenyl) pyridino [2,3-d] pyrimidin-7 (8H) - ketone (ZX-HYT-98)

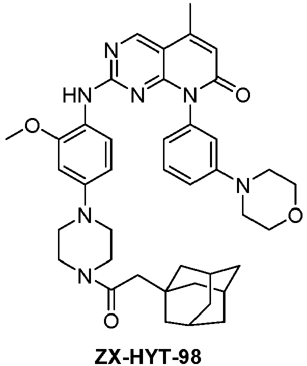

The synthesis method of compound **ZX-HYT-98** referred to Embodiment 18. HRMS (ESI) for C₄₃H₅₄N₈O₃ [M+H]⁺: calcd, 731.4392; found, 731.4395. ¹H NMR (500 MHz, DMSO-*d*₆) δ 8.77 (s, 1H), 8.09 (s, 1H), 7.37 (t, *J* = 8.0 Hz, 1H), 7.29 (d, *J* = 8.8 Hz, 1H), 7.07 (d, *J* = 8.5 Hz, 1H), 6.85 (d, *J* = 2.3 Hz, 1H), 6.69 - 6.63 (m, 1H), 6.57 (d, *J* = 2.5 Hz, 1H), 6.29 (s, 1H), 5.99 (s, 1H), 3.77 (s, 3H), 3.68 (t, *J* = 4.9 Hz, 4H), 3.62 (dq, *J* = 11.0, 5.4, 5.0 Hz, 4H), 3.10 (q, *J* = 5.0 Hz, 4H), 3.04 - 2.94 (m, 4H), 2.43 (s, 3H), 2.14 (t, *J* = 4.5 Hz, 2H), 1.91 (s, 3H), 1.67 - 1.54 (m, 12H).

### Embodiment 99: N-(3-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) -5- (trifluoromethyl) phenyl) -2-aminoacetamide (ZX-HYT-99)

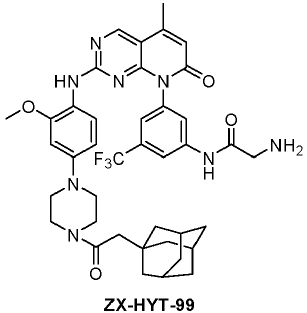

The synthesis method of compound **ZX-HYT-99** referred to Embodiment 18. MS (ESI), *m*/*z:* 759.8 [M+H]⁺. ¹H NMR (400 MHz, DMSO-*d*₆) δ 10.19 (s, 1H), 8.91 (s, 1H), 8.18 (s, 1H), 7.90 (s, 1H), 7.78 (s, 1H), 7.4 (d, *J* = 7.8 Hz, 1H), 6.98 (d, *J* = 7.8 Hz, 1H), 6.57 (s, 1H), 6.32 (s, 1H), 6.06 (s, 1H), 4.12 (s, 2H), 3.79 (s, 3H), 3.70 - 3.55 (m, 4H), 3.11 - 2.92 (m, 4H), 2.46 (s, 3H), 2.16 (s, 2H), 1.93 (s, 3H), 1.74 - 1.50 (m, 12H).

### Embodiment 100: 5-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) - N-cyclopropyl-2-fluorobenzamide (ZX-HYT-100)

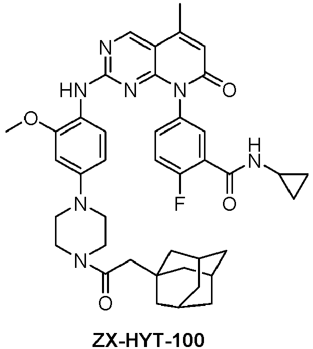

The synthesis method of compound **ZX-HYT-100** referred to Embodiment 18. MS (ESI), *m*/*z:* 720.4 [M+H]⁺. ¹H NMR (600 MHz, Chloroform-*d*) δ 8.65 (s, 1H), 8.01 (dd, *J* = 6.9, 2.7 Hz, 1H), 7.76 (s, 1H), 7.45 - 7.27 (m, 3H), 6.81 (dd, *J* = 12.2, 3.2 Hz, 1H), 6.44 (s, 1H), 6.37 (s, 1H), 6.05 (s, 1H), 3.84 (s, 3H), 3.82 - 3.78 (m, 2H), 3.72 - 3.67 (m, 2H), 3.06 (dt, *J* = 14.9, 4.9 Hz, 4H), 2.93 (tq, *J* = 7.2, 3.6 Hz, 1H), 2.46 (s, 3H), 2.20 (s, 2H), 2.00 - 1.97 (m, 3H), 1.72 - 1.65 (m, 12H), 0.91 - 0.86 (m, 2H), 0.65 - 0.61 (m, 2H).

### Embodiment 101: 8- (3- (1H imidazol-1-yl) phenyl) -2- ((4- (4- (2- ((3R, 5R, 7R) - adamantan-1-yl) acetyl) piperazin-1-yl) -2-methoxyphenyl) amino) -5-methylpyridino [2,3-d] pyrimidin-7 (8H) - one (ZX-HYT-101)

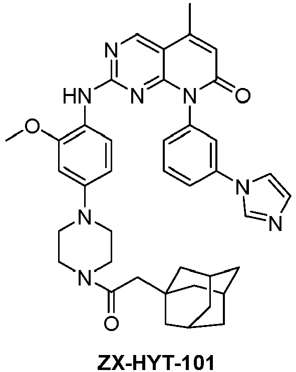

The synthesis method of compound **ZX-HYT-101** referred to Embodiment 18. MS (ESI), *m*/*z:* 685.1 [M+H]⁺. ¹H NMR (400 MHz, DMSO-*d*₆) δ 10.19 (s, 1H), 8.91 (s, 1H), 8.18 (s, 1H), 7.87 (s, 1H), 7.72 (s, 1H), 7.46 - 7.59 (m, 2H),7.4 (d, *J* = 7.8 Hz, 1H), 7.18 (s, 1H), 6.98 (d, *J* = 7.8 Hz, 1H), 6.57 (s, 1H), 6.32 (s, 1H), 6.06 (s, 1H), 3.79 (s, 3H), 3.70 - 3.55 (m, 4H), 3.11 - 2.92 (m, 4H), 2.46 (s, 3H), 2.16 (s, 2H), 1.93 (s, 3H), 1.74 - 1.50 (m, 12H).

### Embodiment 102: 4-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) - N-cyclopropylpyridinamide (ZX-HYT-102)

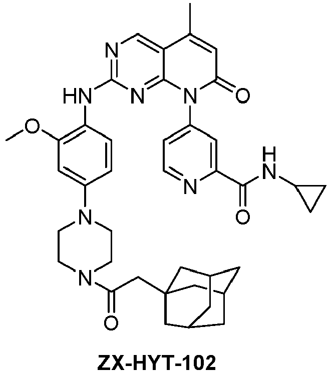

The synthesis method of compound **ZX-HYT-102** referred to Embodiment 18. MS (ESI), *m*/*z:* 703.3 [M+H]⁺. ¹H NMR (600 MHz, Chloroform-*d*) δ 8.72 (d, *J* = 5.1 Hz, 1H), 8.68 (s, 1H), 8.20 (s, 1H), 8.11 (d, *J* = 3.9 Hz, 1H), 7.42 (dd, *J* = 6.1, 4.3 Hz, 1H), 7.26 (s, 1H), 6.43 (s, 1H), 6.37 (s, 1H), 3.84 (s, 3H), 3.81 (t, *J* = 5.2 Hz, 2H), 3.68 (t, *J* = 5.1 Hz, 2H), 3.05 (s, 4H), 2.98 (dq, *J* = 7.2, 3.6 Hz, 1H), 2.48 (s, 3H), 2.20 (s, 2H), 1.99 (s, 3H), 1.73 - 1.65 (m, 12H), 0.94 - 0.89 (m, 2H), 0.72 - 0.67 (m, 2H).

### Embodiment 103: 3-(2-((4-(4-(2-((3R,5R,7R) - adamantan-1-yl) acetyl) piperazin-1-yl) -2-methoxyphenyl) amino) -5-methyl-7-oxypyridino [2,3-d] pyrimidin-8 (7H) - yl) - N-cyclopropylbenzamide (ZX-HYT-103)

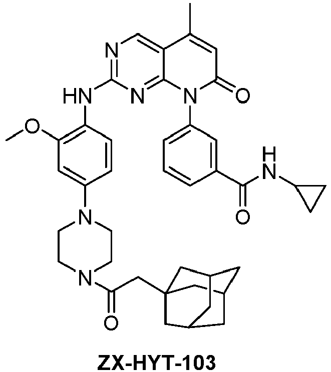

The synthesis method of compound **ZX-HYT-103** referred to Embodiment 18. MS (ESI), *m*/*z:* 702.4 [M+H]⁺. ¹H NMR (500 MHz, Chloroform-*d*) δ 8.63 (s, 1H), 8.10 - 7.97 (m, 1H), 7.83 (s, 1H), 7.64 (t, *J* = 7.8 Hz, 1H), 7.58 (t, *J* = 1.9 Hz, 1H), 7.40 (d, *J* = 7.8 Hz, 1H), 7.26 (s, 1H), 6.50 (d, *J* = 3.2 Hz, 1H), 6.41 (d, *J* = 2.6 Hz, 1H), 6.35 (d, *J* = 1.4 Hz, 1H), 6.02 (s, 1H), 3.83 (s, 3H), 3.82 - 3.76 (m, 2H), 3.73 - 3.64 (m, 2H), 3.12 - 2.99 (m, 4H), 2.84 (tq, *J* = 7.2, 3.7 Hz, 1H), 2.45 (s, 3H), 2.21 (s, 2H), 2.03 - 1.90 (m, 3H), 1.77 - 1.54 (m, 12H), 0.85 - 0.74 (m, 2H), 0.59 - 0.45 (m, 2H).

### Embodiment 104: 5-(2-((4-(4-(2-((3R,5R,7R)- adamantan-1-yl) acetyl) piperazin-1-yl) -2-methoxyphenyl) amino) -5-methyl-7-oxypyridino [2,3-d] pyrimidin-8 (7H) - yl) - N-cyclopropyl-2- (trifluoromethyl) benzamide (ZX-HYT-104)

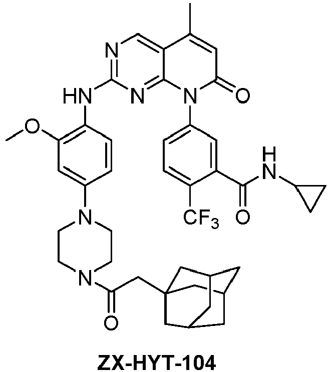

The synthesis method of compound **ZX-HYT-104** referred to Embodiment 18. MS (ESI), *m*/*z:* 770.2 [M+H]⁺. ¹H NMR (600 MHz, Chloroform-*d*) δ 8.66 (s, 1H), 7.90 (d, *J* = 8.2 Hz, 1H), 7.80 (s, 1H), 7.52 - 7.45 (m, 2H), 7.21 (s, 1H), 6.49 - 6.40 (m, 1H), 6.37 (s, 1H), 6.15 (s, 1H), 5.90 (s, 1H), 3.85 (s, 3H), 3.83 - 3.74 (m, 2H), 3.71 - 3.64 (m, 2H), 3.13 - 3.04 (m, 4H), 2.83 (q, *J* = 3.6 Hz, 1H), 2.48 (s, 3H), 2.24 - 2.19 (m, 2H), 1.99 (s, 3H), 1.75 - 1.63 (m, 12H), 0.84 - 0.78 (m, 2H), 0.56 - 0.47 (m, 2H).

### Embodiment 105: Effect of compounds on AKT3 protein levels in H1975OR cells

Cell line: Non-small cell lung cancer H1975-OR cell line, which is an Osimertinib resistant H1975 cell line, and could be obtained through low dose gradual addition culture. Specific method: H1975 cells were spread in a 10 cm culture dish at a convergence rate of 60-70%, added 50 nM of Osimertinib to the culture medium. After the cell state stabilized, the concentration of Osimertinib was gradually double to 3 µM. After genetic detection, the obtained drug-resistant cell line (H1975-OR) showed significantly higher expression of AKT3 compared to the original H1975 cell line.

Conventional Western Blot (immunoblotting) was used for detection, as follows: a certain number of H1975-OR cells were planted on a 96 well plate, adherent cultured in an incubator overnight, and added a certain concentration of compound to act for 24 hours. 1× SDS lysis buffer with protease and phosphatase inhibitors was used to lyse cells. Cell lysates were separated by SDS-PAGE and transferred to PVDF membrane. Then, at room temperature, the PCDF membrane was taken out and immersed in a sealing solution (5% BSA TBS solution containing 0.5% Tween-20) for sealing treatment for 1 hour, and then incubated with a specific primary antibody at 4 °C overnight. The imprints were washed with TBST and incubated with horseradish peroxidase (HRP) labeled secondary antibodies at room temperature for 2 hours. Finally, ECL plus fluorescence detection reagent (Thermo Scientific, Waltham, MA) was used for protein development, and the Amersham Imager 600 system (GE, America) was used for photography. The detection results were processed using ImageJ software to obtain the grayscale value G (Density). The maximum degradation rate (Dmax) of the protein was calculated using the following formula: Dmax=1- (Gmax Gmin)/Gmax × 100%, wherein Gmax=blank control group Density_{(target protein band)}/Density_{(corresponding to GAPDH)}; Gmin was the Density _{(target protein band)}/Density_{(corresponding to GAPDH)} when the maximum degradation value of the target protein was observed in the compound treatment group. The obtained results were presented in Dmax (%), as shown in Table 1.

**Table 1 Ability of compounds to induce degradation of AKT1, AKT2, and AKT3 proteins in H1975-OR cells**

| Compounds No. | Dmax (%) at 1000 nM | | | Compounds No. | Dmax (%) at 1000 nM | | |
|---|---|---|---|---|---|---|---|
| | AKT1 | AKT2 | AKT3 | | AKT1 | AKT2 | AKT3 |
| **ZX-HYT-01** | 59.2 | 27.2 | 67.6 | **ZX-HYT-26** | - | - | - |
| **ZX-HYT-02** | 0 | 0 | 30.8 | **ZX-HYT-27** | 0 | 30.18 | 76.3 |
| **ZX-HYT-03** | 78.9 | 64.8 | 89.1 | **ZX-HYT-28** | - | - | - |
| **ZX-HYT-04** | 0 | 0 | 26.6 | **ZX-HYT-29** | 57.51 | 36.43 | 54.7 |
| **ZX-HYT-05** | 34.4 | 29.9 | 71.9 | **ZX-HYT-30** | 40.53 | 37.13 | 51.9 |
| **ZX-HYT-06** | 0 | 47 | 62.9 | **ZX-HYT-31** | 0 | 30.64 | 55.5 |
| **ZX-HYT-07** | 0 | 0 | 69.2 | **ZX-HYT-32** | 0 | 24.26 | 45.2 |
| **ZX-HYT-08** | 0 | 0 | 66.8 | **ZX-HYT-33** | 0 | 43.73 | 55.3 |
| **ZX-HYT-09** | 0 | 0 | 75.7 | **ZX-HYT-34** | 55.4 | 67.51 | 56.6 |
| **ZX-HYT-10** | 0 | 25.3 | 57.1 | **ZX-HYT-35** | 42.17 | 39.12 | 43.4 |
| **ZX-HYT-11** | 0 | 15.3 | 91.1 | **ZX-HYT-36** | 0 | 0 | 12.3 |
| **ZX-HYT-12** | 56 | 0 | 75.4 | **ZX-HYT-37** | 0 | 0 | 17.5 |
| **ZX-HYT-13** | - | - | - | **ZX-HYT-38** | 0 | 0 | 16.17 |
| **ZX-HYT-14** | - | - | - | **ZX-HYT-39** | 0 | 51 | 55.3 |
| **ZX-HYT-15** | - | - | - | **ZX-HYT-40** | 32 | 30.7 | 53 |
| **ZX-HYT-16** | 0 | 31.37 | 70.3 | **ZX-HYT-43** | 57.5 | 30.5 | 37.9 |
| **ZX-HYT-17** | 46.4 | 45.9 | 76.4 | **ZX-HYT-44** | 22.4 | 23.6 | 40.1 |
| **ZX-HYT-18** | 0 | 0 | 30.6 | **ZX-HYT-45** | 32.8 | 17.6 | 29.1 |
| **ZX-HYT-19** | 0 | 0 | 28 | **ZX-HYT-47** | 0 | 0 | 67.45 |
| **ZX-HYT-20** | - | - | - | **ZX-HYT-48** | 0 | 34.25 | 60.6 |
| **ZX-HYT-21** | 0 | 0 | 6.8 | **ZX-HYT-49** | 0 | 26.13 | 61.6 |
| **ZX-HYT-22** | 0 | 0 | 25.02 | **ZX-HYT-59** | 30.3 | 24.7 | 38.9 |
| **ZX-HYT-23** | 0 | 0 | 41.38 | **ZX-HYT-60** | 13 | 0 | 18.9 |
| **ZX-HYT-24** | 0 | 0 | 51.5 | **ZX-HYT-61** | 14.2 | 31.5 | 15.9 |
| **ZX-HYT-25** | - | - | - | **ZX-HYT-62** | 28.4 | 15.1 | 18.8 |

Embodiment 106: Effect of compound **ZX-HYT-11** on AKT1/2/3 proteins in other tumor cells

Tumor cells (H1975, PC-9, H1299, and A549) was incubated with different concentrations of compound **ZX-HYT-11** for 24 hours, and then analyzed the protein levels of AKT1/2/3 using immunoblotting method as described in Embodiment 105. The results showed (Figure 1) that compound **ZX-HYT-11** could selectively degrade AKT3 protein in the aforementioned cells, without affecting AKT1/2, further demonstrating the effectiveness and universality of this compound in degrading AKT3 protein.

Embodiment 107: Inhibitory activity of compounds on tumor cells proliferation

Tumor cells (see Table 2 to Table 5) were inoculated in 96-well plates in complete culture medium (2000-3000 cells/well). After overnight incubation, the compounds with different concentrations (0.000508 µM - 10 µM) were used to treat the cells separately for 72 hours. CCK-8 (Cell Counting Kit 8, Dojindo Laboratories, Kumamoto, Japan) experiment was used to evaluate cell proliferation. GraphPad Prism 5.0 software (La Jolla, CA) was used to calculate the half-maximal inhibitory concentration (IC50) values by fitting the concentration response curve. Each IC50 value was represented as an average value ± SD. The results were shown in Table 2 to Table 5.

**Table 2: The inhibitory activity of compounds on various tumor cells proliferation**

| Compounds No. | IC₅₀ (µM) | | Compounds No. | IC₅₀ (µM) | |
|---|---|---|---|---|---|
| | **H1975** | **H1975OR** | | **H1975** | **H1975OR** |
| **ZX-HYT-0 1** | 3.27±0.59 8 | 2.014±0.44 4 | **ZX-HYT-2 7** | 7.84±1.35 | 1.67±0.69 |
| **ZX-HYT-0 2** | 0.194±0.0 63 | 0.167±0.02 5 | **ZX-HYT-2 8** | 0.6453 | 0.1775 |
| **ZX-HYT-0 3** | 0.844±0.8 22 | 0.074±0.00 4 | **ZX-HYT-2 9** | 0.98±0.21 | 0.98±0.25 |
| **ZX-HYT-0 4** | 1.768±0.1 87 | 0.083±0.01 9 | **ZX-HYT-3 0** | >10 | 8.84±2.17 |
| **ZX-HYT-0 5** | 5.189±4.5 89 | 0.009±0.00 2 | **ZX-HYT-3 1** | 0.145±0.101 | 0.217±0.036 7 |
| **ZX-HYT-0 6** | 0.837±0.1 1 | 0.125±0.01 2 | **ZX-HYT-3 2** | 1.02±0.24 | 0.21±0.04 |
| **ZX-HYT-0 7** | 5.065±0.8 36 | 0.417±0.08 0 | **ZX-HYT-3 3** | 2.31±0.52 | 0.44±0.14 |
| **ZX-HYT-0 8** | 8.472 | 0.0107±0.0 03 | **ZX-HYT-3 4** | 1.15±0.42 | 0.25±0.12 |
| **ZX-HYT-0 9** | 0.119±0.0 112 | 0.0236±0.0 0351 | **ZX-HYT-3 5** | 2.59±0.2 | 0.32±0.04 |
| **ZX-HYT-1 0** | 0.142±0.0 494 | 0.0196±0.0 0141 | **ZX-HYT-3 6** | 0.219±0.0782 | 0.107±0.008 |
| **ZX-HYT-1 1** | 2.386±2.0 0 | 0.007±0.00 06 | **ZX-HYT-3 7** | 4.149±0.462 | 6.306±0.645 |
| **ZX-HYT-1 2** | 0.554±0.2 13 | 0.136±0.01 65 | **ZX-HYT-3 8** | - | - |
| **ZX-HYT-1 3** | 6.96±6.03 9 | 0.216±0.03 2 | **ZX-HYT-3 9** | 0.009193±0.0 0567 | 0.0102±0.00 4 |
| **ZX-HYT-1 4** | > 10 | > 10 | **ZX-HYT-4 0** | 0.0864±0.029 6 | 0.0609±0.01 4 |
| **ZX-HYT-1 5** | > 10 | > 10 | **ZX-HYT-4 3** | 0.257±0.0423 | 0.172±0.020 4 |
| **ZX-HYT-1 6** | - | - | **ZX-HYT-4 4** | 5.393±0.293 | 8.090±0.432 |
| **ZX-HYT-1 7** | 0.214±0.0 13 | 0.023±0.00 7 | **ZX-HYT-4 5** | 2.403±0.226 | 2.354±0.110 3 |
| **ZX-HYT-1 8** | - | - | **ZX-HYT-4 7** | 0.0365±0.001 2 | 0.0224±0.01 2655 |
| **ZX-HYT-1 9** | 2.293 | 3.465 | **ZX-HYT-4 8** | 0.187±0.0357 | 0.105±0.052 |
| **ZX-HYT-2 0** | - | - | **ZX-HYT-4 9** | 0.0958±0.012 6 | 0.0346±0.21 3 |
| **ZX-HYT-2 1** | - | - | **ZX-HYT-5 9** | 0.121±0.0449 | 0.0939±0.00 4 |
| **ZX-HYT-2 2** | 0.014 | 0.006824 | **ZX-HYT-6 0** | 0.135±0.0948 | 0.107±0.019 4 |
| **ZX-HYT-2 3** | - | - | **ZX-HYT-6 1** | 0.0738±0.045 6 | 0.103±0.059 2 |
| **ZX-HYT-2 4** | 2.671±0.4 71 | 0.734±0.00 594 | **ZX-HYT-6 2** | 0.0551±0.023 2 | 0.0487±0.00 4 |

**Table 3: The inhibitory activity of compounds on PC-9 and other tumor cells proliferation**

| Compounds No. | **IC₅₀ (µM)** | | | |
|---|---|---|---|---|
| | **PC-9** | **A431** | **HCC827** | **H1299** |
| **ZX-HYT-0 1** | 1.568±0.258 | 0.541±0.0589 | 0.141±0.0372 | 10.366±1.717 |
| **ZX-HYT-0 2** | 0.255±0.0394 | 0.102±0.00950 | 0.105±0.00819 | 8.740±1.125 |
| **ZX-HYT-0 3** | 0.0536±0.00753 | 0.0299±0.0028 | 0.0954±0.0331 | 0.876±0.283 |
| **ZX-HYT-0 4** | 0.0181±0.00105 | 0.0102±0.002 | 0.0555±0.0327 | 0.305±0.166 |
| **ZX-HYT-0 5** | 0.00830±0.00033 3 | 0.00806±0.0003 | 0.126±0.0216 | 0.338±0.0855 |
| **ZX-HYT-0 6** | 0.0732±0.00431 | 0.26±0.047 | 0.163±0.0134 | 0.163±0.0134 |
| **ZX-HYT-0 7** | 6.786±3.60 | 0.0839±0.0133 | 0.882±0.0945 | 1.460±0.428 |
| **ZX-HYT-0 8** | 0.109±0.019 | 0.00986±0.0022 | 0.157±0.0679 | 0.212±0.0153 |
| **ZX-HYT-0 9** | 0.215±0.0636 | 0.0106±0.0020 | 0.134±0.0640 | 0.624±0.0448 |
| **ZX-HYT-1 0** | 0.126±0.0177 | 0.0517±0.00 | 0.309±0.139 | 1.81±0.395 |
| **ZX-HYT-1 1** | 0.0839±0.0135 | 0.00930±0.0004 | 0.0932±0.0140 | 0.270±0.0723 |
| **ZX-HYT-1 2** | 0.466±0.0241 | 0.1555±0.0075 | 0.549±0.247 | 2.174±0.419 |
| **ZX-HYT-1 3** | >10 | 2.290±0.0474 | 10.723±3.88 | > 10 |
| **ZX-HYT-1 4** | >10 | > 10 | > 10 | > 10 |
| **ZX-HYT-1 5** | 4.803±0.501 | > 10 | > 10 | > 10 |
| **ZX-HYT-1 7** | 0.0234±0.00578 | 0.0225±0.0035 | 0.186±0.122 | 2.1±0.049 |
| **ZX-HYT-2 0** | 0.2951 | 0.1061 | - | - |
| **ZX-HYT-2 4** | - | 0.212±0.0653 | 1.395±0.248 | - |

**Table 4: The inhibitory activity of compounds on MDA-MB-231 and other tumor cells proliferation**

| Compounds No. | **IC50** (**µM)** | | | | | |
|---|---|---|---|---|---|---|
| | **MDA-MB-231** | **MDA-MB-453** | **BT-549** | **Bel-7402** | **Huh7** | **HCC1937** |
| **ZX-HYT-11** | 0.02935 | >10 | >10 | >10 | 0.6859 | >10 |
| **ZX-HYT-19** | <0.001 | >10 | 0.004209 | 0.041 | - | - |
| **ZX-HYT-50** | 0.1553 | >10 | >10 | >10 | 0.6317 | >10 |
| **ZX-HYT-51** | 0.1449 | >10 | 0.6436 | >3 | >10 | >10 |
| **ZX-HYT-52** | 1.993 | 2.5 | 0.4775 | 0.568 | 1.352 | 0.002-0.005 |
| **ZX-HYT-64** | 0.01515 | >10 | 0.1406 | 0.37 | - | 4 |
| **ZX-HYT-65** | 0.1038 | >10 | >10 | >10 | 0.3351 | >10 |
| **ZX-HYT-67** | - | - | 0.376 | - | >10 | - |
| **ZX-HYT-68** | - | - | 0.03856 | - | >10 | - |
| **ZX-HYT-69** | - | - | 2.996 | - | >10 | - |
| **ZX-HYT-70** | - | - | 1.021 | - | >10 | - |
| **ZX-HYT-71** | - | - | 1.251 | - | >10 | - |
| **ZX-HYT-72** | - | - | 0.6261 | - | 3 | - |
| **ZX-HYT-89** | - | - | 0.02666 | - | 0.12 | - |
| **ZX-HYT-91** | - | - | 0.9371 | - | 3 | - |

**Table 5: The inhibitory activity of compounds on A549 and other tumor cells proliferation**

| Compounds No. | **IC50 (µM**) | | | | | |
|---|---|---|---|---|---|---|
| | **A549** | **HCT116** | **K562** | **MIAPACA-2** | **PANC-1** | **BXPC-3** |
| **ZX-HYT-50** | 0.6682 | 0.01193 | 0.02934 | 0.008645 | 0.01373 | 0.0421 |
| **ZX-HYT-51** | 0.6285 | 0.05528 | 0.2626 | 0.05128 | 0.1248 | 0.3327 |
| **ZX-HYT-52** | 0.8329 | 0.1036 | 0.2501 | 0.07209 | 0.1259 | 0.3033 |
| **ZX-HYT-65** | 1.173 | 0.07291 | 0.2341 | 0.05108 | 0.1786 | 0.447 |

The technical features of the Embodiments above can be combined arbitrarily. To simplify description, all possible combinations of the technical features of the Embodiments above are not described. However, as long as there is no contradiction in the combination of these technical features, they should be considered as falling within the scope of this specification.

The Embodiments above only express several implementations of the present disclosure. The descriptions of the Embodiments are relatively specific and detailed, but may not be construed as the limitation on the patent scope of the present disclosure. It should be noted that a person of ordinary skill in the art may make several variations and improvements without departing from the concept of the present disclosure. These variations and improvements all fall within the protection scope of the present disclosure. Therefore, the patent protection scope of the present disclosure shall be defined by the appended claims.

## Claims

1. Pyridopyrimidine compounds having a structure shown in Formula (I) or their pharmaceutically acceptable salts, or stereoisomers or prodrug molecules, 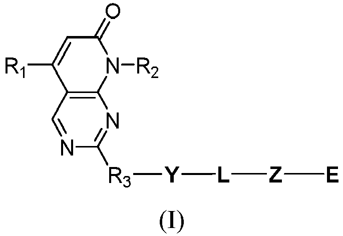 wherein,
E is selected from: H, C₁-C₁₅ alkyl, substituted C₁-C₁₅ alkyl, C₃-C₁₅ cycloalkyl, substituted C₃-C₁₅ cycloalkyl, 3-15 membered heterocycloalkyl, substituted 3-15 membered heterocycloalkyl;
L is absent or is a connecting unit consistingof one or more of the following groups: alkylene, ether, thioether, ester, amine, amide, heteroaryl, cycloalkyl, heterocycloalkyl, -N=N-;
Y is absent or is selected from -O-, -NH-, -NHCO-, -CH₂-, -S-, -CO-;
Z is absent or is selected from: -O-, -NH-, -N(C₁-C₆ alkyl)-, -NHCO-, -CH₂-, -S-, -CO-, -SO-;
R₁ is selected from: H, C₁-C₆ alkyl, halogen or halogen substituted C₁-C₆ alkyl;
R₂ is selected from: C₃-C₁₅ cycloalkyl, substituted C₃-C₁₅ cycloalkyl, 3-15 membered heterocycloalkyl, substituted 3-15 membered heterocycloalkyl, C₆-C₁₀ aryl, substituted C₆-C₁₀ aryl, 5-10 membered heteroaryl, substituted 5-10 membered heteroaryl;
R₃ is 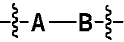 , wherein B is connected to Y through a covalent bond;
A is selected from: -NH-, -NHR-; R is selected from: C₆-C₁₀ aryl, substituted C₆-C₁₀ aryl, 5-10 membered heteroaryl, substituted 5-10 membered heteroaryl;
B is absent or is selected from: C₃-C₁₅ cycloalkyl, substituted C₃-C₁₅ cycloalkyl, 3-15 membered heterocycloalkyl, substituted 3-15 membered heterocycloalkyl, 3-15 membered heterocycloalkyl ketone group, R₈ substituted 3-15 membered heterocycloalkyl ketone group, C₃-C₁₂ cycloalkyl substituted amine group, 3-12 membered heterocycloalkyl substituted amine group,

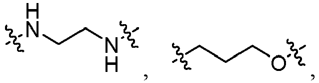

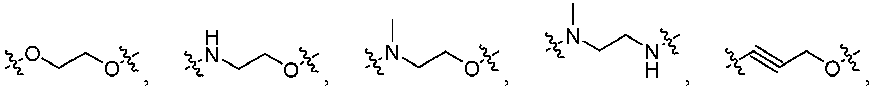

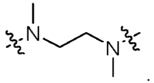

2. The Pyridopyrimidine compounds or their pharmaceutically acceptable salts or stereoisomers or prodrug molecules according to claim 1, wherein E is selected from: H, C₁-C₈ alkyl, R₅ substituted C₁-C₈ alkyl, C₃-C₁₂ cycloalkyl, R₆ substituted C₃-C₁₂ cycloalkyl, 3-12 membered heterocycloalkyl, R₆ substituted 3-12 membered heterocycloalkyl;
R₅ is selected from: halogen, C₃-C₁₂ cycloalkyl, C₁-C₃ alkyl substituted C₃-C₁₂ cycloalkyl, halogen substituted C₃-C₁₂ cycloalkyl;
R₆ is selected from: halogen, C₁-C₆ alkyl, halogen substituted C₁-C₆ alkyl.

3. The Pyridopyrimidine compounds or their pharmaceutically acceptable salts or stereoisomers or prodrug molecules according to claim 2, wherein E is selected from: H, C₁-C₆ alkyl, R₅ substituted C₁-C₆ alkyl, C₃-C₁₀ cycloalkyl, R₆ substituted C₃-C₁₀ cycloalkyl;
R₅ is selected from: halogen, C₆-C₁₀ cycloalkyl, methyl substituted C₆-C₁₀ cycloalkyl, halogen substituted C₆-C₁₀ cycloalkyl;
R₆ is selected from: halogen, C₁-C₃ alkyl.

4. The Pyridopyrimidine compounds or their pharmaceutically acceptable salts or stereoisomers or prodrug molecules according to claim 2, wherein E is selected from: H, cyclopropyl, 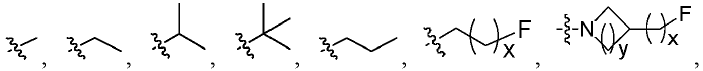 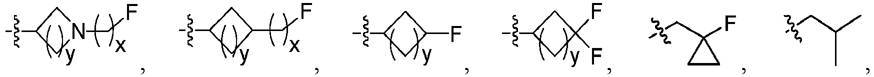 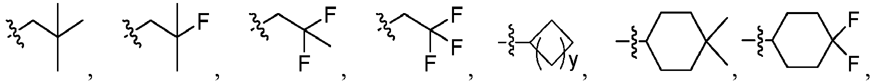 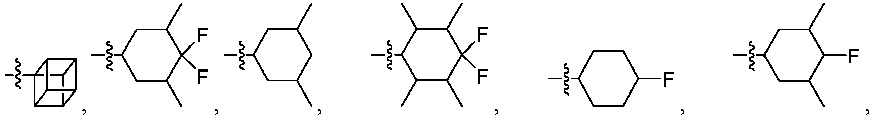 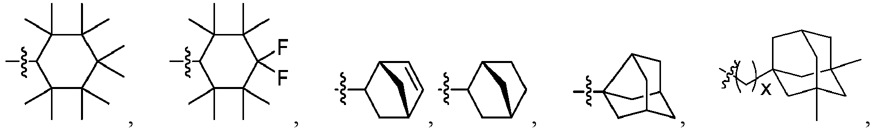 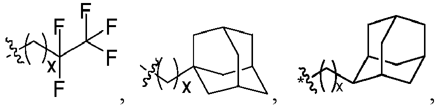 wherein x is an integer from 0 to 3, and y is an integer from 0 to 3.

5. The Pyridopyrimidine compounds or their pharmaceutically acceptable salts or stereoisomers or prodrug molecules according to claim 1, wherein L is selected from: 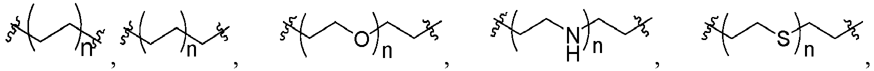 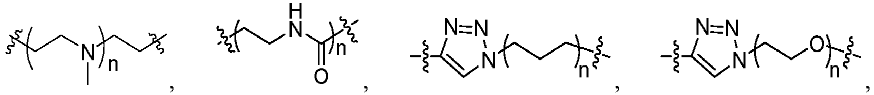 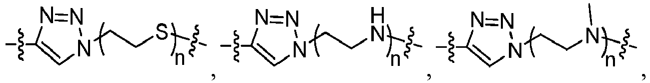 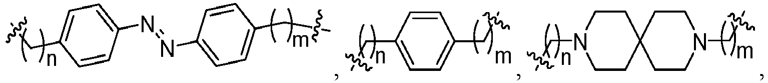 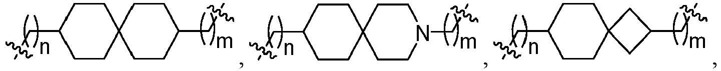 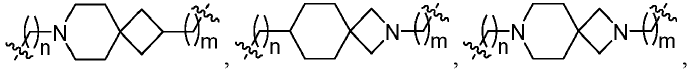 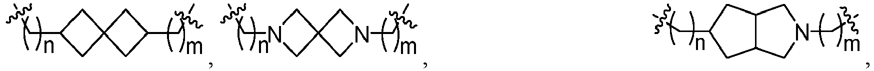 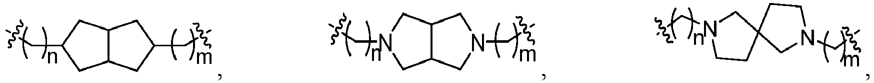 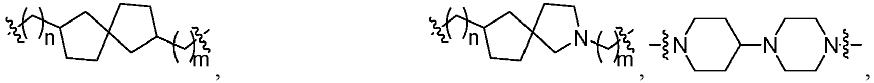 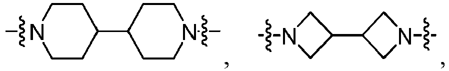 wherein n and m are independently integers from 0 to 14.

6. The Pyridopyrimidine compounds or their pharmaceutically acceptable salts or stereoisomers or prodrug molecules according to claim 5, wherein L is selected from: 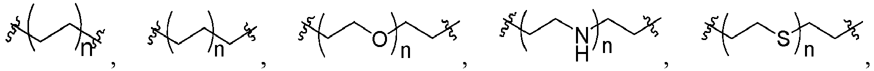 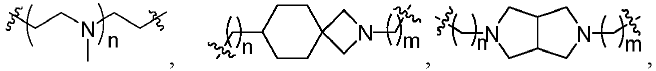 wherein n is an integer from 0 to 7, and m is an integer from 0 to 3.

7. The Pyridopyrimidine compounds or their pharmaceutically acceptable salts or stereoisomers or prodrug molecules according to claim 6, wherein L is selected from: 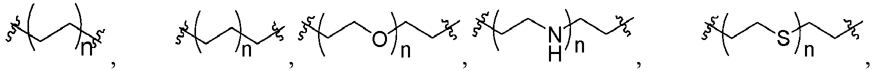 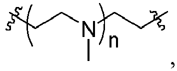 or L is absent, wherein n is an integer from 2 to 7.

8. The Pyridopyrimidine compounds or their pharmaceutically acceptable salts or stereoisomers or prodrug molecules according to claim 1, wherein Y is selected from: -CH₂-, -CO-, -O-, or Y is absent; Z is selected from: -NHCO-, -NH-, or Z is absent.

9. The Pyridopyrimidine compounds or their pharmaceutically acceptable salts or stereoisomers or prodrug molecules according to claim 1, wherein R₁ is selected from: H, halogen, C₁-C₃ alkyl.

10. The Pyridopyrimidine compounds or their pharmaceutically acceptable salts or stereoisomers or prodrug molecules according to claim 1, wherein
R₂ is selected from: C₅-C₁₀ cycloalkyl, R₉ substituted C₅-C₁₀ cycloalkyl, 5-10 membered heterocycloalkyl, R₉ substituted 5-10 membered heterocycloalkyl, C₆-C₁₀ aryl, R₉ substituted C₆-C₁₀ aryl, 5-10 membered heteroaryl, R₉ substituted 5-10 membered heteroaryl, 5-10 membered heteroaryl ketone, and R₉ substituted 5-10 membered heteroaryl ketone;
R₉ is selected from: amino, -N(C₁-C₆ alkyl)₂, halogen, C₁-C₆ alkyl, C₁-C₆ alkoxy, halogen substituted C₁-C₆ alkyl, halogen substituted C₁-C₆ alkoxy, -NH(R₄), -N(R₄)₂, - O(R₄), -C=O-NH(R₄), -C=O-NH(C₃-C₆ cycloalkyl), R₁₀ substituted C₁-C₆ alkyl, C₃-C₁₀ cycloalkyl, 3-10 membered heterocycloalkyl, R₁₀ substituted C₃-C₁₀ cycloalkyl, R₁₀ substituted 3-10 membered heterocycloalkyl, -NH(R₄) Substituted 3-10 membered heterocycloalkyls, 5-10 membered heteroaryl groups, -COR₁₁;
R₄ is absent or is selected from: H, amino, ester, carboxyl, hydroxyl, thiol, sulfone, sulfoxide, C₁-C₁₅ alkyl, R₁₀ substituted C₁-C₁₅ alkyl, C₃-C₁₅ cycloalkyl, 3-15 membered heterocycloalkyl, R₁₀ substituted C₃-C₁₅ cycloalkyl, R₁₀ substituted 3-15 membered heterocycloalkyl, - COR₁₁;
R₁₀ is selected from: C₁-C₆ alkyl, amino substituted C₁-C₆ alkyl, C₃-C₆ cycloalkyl, dimethylamino substituted C₁-C₆ alkyl, 3-6 membered heterocycloalkyl, dimethylamino, C₁-C₃ alkoxy substituted C₁-C₆ alkyl, hydroxyl substituted C₁-C₆ alkyl, C₁-C₆ alkyl substituted 3-6 membered heterocycloalkyl, C₁-C₆ alkyl acyl, hydroxyl, C1-C6 alkyl substituted C₃-C₆ cycloalkyl, dimethylaminoethyl substituted 5-6 membered heterocycloalkyl, C₁-C₆ alkoxy;
R₁₁ is selected from: vinyl, C₁-C₆ alkyl, amino substituted C₁-C₆ alkyl, C₃-C₆ cycloalkyl, amino substituted C₃-C₆ cycloalkyl, halogen substituted C₃-C₆ cycloalkyl, 3-6 membered heterocycloalkyl, C₁-C₆ alkyl substituted 3-6 membered heterocycloalkyl, dimethylamine substituted C₁-C₆ alkyl, and dimethylamine ethyl substituted 5-6 membered heterocycloalkyl.

11. The Pyridopyrimidine compounds or their pharmaceutically acceptable salts or stereoisomers or prodrug molecules according to claim 10, wherein R₂ is selected from: phenyl, 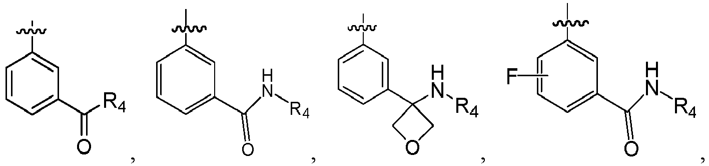 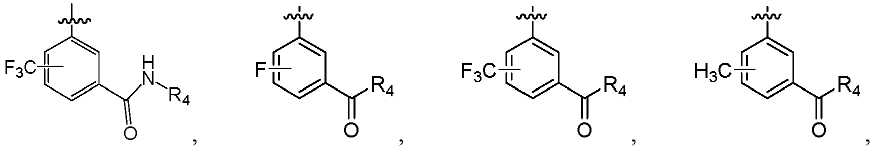 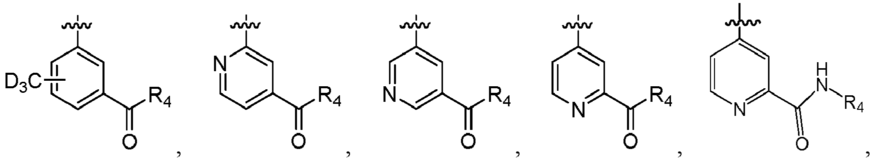 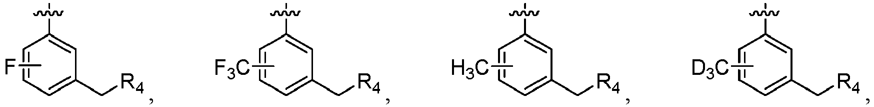 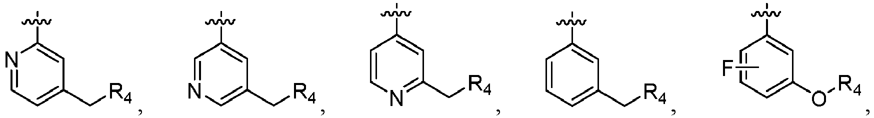 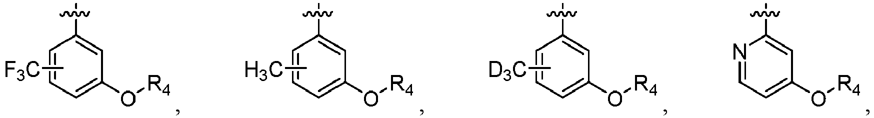 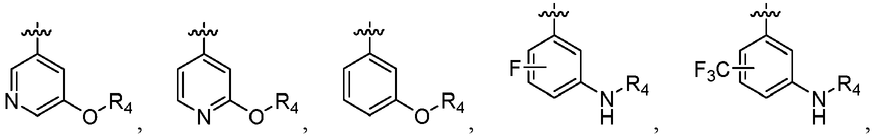 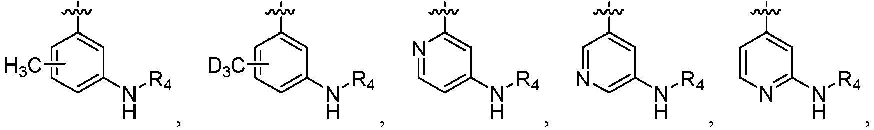 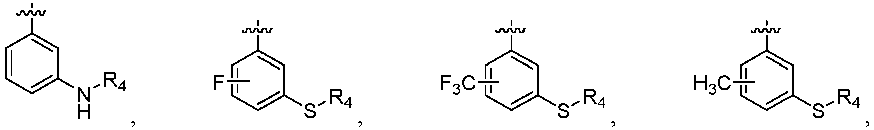 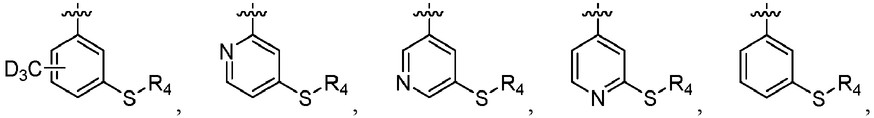 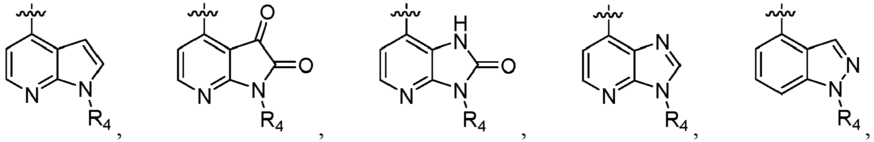 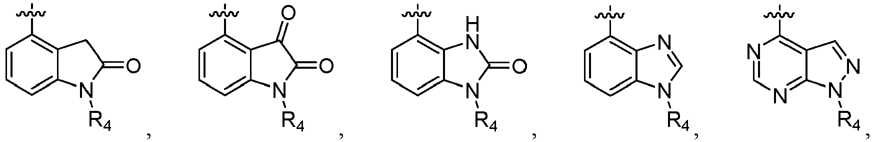 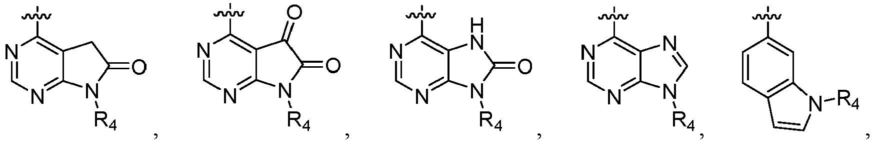 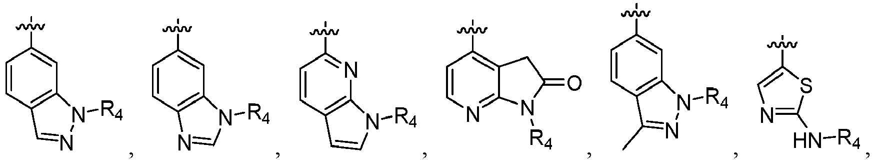 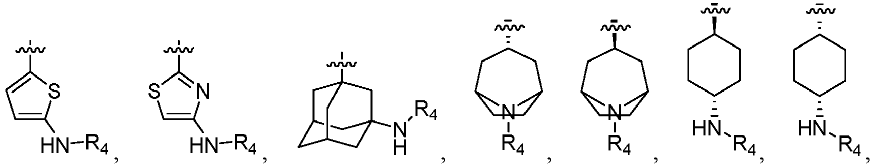 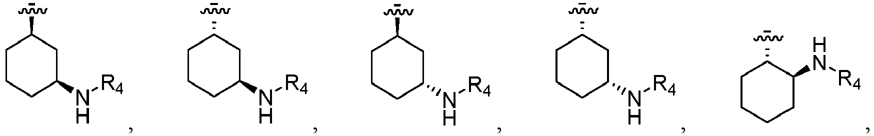 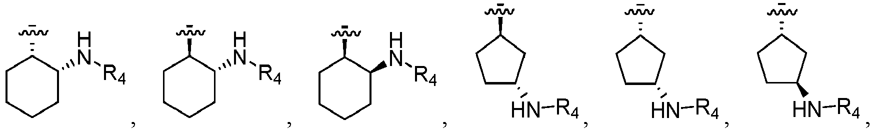 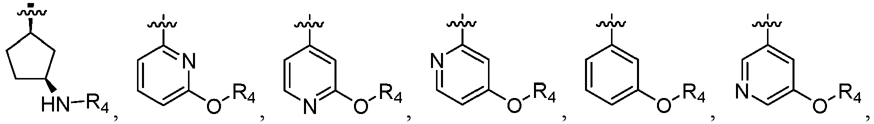 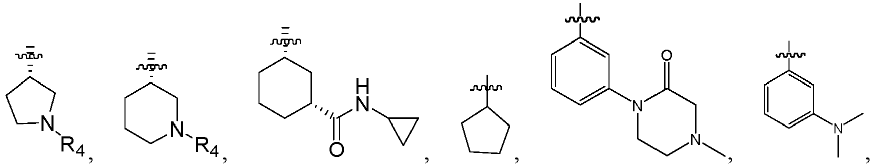 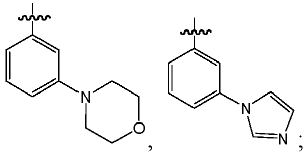 wherein R₄ is selected from: H,

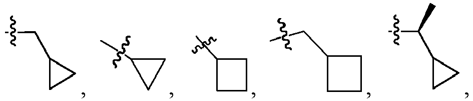

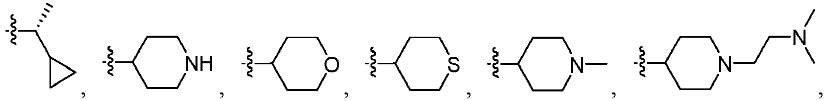

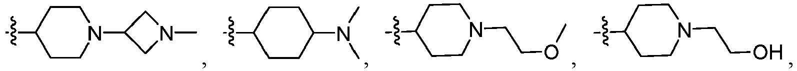

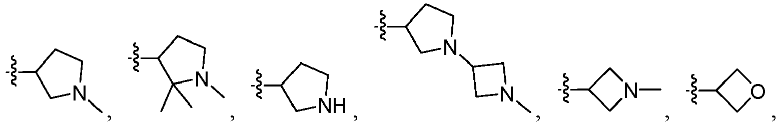

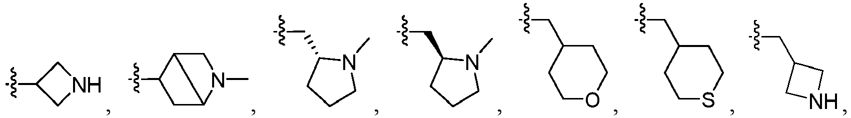

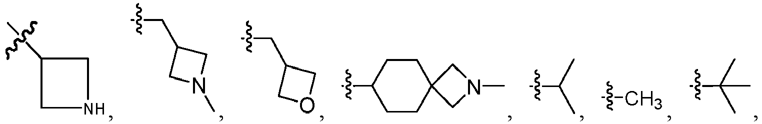

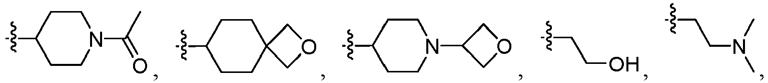

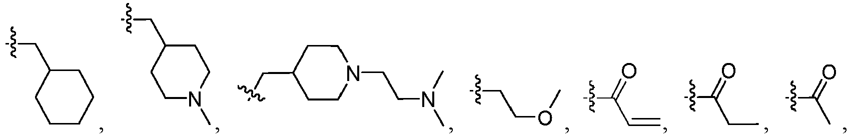

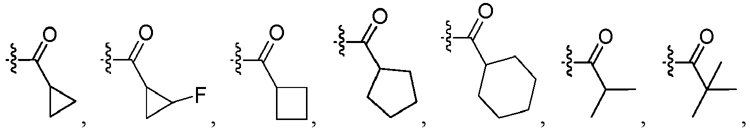

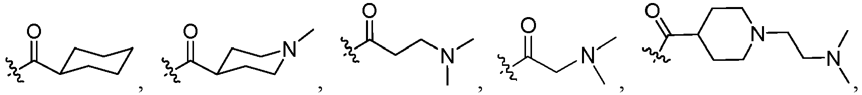

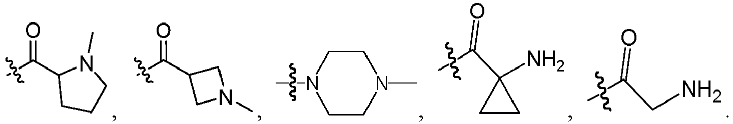

12. The Pyridopyrimidine compounds or their pharmaceutically acceptable salts or stereoisomers or prodrug molecules according to claim 10, wherein
R₂ is selected from: C₅-C₈ cycloalkyl, R₉ substituted C₅-C₈ cycloalkyl, 5-8-membered heterocycloalkyl, R₉ substituted 5-8-membered heterocycloalkyl, phenyl, R₉ substituted phenyl, 5-6-membered heteroaryl group, R₉ substituted 5-6-membered heteroaryl;
R₉ is selected from: H, dimethylamino, amino, halogen, C₁-C₃ alkyl, C₁-C₃ alkoxy, halogen substituted C₁-C₃ alkyl, halogen substituted C₁-C₃ alkoxy, -NH(R₄), -N(R₄)₂, - OR₄, -C=O-NH(cyclopropyl), R₁₀ substituted C₁-C₃ alkyl, C₃-C₆ cycloalkyl, 3-6 membered heterocycloalkyl, R₁₀ substituted C₃-C₆ cycloalkyl, R₁₀ substituted 3-6 membered heterocycloalkyl, -NH(R₄) substituted 3-6 membered heterocycloalkyl, - COR₁₁;
R₄ is selected from: H, C₁-C₆ alkyl, R₁₀ substituted C₁-C₆ alkyl, C₃-C₆ cycloalkyl, 3-6 membered heterocycloalkyl, R₁₀ substituted C₃-C₆ cycloalkyl, R₁₀ substituted 3-6 membered heterocycloalkyl, - CH₂R₁₁, - COR₁₁;
R₁₀ is selected from: C₁-C₃ alkyl, dimethylamino, C₃-C₆ cycloalkyl, 3-6 membered heterocycloalkyl, C₁-C₃ alkyl substituted 3-6 membered heterocycloalkyl, C₁-C₃ alkyl substituted C₃-C₆ cycloalkyl;
R₁₁ is selected from: vinyl, C₁-C₄ alkyl, C₃-C₆ cycloalkyl, and halogen substituted C₃-C₆ cycloalkyl.

13. The Pyridopyrimidine compounds or their pharmaceutically acceptable salts or stereoisomers or prodrug molecules according to claim 12, wherein
R₂ is selected from: C₅-C₆ cycloalkyl, R₉ substituted C₅-C₆ cycloalkyl, 5-6-membered heterocycloalkyl, R₉ substituted 5-6-membered heterocycloalkyl, phenyl, R₉ substituted phenyl;
R₉ is selected from: H, dimethylamino, amino, C₁-C₃ alkyl, -NH(R₄), -OR₄, - C=O-NH (cyclopropyl), R₁₀ substituted C₁-C₃ alkyl, C₃-C₆ cycloalkyl, 5-6 membered heterocycloalkyl, R₁₀ substituted C₃-C₆ cycloalkyl, R₁₀ substituted 5-6 membered heterocycloalkyl, - COR₁₁,
R₄ is selected from: H, C₁-C₃ alkyl, R₁₀ substituted C₁-C₃ alkyl, C₃-C₆ cycloalkyl, 5-6 membered heterocycloalkyl, R₁₀ substituted C₃-C₆ cycloalkyl, R₁₀ substituted 5-6 membered heterocycloalkyl, -CH₂R₁₁, - COR₁₁;
R₁₀ is selected from: C₁-C₃ alkyl, C₃-C₆ cycloalkyl;
R₁₁ is selected from: vinyl, C₁-C₄ alkyl, C₃-C₆ cycloalkyl.

14. The Pyridopyrimidine compounds or their pharmaceutically acceptable salts or stereoisomers or prodrug molecules according to claim 12, wherein
R₂ is selected from: 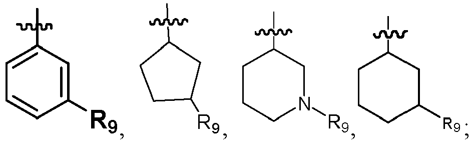
wherein, R₉ is selected from: H, dimethylamino, C₁-C₃ alkyl, -NH(R₄), -OR₄, -COR₁₁;
R₄ is selected from: H, methylpiperidyl, - CH₂R₁₁, - COR₁₁; R₁₁ is selected from: vinyl, C₁-C₄ alkyl, cyclopropyl, cyclobutyl, and cyclopentyl.

15. The Pyridopyrimidine compounds or their pharmaceutically acceptable salts or stereoisomers or prodrug molecules according to claim 1, wherein
A is selected from: -NH-, -NHR-; R is selected from: phenyl, R₇ substituted phenyl, 6-membered heteroaryl, R₇ substituted 6-membered heteroaryl;
R₇ is selected from: C₁-C₆ alkyl, C₃-C₆ cycloalkyl, halogen, C₁-C₆ alkoxy, halogen substituted C₁-C₆ alkoxy, deuterated C₁-C₆ alkoxy, halogen substituted C₁-C₆ alkyl, cyano substituted C₁-C₆ alkyl, deuterated C₁-C₆ alkyl, trifluoromethyl substituted C₃-C₆ cycloalkyl, C₁-C₆ cycloalkyl substituted by C₁-C₆ alkyl, hydroxyl, amino, -SO (C₁-C₆ alkyl), -S(O)₂ (C₁-C₆ alkyl), C₁-C₆ alkylthio;
B is absent or is selected from: C₃-C₁₂ cycloalkyl, R₈ substituted C₃-C₁₂ cycloalkyl, 3-12 membered heterocycloalkyl, R₈ substituted 3-12 membered heterocycloalkyl, 3-12 membered heterocycloalkyl ketone group, R₈ substituted 3-12 membered heterocycloalkyl ketone group, C₃-C₁₂ cycloalkyl substituted amine group, 3-12 membered heterocycloalkyl substituted amine group, 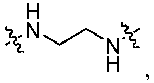 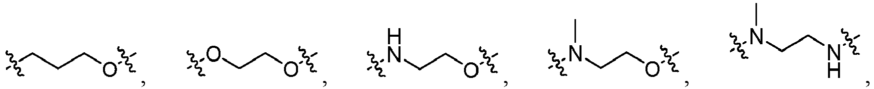 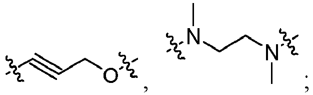
R₈ is selected from: C₁-C₆ alkyl, C₃-C₁₂ cycloalkyl, 3-12 membered heterocycloalkyl, amino, cyano substituted C₁-C₆ alkyl, halogen, C₁-C₆ alkoxy, halogen substituted C₁-C₆ alkyl, hydroxyl, amino.

16. The Pyridopyrimidine compounds or their pharmaceutically acceptable salts or stereoisomers or prodrug molecules according to claim 15, wherein
A is selected from: -NH-, 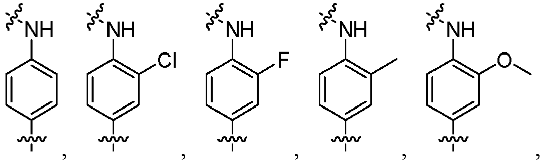 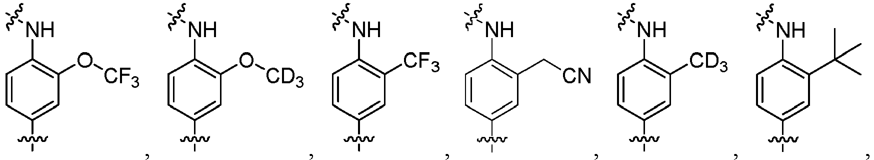 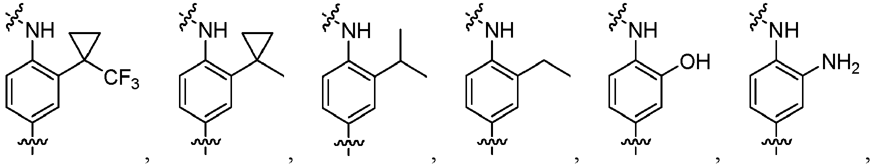 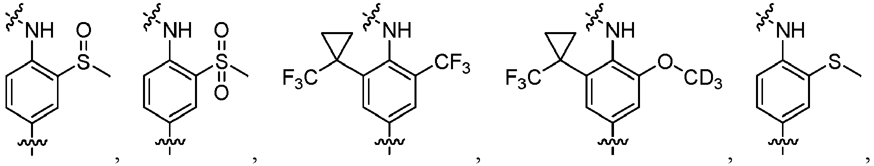 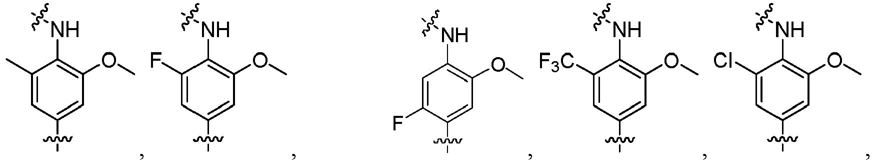 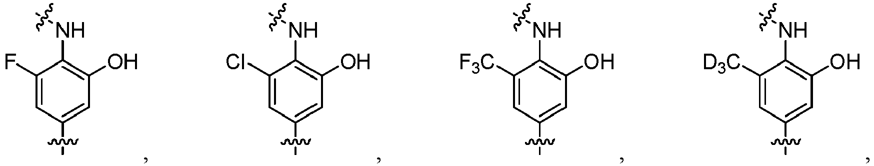 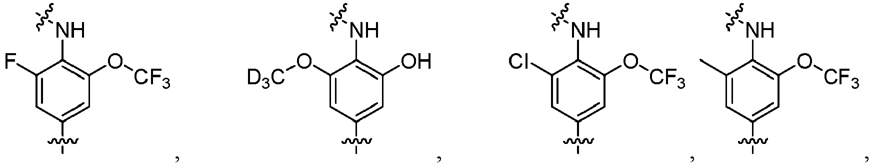 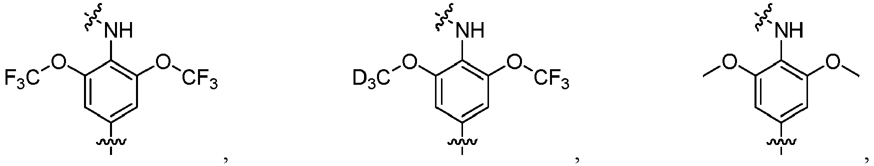 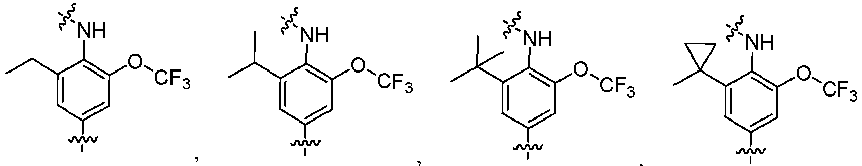 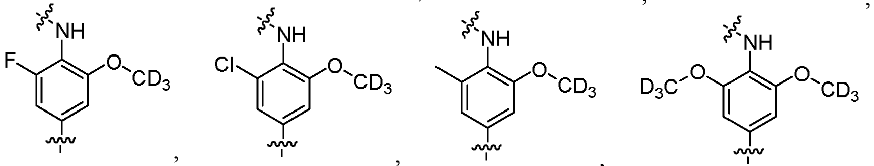 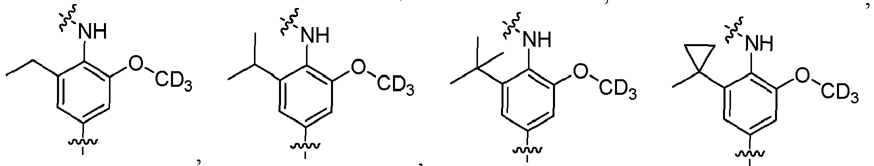 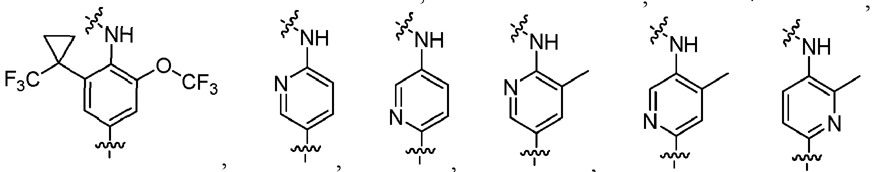 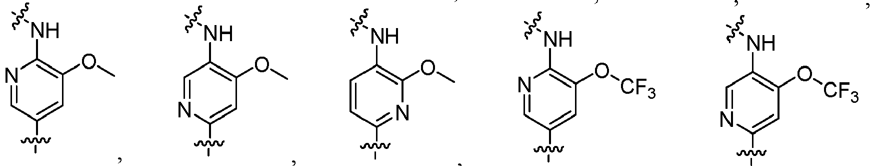 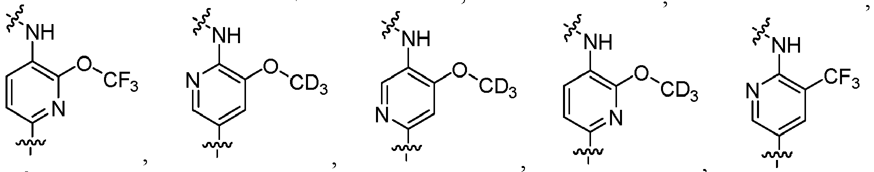 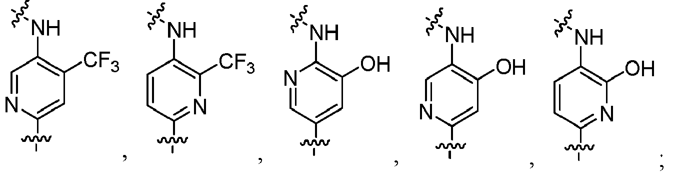
B is absent or is selected from:

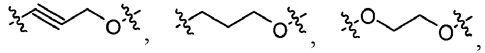

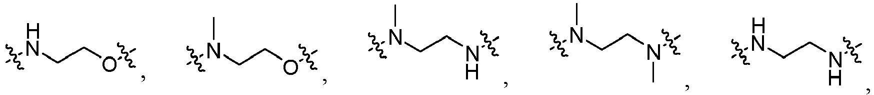

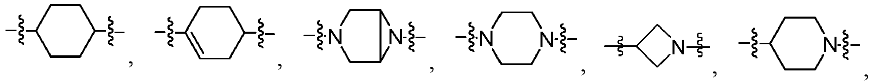

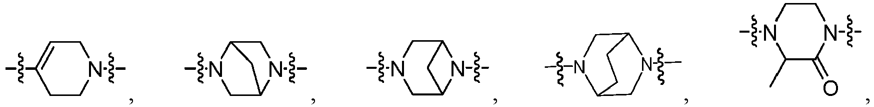

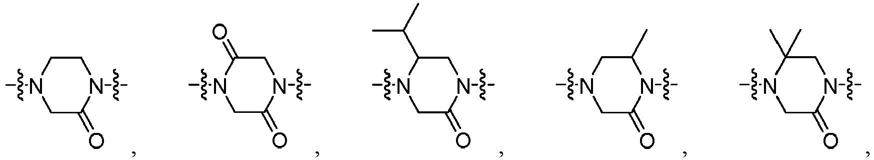

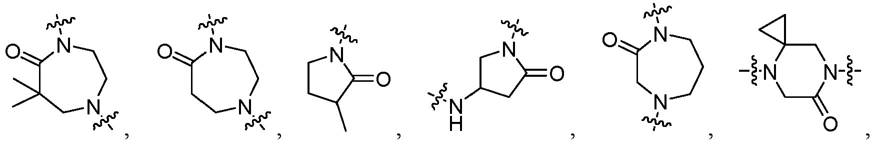

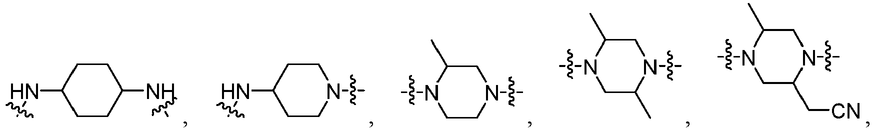

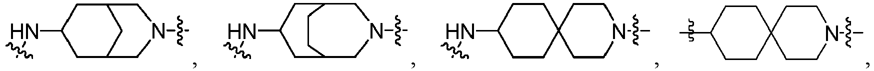

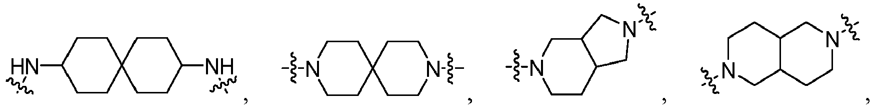

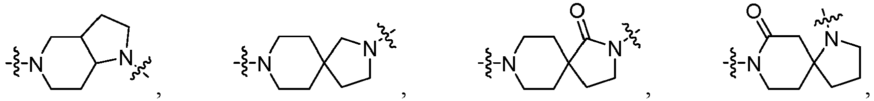

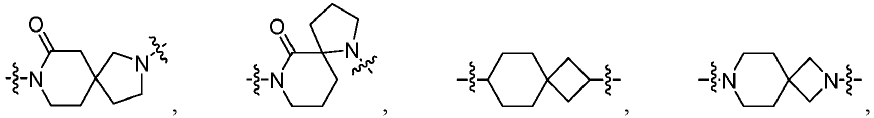

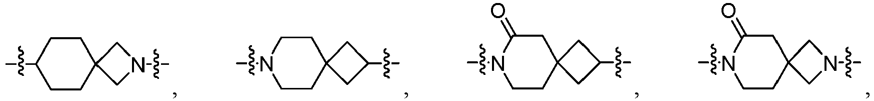

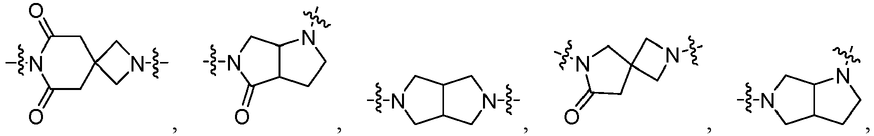

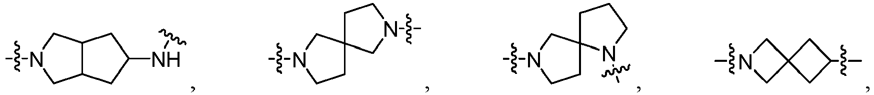

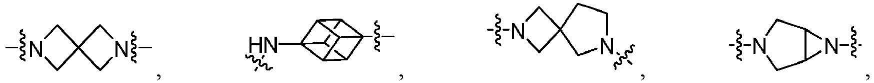

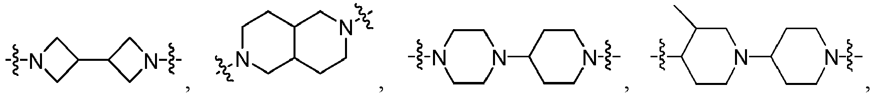

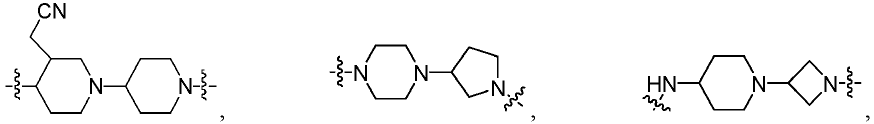

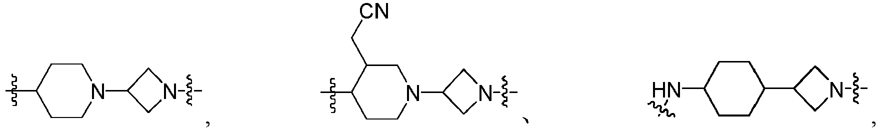

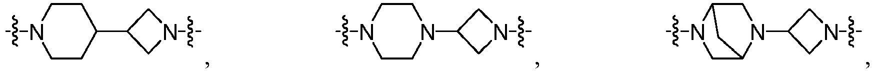

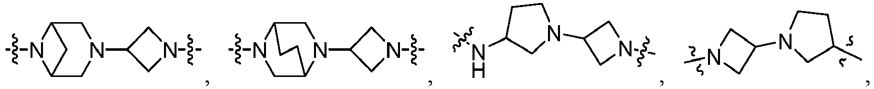

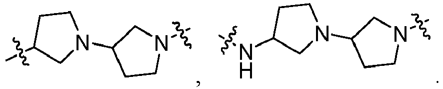

17. The Pyridopyrimidine compounds or their pharmaceutically acceptable salts or stereoisomers or prodrug molecules according to claim 15, wherein
A is selected from: -NH-, -NHR-; wherein R is selected from: phenyl, 6-membered heteroaryl, R₇ substituted phenyl, R₇ substituted 6-membered heteroaryl;
R₇ is selected from: C₁-C₃ alkyl, halogen, C₁-C₃ alkoxy, halogen substituted C₁-C₃ alkoxy, halogen substituted C₁-C₃ alkyl, cyano substituted C₁-C₃ alkyl;
B is absent or is selected from: C₄-C₁₂ cycloalkyl, R₈ substituted C₄-C₁₂ cycloalkyl, 4-12 membered heterocycloalkyl, R₈ substituted 4-12 membered heterocycloalkyl, 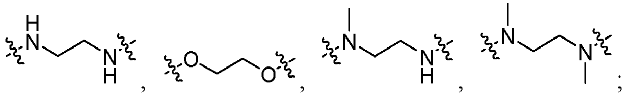
R₈ is selected from: C₁-C₃ alkyl, C₄-C₆ cycloalkyl, 4-6 membered heterocycloalkyl, cyano substituted C₁-C₃ alkyl, halogen, C₁-C₃ alkoxy, halogen substituted C₁-C₃ alkyl.

18. The Pyridopyrimidine compounds or their pharmaceutically acceptable salts or stereoisomers or prodrug molecules according to claim 17, wherein
A is selected from: -NH-, -NHR-; wherein R is selected from: phenyl, 6-membered azaaryl group, R₇ substituted phenyl, R₇ substituted 6-membered azaaryl group;
R₇ is selected from: C₁-C₃ alkoxy;
B is absent or is selected from: C₄-C₈ cycloalkyl, R₈ substituted C₄-C₁₀ cycloalkyl, 4-10 membered heterocycloalkyl, R₈ substituted 4-10 membered heterocycloalkyl, 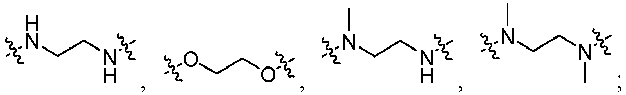
R₈ is selected from: C₁-C₃ alkyl, C₁-C₃ alkoxy.

19. The Pyridopyrimidine compounds or their pharmaceutically acceptable salts or stereoisomers or prodrug molecules according to claim 18, wherein
R₃ is selected from:

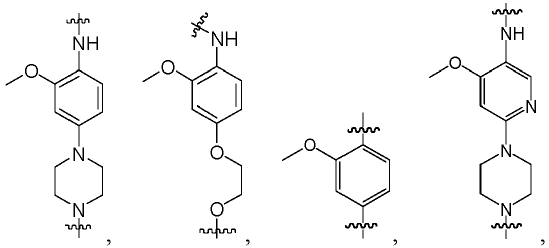

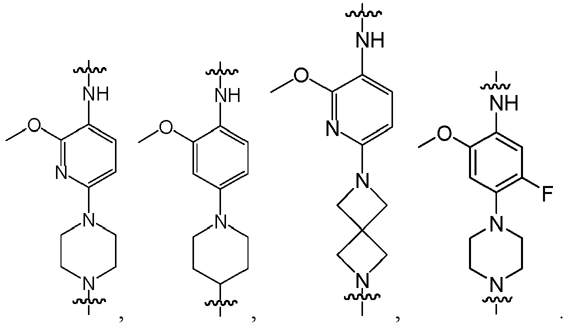

20. The Pyridopyrimidine compounds or their pharmaceutically acceptable salts or stereoisomers or prodrug molecules according to claim 1, wherein the compound is selected from:

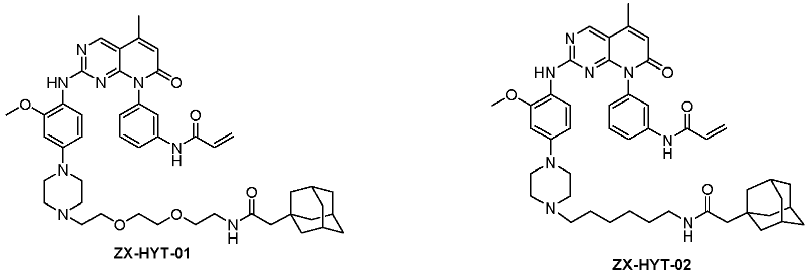

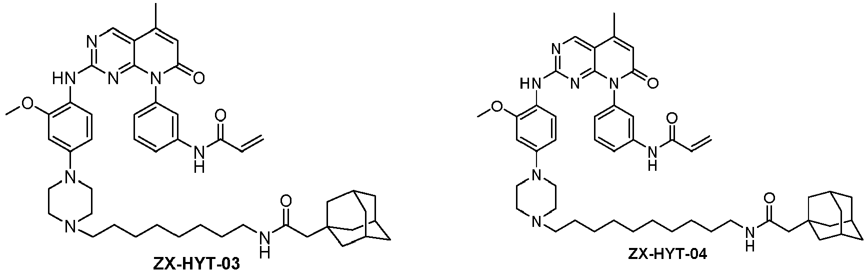

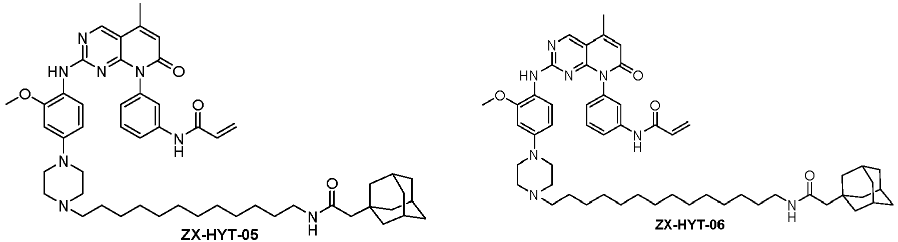

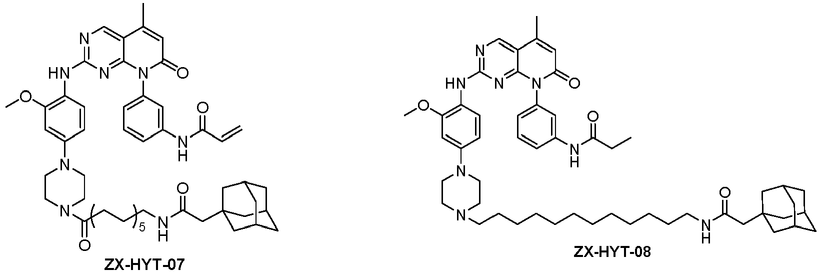

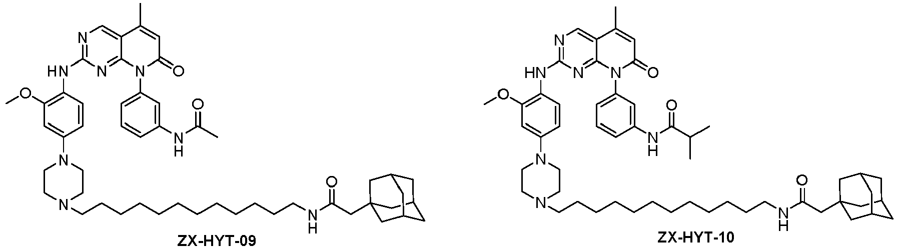

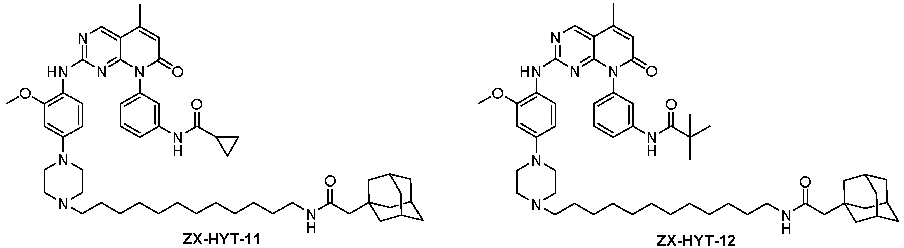

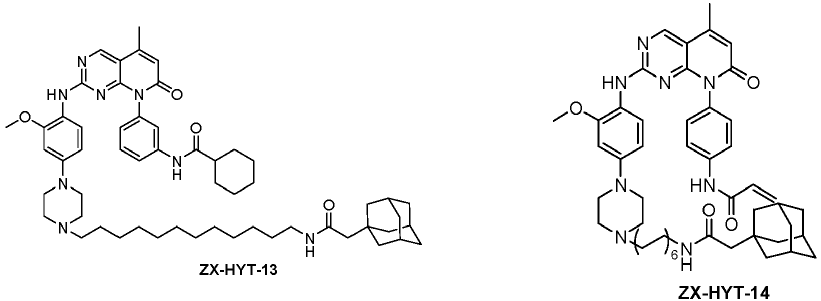

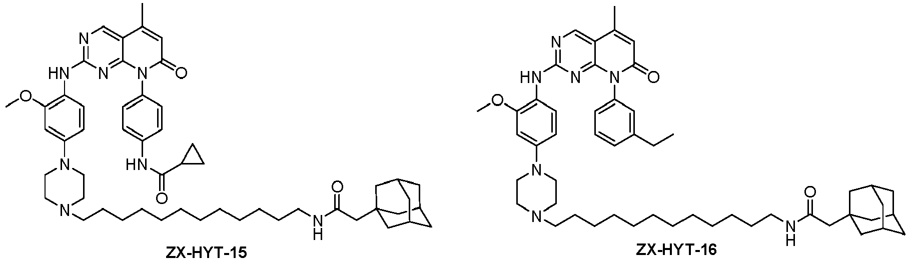

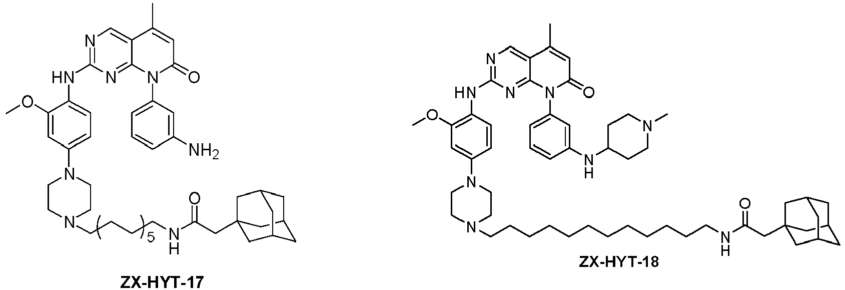

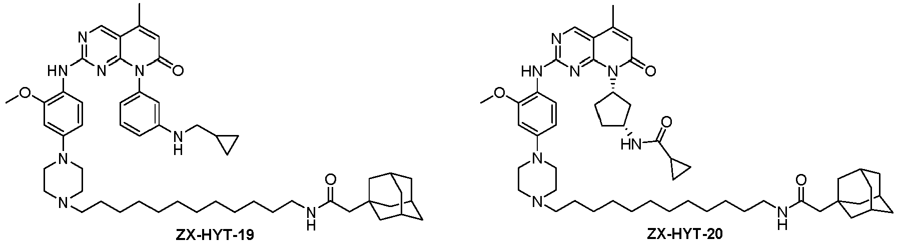

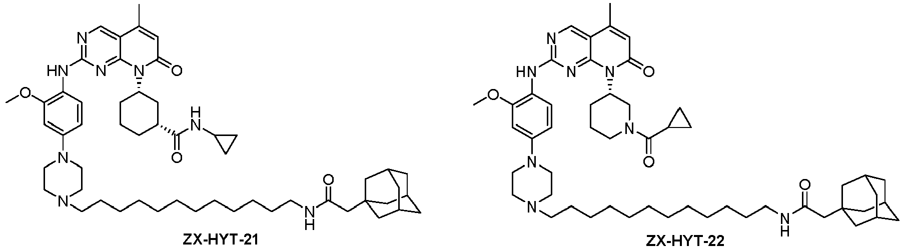

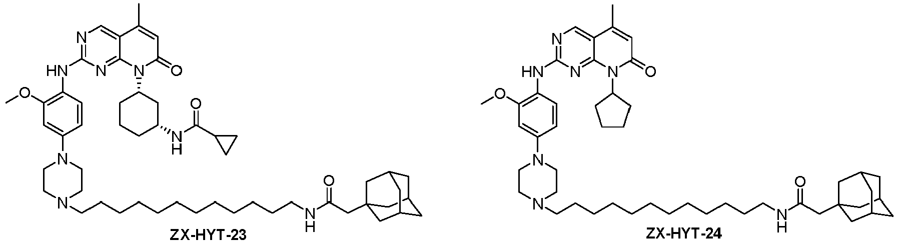

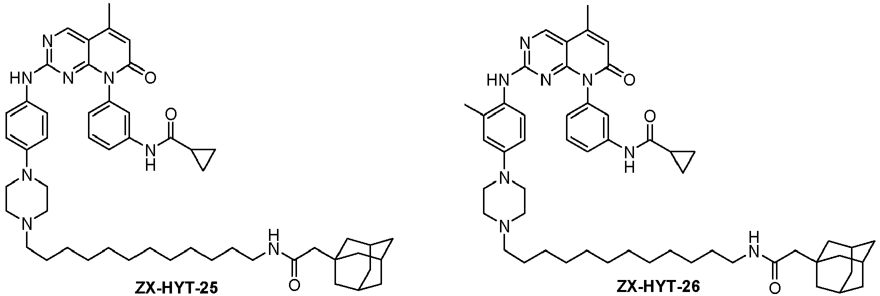

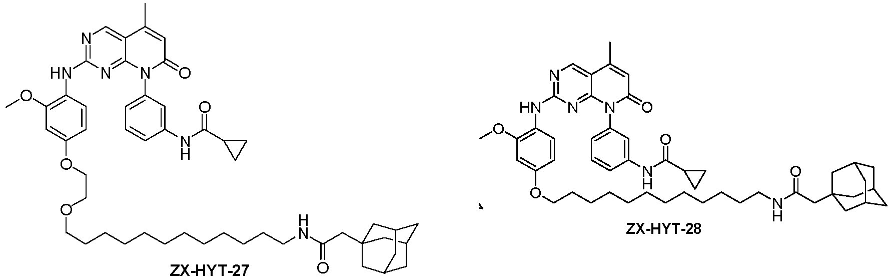

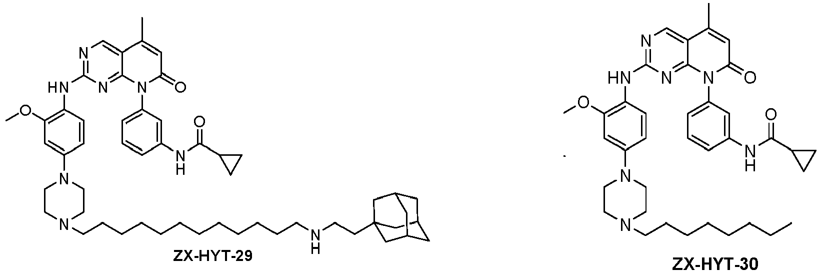

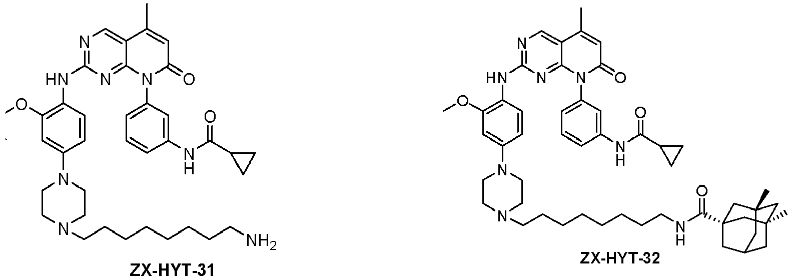

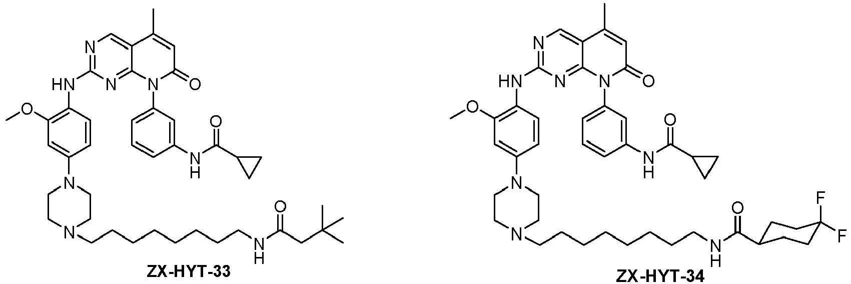

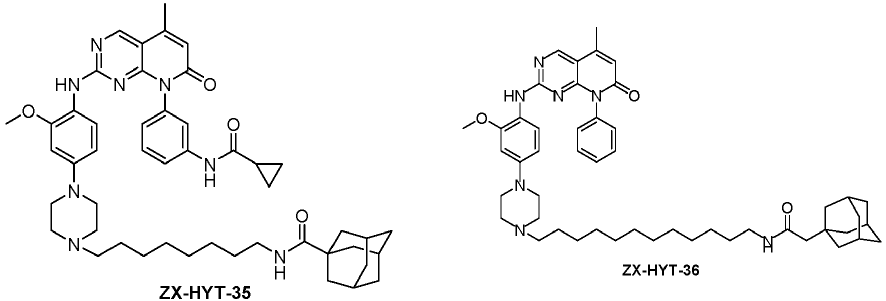

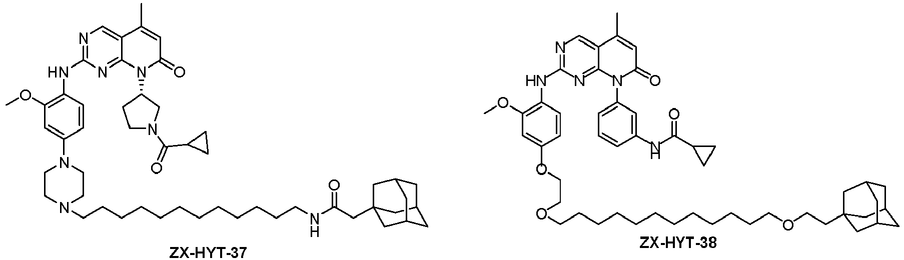

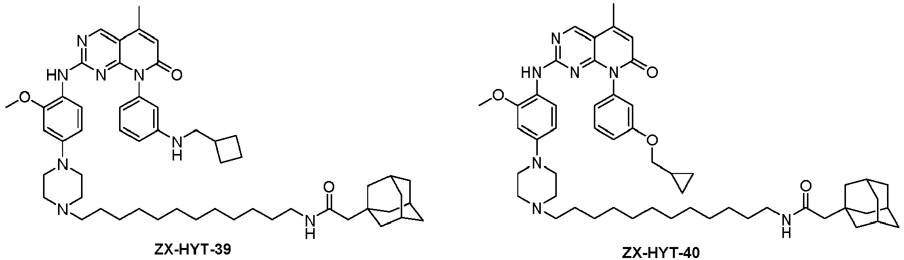

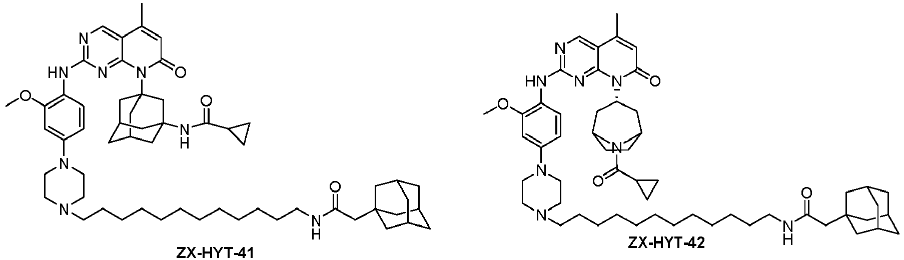

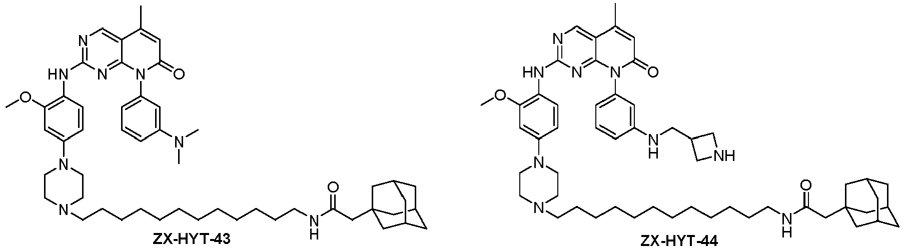

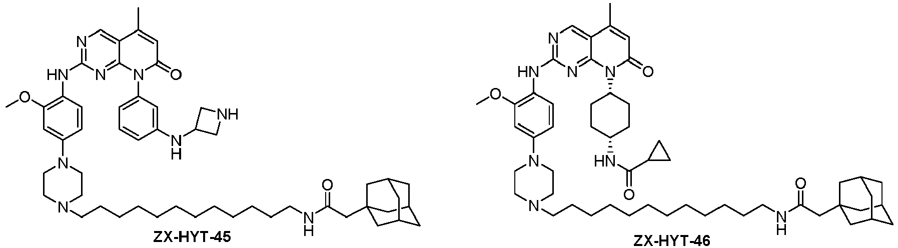

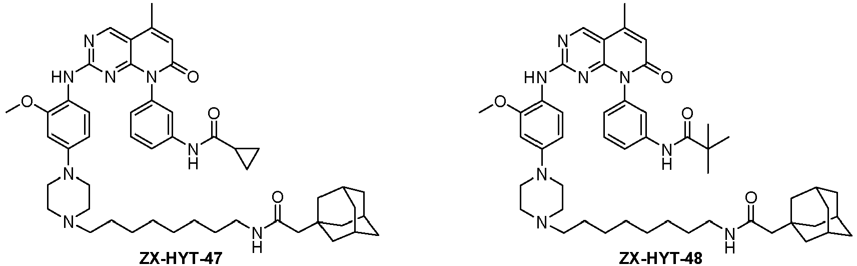

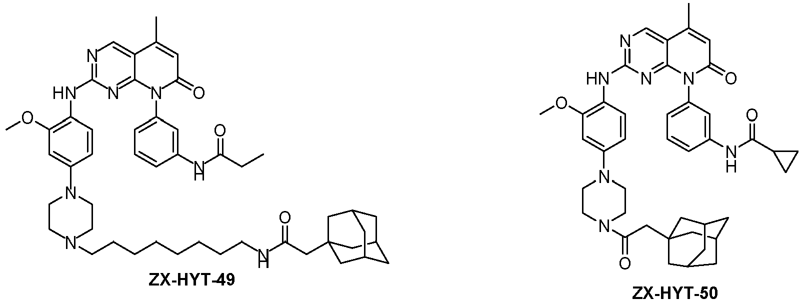

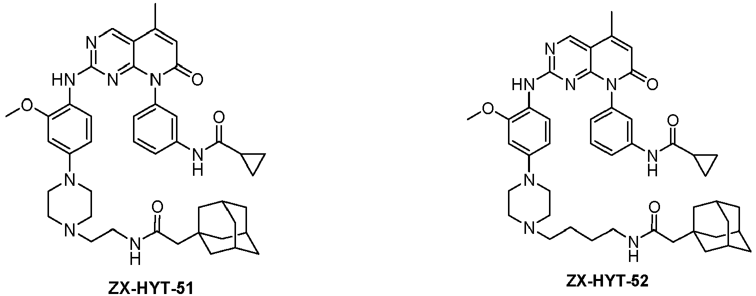

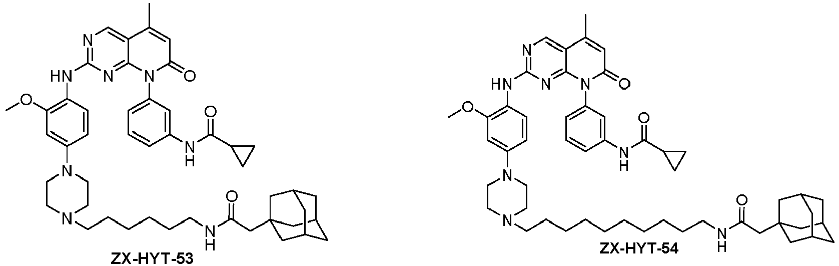

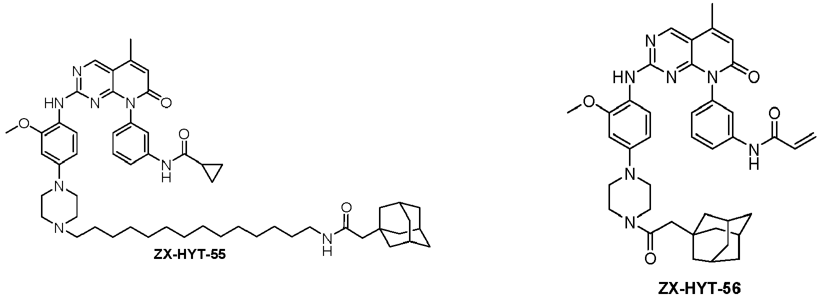

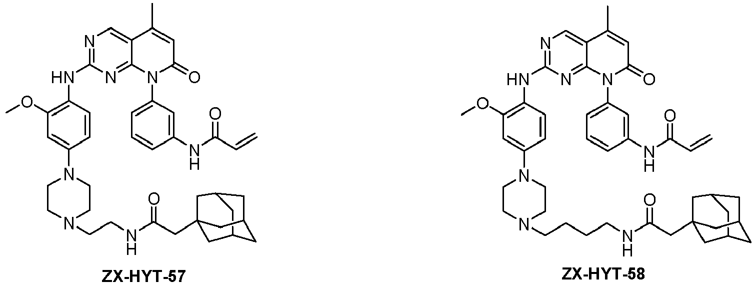

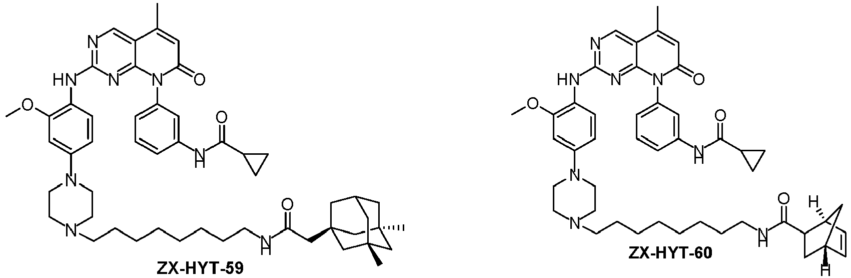

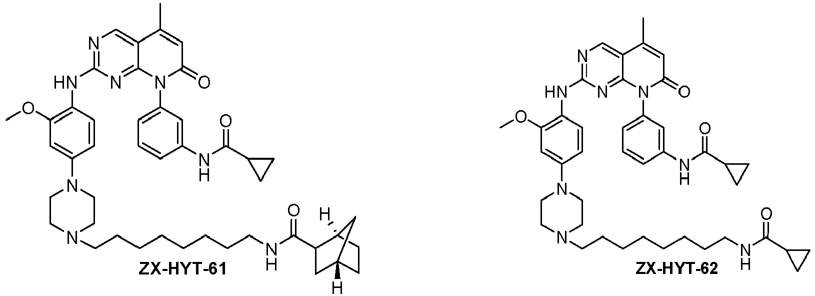

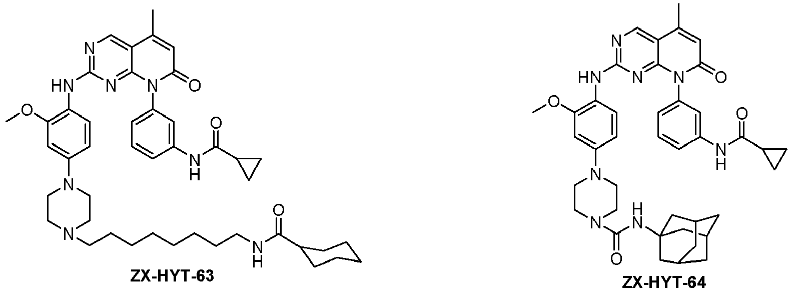

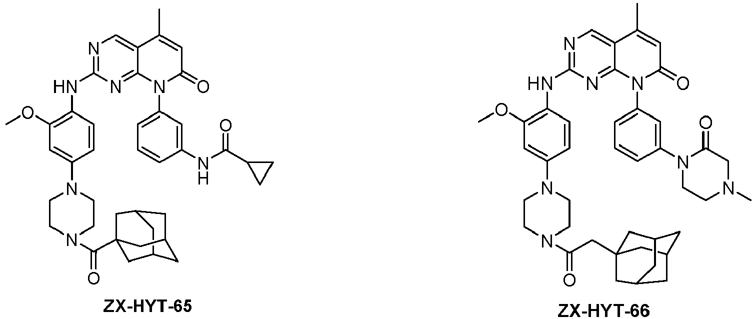

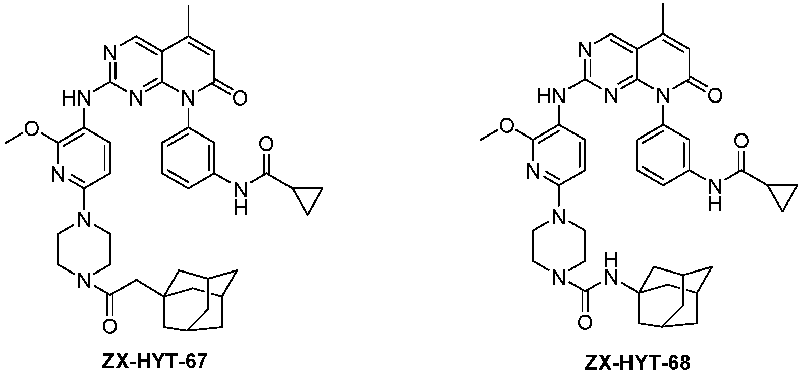

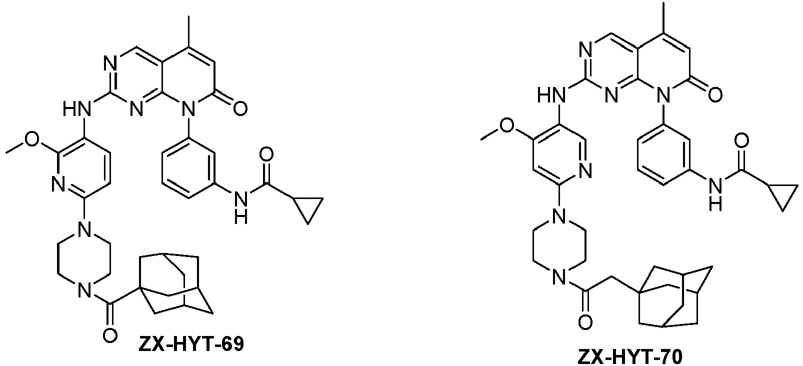

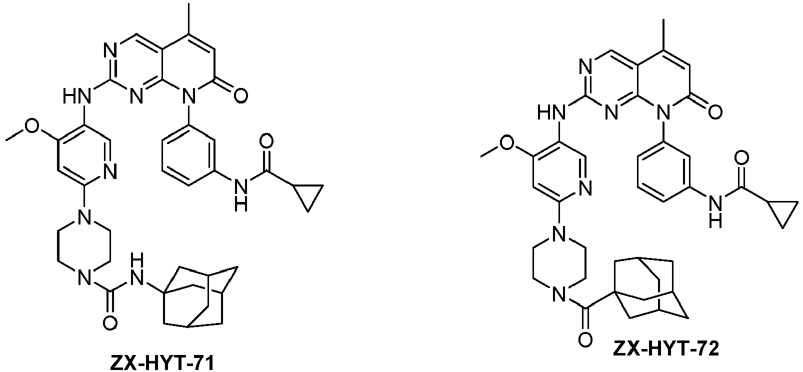

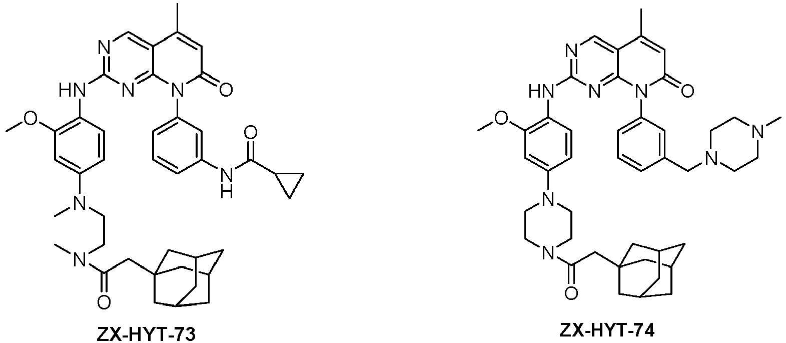

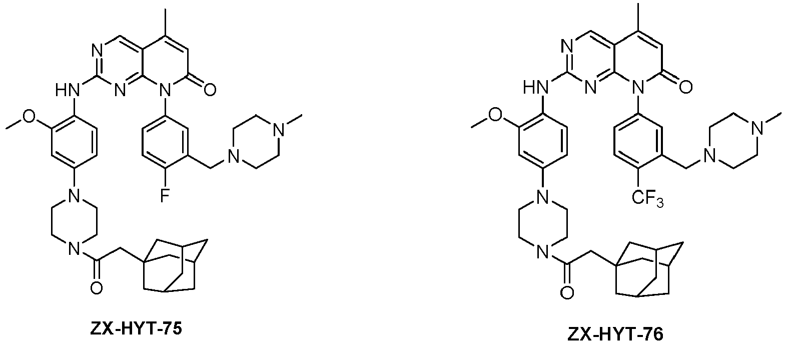

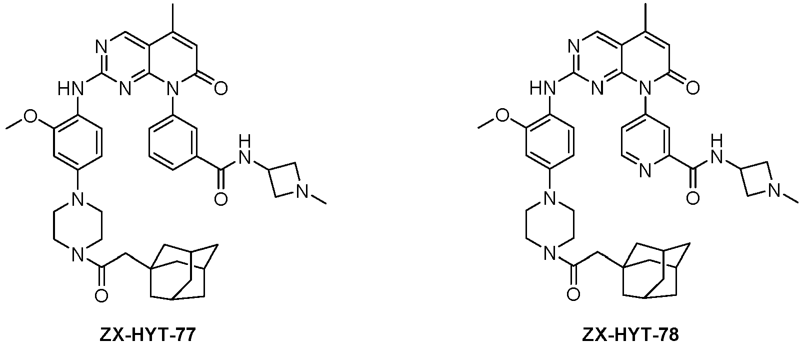

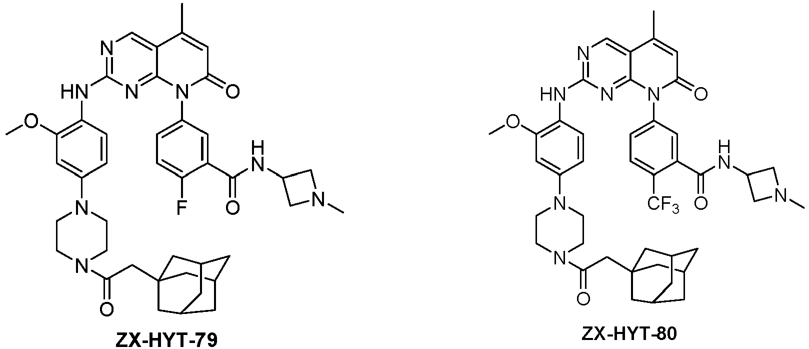

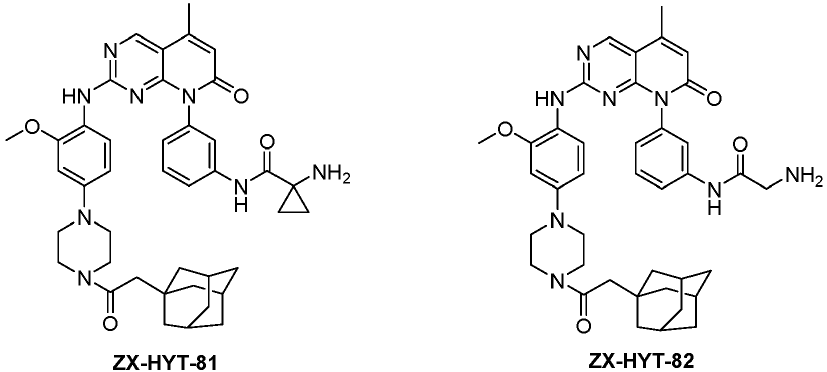

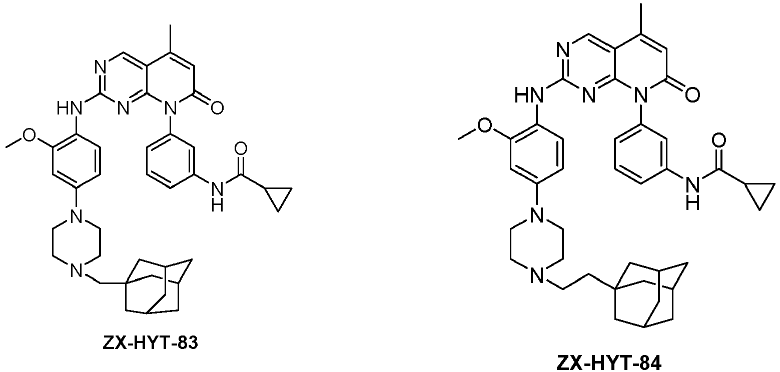

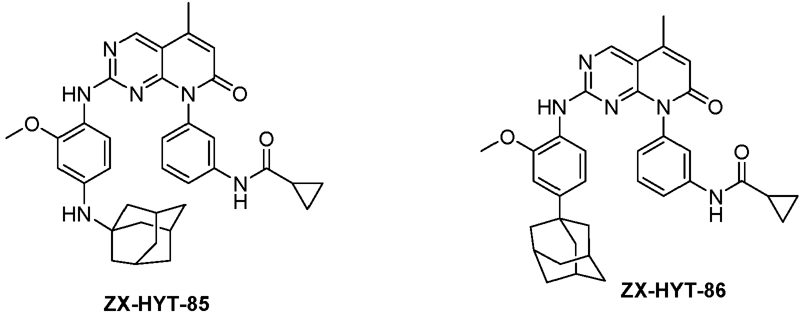

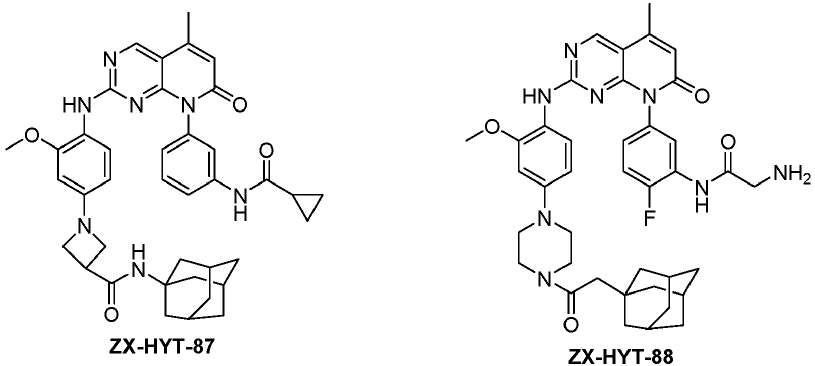

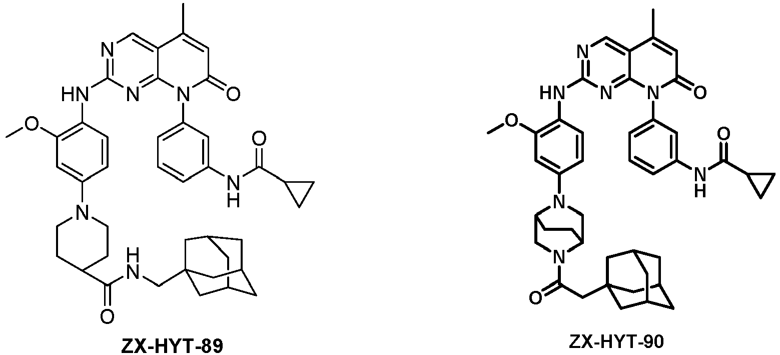

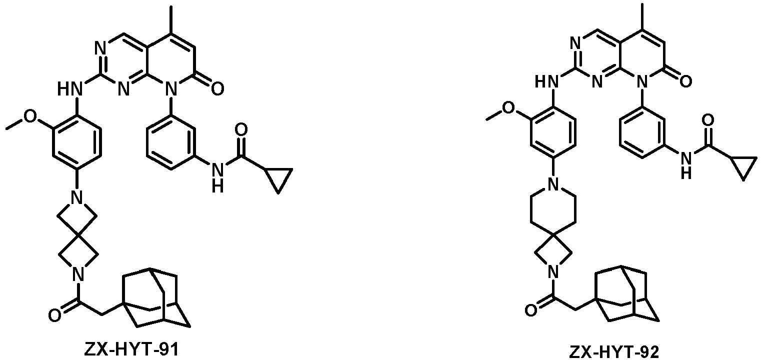

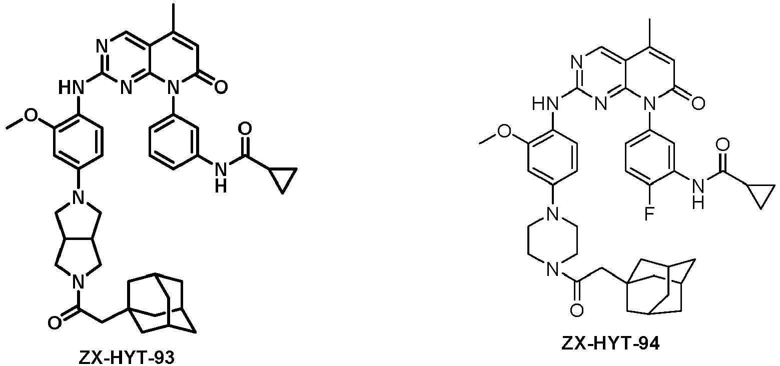

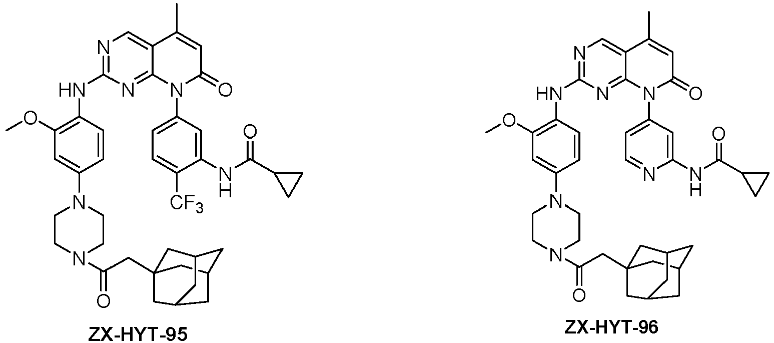

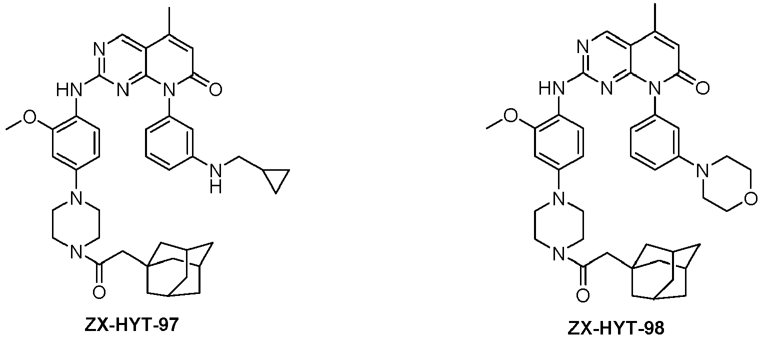

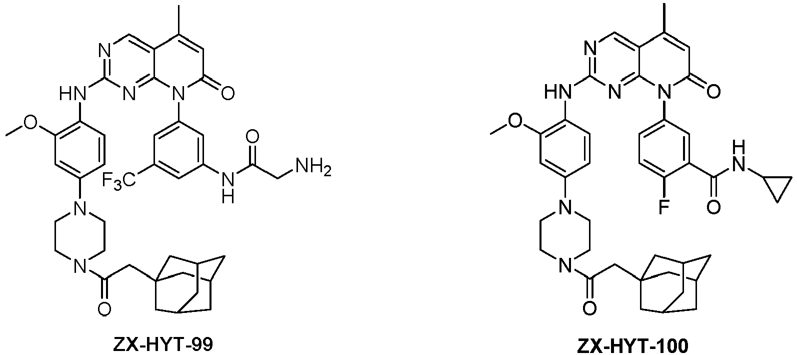

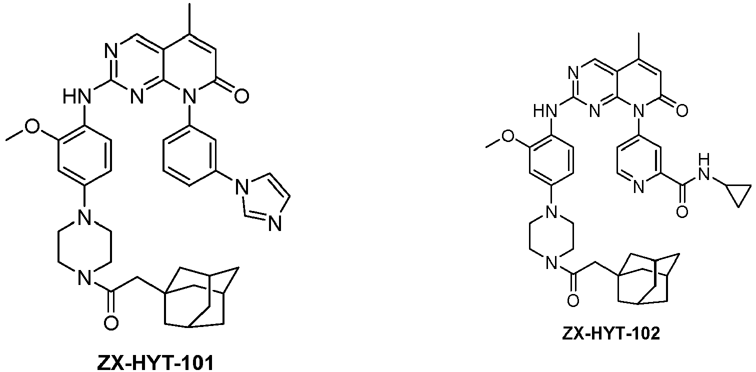

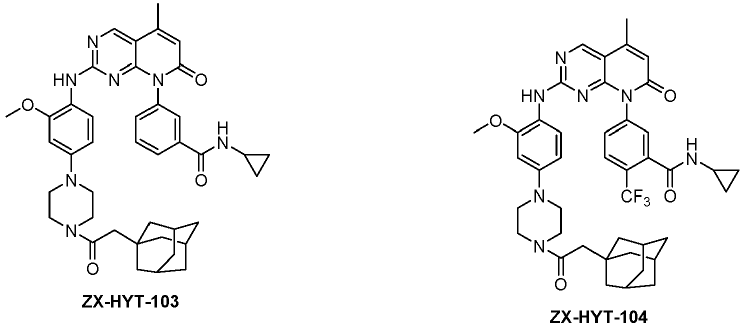

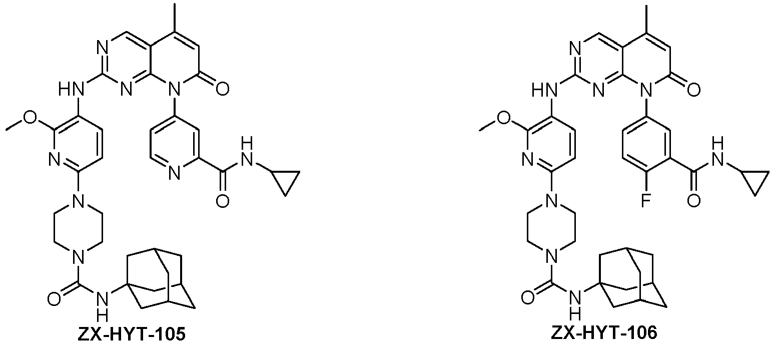

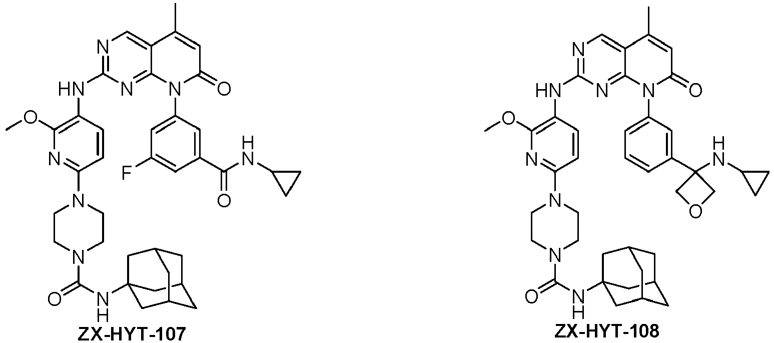

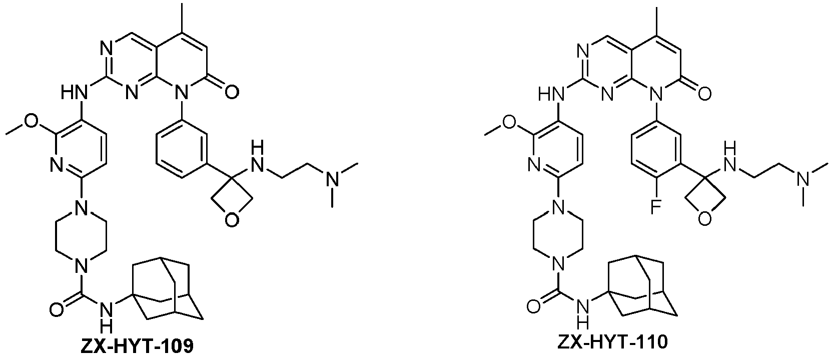

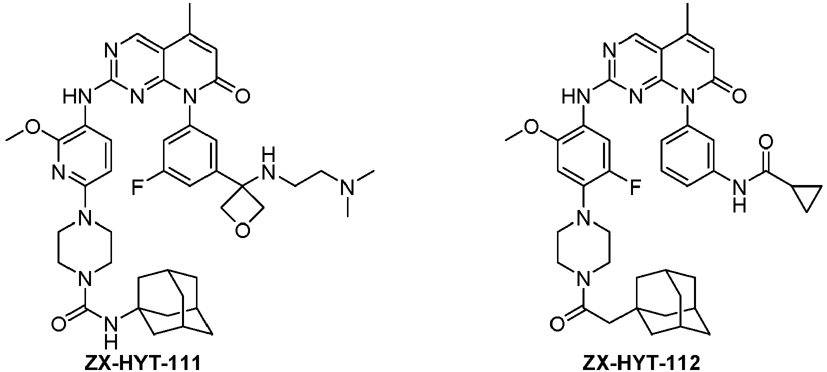

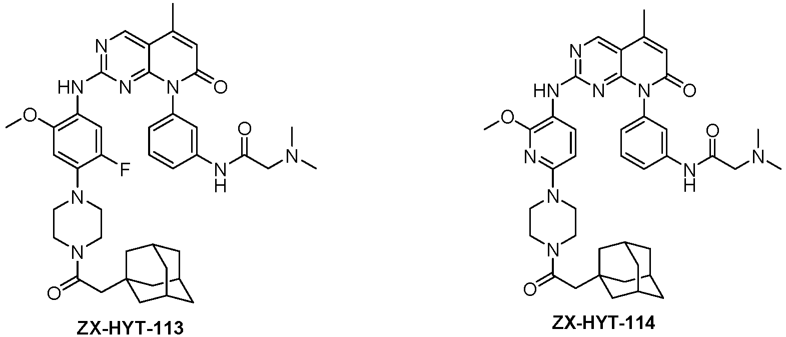

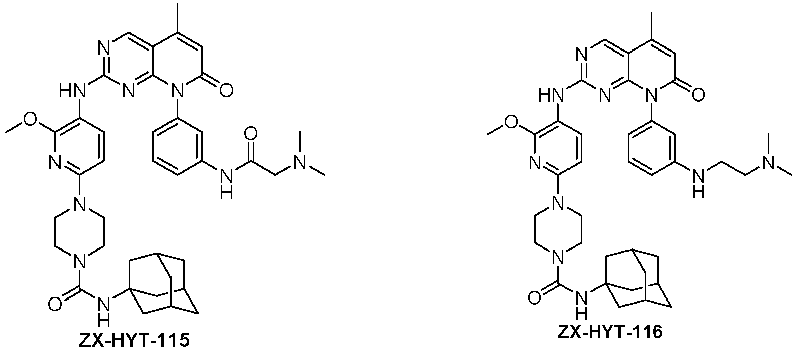

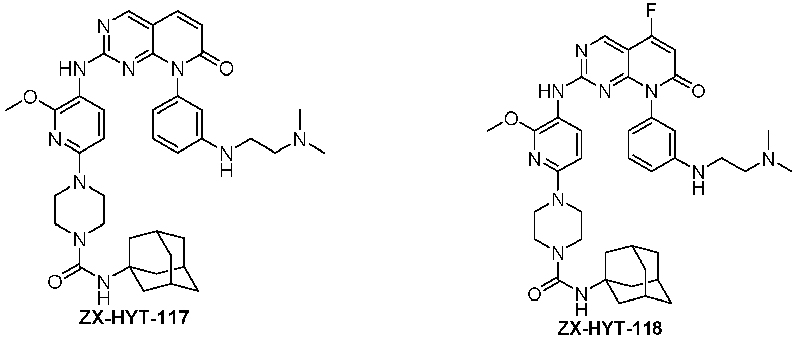

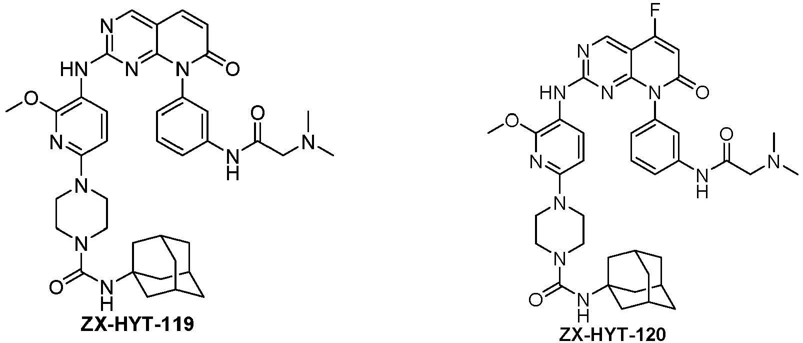

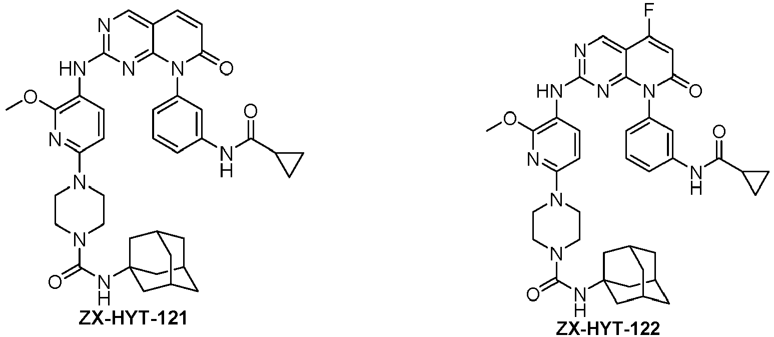

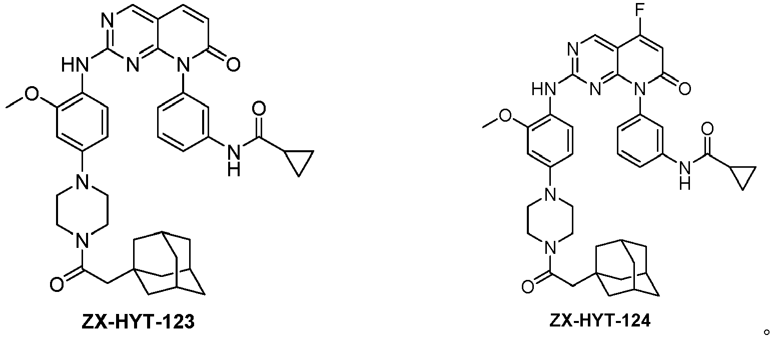

21. An application of the Pyridopyrimidine compounds or their pharmaceutically acceptable salts or stereoisomers or prodrug molecules according to any one of claims 1-20 in the preparation of AKT3 protein degradation agent.

22. An application of the Pyridopyrimidine compounds or their pharmaceutically acceptable salts or stereoisomers or prodrug molecules according to any one of claims 1-20 in the preparation of drugs for preventing and/or treating diseases related to abnormal expression of AKT3 protein.

23. The application according to claim 22, wherein the diseases related to abnormal expression of AKT3 protein include: tumor, cardiovascular disease, diabetes, hypertension, muscular dystrophy, Parkinson's disease and Alzheimer's disease.

24. An application of the Pyridopyrimidine compounds or their pharmaceutically acceptable salts or stereoisomers or prodrug molecules according to any one of claims 1-20 in the preparation of drugs for preventing and/or treating tumors or preventing postoperative recurrence of tumors.

25. The application according to claim 24, wherein the tumors are: non-small cell lung cancer, malignant melanoma, prostate cancer, kidney cancer, liver cancer, bladder cancer, ovarian cancer, colon cancer, rectal cancer, breast cancer, cervical cancer, lung cancer, laryngeal cancer, nasopharyngeal cancer, pancreatic cancer, multiple myeloma, B lymphoma, leukemia, skin squamous cell carcinoma.

26. An AKT3 protein degrading agent, wherein its active ingredient comprises the Pyridopyrimidine compounds or their pharmaceutically acceptable salts or stereoisomers or prodrug molecules according to any one of claims 1-20.

27. A pharmaceutical composition for treating and/or preventing tumors or preventing postoperative recurrence of tumors, being prepared from active ingredients and pharmaceutically acceptable carriers or excipients, wherein the active ingredients include the Pyridopyrimidine compounds or their pharmaceutically acceptable salts or stereoisomers or prodrug molecules according to any one of claims 1-20.
